# EUROPEAN PATENT SPECIFICATION

(11) **EP 4 212 522 B1**
(45) Date of publication and mention of the grant of the patent: **02.12.2026**
(21) Application number: 21867047.9
(22) Date of filing: 02.09.2021
(51) Int. Cl.: C07D 401/14, A61K 31/506, A61P 35/00, C07D 471/10, C07D 417/14, C07D 487/10

(54) **COMPOUNDS FOR SUPPRESSING EGFR MUTANT CANCER AND PHARMACEUTICAL USE THEREOF**
VERBINDUNGEN ZUR UNTERDRÜCKUNG VON EGFR-MUTANTEM KREBS UND PHARMAZEUTISCHE VERWENDUNG DAVON
COMPOSÉS DESTINÉS À ÉLIMINER LE CANCER MUTANT DE L'EGFR ET LEUR UTILISATION PHARMACEUTIQUE

(30) Priority: 11.09.2020 KR 20200117186
(43) Date of publication of application: 19.07.2023
(73) Proprietor: J2H Biotech Inc., Suwon-Si, Gyeonggi-do 16648 (KR)
(72) Inventor: RYU, Hyung-Chul, Hwaseong-si, Gyeonggi-do 18526 (KR); KIM, Jae-Sun, Suwon-si, Gyeonggi-do 16658 (KR); LIM, Jee-Woong, Seongnam-si, Gyeonggi-do 13600 (KR); LEE, Ju Young, Suwon-si, Gyeonggi-do 16424 (KR); CHOI, Kwanghyun, Gunpo-si, Gyeonggi-do 15866 (KR); RAJESH, Rengasamy, Yongin-si, Gyeonggi-do 16948 (KR); CHANG, Duk-Ho, Suwon-si, Gyeonggi-do 16567 (KR); GWON, Hyeok Jun, Seongnam-si, Gyeonggi-do 13607 (KR); KANG, Hyo Jin, Suwon-si, Gyeonggi-do 16627 (KR)
(74) Representative: Graf von Stosch Patentanwaltsgesellschaft mbH
(86) International application number: PCT/KR2021/011898
(87) International publication number: WO 2022/055181

(56) References cited:
- WO-A1-2019/190259
- WO-A1-2022/194269
- WO-A1-2022/268229
- SUN NING; REN CHAOWEI; KONG YING; ZHONG HUI; CHEN JINJU; LI YAN; ZHANG JIANSHUI; ZHOU YUEDONG; QIU XING; LIN HAIFAN; SONG XIAOLING: "Development of a Brigatinib degrader (SIAIS117) as a potential treatment for ALK positive cancer resistance", EUROPEAN JOURNAL OF MEDICINAL CHEMISTRY, ELSEVIER, AMSTERDAM, NL, vol. 193, 29 February 2020 (2020-02-29), AMSTERDAM, NL , XP086118072, ISSN: 0223-5234, DOI: 10.1016/j.ejmech.2020.112190
- HYEONJEONG CHOE, BYEONG UK JEON, MYOUNG EUN JUNG, MOON-KOOK JEON, INJI SHIN, BYOUNG CHUL CHO, GILDON CHOI, CHONG HAK CHAE, KWANGHO: "Structure-Activity Relationship Study of 2,4-Dianilinopyrimidine Containing Methanesulfonamide (TRE-069) as Potent and Selective Epidermal Growth Factor Receptor T790M/C797S Mutant Inhibitor for Anticancer Treatment", BULL. KOREAN CHEM. SOC., vol. 38, no. 11, 1 November 2017 (2017-11-01), pages 1353 - 1357, XP055561541, ISSN: 1229-5949, DOI: 10.1002/bkcs.11287
- CHELSEA E. POWELL, YANG GAO, LI TAN, KATHERINE A. DONOVAN, RADOSłAW P. NOWAK, AMANDA LOEHR, MAGDA BAHCALL, ERIC S. FISCHER, P: "Chemically Induced Degradation of Anaplastic Lymphoma Kinase (ALK)", JOURNAL OF MEDICINAL CHEMISTRY, AMERICAN CHEMICAL SOCIETY, US, vol. 61, no. 9, 10 May 2018 (2018-05-10), US , pages 4249 - 4255, XP055628330, ISSN: 0022-2623, DOI: 10.1021/acs.jmedchem.7b01655
- CHENGWEI ZHANG, HAN XIAO-RAN, YANG XIAOBAO, JIANG BIAO, LIU JING, XIONG YUE, JIN JIAN: "Proteolysis Targeting Chimeras (PROTACs) of Anaplastic Lymphoma Kinase (ALK)", EUROPEAN JOURNAL OF MEDICINAL CHEMISTRY, ELSEVIER, AMSTERDAM, NL, vol. 151, 1 May 2018 (2018-05-01), AMSTERDAM, NL , pages 304 - 314, XP055581975, ISSN: 0223-5234, DOI: 10.1016/j.ejmech.2018.03.071
- CHUNG HYO KANG, DONG HO LEE, CHONG OCK LEE, JAE DU HA, CHI HOON PARK, JONG YEON HWANG: "Induced protein degradation of anaplastic lymphoma kinase (ALK) by proteolysis targeting chimera (PROTAC)", BIOCHEMICAL AND BIOPHYSICAL RESEARCH COMMUNICATIONS, ELSEVIER, AMSTERDAM NL, vol. 505, no. 2, 28 September 2018 (2018-09-28), Amsterdam NL , pages 542 - 547, XP055765181, ISSN: 0006-291X, DOI: 10.1016/j.bbrc.2018.09.169

## Description

### [Technical Field]

The present disclosure relates to a group of compounds for inhibiting, i.e. treating or preventing cancers exhibiting EGFR mutations. The present disclosure also relates to pharmaceutical compositions comprising such compound. The present disclosure also relates to a pharmaceutical composition for use in methods for treating or preventing cancers exhibiting EGFR mutations, in particular, cancers exhibiting EGFR mutations of any one or more of del19, T790M, C797S, and L858R, using such compound. That is, the present disclosure relates to the medical use of a pharmaceutical composition comprising the compounds according to the present invention for treating or preventing said cancer.

### [Background Art]

Epidermal growth factor receptor (EGFR) is a protein composed of a receptor part and a tyrosine kinase part, and serves to transmit signals from outside the cell to the inside of the cell through the cell membrane. EGFR plays an essential role in normal cell regulation through intracellular signal transduction. However, overexpression of EGFR or activating EGFR mutations characterized by ligand-independent tyrosine kinase activity are known to induce growth, differentiation, angiogenesis, metastasis, resistance expression, etc. of cancer cells by abnormally activating the cell signaling system. It has been reported that EGFR is abnormally overexpressed or frequently mutated in most solid cancer cells, which is associated with poor prognosis. For example, lung cancer, liver cancer, esophageal cancer, stomach cancer, colon cancer, small intestine cancer, pancreatic cancer, melanoma, breast cancer, oral cancer, brain tumor, thyroid cancer, parathyroid cancer, kidney cancer, cervical cancer, sarcoma, prostate cancer, urethral cancer, bladder cancer, testicular cancer, blood cancer, lymphoma, skin cancer, psoriasis, and fibroadenoma are known to be associated with EGFR mutations (Cancer Communications. 2020;40:43-59).

Among them, EGFR activating mutations such as the L858R point mutation in exon 21 of the EGFR tyrosine kinase domain or the in-frame deletion in exon 19 are known to be important causes of non-small cell lung cancer. Therefore, according to the prediction that the anticancer effect will be excellent if cancer cell signaling through the epidermal growth factor receptor is blocked, research for developing anticancer drugs targeting the epidermal growth factor receptor is being actively conducted.

The first drug developed as an EGFR tyrosine kinase inhibitor among small molecules is Gefitinib, which is a reversible inhibitor that selectively inhibits EGFR (Erb-B1) among EGFR subtypes. Another drug with such characteristics is Erlotinib, and this EGFR targeting therapy is mainly used for patients with EGFR activating mutations for non-small cell lung cancer (NSCLC) as a main indication.

However, it has been reported that NSCLC patients with EGFR activating mutations treated with Gefitinib or Erlotinib develop resistance to the drug after about 8 to 16 months, and about 60% of them show resistance due to the EGFR T790M mutation. (Helena A. Yu et al., Clin Cancer Res. 19(8), 2240, 2013).

Irreversible inhibitors have been proposed to overcome resistance to existing EGFR inhibitors such as Gefitinib or Erlotinib. However, since EGFR irreversible inhibitors also have high activity against EGFR WT (wild-type), which also exists in normal cells, serious side effects are caused when doses to overcome resistance due to EGFR T790M mutation are administered. It indicates the limitations of clinical application.

As an alternative to this, a number of drugs including Osimertinib, Olmutinib, Naquotinib, and Avitinib, which are EGFR mutation-selective inhibitors, are under development in the clinical stage. However, according to the clinical results of Osimertinib for non-small cell lung cancer patients with EGFR-resistant mutations, after about 10 months, other resistance mechanisms are activated, resulting in drug resistance, among which the C797S mutation appears at a high rate of 20% or more. The C797S mutation is a point mutation in which cysteine 773 (Cys773), which forms a covalent bond with irreversible inhibitors of EGFR, is changed to serine. As a result, it is impossible to form a covalent bond with irreversible inhibitors of EGFR, thereby causing a decrease in reactivity to the drug.

As described above, the development of EGFR-targeted therapeutics shows limitations that cannot maintain drug efficacy over a certain period of time due to the expression of primary and secondary resistance. In particular, researches on the EGFR C797S mutation have not presented even a substance under clinical study other than reports on early-stage preclinical studies, so effective treatment for this is urgently required.

WO 2022/268229 A1 relates to organic compounds targeting ALK, ROS1 and EGFR protein and mutein thereof and their use for preparing a pharmaceutical composition, in particular for inhibiting and reducing EGFR degradation.

Sun Ming et al. (Eur. J. of Med. Chem., vol. 193, Feb. 2020, XP086118072) describes the development of a Brigatinib degrader as a potential treatment of ALK positive cancer resistance, in particular effective in the treatment of non-small cell lung cancer.

Hyeonjeong Choe et al. (Bull. Korean Chem. Soc., vol. 38, no. 11, 2017, pp. 1353-1357, XP055561541) describes the structure-activity relationship of 2,4-dianillinopyrimidine containing methanesulfonamide as potent and selective EGFR mutant inhibitor for anti-cancer treatment.

### [Disclosure]

### [Technical Problem]

Therefore, a problem to be solved by the present disclosure is to provide a compound having an activity suppressing, inhibiting and/or degrading EGFR mutation-expressing cancer, pharmaceutical compositions comprising the compound as an active ingredient, and its medical use for treating or preventing cancer showing EGFR mutation(s).

Another problem to be solved by the present disclosure is to provide the use in a method for treating or alleviating EGFR mutation-expressing cancer, comprising administering to a patient in need of treatment, improvement or prevention of EGFR mutation-expressing cancer the compound according to the present invention, wherein it inhibits EGFR mutation-expressing cancer cells.

### [Technical Solution]

### Summary

In order to solve the above problem, one embodiment of the present invention provides a list of compounds as defined by the appended claim set. More generally, the disclosure refers to the following Chemical Formula 1 or a pharmaceutically acceptable salt thereof.

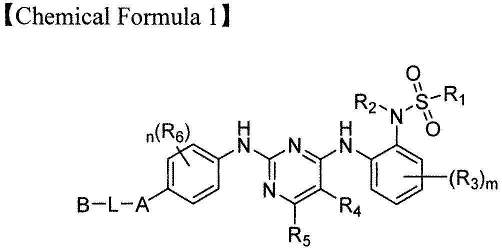

In the Chemical Formula 1,
R₁ and R₂ are each independently hydrogen, C1~C6 alkyl, CD₃, or C1~C6 haloalkyl,
R₃ to R₆ are each independently hydrogen, halogen, cyano, C1~C6 alkyl, C1~C6 alkoxy, C1~C6 haloalkyl, C1~C6 haloalkoxy, C3~C6 cycloalkyloxy, C3~C6 heterocycloalkyloxy, or - C(=O)OR₇, wherein R₇ is C1~C5 alkyl,
m and n are each independently 0, 1, 2, 3, or 4,
A is a divalent 4-7 membered heterocyclic ring, 4-7 membered spiro heterocyclic ring, or 8-10 membered bicyclic heterocyclic ring,
L is a group linking A and B by a covalent bond,
B is 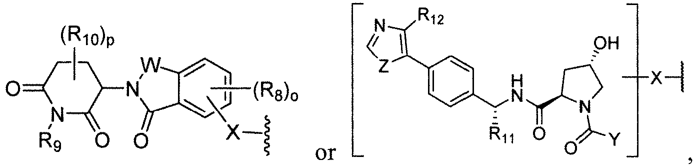
X is direct bond, (CH₂)₁₋₆, O-(CH₂)₀₋₆, C(O)-(CH₂)₀₋₆, N(R₁₃)-(CH₂)₀₋₆, C(O)-N(R₁₃)-(CH₂)₀₋₆, N(R₁₃)-C(O)-(CH₂)₀₋₆, N(R₁₃), N(R₁₃)-(CHR₁₄), or O-(CHR₁₄),
W is C(R₁₄)₂ or C(O),
R₈ is hydrogen, OH, halogen, C1~C6 alkyl, C1~C6 alkoxy, C1~C6 haloalkyl, or C1~C6 haloalkoxy,
R₉ and R₁₀ are each independently hydrogen or C1~C6 alkyl,
R₁₃ and R₁₄ are each independently hydrogen or C1~C6 alkyl,
o and p are each independently 1, 2, or 3,
Y is C1~C6 alkyl, C1~C6 haloalkyl, C3~C10 aryl, C3~C10 heteroaryl, or 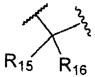 (wherein when Y is 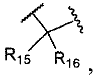 X is connected as 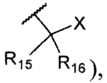
Z is O or S,
R₁₁ is hydrogen, C1~C6 alkyl, or (CH₂)₁₋₆-C(O) (wherein when R₁₁ is (CH₂)₁₋₆-C(O), X can be connected as (CH₂)₁₋₆-C(O)-X),
R₁₂ is hydrogen, C1~C6 alkyl, or C1~C6 cycloalkyl,
R₁₅ is hydrogen, C1~C6 alkyl, C1~C6 haloalkyl, or C1~C6 cycloalkyl,
R₁₆ is hydrogen, C1~C4 alkyl, N-(R₁₇)R₁₈, C3~C10 aryl, or C3~C10 heteroaryl, wherein R₁₇ and R₁₈ are each independently hydrogen, C1~C6 alkyl, C1~C6 haloalkyl, or -C(O).

Compounds of the present disclosure are excellent in degrading EGFR mutant proteins in cancers with EGFR mutations. Therefore, a preventive or therapeutic effect on cancers exhibiting EGFR mutations can be expected.

In particular, the compounds of the present disclosure have the advantage of differentially degrading mutant EGFR proteins including C797S and the like rather than EGFR proteins of cells having normal EGFR.

The compounds according to the present disclosure are particularly useful for degradation of EGFR mutant proteins exhibiting any one or more mutations among Del19, T790M, C797S, and L858R, and are particularly useful for degradation of Del19/T790M/C797S and L858R/T790M/C797S mutant proteins. Mutant EGFR including C797S is a resistance mutation that appears after treatment with Tagrisso (Osimertinib), a third-generation therapeutic agent. The compounds of the present disclosure have the advantage of being useful for inhibiting, degrading, and the like in lung cancer having the C797S mutation of EGFR, in particular, non-small cell lung cancer (NSCLC) cells.

The present disclosure, the right moiety in the chemical structure serves to bind and/or inhibit mutant EGFR protein, etc., and the left moiety serves as an E3 ligase binder to induce ubiquitin-proteasome system (UPS). This is expected to further promote degradation and/or inhibition of the protein, but the present invention is not limited to these theoretically predicted mechanisms.

The present disclosure also provides a pharmaceutical composition comprising a compound of Chemical Formula 1 or a pharmaceutically acceptable salt thereof, and a pharmaceutically acceptable carrier or additive. The invention refers to a pharmaceutical composition comprising a compound or a pharmaceutically acceptable salt thereof and a pharmaceutically acceptable carrier as defined by the appended claim set.

The present disclosure also provides a method for treating cancer exhibiting an EGFR mutation, comprising administering to a subject a therapeutically effective amount of a compound of Chemical Formula 1 or a pharmaceutically acceptable salt thereof. Cancer exhibiting the EGFR mutation is, for example, lung cancer, liver cancer, esophageal cancer, stomach cancer, colon cancer, small intestine cancer, pancreatic cancer, melanoma, breast cancer, oral cancer, brain tumor, thyroid cancer, parathyroid cancer, kidney cancer, cervical cancer, sarcoma, prostate cancer, urethral cancer, bladder cancer, testicular cancer, blood cancer, lymphoma, skin cancer, psoriasis, fibroadenoma, and the like. Preferably, the cancer exhibiting an EGFR mutation is lung cancer. More specifically, the cancer exhibiting an EGFR mutation is non-small cell lung cancer.

According to the invention, a pharmaceutical composition comprising a compound or a pharmaceutically acceptable salt thereof and a pharmaceutically acceptable carrier for use in a method of treating cancer is provided as defined by the appended claim set.

That is, the present disclosure provides a medical use characterized by using the compound of Chemical Formula 1 or a pharmaceutically acceptable salt thereof according to the present disclosure as an active ingredient. The medical use of the present disclosure is for the treatment or prevention of a disease or condition described herein.

The compounds, pharmaceutical compositions comprising such compounds, and their medical uses are more fully described in the detailed description that follows.

### Detailed Description

The following description is illustrative only.

### Definition

The following terms used herein are defined as follows.

As used herein, the terms "substituent", "radical", "group", "moiety", and "fragment" may be used interchangeably.

As used herein, the term "alkyl" means a saturated straight chain or branched non-cyclic hydrocarbon, unless the context clearly dictates otherwise, having from 1 to 10 carbon atoms. "Lower alkyl" means alkyl having from 1 to 4 carbon atoms. Representative saturated straight chain alkyls include -methyl, -ethyl, -n-propyl, -n-butyl, -n-pentyl, -n-hexyl, -n-heptyl, -n-octyl, - n-nonyl and -n-decyl, while saturated branched alkyls include -isopropyl, - sec-butyl, -isobutyl, - tert-butyl, -isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl and the like.

As used herein, if the term "C₁₋₆", "C1-6", or "C1-C6" is used, it means the number of carbon atoms is from 1 to 6. For example, C₁₋₆alkyl means an alkyl which carbon number is any integer of from 1 to 6.

As used herein, the terms "halogen" and "halo" mean fluorine, chlorine, bromine or iodine. In a specific example of the present disclosure, the halogen is chlorine or fluorine.

As used herein, the term "haloalkyl", "haloalkoxy", "haloalkenyl", or "haloalkynyl" mean an alkyl, alkoxy, alkenyl or alkynyl group, wherein one or more hydrogen atoms are substituted with halogen atoms. For example, the haloalkyl includes -CF₃, -CHF₂, -CH₂F, -CBr₃, -CHBr₂, - CH₂Br, -CC1₃, -CHC1₂, -CH₂CI, -CI₃, -CHI₂, -CH₂I, -CH₂-CF₃, -CH₂-CHF₂, -CH₂-CH₂F, -CH₂-CBr₃, -CH₂-CHBr₂, -CH₂-CH₂Br, -CH₂-CC1₃, -CH₂-CHC1₂, -CH₂-CH₂CI, -CH₂-CI₃, -CH₂-CHI₂, -CH₂-CH₂I, and the like, wherein alkyl and halogen are as described above. In an example of the present disclosure, haloalkyl is -CF₃.

As used herein, the term "alkoxy" means -O-(alkyl) including -OCH₃, -OCH₂CH₃, - O(CH₂)₂CH₃, -O(CH₂)₃CH₃, -O(CH₂)₄CH₃, -O(CH₂)₅CH₃, and the like, wherein alkyl is as defined above. As used herein, the term "lower alkoxy" means -O-(lower alkyl), wherein lower alkyl is as defined above.

The terms "aryloxy", "cycloalkyloxy" or "heterocycloalkyloxy" are RO-, where R is aryl, cycloalkyl or heterocycloalkyl, respectively, as defined herein. "Arylthio" is RS-, where R is aryl as defined above.

As used herein, the term "aryl" means a carbocyclic aromatic group containing from 3 to 10 ring atoms. Representative examples include, but are not limited to, phenyl, tolyl, xylyl, naphthyl, tetrahydronaphthyl, anthracenyl, fluorenyl, indenyl, and azulenyl. A carbocyclic aromatic group can be unsubstituted or optionally substituted.

As used herein, the term "cycloalkyl" means a monocyclic or polycyclic saturated ring having carbon and hydrogen atoms and having no carbon-carbon multiple bonds. Examples of monocyclic rings include, but are not limited to, (C₃-C₇)cycloalkyl groups, including cyclopropyl, cyclobutyl, cyclopentyl, cyclohexyl, and cycloheptyl. Examples of polycyclic rings include, but are not limited to, fused bicyclic rings (bicyclic hydrocarbon rings) such as octahydropentalene and decahydronaphthalene; spiro rings such as spiro[3.3]heptane, spiro[3.4]octane, spiro[3.5]nonane, spiro[4.4]nonane, spiro[4.5]decane, and spiro[5.5]undecane; and bridged bicycle rings such as bicyclo[2.1.1]hexane, bicyclo[2.2.1]heptane, and bicyclo[2.2.2]octane. A cycloalkyl group can be unsubstituted or optinally substituted. In an example of the present disclosure, the cycloalkyl group is monocyclic ring. In an example of the present disclosure, cycloalkyl is cyclopropyl. In an example of the present disclosure, "spiro hydrocarbon ring" or "spiro ring" is spiro[3.3]heptane, spiro[3.4]octane, spiro[4.3]octane, spiro[4.5]decane, spiro[4.4]decane, or spiro[5.5]undecane.

As used herein, the term "heteroaryl" means an aromatic heterocycle ring of 3- to 10-members and having at least one heteroatom selected from nitrogen, oxygen and sulfur, and containing at least 1 carbon atom, including both mono- and bicyclic ring systems. Representative heteroaryls are furan, 4H-pyran, pyrrole, imidazole, pyrazole, triazole, tetrazole, pyridine, pyrimidine, pyridazine, pyrazine, triazine, thiophene, ozaxole, isoxazole, thiazole, isothiazole, oxadiazole, benzofuran, benzothiophene, quinoline, indole, benzoxazole, benzimidazole, benzothiazole, cinnoline, phthalazine, quinazoline, 1H-azepine and the like.

The term "heterocycle" or "heterocycloalkyl" means a 3- to 7-membered monocyclic, or 6- to 12-membered bicyclic, saturated heterocyclic ring which contains from 1 to 4 heteroatoms independently selected from nitrogen, oxygen and sulfur, and wherein the nitrogen and sulfur heteroatoms can be optionally oxidized, and the nitrogen heteroatom can be optionally quatemized. Representative heterocycles include oxiran, oxetan, tetrahydrofuran, tetrahydropyran, 1,4-dioxane, aziridine, azetidine, pyrrolidine, piperidine, piperazine, pyrrolidinone, hydantoine, valerolactam, thiirane, thietane, tetrahydrothiophene, tetrahydrothiopyra, morpholine, tetrahydropyridine, and tetrahydropyrimidine. Heterocycles include a bicyclic ring in which part of the heterocycle is fused to a benzene or cyclopenta-1,3-diene ring. The heterocycle can be attached via any heteroatom or carbon atom. In addition, heterocycles include fused bicyclic rings, spiro rings and bridged bicyclic rings in which one or more carbon atoms of the aforementioned polycyclic rings are replaced with nitrogen, oxygen or sulfur atoms. For example, when the heteroatom is nitrogen, these include fused heterobicyclic rings (bicyclic hetero rings) such as octahydrocyclopenta[c]pyrrole, octahydropyrrolo[3,4-c]pyrrole, decahydroisoquinoline, and decahydro-2,6-naphthyridine; spiro (hetero)rings such as 2-azaspiro[3.3]heptane, 2,6-diazaspiro[3.3]heptane, 2-azaspiro[3.4]octane, 2,6-diazaspiro[3.4]octane, 2-azaspiro[3.5]nonane, 2,7-diazaspiro[3.5]nonane, 2-azaspiro[4.4]nonane, 2,7-diazaspiro[4.4]nonane, 8-azaspiro[4.5]decane, 2,8-diazaspiro[4.5]decane, 3-azaspiro[5.5]undecane, and 3,9-diazaspiro[5.5]undecane; and bridged heterobicyclic rings such as 2-azabicyclo[2.1.1]hexane, 2-azabicyclo[2.2.1]heptane, 2,5-diazabicyclo[2.2.1]heptane, 2-azabicyclo[2.2.2]octane, and 2,5-diazabicyclo[2.2.2]octane. In an embodiment of the present invention, the spiro heteroring is 2-azaspiro[3.3]heptane, 2,6-diazaspiro[3.3]heptane, 3-azaspiro[5.5]undecane, 3,9-diazaspiro[5.5]undecane, or 1,4-dioxaspiro[4.5]decane.

"Heterocycle fused to a phenyl" means a heterocycle attached to two adjacent carbon atoms of a phenyl ring, wherein the heterocycle is as defined above.

In this specification, "*" or " 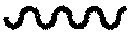 " means connected to another moiety.

As used herein, the term "pharmaceutically acceptable salt(s)" refers to a salt prepared from active compounds according to the present disclosure with relatively non-toxic acids or bases, depending on the particular substituents of those compounds. When the compounds have a relatively acidic group, base-added salts can be obtained by contacting the neutral compounds with a sufficient amount of the desired base and a pure or inert solvent. Suitable pharmaceutically acceptable base addition salts include sodium, potassium, calcium, aluminum, organic amino, magnesium salts and the like. When the compounds have a relatively basic group, acid-added salts can be obtained by contacting the neutral compounds with a sufficient amount of the desired acid and pure or inert solvent. Suitable pharmaceutically acceptable acid addition salts include salts derived from non-toxic organic acids including acetic acid, propionic acid, isobutyl acid, oxalic acid, maleic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, and the like, and non-toxic inorganic acids including hydrochloric acid, hydrobromic acid, nitric acid, carbonic acid, monohydrogencarbonic acid, phosphoric acid, monohydrogenphosphric acid, dihydrogenphosphoric acid, sulfuric acid, monohydrogensulfuric acid, hydrogen iodide, phosphorous acid and the like. Also it includes a salt of amino acid such as arginate or its analogues, and it also includes analogues of organic acid such as glucuronic or galacturonic acid. Some specific compounds of this disclosure have both basic and acidic functionality for the conversion of compounds with a basic or acidic portion (addition) salts. Other examples of salts are well known through literature known in the art to which the present invention pertains.

As used herein, "effective amount" refers to an amount of a compound of the invention sufficient to slow or minimize the progression of a cancer with EGFR mutation or to provide a therapeutic benefit in the treatment or management of a cancer with EGFR mutation. "Effective amount" also refers to an amount sufficient to inhibit or reduce EGFR mutation proteins, either *in vitro* or *in vivo.*

As used herein, the term "treatment" may be any one or more of preventive treatment, palliative treatment, and/or restorative treatment.

As used herein, the phrase "compound(s) of this/the disclosure" includes any compound(s) of Chemical Formula 1, as well as clathrates, hydrates, solvates, or polymorphs thereof. And, even if the term "compound(s) of the disclosure" does not mention its pharmaceutically acceptable salt, the term includes salts thereof. The phrase "Compound(s) of this/the invention" refers to compounds as defined by the appended claim set. In one embodiment, the compounds of this disclosure include stereo-chemically pure compounds, e.g., those substantially free (e.g., greater than 85% ee, greater than 90% ee, greater than 95% ee, greater than 97% ee, or greater than 99% ee) of other stereoisomers. That is, if the compounds of Chemical Formula 1 according to the present disclosure or salts thereof or compounds according to the invention as defined by the appended claim set are tautomeric isomers and/or stereoisomers (e.g., geometrical isomers and conformational isomers), such isolated isomers and their mixtures, also are included in the scope of this disclosure/invention. If the compounds of the present disclosure/invention or salts thereof have an asymmetric carbon in their structures, their active optical isomers and their racemic mixtures also are included in the scope of this disclosure/invention.

As used herein, the term "polymorph" refers to solid crystalline forms of a compound of this disclosure or complex thereof. Different polymorphs of the same compound can exhibit different physical, chemical and/or spectroscopic properties. Different physical properties include stability (e.g., to heat or light), compressibility and density (important in formulation and product manufacturing), and dissolution rates (which can affect bioavailability). Differences in stability can result from changes in chemical reactivity (e.g., differential oxidation, such that a dosage form discolors more rapidly when comprised of one polymorph than when comprised of another polymorph) or mechanical characteristics (e.g., tablets crumble on storage as a kinetically favored polymorph converts to thermodynamically more stable polymorph) or both (e.g., tablets of one polymorph are more susceptible to breakdown at high humidity). Different physical properties of polymorphs can affect their processing. For example, one polymorph might be more likely to form solvates or might be more difficult to filter or wash free of impurities than another due to, for example, the shape or size distribution of particles of it.

As used herein, the term "solvate" means a compound or its salt according to this disclosure/invention that further includes a stoichiometric or non-stoichiometric amount of a solvent bound by non-covalent intermolecular forces. Preferred solvents are volatile, non-toxic, and acceptable for administration to humans in trace amounts.

As used herein, the term "hydrate" means a compound or its salt according to this disclosure/invention that further includes a stoichiometric or non-stoichiometric amount of water bound by non-covalent intermolecular forces.

As used herein, the term "clathrate" means a compound or its salt in the form of a crystal lattice that contains spaces (e.g., channels) that have a guest molecule (e.g., a solvent or water) trapped within.

As used herein, the term "purified" means that when isolated, the isolate is greater than 90% pure, in one embodiment greater than 95% pure, in another embodiment greater than 99% pure and in another embodiment greater than 99.9% pure.

The term "hydrido" refers to a single -H atom (H) and is used interchangeably with the symbol "H" or the term "hydrogen".

If a substituent is described as "optionally substituted", the substituent may be (1) unsubstituted or (2) substituted with one or more of the defined substituents. If the substitutable position is unsubstituted, the default substituent is hydrogen.

As used herein, the singular "a" and "an" may include the plural forms unless the context clearly dictates otherwise.

The term "pharmaceutically acceptable" means suitable for use as a pharmaceutical preparation, and generally considered safe for such use. The term also means that it has been officially approved by the governing body of a country for this use, or is listed in the Korean Pharmacopoeia or the United States Pharmacopoeia.

### Compound of the present invention

More generally, the present disclosure provides a compound having a structure of Chemical Formula 1 or a pharmaceutically acceptable salt thereof.

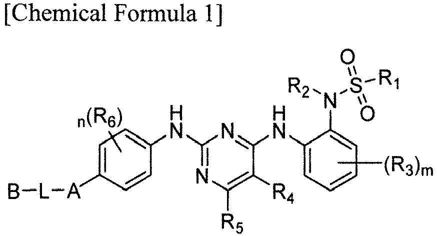

In the Chemical Formula 1,
R₁ and R₂ are each independently hydrogen, C1~C6 alkyl, CD₃, or C1~C6 haloalkyl,
R₃ to R₆ are each independently hydrogen, halogen, cyano, C1~C6 alkyl, C1~C6 alkoxy, C1~C6 haloalkyl, C1~C6 haloalkoxy, C3~C6 cycloalkyloxy, C3~C6 heterocycloalkyloxy, or - C(=O)OR₇, wherein R₇ is C1~C5 alkyl,
m and n are each independently 0, 1, 2, 3, or 4,
A is a divalent 4-7 membered heterocyclic ring, 4-7 membered spiro heterocyclic ring, or 8-10 membered bicyclic heterocyclic ring,
L is a group linking A and B by a covalent bond,
B is 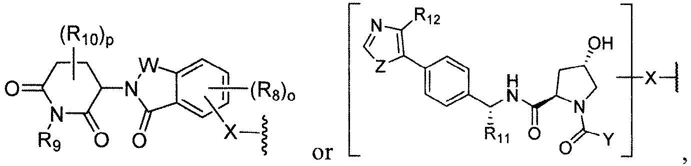
X is direct bond, (CH₂)₁₋₆, O-(CH₂)₀₋₆, C(O)-(CH₂)₀₋₆, N(R₁₃)-(CH₂)₀₋₆, C(O)-N(R₁₃)-(CH₂)₀₋₆, N(R₁₃)-C(O)-(CH₂)₀₋₆, N(R₁₃), N(R₁₃)-(CHR₁₄), or O-(CHR₁₄),
W is C(R₁₄)₂ or C(O),
R₈ is hydrogen, OH, halogen, C1~C6 alkyl, C1~C6 alkoxy, C1~C6 haloalkyl, or C1~C6 haloalkoxy,
R₉ and R₁₀ are each independently hydrogen or C1~C6 alkyl
R₁₃ and R₁₄ are each independently hydrogen or C1~C6 alkyl,
o and p are each independently 1, 2, or 3,
Y is C1~C6 alkyl, C1~C6 haloalkyl, C3~C10 aryl, C3~C10 heteroaryl, or 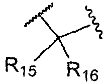 (wherein when Y is 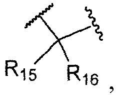 X is connected as 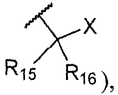
Z is O or S,
R₁₁ is hydrogen, C1~C6 alkyl, or (CH₂)₁₋₆-C(O) (wherein when R₁₁ is (CH₂)₁₋₆-C(O), X is connected as (CH₂)₁₋₆-C(O)-X),
R₁₂ is hydrogen, C1~C6 alkyl, or C1~C6 cycloalkyl,
R₁₅ is hydrogen, C1~C6 alkyl, C1~C6 haloalkyl, or C1~C6 cycloalkyl,
R₁₆ is hydrogen, C1~C4 alkyl, N-(R₁₇)R₁₈, C3~C10 aryl, or C3~C10 heteroaryl, wherein R₁₇ and R₁₈ are each independently hydrogen, C1~C6 alkyl, C1~C6 haloalkyl, or -C(O).

In a specific example, the present disclosure provides a compound represented by the Chemical Formula 1 or a pharmaceutically acceptable salt thereof, wherein
R₁ and R₂ are each independently hydrogen, C1~C6 alkyl, or C1~C6 haloalkyl,
R₃ is hydrogen, or C1~C6 alkyl,
R₄ is hydrogen, halogen (preferably, Cl), or C1~C6 alkyl,
R₅ is hydrogen, or C1~C6 alkyl,
R₆ is hydrogen, halogen, C1~C6 alkyl, C1~C6 alkoxy, or C1~C6 haloalkoxy,
m and n are each independently 0, 1, or 2,
A is 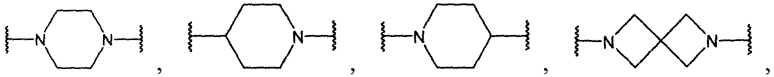 or 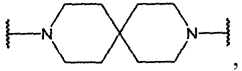
L is a group linking A and B by a covalent bond,
B is 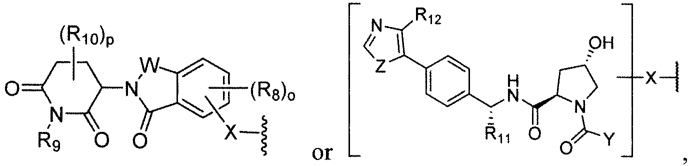
X is direct bond, (CH₂)₁₋₆, O-(CH₂)₀₋₆, C(O)-(CH₂)₀₋₆, N(R₁₃)-(CH₂)₀₋₆, C(O)-N(R₁₃)-(CH₂)₀₋₆, N(R₁₃)-C(O)-(CH₂)₀₋₆, N(R₁₃), N(R₁₃)-(CHR₁₄), or O-(CHR₁₄),
W is C(R₁₄)₂ or C(O),
R₈ is hydrogen, OH, halogen, C1~C6 alkyl, C1~C6 alkoxy, C1~C6 haloalkyl, or C1~C6 haloalkoxy,
R₉ and R₁₀ are each independently hydrogen or C1~C6 alkyl,
R₁₃ and R₁₄ are each independently hydrogen or C1~C6 alkyl,
o and p are each independently 1, 2, or 3,
Y is C1~C6 alkyl, or C1~C6 haloalkyl, or 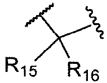 (wherein when Y is 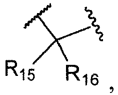 X is connected as 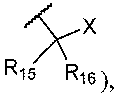
Z is O or S,
R₁₁ is hydrogen, C1~C6 alkyl, or (CH₂)₁₋₆-C(O),
R₁₂ is hydrogen, C1~C6 alkyl, or C1~C6 cycloalkyl,
R₁₅ is hydrogen, C1~C6 alkyl, C1~C6 haloalkyl, or C1~C6 cycloalkyl,
R₁₆ is hydrogen, C1~C4 alkyl, N-(R₁₇)R₁₈, C3~C10 aryl, or C3~C10 heteroaryl, wherein R₁₇ and R₁₈ are each independently hydrogen, C1~C6 alkyl, C1~C6 haloalkyl, or -C(O).

In the other example, the present disclosure provides a compound represented by the Chemical Formula 1 or a pharmaceutically acceptable salt thereof, wherein L is a direct bond or divalent, saturated or unsaturated C1~C50 hydrocarbon chain; wherein at least one of the methylene units of L may be replaced with one or more selected from the group consisting of -Cy-, -O-, -N(R₂₀)-, -S-, -OC(O)-, -C(O)O-, -C(O)-, -S(O)-, -S(O)₂-, -N(R₂₀)S(O)₂-, -S(O)₂N(R₂₀)-, - N(R₂₀)C(O)-, -C(O)N(R₂₀)-, -OC(O)N(R₂₀)-, -N(R₂₀)C(O)-, -(CH₂OCH₂)-, -(OCH₂CH₂)-, - (CH₂CH₂O)-, -(C(O)CH₂CH₂)-, and -(CH₂CH₂C(O))-; here, -Cy- is each independently a divalent ring substituent, which is phenylene, 4-7-membered spiro hydrocarbon ring, 8-10-membered bicyclic hydrocarbon ring, 4-7-membered heterocyclic ring, 4-7-membered spiro heterocyclic ring, 8-10-membered bicyclic heterocyclic ring, 5-6-membered heteroarylene, or 8-10-membered bicyclic heteroarylene, wherein R₂₀ is hydrogen or C1~C4 alkyl.

Specifically, in the other example, the present disclosure provides a compound represented by the Chemical Formula 1 or a pharmaceutically acceptable salt thereof, wherein the X-L of the Chemical Formula 1 is -K1-K2-K3-K4-K5-,
K1 is C1~C6 alkyl, NH, O, CC, N-C1~C6 alkyl, NH-C1~C6 alkyl, O-C1~C6 alkyl, 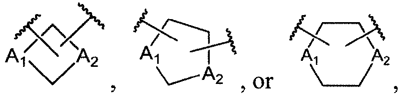
K2 is direct bond, C(O), or O,
K3 is direct bond, (CH₂)ₙ₁, or (CH₂)ₙ₁-O, wherein n1 is an integer of from 0 to 10,
K4 is direct bond, (CH₂CH₂O)ₙ₂, 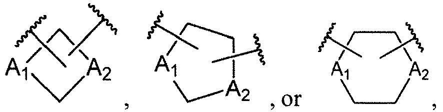 wherein n2 is an integer of from 0 to 5,
K5 is direct bond, (CH₂)ₙ₃, NH or N-C1~C3 alkyl, wherein n3 is an integer of from 0 to 3, and
one of A₁ and A₂ is N and the other is CH, or both A₁ and A₂ are N.

More specifically, another example of the present disclosure provides a compound or a pharmaceutically acceptable salt thereof, wherein X-L of the Chemical Formula 1 is the X-L moiety used in the following examples. X-L used in the examples may be connected to various Bs (except for X) disclosed herein on the left side, and various '-A-EGFR-binding moieties' disclosed herein may be connected to the right side.

More specifically, in the other example, the present disclosure provides a compound represented by the Chemical Formula 1 or a pharmaceutically acceptable salt thereof, wherein the A is 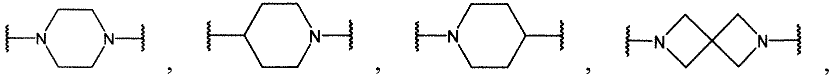 or

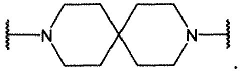

Specifically, one example of the present disclosure provides a compound represented by the Chemical Formula 1a below or a pharmaceutically acceptable salt thereof

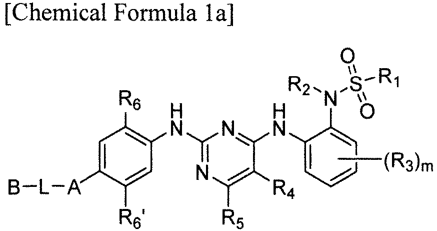

In the Chemical Formula 1a,
R₁ and R₂ are each independently hydrogen, C1~C6 alkyl, or C1~C6 haloalkyl,
R₃ to R₆ are each independently hydrogen, halogen, cyano, C1~C6 alkyl, C1~C6 alkoxy, C1~C6 haloalkyl, C1~C6 haloalkoxy, C3~C6 cycloalkyloxy, C3~C6 heterocycloalkyloxy, or - C(=O)OR₇, wherein R₇ C1~C5 alkyl,
R₆' is halogen or C1~C6 alkyl,
m is 0, 1, 2, 3, or 4,
A is a divalent 4-7 membered heterocyclic ring, 4-7 membered spiro heterocyclic ring, or 8-10 membered bicyclic heterocyclic ring,
L is a group linking A and B by a covalent bond,
B is 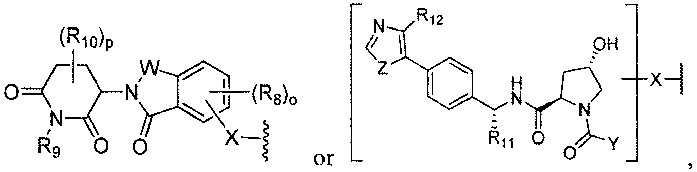
X is direct bond, (CH₂)₁₋₆, O-(CH₂)₀₋₆, C(O)-(CH₂)₀₋₆, N(R₁₃)-(CH₂)₀₋₆, C(O)-N(R₁₃)-(CH₂)₀₋₆, N(R₁₃)-C(O)-(CH₂)₀₋₆, N(R₁₃), N(R₁₃)-(CHR₁₄), or O-(CHR₁₄),
W is C(R₁₄)₂ or C(O),
R₈ is hydrogen, OH, halogen, C1~C6 alkyl, C1~C6 alkoxy, C1~C6 haloalkyl, or C1~C6 haloalkoxy,
R₉ and R₁₀ are each independently hydrogen or C1~C6 alkyl
R₁₃ and R₁₄ are each independently hydrogen or C1~C6 alkyl,
o and p are each independently 1, 2, or 3,
Y is C1~C6 alkyl, C1~C6 haloalkyl, C3~C10 aryl, C3~C10 heteroaryl, or 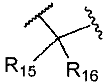 (wherein when Y is 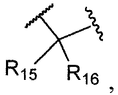 X is 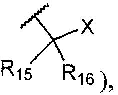
Z is O or S,
R₁₁ is hydrogen, C1~C6 alkyl, or (CH₂)₁₋₆-C(O),
R₁₂ is hydrogen, C1~C6 alkyl, or C1~C6 cycloalkyl,
R₁₅ is hydrogen, C1~C6 alkyl, C1~C6 haloalkyl, or C1~C6 cycloalkyl,
R₁₆ is hydrogen, C1~C4 alkyl, N-(R₁₇)R₁₈, C3~C10 aryl, or C3~C10 heteroaryl, wherein R₁₇ and R₁₈ are each independently hydrogen, C1~C6 alkyl, C1~C6 haloalkyl, or -C(O).

Among the compounds of Chemical Formula 1, when they have the same structure as Chemical Formula 1a, bioavailability is significantly improved as described later. As shown in the exemplary experimental results described later, Compounds 74, 76, 77, 80 and 81 showed significantly improved AUC compared to the corresponding compounds 45, 71, 51, 65 and 53.

Specifically, one example of the present disclosure also provides a compound represented by the Chemical Formula 1 or Chemical Formula 1a or a pharmaceutically acceptable salt thereof, wherein X is O-(CH₂)₀₋₆, N(R₁₃)-(CHR₁₄), or O-(CHR₁₄), and R₁₄ is C1~C6 alkyl (preferably, methyl) (the rest of the substituents are the same as defined in Chemical Formula 1 or 1a). As shown in the exemplary experimental results described later, Compounds 69, 70, 75, 78, and 82 showed significantly improved AUC compared to the corresponding compounds 53 and 65. These compounds are compounds in which K1 of -K1-K2-K3-K4-K5- is *-O-C1~C6 alkyl-*, or *-NH-C1~C6 alkyl-* (K2, K3, K4, and K5 are the same as the previous definition), wherein O or NH is connected to the 2nd or 3rd carbon other than the terminal of C1~C6 alkyl.

The present inventors performed various evaluation experiments after synthesizing various compounds in order to secure compounds with good therapeutic or preventive effects for cancer showing EGFR mutations and reduced other side effects and their use by having particularly high inhibitory, degrading and/or binding activity to the mutant EGFR protein, and also high selectivity thereto, and preferably effectively degrading the mutant EGFR protein. The present inventors also evaluated pharmacokinetic aspects such as bioavailability in addition to pharmacological aspects. Finally, the present invention was completed by confirming that the compounds of the present invention are suitable for the purpose of the present invention.

The present invention provides the compounds of Examples described below and as defined by the appended claim set and pharmaceutically acceptable salts thereof.

Preferably, another embodiment of the present invention provides a compound and a pharmaceutically acceptable salt thereof, wherein the compound of Chemical Formula 1 is a compound of Table 1 below. The compounds of Table 1 below are more preferable in various aspects such as drug efficacy and bioavailability.

**[Table 1]**

| Comp ound no. | IUPAC Name |
|---|---|
| 11 | (2R,4R)-1-((S)-2-(2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)acetamido)-3,3-dimethylbutanoyl)-4-hydroxy-N-(4-(4-methylthiazol-5-yl)benzyl)pyrrolidin-2-carboxamide |
| 19 | (2R,4R)-1-((S)-2-(2-(4-(1-(4-((5-chloro-4-((2-(N-methylmethylsulfonamido)phenyl)amino)pyrimidin-2-yl)amino)-2-fluorophenyl)piperidin-4-yl)piperazin-1-yl)acetamido)-3,3-dimethylbutanoyl)-4-hydroxy-N-(4-(4-methylthiazol-5-yl)benzyl)pyrrolidin-2-carboxamide |
| 20 | (2R,4R)-1-((S)-2-(3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanamido)-3,3-dimethylbutanoyl)-4-hydroxy-N-(4-(4-methylthiazol-5-yl)benzyl)pyrrolidin-2-carboxamide |
| 22 | 2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1-oxoisoindolin-4-yl)acetamide |
| 24 | 2-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)-N-(2-(2,6-dioxopiperidin-3-yl)-1-oxoisoindolin-4-yl)acetamide |
| 26 | 2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)-N-(2-(2,6-dioxopiperidin-3-yl)-1-oxoisoindolin-4-yl)acetamide |
| 27 | (2R,4R)-1-((S)-2-(5-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)acetamido)-3,3-dimethylbutanoyl)-4-hydroxy-N-(4-(4-methylthiazol-5-yl)benzyl)pyrrolidin-2-carboxamide |
| 28 | (2R,4R)-1-((S)-2-(2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamide |
| 31 | (2R,4R)-1-((S)-2-(2-(9-(4-((5-chloro-4-((2-(N-methylmethylsulfonamido)phenyl)amino)pyrimidin-2-yl)amino)-3-methoxyphenyl)-3,9-diazaspiro[5,5]undecan-3-yl)acetamido)-3,3-dimethylbutanoyl)-4-hydroxy-N-(4-(4-methylthiazol-5-yl)benzyl)pyrrolidin-2-carboxamide |
| 35 | (2S,4R)-1-((S)-2-(2-(4-((4-(3-chloro-4-((5-chloro-4-((2-(N-methylmethylsulfonamido)phenyl)amino)pyrimidin-2-yl)amino)phenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamide |
| 36 | (2S,4R)-1-((S)-2-(2-(4-((4-(4-((5-chloro-4-((2-(N-methylmethylsulfonamido)phenyl)amino)pyrimidin-2-yl)amino)2-fluorophenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamide |
| 39 | N-(2-((5-chloro-2-((4-(4-(2-(3-((2-(2,6-dioxopiperidin-3-yl)-1-oxoisoindolin-4-yl)oxy)azetidin-1-yl)ethyl)piperazin-1-yl)-3-fluorophenyl)amino)pyrimidin-4-yl)amino)phenyl)-N-methylmethanesulfonamide |
| 40 | N-(2-((5-chloro-2-((4-(4-(2-((1-(2-(2,6-dioxopiperidin-3-yl)-1,3-dioxoisoindolin-5-yl)azetidin-3-yl)oxy)ethyl)piperazin-1-yl)-3-fluorophenyl)amino)pyrimidin-4-yl)amino)phenyl)-N-methylmethanesulfonamide |
| 43 | N-(2-((5-chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-3-fluorophenyl)amino)pyrimidin-4-yl)amino)phenyl)-N-methylmethanesulfonamide |
| 45 | N-(2-((5-chloro-2-((4-(4-(4-(2-((2-(2,6-dioxopiperidin-3-yl)-1,3-dioxoisoindolin-4-yl)amino)ethyl)piperazin-1-yl)piperidin-1-yl)-2-methoxyphenyl)amino)pyrimidin-4-yl)amino)phenyl)-N-methylmethanesulfonamide |
| 46 | N-(2-((5-chloro-2-((4-(4-((1-(2-((2-(2,6-dioxopiperidin-3-yl)-1,3-dioxoisoindolin-4-yl)oxy)ethyl)piperidin-4-yl)methyl)piperazin-1-yl)-3-fluorophenyl)amino)pyrimidin-4-yl)amino)phenyl)-N-methylmethanesulfonamide |
| 47 | 2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1-oxoisoindolin-5-yl)acetamide |
| 51 | N-(2-((5-chloro-2-((4-(4-(4-(2-(1-(2-(2,6-dioxopiperidin-3-yl)-1,3-dioxoisoindolin-5-yl)piperidin-4-yl)ethyl)piperazin-1-yl)piperidin-1-yl)-2-methoxyphenyl)amino)pyrimidin-4-yl)amino)phenyl)-N-methylmethanesulfonamide |
| 53 | N-(2-((5-chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-2-methoxyphenyl)amino)pyrimidin-4-yl)amino)phenyl)-N-methylmethanesulfonamide |
| 54 | N-(2-((5-chloro-2-((4-(4-((1-(3-(2-(2,6-dioxopiperidin-3-yl)-1,3-dioxoisoindolin-4-yl)propanoyl)piperidin-4-yl)methyl)piperazin-1-yl)-2-methoxyphenyl)amino)pyrimidin-4-yl)amino)phenyl)-N-methylmethanesulfonamide |
| 59 | N-(2-((5-chloro-2-((4-(4-((1-((1-(2-(2,6-dioxopiperidin-3-yl)-1,3-dioxoisoindolin-5-yl)piperidin-4-yl)methyl)piperidin-4-yl)methyl)piperazin-1-yl)-2-methoxyphenyl)amino)pyrimidin-4-yl)amino)phenyl)-N-methylmethanesulfonamide |
| 62 | N-(2-((5-chloro-2-((4-(4-((4-(((2-(2,6-dioxopiperidin-3-yl)-1,3-dioxoisoindolin-4-yl)amino)methyl)piperidin-1-yl)methyl)piperidin-1-yl)-2-methoxyphenyl)amino)pyrimidin-4-yl)amino)phenyl)-N-methylmethanesulfonamide |
| 63 | N-(2-((5-chloro-2-((4-(4-((4-(((2-(2,6-dioxopiperidin-3-yl)-1,3-dioxoisoindolin-5-yl)amino)methyl)piperidin-1-yl)methyl)piperidin-1-yl)-2-methoxyphenyl)amino)pyrimidin-4-yl)amino)phenyl)-N-methylmethanesulfonamide |
| 65 | N-(2-((5-chloro-2-((4-(4-((1-((2-(2,6-dioxopiperidin-3-yl)-1,3-dioxoisoindolin-4-yl)glycyl)piperidin-4-yl)methyl)piperazin-1-yl)-2-methoxyphenyl)amino)pyrimidin-4-yl)amino)phenyl)-N-methylmethanesulfonamide |
| 66 | N-(2-((5-chloro-2-((4-(4-((1-((2-(2,6-dioxopiperidin-3-yl)-1,3-dioxoisoindolin-5-yl)glycyl)piperidin-4-yl)methyl)piperazin-1-yl)-2-methoxyphenyl)amino)pyrimidin-4-yl)amino)phenyl)-N-methylmethanesulfonamide |
| 68 | N-(2-((5-chloro-2-((4-(4-((4-(2-(2,6-dioxopiperidin-3-yl)-1,3-dioxoisoindolin-5-yl)piperidin-4-yl)methyl)piperazin-1-yl)-2-methoxyphenyl)amino)pyrimidin-4-yl)amino)phenyl)-N-methylmethanesulfonamide |
| 69 | N-(2-((5-chloro-2-((4-(4-((1-((2-(2,6-dioxopiperidin-3-yl)-1,3-dioxoisoindolin-4-yl)oxy)propanoyl)piperidin-4-yl)methyl)piperazin-1-yl)-2-methoxyphenyl)amino)pyrimidin-4-yl)amino)phenyl)-N-methylmethanesulfonamide |
| 70 | N-(2-((5-chloro-2-((4-(4-((1-((2-(2,6-dioxopiperidin-3-yl)-1,3-dioxoisoindolin-4-yl)-L-alanyl)piperidin-4-yl)methyl)piperazin-1-yl)-2-methoxyphenyl)amino)pyrimidin-4-yl)amino)phenyl)-N-methylmethanesulfonamide |
| 71 | N-(2-((5-chloro-2-((4-(4-(4-((1-(2-(2,6-dioxopiperidin-3-yl)-1,3-dioxoisoindolin-5-yl)piperidin-4-yl)methyl)piperazin-1-yl)piperidin-1-yl)-2-methoxyphenyl)amino)pyrimidin-4-yl)amino)phenyl)-N-methylmethanesulfonamide |
| 74 | N-(2-((5-chloro-2-((4-(4-(4-(2-((2-(2,6-dioxopiperidin-3-yl)-1,3-dioxoisoindolin-4-yl)amino)ethyl)piperazin-1-yl)piperidin-1-yl)-2-methoxy-5-methylphenyl)amino)pyrimidin-4-yl)amino)phenyl)-N-methylmethanesulfonamide |
| 75 | N-(2-((5-chloro-2-((4-(4-((1-((2-(2,6-dioxopiperidin-3-yl)-1,3-dioxoisoindolin-4-yl)-L-alanyl)piperidin-4-yl)methyl)piperazin-1-yl)-2-methoxy-5-methylphenyl)amino)pyrimidin-4-yl)amino)phenyl)-N-methylmethanesulfonamide |
| 76 | N-(2-((5-chloro-2-((4-(4-(4-((1-(2-(2,6-dioxopiperidin-3-yl)-1,3-dioxoisoindolin-5-yl)piperidin-4-yl)methyl)piperazin-1-yl)piperidin-1-yl)-2-methoxy-5-methylphenyl)amino)pyrimidin-4-yl)amino)phenyl)-N-methylmethanesulfonamide |
| 77 | N-(2-((5-chloro-2-((4-(4-(4-(2-(1-(2-(2,6-dioxopiperidin-3-yl)-1,3-dioxoisoindolin-5-yl)piperidin-4-yl)ethyl)piperazin-1-yl)piperidin-1-yl)-2-methoxy5-methylphenyl)amino)pyrimidin-4-yl)amino)phenyl)-N-methylmethanesulfonamide |
| 78 | N-(2-((5-chloro-2-((4-(4-((4-((2-(2,6-dioxopiperidin-3-yl)-1,3-dioxoisoindolin-4-yl)-L-alanyl)piperazin-1-yl)methyl)piperidin-1-yl)-2-methoxy-5-methylphenyl)amino)pyrimidin-4-yl)amino)phenyl)-N-methylmethanesulfonamide |
| 80 | N-(2-((5-chloro-2-((4-(4-((1-((2-(2,6-dioxopiperidin-3-yl)-1,3-dioxoisoindolin-4-yl)glycyl)piperidin-4-yl)methyl)piperazin-1-yl)-2-methoxy-5-methylphenyl)amino)pyrimidin-4-yl)amino)phenyl)-N-methylmethanesulfonamide |
| 81 | N-(2-((5-chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-2-methoxy-5-methylphenyl)amino)pyrimidin-4-yl)amino)phenyl)-N-methylmethanesulfonamide |
| 82 | N-(2-((5-chloro-2-((4-(4-((1-((2S)-2-((2-(2,6-dioxopiperidin-3-yl)-1,3-dioxoisoindolin-4-yl)oxy)propanoyl)piperidin-4-yl)methyl)piperazin-1-yl)-2-methoxy-5-methylphenyl)amino)pyrimidin-4-yl)amino)phenyl)-N-methylmethanesulfonamide |

In another embodiment, the present invention provides a pharmaceutical composition comprising a therapeutically effective amount of a compound as defined by the appended claim set or a pharmaceutically acceptable salt thereof according to the present invention, and a pharmaceutically acceptable carrier.

### Medical uses and methods of treatment of the compounds according to the present invention

The present invention further provides use in methods for treating a disease or condition in a subject having or susceptible to having such a disease or condition as defined by the appended claims, by administering to the subject a therapeutically-effective amount of one or more compounds as described above. In one embodiment, the treatment is preventative treatment. In another embodiment, the treatment is palliative treatment. In another embodiment, the treatment is restorative treatment.

### 1. Diseases or conditions

The compounds of the present invention for inhibiting or/and degrading EGFR mutant protein are useful for various therapeutic or prophylactic uses. These compounds can be used to treat cancers showing an EGFR mutant or to prevent exacerbation of these diseases. Accordingly, the present invention provides use in a method of inhibiting, suppressing, and/or degrading EGFR mutant protein activity in a cell. In this method, the cells are contacted with an effective amount of a compound of the present invention. In one embodiment, the cell is in a subject (e.g., a lung cancer patient). Use in the method of the present invention comprises administering a pharmaceutical composition comprising a therapeutically or prophylactically effective amount of a compound of the present invention to a subject in need of treatment or prevention of a cancer disease showing an EGFR mutation.

The present invention provides use in a method for inhibiting, degrading or inhibiting EGFR mutant protein activity in cancer cells exhibiting EGFR mutations. For example, the present invention can be used for treatment or prevention of lung cancer, liver cancer, esophageal cancer, gastric cancer, colon cancer, small intestine cancer, pancreatic cancer, melanoma, breast cancer, oral cancer, brain tumor, thyroid cancer, parathyroid cancer, kidney cancer, cervical cancer, sarcoma, prostate cancer, urethral cancer, bladder cancer, testicular cancer, blood cancer, lymphoma, skin cancer, fibroadenoma, and the like, which have EGFR mutations.

A compound of the present invention can be administered to the subject in the form of a pharmaceutical composition described herein.

In another embodiment, the present invention provides use in a method for treating or preventing cancer exhibiting an EGFR mutation in a subject, and cancers exhibiting such an EGFR mutation include lung cancer, liver cancer, esophageal cancer, gastric cancer, colon cancer, small intestine cancer, pancreatic cancer, melanoma, breast cancer, oral cancer, brain tumor, thyroid cancer, parathyroid cancer, kidney cancer, cervical cancer, sarcoma, prostate cancer, urethral cancer, bladder cancer, testicular cancer, blood cancer, lymphoma, skin cancer, fibroadenoma, and the like. Such methods comprise administering to a subject in need of treatment an amount of a compound of the present invention sufficient to inhibit EGFR mutant protein activity, i.e., a therapeutically effective amount.

In particular, the compounds of the present invention are useful for degradation of EGFR mutant proteins exhibiting any one or more mutations among Del19, T790M, C797S, and L858R, and are particularly useful for degradation of Del19/T790M/C797S and L858R/T790M/C797S EGFR mutant proteins. Cancers with these mutant EGFRs may be lung cancers, particularly non-small cell lung cancer (NSCLC).

### 2. Subjects

Suitable subjects to be treated according to the present invention include mammalian subjects. Mammals according to the present disclosure include human, canine, feline, bovine, caprine, equine, ovine, porcine, rodents, lagomorphs, primates, and the like, and encompass mammals *in utero.* Subjects can be of both sexes and can be at any stage of development.

In one embodiment, the suitable subject to be treated according to the present invention is human.

### 3. Administration and Dosing

The compounds of the present invention are generally administered in a therapeutically effective amount.

The compounds of the present invention can be administered by any suitable route in the form of a pharmaceutical composition adapted to such a route, and in a dose effective for the treatment intended. An effective dosage is typically in the range of about 0.001 to about 100 mg per kg body weight per day, preferably about 0.01 to about 50 mg/kg/day, in single or divided doses. Depending on age, species and disease or condition being treated, dosage levels below the lower limit of this range may be suitable. In other cases, still larger doses may be used without harmful side effects. Larger doses may also be divided into several smaller doses, for administration throughout the day.

### Pharmaceutical compositions, dosage forms and administration routes

For the treatment of the diseases or conditions referred to above, the compounds described herein or pharmaceutically acceptable salts thereof can be administered as follows:

### Oral administration

The compounds of the present invention may be administered orally, including by swallowing, so that the compound enters the gastrointestinal tract, or absorbed into the blood stream directly from the mouth (e.g., buccal or sublingual administration).

Suitable compositions for oral administration include solid, liquid, gel or powder formulations, and have a dosage form such as tablet, lozenge, capsule, granule or powder.

Compositions for oral administration may optionally be enteric coated and may exhibit delayed or sustained release through the enteric coating. That is, the composition for oral administration according to the present invention may be a formulation having an immediate or modified release pattern.

Liquid formulations can include solutions, syrups and suspensions, which can be used in soft or hard capsules. Such formulations may include a pharmaceutically acceptable carrier, for example, water, ethanol, polyethylene glycol, cellulose, or an oil. The formulation may also include one or more emulsifying agents and/or suspending agents.

In a tablet dosage form the amount of drug, active ingredient, present may be from about 0.05% to about 95% by weight, more typically from about 2% to about 50% by weight of the dosage form. In addition, tablets may contain a disintegrant, comprising from about 0.5% to about 35% by weight, more typically from about 2% to about 25% of the dosage form. Examples of disintegrants include lactose, starch, sodium starch glycolate, crospovidone, croscarmellose sodium, maltodextrin, or mixtures thereof.

Suitable lubricants, for use in a tablet, may be present in amounts from about 0.1% to about 5% by weight, and include talc, silicon dioxide, stearic acid, calcium, zinc or magnesium stearate, sodium stearyl fumarate and the like.

Suitable binders, for use in a tablet, include gelatin, polyethylene glycol, sugars, gums, starch, polyvinyl pyrrolidone, hydroxypropyl cellulose, hydroxypropylmethyl cellulose and the like. Suitable diluents, for use in a tablet, include mannitol, xylitol, lactose, dextrose, sucrose, sorbitol, microcrystalline cellulose and starch.

Suitable solubilizers, for use in a tablet, may be present in amounts from about 0.1% to about 3% by weight, and include polysorbates, sodium lauryl sulfate, sodium dodecyl sulfate, propylene carbonate, diethyleneglycol monoethyl ether, dimethyl isosorbide, polyethylene glycol (natural or hydrogenated) castor oil, HCOR^{™} (Nikkol), oleyl ester, Gelucire^{™}, caprylic/caprylic acid mono/diglyceride, sorbitan fatty acid esters, and Solutol HS^{™}.

### Parenteral Administration

Compounds of the present disclosure may be administered directly into the blood stream, muscle, or internal organs. Suitable means for parenteral administration include intravenous, intramuscular, subcutaneous intraarterial, intraperitoneal, intrathecal, intracranial, and the like. Suitable devices for parenteral administration include injectors (including needle and needle-free injectors) and infusion methods.

Compositions for parenteral administration may be formulated as immediate or modified release, including delayed or sustained release.

Most parenteral formulations are liquid compositions, and the liquid composition is an aqueous solution containing the active ingredient according to the present invention, a salt, a buffering agent, an isotonic agent, and the like.

Parenteral formulations may also be prepared in a dehydrated form (e.g., by lyophilization) or as sterile non-aqueous solutions. These formulations can be used with a suitable vehicle, such as sterile water. Solubility-enhancing agents may also be used in preparation of parenteral solutions.

### Topical Administration

Compounds of the present invention may be administered topically to the skin or transdermally. Formulations for this topical administration can include lotions, solutions, creams, gels, hydrogels, ointments, foams, implants, patches and the like. Pharmaceutically acceptable carriers for topical administration formulations can include water, alcohol, mineral oil, glycerin, polyethylene glycol and the like. Topical administration can also be performed by electroporation, iontophoresis, phonophoresis and the like.

Compositions for topical administration may be formulated as immediate or modified release, including delayed or sustained release.

### [Advantageous Effects]

The present invention relates to compounds capable of exhibiting various pharmacological activities by inhibiting or degrading the activity of EGFR mutant proteins, pharmaceutical compositions comprising them as an active ingredient, and their medical uses (for the treatment or prevention of cancers showing EGFR mutations, especially lung cancer) and their use in a treatment method comprising administering them to a subject in need of treatment or prevention. The compounds according to the present invention or pharmaceutically acceptable salts thereof are excellent in various aspects such as safety due to high selectivity for mutant EGFR, and may exhibit excellent medical effects in terms of activity inhibition or degradation of EGFR mutant.

### [Brief Description of Figures]

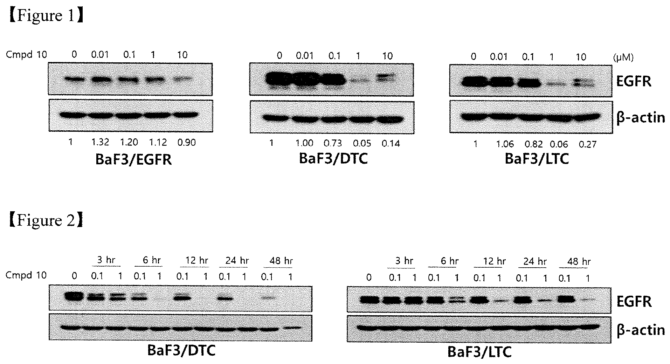

Figure 1 is a result of a Western blot experiment showing protein degrading ability for normal EGFR, Del19/T790M/C797S mutant EGFR (EGFR^{del19+T790M+C797S})(DTC), and L858R/T790M/C797S mutant EGFR (EGFR^{L858R+T790M+C797S})(LTC) depending on the concentration of the compound of Example 10.
Figure 2 is a result of Western blot experiment showing the protein degradation for Del19/T790M/C797S mutant EGFR (EGFR^{del19+T790M+C797S)} and L858R/T790M/C797S mutant EGFR (EGFR^{L858R+T790M+C797S}) according to concentration of Compound 10 (not claimed) and treatment time.

### [Mode for Invention]

Hereinafter, the present invention is described in considerable detail with examples to help those skilled in the art understand the present invention. However, the following examples are offered by way of illustration.

### Preparation of compounds of the present invention

Hereinafter, the synthesis process of some compounds of the present invention will be described, and the other compounds not mentioned below can be prepared by substituting starting materials, intermediates and/or reactants in a similar manner.

### Example 1: Synthesis of 5-(4-(4-((5-chloro-4-((2-(N-methylmethylsulfonamido)phenyl)amino)pyrimidin-2-yl)amino)-3-methoxyphenyl)piperazin-1-yl)-N-(2-(2,6-dioxopiperidin-3-yl)-1,3-dioxoisoindolin-4-yl)pentanamide (Compound 1) (not claimed)

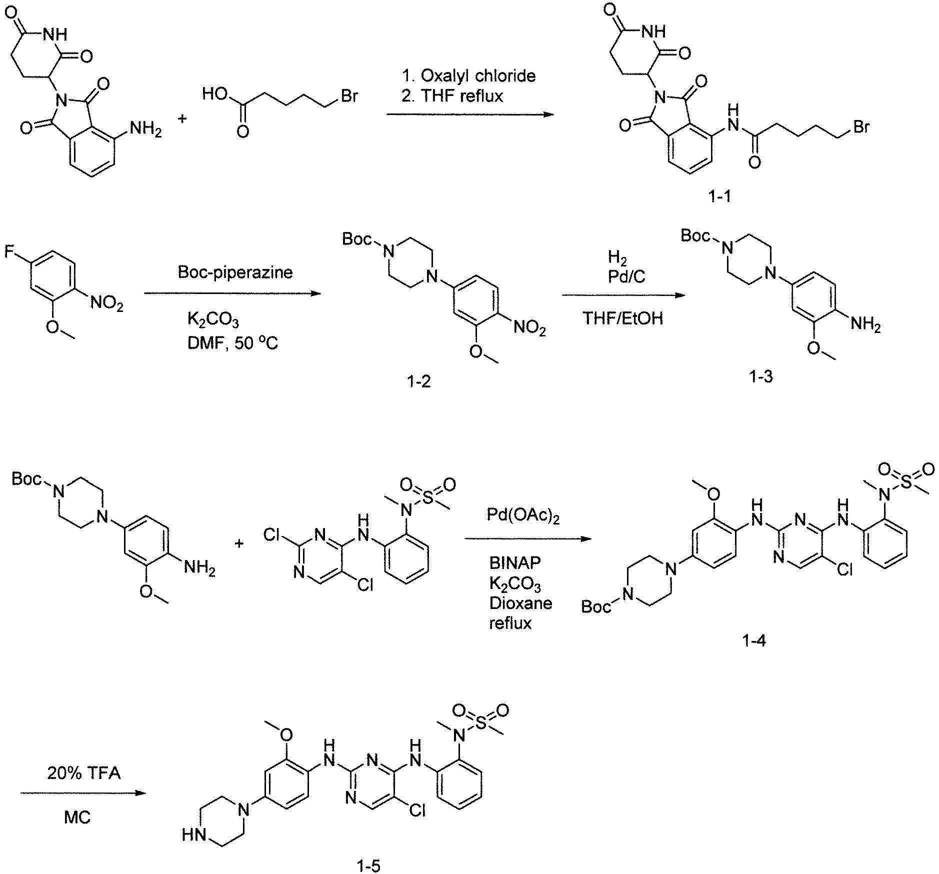

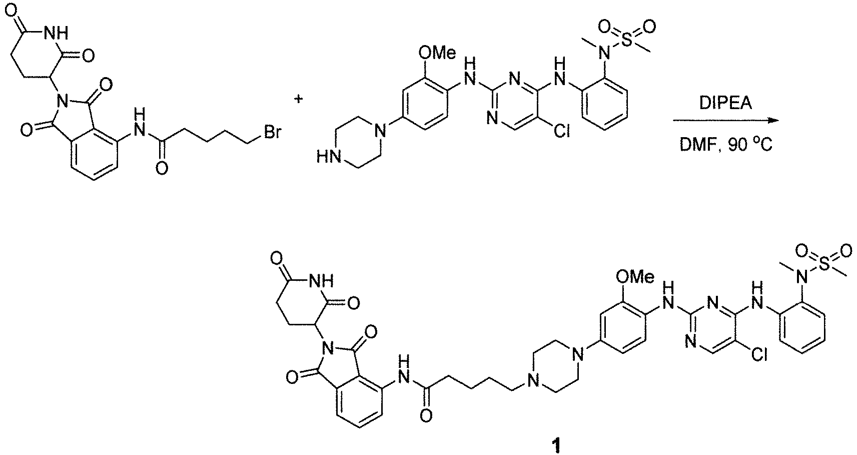

### Step 1: Synthesis of 5-bromo-N-(2-(2,6-dioxopiperidin-3-yl)-1,3-dioxoisoindolin-4-yl)pentanamide (1-1)

To a solution of 5-bromopentanoic acid (398 mg, 2.20 mmol) in dichloromethane (5 mL) was added oxalyl chloride (964 mL, 11.00 mmol), followed by 1 drop of N,N-dimethylformamide. The solution was concentrated for 1 hour. Tetrahydrofuran (10 mL) and 4-amino-2-(2,6-dioxopiperidin-3-yl)isoindolin-1,3-dione (200 mg, 0.73 mmol) were added, and stirred overnight at 75 °C. It was extracted by diluting with ethyl acetate and brine, and dried over anhydrous magnesium sulfate. After filtering and concentrating, the product was purified with a silica gel column to obtain the title compound (244.1 mg, 77%).

### Step 2: Synthesis of tertiary butyl 4-(3-methoxy-4-nitrophenyl)piperazin-1-carboxylate (1-2)

A mixture of 4-fluoro-2-methoxy-1-nitrobenzene (5.0 g, 29.22 mmol), Boc-piperazine (6.5 g, 35.06 mmol) and potassium carbonate (6.0 g, 43.24 mmol) in N,N-dimethylformamide ( 43 mL) was stirred at room temperature. After stirring overnight, potassium carbonate (3.0 g) was added and stirred for 2 days. Water was added to the reaction mixture, and the mixture was stirred, filtered, washed with water, and vacuum dried to obtain the title compound (9.5 g, 96%).

### Step 3: Synthesis of tertiary butyl 4-(4-amino-3-methoxyphenyl)piperazin-1-carboxylate (1-3)

A mixture of tertiary butyl 4-(3-methoxy-4-nitrophenyl)piperazin-1-carboxylate (500 mg, 1.48 mmol) in tetrahydrofuran/ethanol (5/5 mL) was purged with argon gas and 10% Pd/C (50 mg) was added. After substituted with hydrogen gas, it was stirred for 4 hours, filtered, and concentrated to obtain the title compound (450 mg, 99%).

### Step 4: Synthesis of tertiary butyl 4-(4-((5-chloro-4-((2-(N-methylmethylsulfonamido)phenyl)amino)pyrimidin-2-yl)amino)-3-methoxyphenyl)piperazin-1-carboxylate (1-4)

A mixture of tertiary butyl 4-(4-amino-3-methoxyphenyl)piperazin-1-carboxylate (450 mg, 1.46 mmol), N-(2-((2,5-dichloropyrimidin-4-yl)amino)phenyl)-N-methylmethanesulfonamide (508 mg, 1.46 mmol), BINAP (255 mg, 0.41 mmol) and potassium carbonate (464 mg, 3.37 mmol) in 1,4-dioxane (5 mL) was purged with argon gas and Pd(OAc)₂ (50 mg, 0.20 mmol) was added. The mixture was stirred under reflux overnight, cooled to room temperature, and filtered through Celite. It was extracted by diluting with ethyl acetate and brine, and dried over anhydrous magnesium sulfate. After filtering and concentrating, the title compound (700 mg, 77%) was obtained by purification using a silica gel column.

### Step 5: Synthesis of N-(2-((5-chloro-2-((2-methoxy-4-(piperazin-1-yl)phenyl)amino)pyrimidin-4-yl)amino)phenyl)-N-methylmethanesulfonamide (1-5)

Tertiary butyl 4-(4-((5-chloro-4-((2-(N-methylmethylsulfonamido)phenyl)amino)pyrimidin-2-yl)amino)-3-methoxyphenyl)piperazin-1-carboxylate (2.9 g, 4.68 mmol) was added to a reactor containing 20% trifluoroacetic acid/dichloromethane (46 mL), stirred for 3 hours, and neutralized with an aqueous solution of sodium bicarbonate. After extraction with dichloromethane, drying over anhydrous magnesium sulfate, filtration, and concentration, the title compound (2.4 g, 98%) was obtained.

### Step 6: Synthesis of Compound 1

To a solution of 5-bromo-N-(2-(2,6-dioxopiperidin-3-yl)-1,3-dioxoisoindolin-4-yl)pentanamide (67 mg, 0.15 mmol) and N-(2-((5-chloro -2-((2-methoxy-4-(piperazin-1-yl)phenyl)amino)pyrimidin-4-yl)amino)phenyl)-N-methylmethanesulfonamide (80 mg, 0.15 mmol) in N,N-dimethylformamide (1.5 mL), diisopropylethylamine (67 mL, 0.39 mmol) was added and stirred at 90 °C. After 16 hours, it was concentrated and purified by column to obtain the title compound (95 mg, 66%).

### Example 2: Synthesis of 5-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1,3-dioxoisoindolin-4-yl)pentanamide (Compound 2)

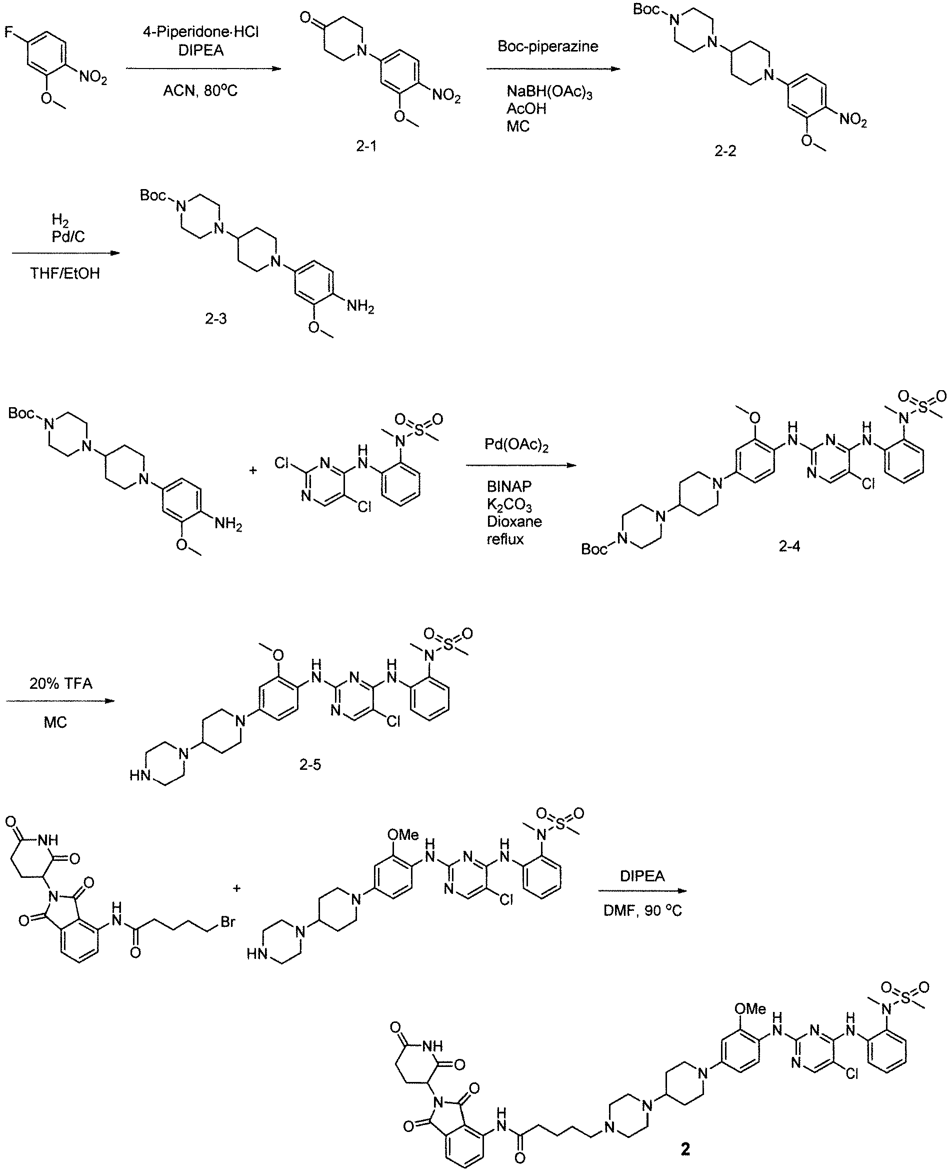

### Step 1: Synthesis of 1-(3-methoxy-4-nitrophenyl)piperidin-4-one (2-1)

A mixture of 4-fluoro-2-methoxy-1-nitrobenzene (500 mg, 2.92 mmol), piperidone hydrochloride (470 mg, 3.47 mmol) and potassium carbonate (799 mg, 5.78 mmol) in dimethylsulfoxide (6 mL) was stirred overnight at 80 °C. The reaction mixture was diluted with water and extracted with ethyl acetate. The organic layer was washed sequentially with water and saturated brine, dried over anhydrous magnesium sulfate, filtered, and concentrated. The residue was purified by column to obtain the title compound (370 mg, 50%).

### Step 2: Synthesis of tertiary butyl 4-(1-(3-methoxy-4-nitrophenyl)piperidin-4-yl)piperazin-1-carboxylate (2-2)

To a mixture of 1-(3-methoxy-4-nitrophenyl)piperidin-4-one (460 mg, 1.84 mmol) and N-Boc-piperazine (1.0 g, 5.51 mmol) in dichloromethane (18 mL), acetic acid (0.3 mL, 5.51 mmol) was added and stirred for 1 hour and 30 minutes. After adding NaBH(OAc)₃ (580 mg, 2.76 mmol) and stirring for 5 hours, the pH was adjusted to ~8 with an aqueous solution of sodium hydrogen carbonate, followed by extraction with dichloromethane. The organic layer was dried over anhydrous magnesium sulfate, filtered, and concentrated, and the residue was purified by column to obtain the title compound (650 mg, 84%).

### Step 3: Synthesis of tertiary butyl 4-(1-(4-amino-3-methoxyphenyl)piperidin-4-yl)piperazin-1-carboxylate (2-3)

A mixture of tertiary butyl 4-(1-(3-methoxy-4-nitrophenyl)piperidin-4-yl)piperazin-1-carboxylate (650 mg, 1.55 mmol) in tetrahydrofuran/ethanol (7/7 mL) was purged with argon gas, and 10% Pd/C (65 mg) was added. After replacing with hydrogen gas and stirring for 4 hours, the mixture was filtered through celite and concentrated to obtain the title compound (650 mg, 100%).

### Step 4: Synthesis of tertiary butyl 4-(1-(4-((5-chloro-4-((2-(N-methylmethylsulfonamido)phenyl)amino)pyrimidin-2-yl)amino)-3-methoxyphenyl)piperidin-4-yl)piperazin-1-carboxylate (2-4)

A mixture of tertiary butyl 4-(1-(4-amino-3-methoxyphenyl)piperidin-4-yl)piperazin-1-carboxylate (640 mg, 1.64 mmol), N-(2-((2,5-dichloropyrimidin-4-yl)amino)phenyl)-N-methylmethanesulfonamide (570 mg, 1.64 mmol), BINAP (285 mg, 0.46 mmol) and potassium carbonate (521 mg, 3.77 mmol) in 1,4-dioxane (6.5 mL) was purged with argon gas, and Pd(OAc)₂ (52 mg, 0.23 mmol) was added. After stirring under reflux overnight and cooling to room temperature, extraction was performed by diluting with ethyl acetate and brine. After drying over anhydrous magnesium sulfate, filtering and concentration, the title compound (610 mg, 53%) was obtained by purification with a silica gel column.

### Step 5: Synthesis of N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (2-5)

20% trifluoroacetic acid/dichloromethane (9 mL) was added to a reactor containing tertiary butyl 4-(1-(4-((5-chloro-4-((2-(N-methylmethylsulfonamido)phenyl)amino)pyrimidin-2-yl)amino)-3-methoxyphenyl)piperidin-4-yl)piperazin-1-carboxylate (610 g, 0.87 mmol), stirred for 3 hours, and then neutralized with an aqueous solution of sodium bicarbonate. After extraction with dichloromethane, drying over anhydrous magnesium sulfate, filtration, and concentration, the title compound (430 mg, 82%) was obtained.

### Step 6: Synthesis of Compound 2

To a solution of 5-bromo-N-(2-(2,6-dioxopiperidin-3-yl)-1,3-dioxoisoindolin-4-yl)pentanamide (51 mg, 0.12 mmol) and N-(2-((5-chloro -2-((2-methoxy-4-(4-(piperazin-1-yl)piperidin-1-yl)phenyl)amino)pyrimidin-4-yl)amino)phenyl)-N-methylmethanesulfonamide (70 mg, 0.12 mmol) in N,N-dimethylformamide (1.5 mL), diisopropylethylamine (51 mL, 0.29 mmol) was added and stirred at 90 °C. After 16 hours, it was concentrated and purified by column to obtain the title compound (95 mg, 66%).

### Example 3: Synthesis of N-(2-((5-chloro-2-((4-(4-((4-((2-(2,6-dioxopiperidin-3-yl)-1,3-dioxoisoindolin-4-yl)oxy)butyl)(methyl)amino)piperidin-1-yl)-2-methoxyphenyl)amino)pyrimidin-4-yl)amino)phenyl)-N-methylmethanesulfonamide (Compound 3) (not claimed)

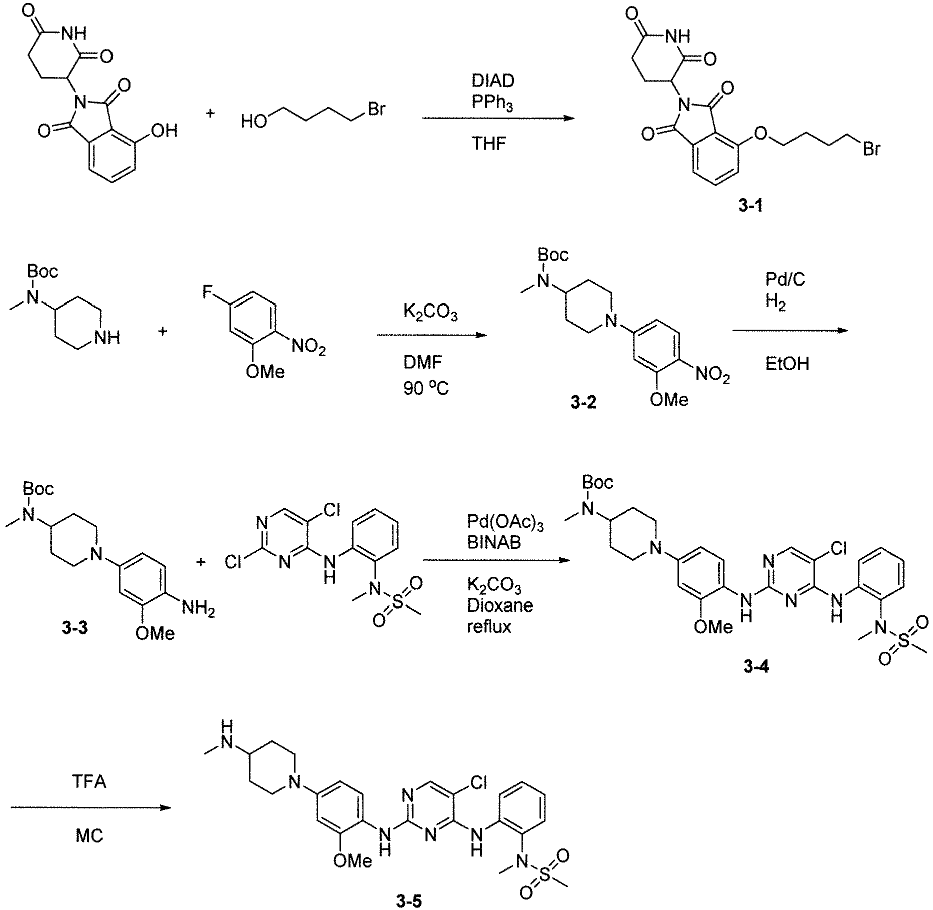

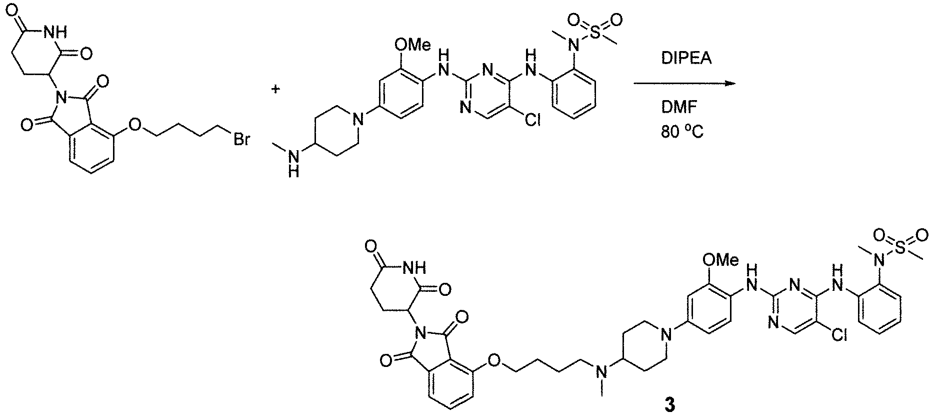

### Step 1: Synthesis of 4-(4-bromobutoxy)-2-(2,6-dioxopiperidin-3-yl)isoindolin-1,3-dione (3-1)

2-(2,6-dioxopiperidin-3-yl)-4-hydroxyisoindolin-1,3-dione (500 mg, 1.82 mmol) was dissolved in tetrahydrofuran (9 mL) and 4-bromobutan-1-ol (0.289 mL, 2.74 mmol) and triphenylphosphine (717 mg, 2.74 mmol) were added. Diisopropyl azodicarboxylate (0.538 m, 2.74 mmol) was added slowly and stirred overnight. The reaction mixture was concentrated, diluted with isopropanol and stirred for 2 hours. The precipitated solid was filtered, washed with isopropanol, and dried to obtain the title compound (510 mg, 69%).

### Step 2: Synthesis of tertiary butyl(1-(3-methoxy-4-nitrophenyl)piperidin-4-yl)(methyl)carbamate (3-2)

The title compound (3.90 g, 91%) was obtained using a method similar to the Step 2 synthesis method of Example 1 above with 4-fluoro-2-methoxy-1-nitrobenzene (2.0 g, 11.69 mmol) and tertiary butyl methyl(piperidin-4-yl)carbamate (2.76 g, 12.86 mmol) instead of 4-fluoro-2-methoxy-1-nitrobenzene and Boc-piperazine, respectively.

### Step 3 Synthesis of tertiary butyl(1-(4-amino-3-methoxyphenyl)piperidin-4-yl)(methyl)carbamate (3-3)

The title compound (1.65 g, yield 94%) was obtained using a method similar to the Step 3 synthesis method of Example 1 above with tertiary butyl(1-(3-methoxy-4-nitrophenyl)piperidin-4-yl)(methyl)carbamate (2.0 g, 7.99 mmol) instead of tertiary butyl 4-(3-methoxy-4-nitrophenyl)piperazin-1-carboxylate.

### Step 4: Synthesis of tertiary butyl(1-(4-((5-chloro-4-((2-(N-methylmethylsulfonamido)phenyl)amino)pyrimidin-2-yl)amino)-3-methoxyphenyl)piperidin-4-yl)(methyl)carbamate (3-4)

The title compound (1.52 g, yield 53%) was obtained using a method similar to the Step 4 synthesis method of Example 1 above with tertiary butyl(1-(4-amino-3-methoxyphenyl)piperidin-4-yl)(methyl)carbamate (1.5 g, 4.47 mmol) and N-(2-((2,5-dichloropyrimidin-4-yl)amino)phenyl)-N-methylmethanesulfonamide (1.63 g, 4.47 mmol) instead of tertiary butyl 4-(4-amino-3-methoxyphenyl)piperazin-1-carboxylate and N-(2-((2,5-dichloropyrimidin-4-yl)amino)phenyl)-N-methylmethanesulfonamide, respectively.

### Step 5: Synthesis of N-(2-((5-chloro-2-((2-methoxy-4-(4-(methylamino)piperidin-1-yl)phenyl)amino)pyrimidin-4-yl)amino)phenyl)-N-methylmethanesulfonamide (3-5)

The title compound (410 mg, yield 97%) was obtained using a method similar to the Step 5 synthesis method of Example 1 above with tertiary butyl(1-(4-((5-chloro-4-((2-(N-methylmethylsulfonamido)phenyl)amino)pyrimidin-2-yl)amino)-3-methoxyphenyl)piperidin-4-yl)(methyl)carbamate (0.5 g, 0.774 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 6: Synthesis of Compound 3

The title compound (21 mg, yield 16%) was obtained using a method similar to the Step 6 synthesis method of Example 1 above with 4-(4-bromobutoxy)-2-(2,6-dioxopiperidin-3-yl)isoindolin-1,3-dione (60 mg, 0.15 mmol) and N-(2-((5-chloro-2-((2-methoxy-4-(4-(methylamino)piperidin-1-yl)phenyl)amino)pyrimidin-4-yl)amino)phenyl)-N-methylmethanesulfonamide (80 mg, 0.15 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 4: Synthesis of 11-(4-(4-((5-chloro-4-((2-(N-methylmethylsulfonamido)phenyl)amino)pyrimidin-2-yl)amino)-3-methoxyphenyl)piperazin-1-yl)-N-(2-(2,6-dioxopiperidin-3-yl)-1,3-dioxoisoindolin-4-yl)undecanamide (Compound 4) (not claimed)

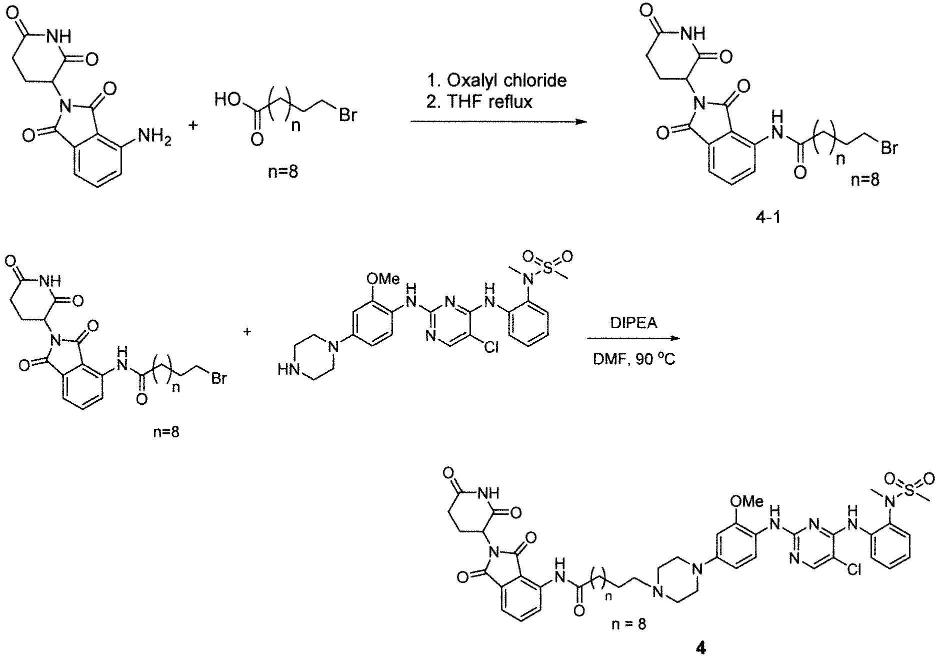

### Step 1: Synthesis of 11-bromo-N-(2-(2,6-dioxopiperidin-3-yl)-1,3-dioxoisoindolin-4-yl)undecanamide (4-1)

The title compound (236 mg, yield 62%) was obtained using a method similar to the Step 1 synthesis method of Example 1 above with 11-bromoundecanoic acid (583 mg, 2.20 mmol) instead of 5-bromopentanoic acid.

### Step 2: Synthesis of Compound 4

The title compound (94 mg, yield 64%) was obtained using a method similar to the Step 6 synthesis method of Example 1 above with 11-bromo-N-(2-(2,6-dioxopiperidin-3-yl)-1,3-dioxoisoindolin-4-yl)undecanamide (80 mg, 0.15 mmol) and N-(2-((5-chloro-2-((2-methoxy-4-(piperazin-1-yl)phenyl)amino)pyrimidin-4-yl)amino)phenyl)-N-methylmethanesulfonamide (80 mg, 0.15 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 5: Synthesis of 11-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1,3-dioxoisoindolin-4-yl)undecanamide (Compound 5) (not claimed)

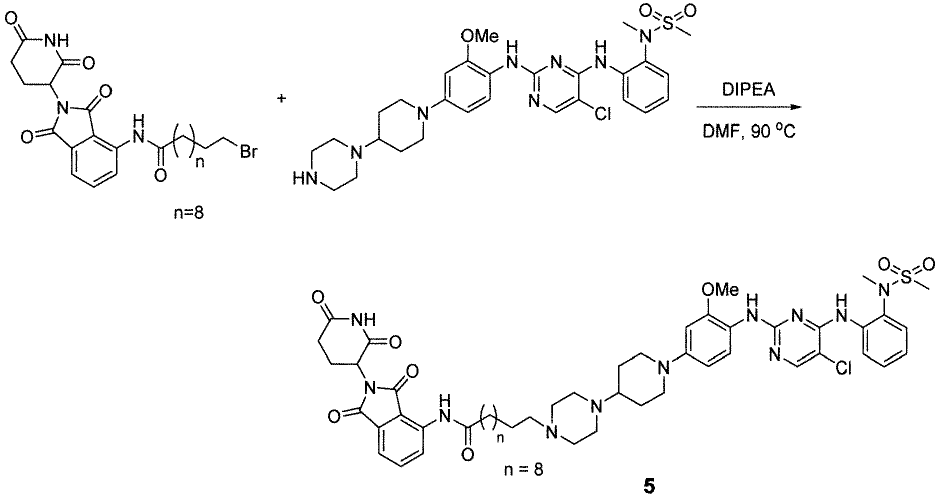

The title compound (65 mg, yield 54%) was obtained using a method similar to the Step 6 synthesis method of Example 1 above with 11-bromo-N-(2-(2,6-dioxopiperidin-3-yl)-1,3-dioxoisoindolin-4-yl)undecanamide (60 mg, 0.12 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (70 mg, 0.12 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 6: Synthesis of (2R,4R)-1-((S)-2-(5-(4-(4-((5-chloro-4-((2-(N-methylmethylsulfonamido)phenyl)amino)pyrimidin-2-yl)amino)-3-methoxyphenyl)piperazin-1-yl)pentanamido)3,3-dimethylbutanoyl)4-hydroxy-N-(4-(4-methylthiazol-5-yl)benzyl)pyrrolidin-2-carboxamide (Compound 6)

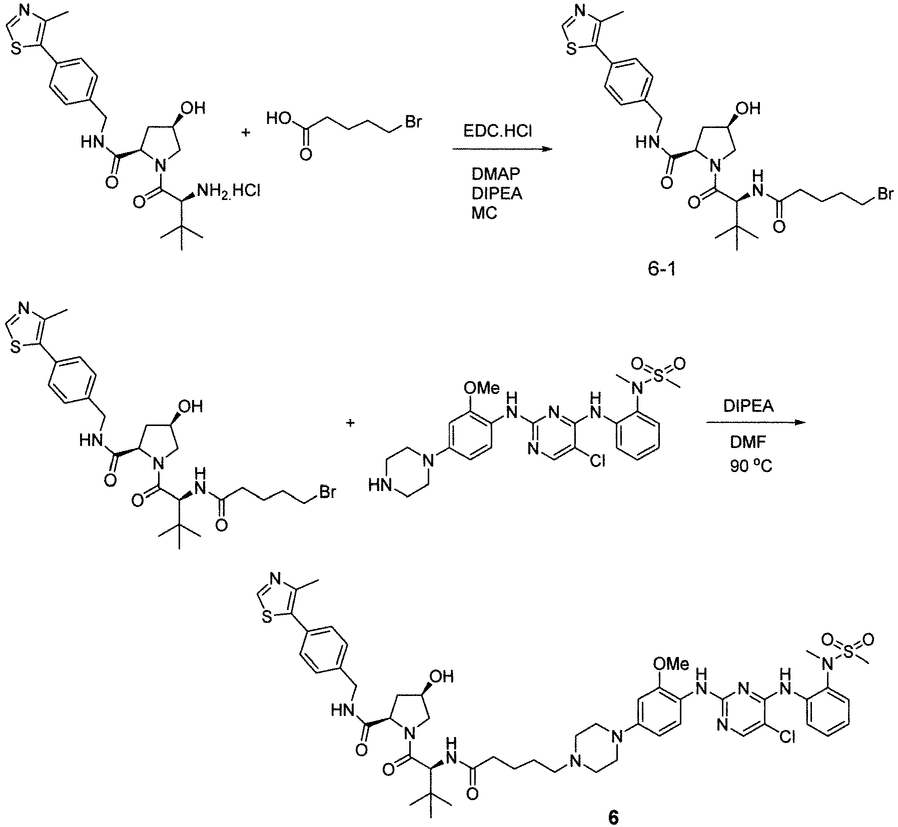

### Step 1: Synthesis of (2R, 4R)-1-((S)-2-(5-bromopentanamido)-3,3-dimethylbutanoyl)-4-hydroxy-N-(4-(4-methylthiazol-5-yl)benzyl)pyrrolidin-2-carboxamide (6-1)

To a mixture of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride (200 mg, 0.43 mmol), 5-bromopentanoic acid (155 mg, 0.86 mmol) and dimethylaminopyridine (11 mg, 0.086 mmol) in dichloromethane (4 mL), diisopropylethylamine (149 mL, 0.86 mmol) and stirred for 5 minutes, and then 1-(3-dimethylaminopropyl)-3-ethylcarbodiimide hydrochloride (107 mg, 0.56 mmol) was added. After stirring overnight, it was diluted with water and extracted with dichloromethane. The organic layer was dried over anhydrous magnesium sulfate, filtered, concentrated, and then purified with a silica gel column to obtain the title compound (203 mg, 80%).

### Step 2: Synthesis of Compound 6

The title compound (46 mg, yield 34%) was obtained using a method similar to the Step 6 synthesis method of Example 1 above with (2R, 4R)-1-((S)-2-(5-bromopentanamido)-3,3-dimethylbutanoyl)-4-hydroxy-N-(4-(4-methylthiazol-5-yl)benzyl)pyrrolidin-2-carboxamide (80 mg, 0.14 mmol) and N-(2-((5-chloro-2-((2-methoxy-4-(piperazin-1-yl)phenyl)amino)pyrimidin-4-yl)amino)phenyl)-N-methylmethanesulfonamide (70 mg, 0.14 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 7: Synthesis of (2R,4R)-1-((S)-2-(5-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)pentanamido)-3,3-dimethylbutanoyl)-4-hydroxy-N-(4-(4-methylthiazol-5-yl)benzyl)pyrrolidin-2-carboxamide (Compound 7)

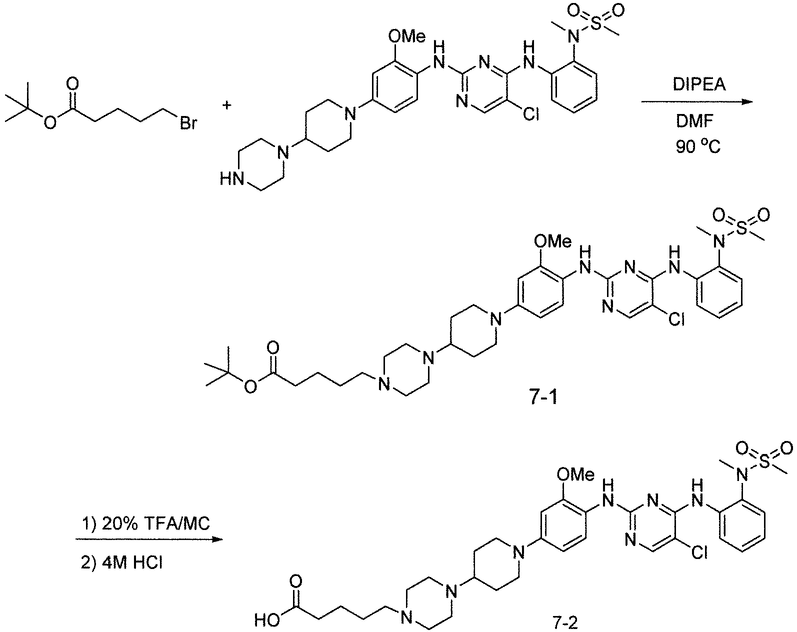

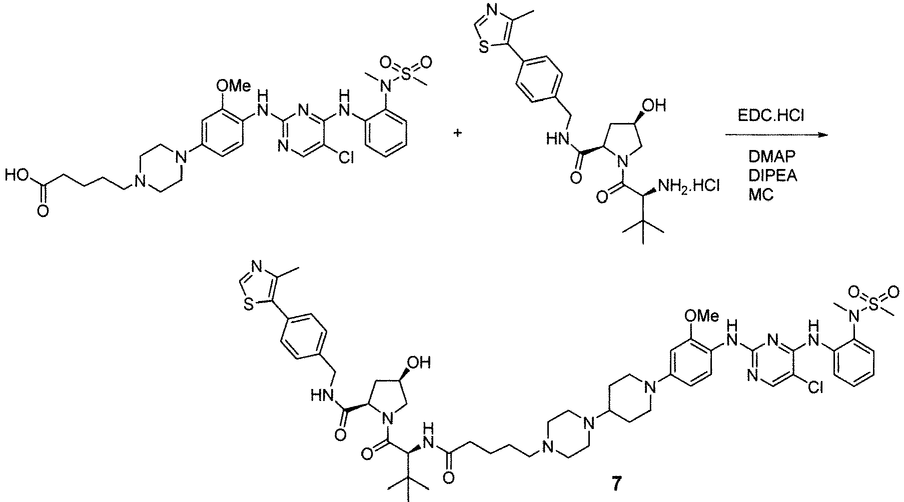

### Step 1: Synthesis of tertiary butyl-5-(4-(1-(4-((5-chloro-4-((2-(N-methylmethylsulfonamido)phenyl)amino)-3-methoxyphenyl)piperidin-4-yl)piperazin-1-yl)pentanoate (7-1)

The title compound (71 mg, yield 56%) was obtained using a method similar to the Step 6 synthesis method of Example 1 above with tertiary butyl 5-bromopentanoate (43 mg, 0.18 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (100 mg, 0.17 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Step 2: Synthesis of 5-(4-(4-((5-chloro-4-((2-(N-methylmethylsulfonamido)phenyl)amino)pyrimidin-2-yl)amino)-3-methoxyphenyl)piperazin-1-yl)pentanoic acid (7-2)

The compound obtained in Step 1 was dissolved in 20% trifluoroacetic acid/dichloromethane (1 mL) and stirred overnight. The reaction mixture was concentrated, diluted with 4N 1,4-dioxane (1.5 mL), stirred for 1 hour, and then concentrated and used in the next reaction.

### Step 3: Synthesis of Compound 7

The title compound (60 mg, yield 57%) was obtained using a method similar to the Step 1 synthesis method of Example 6 above with (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-(4-(4-((5-chloro-4-((2-(N-methylmethylsulfonamido)phenyl)amino)pyrimidin-2-yl)amino)-3-methoxyphenyl)piperazin-1-yl)pentanoic acid obtained in Step 2 instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentaoic acid, respectively.

### Example 8: Synthesis of 2-(2-(2-(4-(4-((5-chloro-4-((2-(N-methylmethylsulfonamido)phenyl)amino)pyrimidin-2-yl)amino)-3-methoxyphenyl)piperazin-1-yl)ethoxy)ethoxy)-N-(2-(2,6-dioxopiperidin-3-yl)-1,3-dioxoisoindolin-4-yl)acetamide (Compound 8) (not claimed)

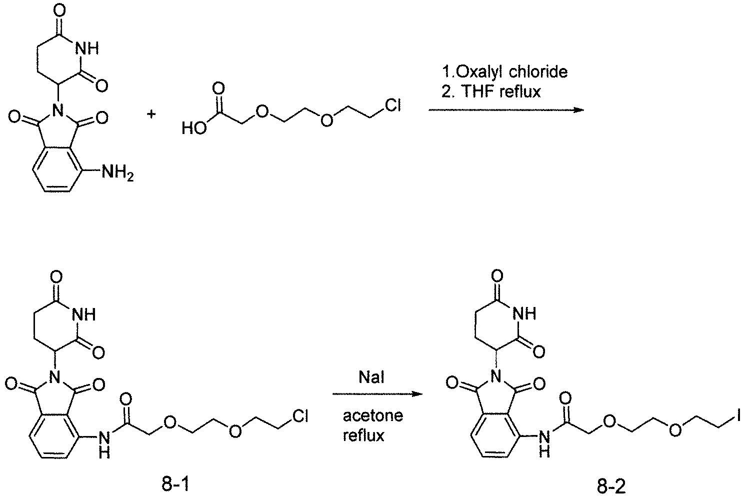

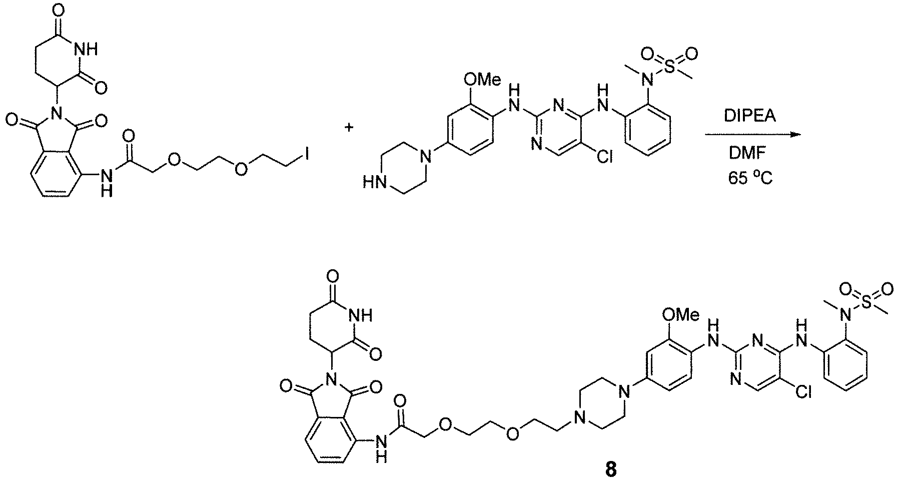

### Step 1: Synthesis of 2-(2-(2-chloroethoxy)ethoxy)-N-(2-(2,6-dioxopiperidin-3-yl)-1,3-dioxoisoindolin-4-yl)acetamide (8-1)

The title compound (404 mg, yield 94%) was obtained using a method similar to the Step 1 synthesis method of Example 1 above with 2-(2-(2-chloroethoxy)ethoxy)acetic acid (535 mg, 2.93 mmol) and 4-amino-2-(2,6-dioxopiperidin-3-yl)isoindolin-1,3-dione (268 mg, 0.98 mmol) instead of 5-bromopentanoic acid and 4-amino-2-(2,6-dioxopiperidin-3-yl)isoindolin-1,3-dione (200 mg, 0.73 mmol), respectively.

### Step 2: Synthesis of N-(2-(2,6-dioxopiperidin-3-yl)-1,3-dioxoisoindolin-4-yl)-2-(2-(2-iodoethoxy)ethoxy)acetamide (8-2)

A mixture of 2-(2-(2-chloroethoxy)ethoxy)-N-(2-(2,6-dioxopiperidin-3-yl)-1,3-dioxoisoindolin-4-yl)acetamide (400 mg, 0.91 mmol) and sodium iodide (1.3 g, 9.14 mmol) in acetone (6 mL) was stirred under reflux overnight. The reaction mixture was concentrated and extracted by diluting with water and dichloromethane. The organic layer was dried over anhydrous magnesium sulfate and filtered through silica gel. After concentration, the title compound (456 mg, 95%) was obtained by recrystallization from dichloromethane/methyl tertiary butyl ether/heptane.

### Step 3: Synthesis of Compound 8

The title compound (96 mg, yield 77%) was obtained using a method similar to the Step 6 synthesis method of Example 1 above with N-(2-(2,6-dioxopiperidin-3-yl)-1,3-dioxoisoindolin-4-yl)-2-(2-(2-iodoethoxy)ethoxy)acetamide (71 mg, 0.14 mmol) and N-(2-((5-chloro-2-((2-methoxy-4-(piperazin-1-yl)phenyl)amino)pyrimidin-4-yl)amino)phenyl)-N-methylmethanesulfonamide (70 mg, 0.14 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 9: Synthesis of 2-(2-(2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)ethoxy)ethoxy)-N-(2-(2,6-dioxopiperidin-3-yl)-1,3-dioxoisoindolin-4-yl)acetamide (Compound 9) (not claimed)

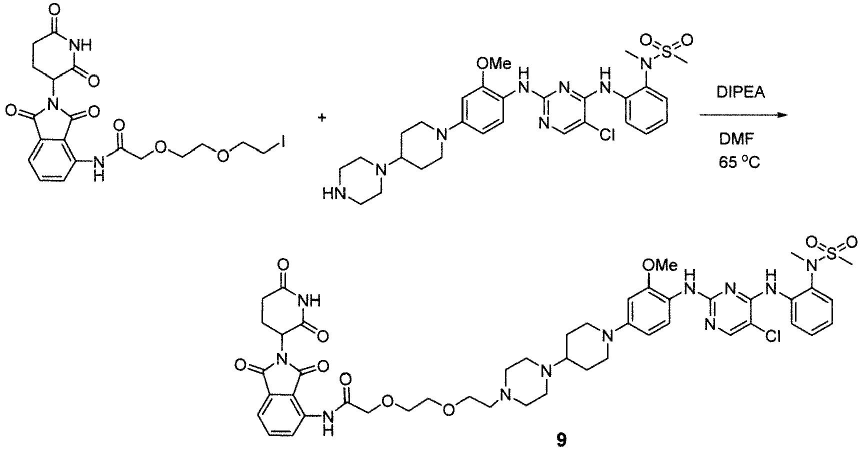

The title compound (59 mg, yield 51%) was obtained using a method similar to the Step 6 synthesis method of Example 1 above with N-(2-(2,6-dioxopiperidin-3-yl)-1,3-dioxoisoindolin-4-yl)-2-(2-(2-iodoethoxy)ethoxy)acetamide (61 mg, 0.12 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (70 mg, 0.12 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 10: Synthesis of (2R,4R)-1-((S)-2-(2-(2-(2-(4-(4-((5-chloro-4-((2-(N-methylmethylsulfonamido)phenyl)amino)pyrimidin-2-yl)amino)-3-methoxyphenyl)piperazin-1-yl)ethoxy)ethoxy)acetamido)-3,3-dimethylbutanoyl)-4-hydroxy-N-(4-(4-methylthiazol-5-yl)benzyl)pyrrolidin-2-carboxamide (Compound 10) (not claimed)

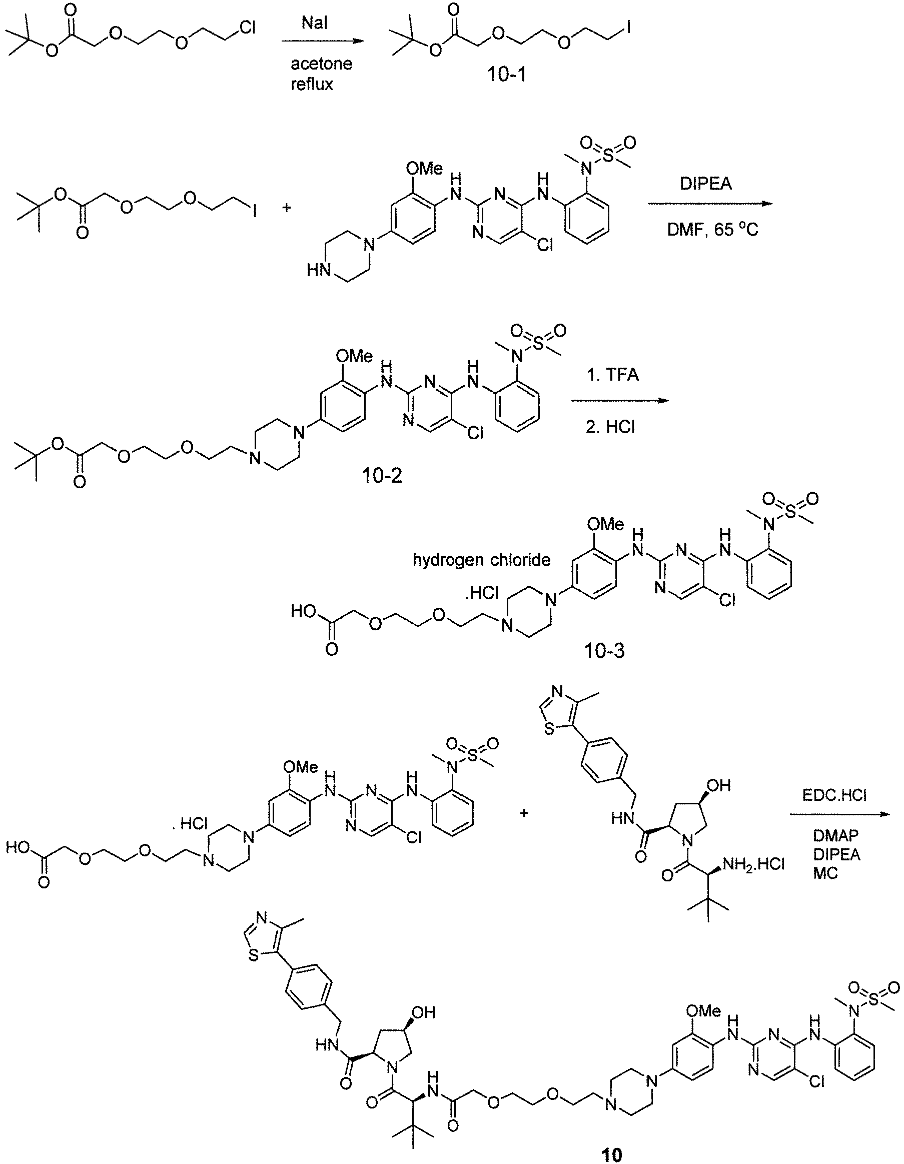

### Step 1: Synthesis of tertiary butyl 2-(2-(2-iodoethoxy)ethoxy)acetate (10-1)

A mixture of tertiary butyl 2-(2-(2-chloroethoxy)ethoxy)acetate (300 mg, 1.26 mmol) and sodium iodide (754 mg, 5.02 mmol) in acetone (8 mL) was stirred under reflux overnight. The reaction mixture was concentrated and extracted by diluting with water and dichloromethane. The organic layer was dried over anhydrous magnesium sulfate, filtered, concentrated, and purified by a column to obtain the title compound (387 mg, 93%).

### Step 2: Synthesis of tertiary butyl 2-(2-(2-(4-(4-((5-chloro-4-((2-(N-methylmethylsulfonamido)phenyl)amino)pyrimidin-2-yl)amino)-3-methoxyphenyl)piperazin-1-yl)ethoxy)ethoxy)acetate (10-2)

The title compound (198 mg, yield 91%) was obtained using a method similar to the Step 6 synthesis method of Example 1 above with tertiary butyl 2-(2-(2-iodoethoxy)ethoxy)acetate (100 mg, 0.30 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (160 mg, 0.30 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Step 3: Synthesis of 2-(2-(2-(4-(4-((5-chloro-4-((2-(N-methylmethylsulfonamido)phenyl)amino)pyrimidin-2-yl)amino)-3-methoxyphenyl)piperazin-1-yl)ethoxy)ethoxy)acetic acid hydrochloride (10-3)

The title compound was obtained using a method similar to the Step 2 synthesis method of Example 7 above with tertiary butyl 2-(2-(2-(4-(4-((5-chloro-4-((2-(N-methylmethylsulfonamido)phenyl)amino)pyrimidin-2-yl)amino)-3-methoxyphenyl)piperazin-1-yl)ethoxy)ethoxy)acetate (195 mg, 0.27 mmol) instead of tertiary butyl-5-(4-(1-(4-((5-chloro-4-((2-(N-methylmethylsulfonamido)phenyl)amino)-3-methoxyphenyl)piperidin-4-yl)piperazin-1-yl)pentanoate, and then was used in the next reaction.

### Step 4: Synthesis of Compound 10

The title compound (99 mg, yield 41%) was obtained using a method similar to the Step 1 synthesis method of Example 6 above with (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride (105 mg, 0.23 mmol) and 2-(2-(2-(4-(4-((5-chloro-4-((2-(N-methylmethylsulfonamido)phenyl)amino)pyrimidin-2-yl)amino)-3-methoxyphenyl)piperazin-1-yl)ethoxy)ethoxy)acetic acid hydrochloride instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentanoic acid, respectively.

### Example 11: Synthesis of (2R,4R)-1-((S)-2-(2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)acetamido)-3,3-dimethylbutanoyl)-4-hydroxy-N-(4-(4-methylthiazol-5-yl)benzyl)pyrrolidin-2-carboxamide (Compound 11)

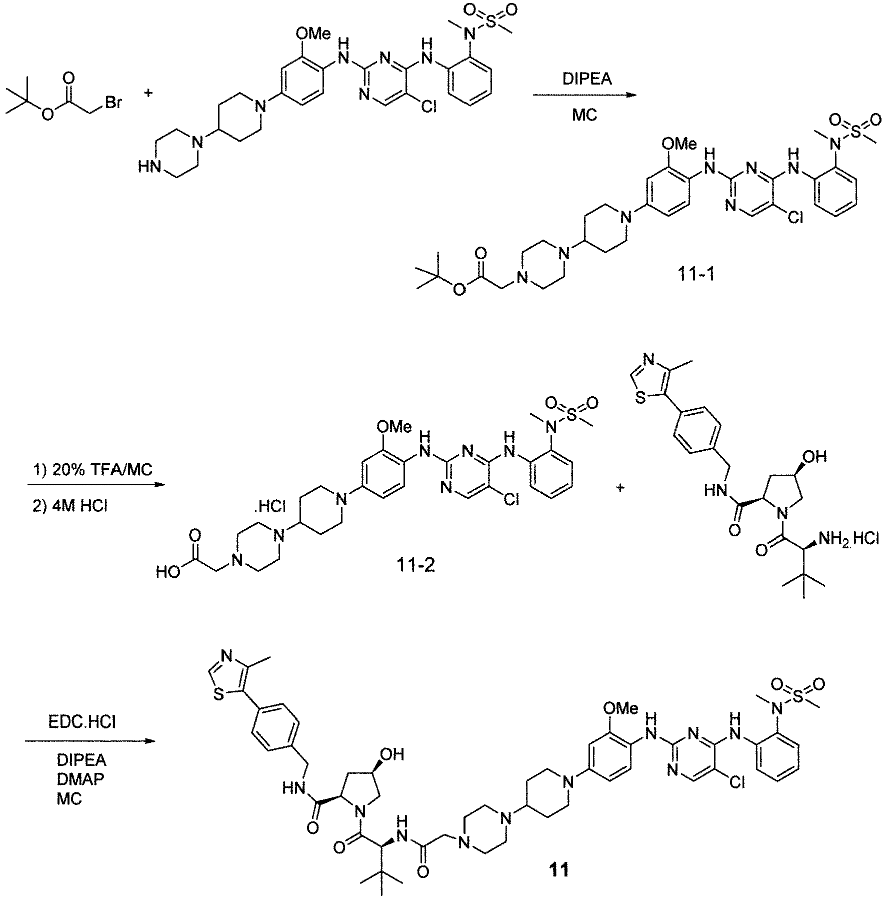

### Step 1: Synthesis of tertiary butyl 2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)acetate (11-1)

To a solution of tertiary butyl 2-bromoacetate (34 mg, 0.17 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (100 mg, 0.17 mmol) in dichloromethane (1.5 mL), diisopropylethylamine (72 µL, 0.43 mmol) was added and stirred for 4 hours. Extraction was performed by dilution with water and dichloromethane. The organic layer was dried over anhydrous magnesium sulfate, filtered, and concentrated, and used in the next reaction.

### Step 2: Synthesis of 2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)acetic acid hydrochloride (11-2)

The title compound was obtained using a method similar to the Step 2 synthesis method of Example 7 above with tertiary butyl 2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)acetate (122 mg, 0.17 mmol) instead of tertiary butyl-5-(4-(1-(4-((5-chloro-4-((2-(N-methylmethylsulfonamido)phenyl)amino)-3-methoxyphenyl)piperidin-4-yl)piperazin-1-yl)pentanoate, and then used in the next reaction.

### Step 3: Synthesis of Compound 11

The title compound (40 mg, yield 28%) was obtained using a method similar to the Step 1 synthesis method of Example 6 above with (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride (62 mg, 0.13 mmol) and 2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)acetic acid hydrochloride instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentanoic acid, respectively.

### Example 12: Synthesis of N-(2-((5-chloro-2-((4-(4-(2-(2-((2-(2,6-dioxopiperidin-3-yl)-1,3-dioxoisoindolin-4-yl)oxy)ethoxy)ethyl)piperazin-1-yl)-2-methoxyphenyl)amino)pyrimidin-4-yl)amino)phenyl)N-methylmethanesulfonamide (Compound 12) (not claimed)

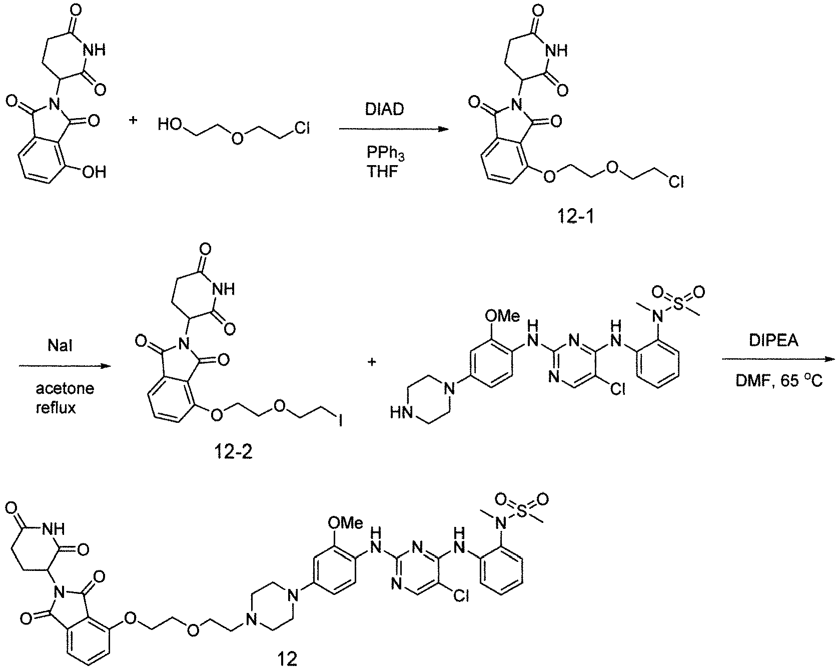

### Step 1: Synthesis of 4-(2-(2-chloroethoxy)ethoxy)-2-(2,6-dioxopiperidin-3-yl)isoindolin-1,3-dione (12-1)

The title compound (257 mg, yield 80%) was obtained using a method similar to the Step 1 synthesis method of Example 3 above with 2-(2,6-dioxopiperidin-3-yl)-4-hydroxyisoindolin-1,3-dione (200 mg, 0.73 mmol) and 2-(2-chloroethoxy)ethan-1-ol (136 mg, 1.10 mmol) instead of 2-(2,6-dioxopiperidin-3-yl)-4-hydroxyisoindolin-1,3-dione and 4-bromobutan-1-ol, respectively.

### Step 2: Synthesis of 2-(2,6-dioxopiperidin-3-yl)-4-(2-(2-iodoethoxy)ethoxy)isoindolin-1,3-dione (12-2)

The title compound (215 mg, yield 71%) was obtained using a method similar to the Step 2 synthesis method of Example 8 above with 4-(2-(2-chloroethoxy)ethoxy)-2-(2,6-dioxopiperidin-3-yl)isoindolin-1,3-dione (250 mg, 0.57 mmol) instead of 2-(2-(2-chloroethoxy)ethoxy)-N-(2-(2,6-dioxopiperidin-3-yl)-1,3-dioxoisoindolin-4-yl)acetamide.

### Step 3: Synthesis of Compound 12

The title compound (65 mg, yield 50%) was obtained using a method similar to the Step 6 synthesis method of Example 1 above with 2-(2,6-dioxopiperidin-3-yl)-4-(2-(2-iodoethoxy)ethoxy)isoindolin-1,3-dione (80 mg, 0.15 mmol) and N-(2-((5-chloro-2-((2-methoxy-4-(piperazin-1-yl)phenyl)amino)pyrimidin-4-yl)amino)phenyl)-N-methylmethanesulfonamide (80 mg, 0.15 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 13: Synthesis of N-(2-((5-chloro-2-((4-(4-(4-(2-(2-((2-(2,6-dioxopiperidin-3-yl)-1,3-dioxoisoindolin-4-yl)oxy)ethoxy)ethyl)piperazin-1-yl)piperidin-1-yl)-2-methoxyphenyl)amino)pyrimidin-4-yl)amino)phenyl)N-methylmethanesulfonamide Compound 13)

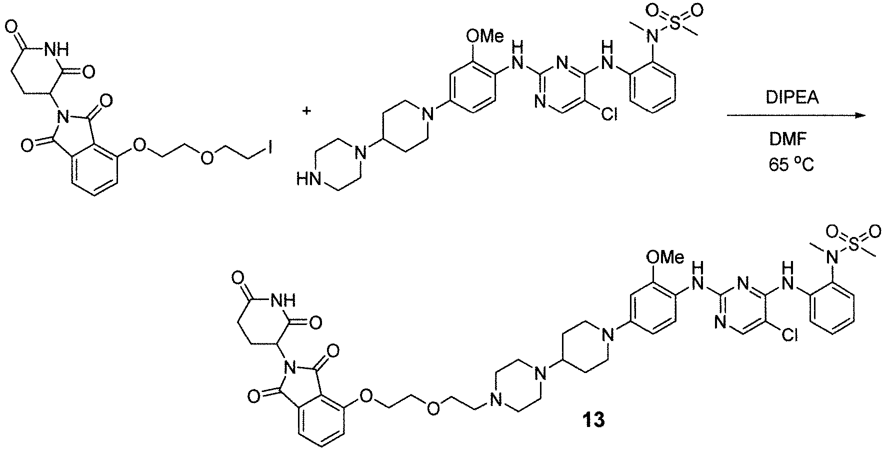

The title compound (79 mg, yield 72%) was obtained using a method similar to the Step 6 synthesis method of Example 1 above with 2-(2,6-dioxopiperidin-3-yl)-4-(2-(2-iodoethoxy)ethoxy)isoindolin-1,3-dione (61 mg, 0.12 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (70 mg, 0.12 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 14: Synthesis of N-(2-((5-chloro-2-((4-(4-(4-((2-(2,6-dioxopiperidin-3-yl)-1,3-dioxoisoindolin-4-yl)oxy)butyl)piperazin-1-yl)-2-methoxyphenyl)amino)pyrimidin-4-yl)amino)phenyl)-N-methylmethanesulfonamide (Compound 14)

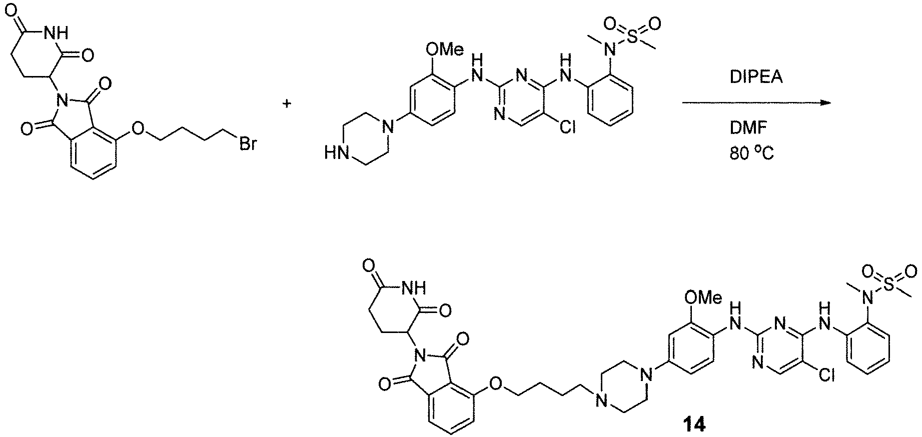

The title compound (25 mg, yield 20%) was obtained using a method similar to the Step 6 synthesis method of Example 1 above with 4-(4-bromobutoxy)-2-(2,6-dioxopiperidin-3-yl)isoindolin-1,3-dione (60 mg, 0.15 mmol) and N-(2-((5-chloro-2-((2-methoxy-4-(piperazin-1-yl)phenyl)amino)pyrimidin-4-yl)amino)phenyl)-N-methylmethanesulfonamide (76 mg, 0.15 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 15: Synthesis of N-(2-((5-chloro-2-((4-(4-(4-(4-((2-(2,6-dioxopiperidin-3-yl)-1,3-dioxoisoindolin-4-yl)oxy)butyl)piperazin-1-yl)piperidin-1-yl)-2-methoxyphenyl)amino)pyrimidin-4-yl)amino)phenyl)-N-methylmethanesulfonamide (Compound 15)

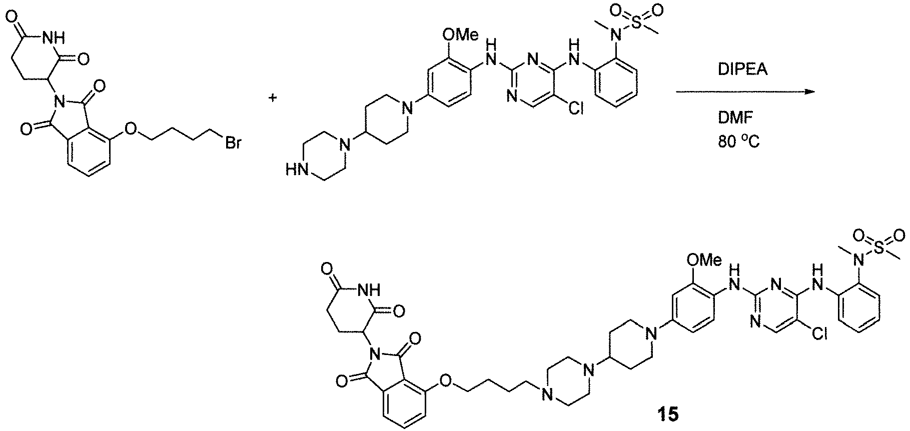

The title compound (23 mg, yield 17%) was obtained using a method similar to the Step 6 synthesis method of Example 1 above with 4-(4-bromobutoxy)-2-(2,6-dioxopiperidin-3-yl)isoindolin-1,3-dione (60 mg, 0.15 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (88 mg, 0.15 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 16: Synthesis of N-(2-((5-chloro-2-((4-(4-(2-(2-((2-(2,6-dioxopiperidin-3-yl)-1,3-dioxoisoindolin-4-yl)amino)ethoxy)ethyl)piperazin-1-yl)-2-methoxyphenyl)amino)piperidin-4-yl)amino)phenyl)-N-methylmethanesulfonamide (Compound 16)

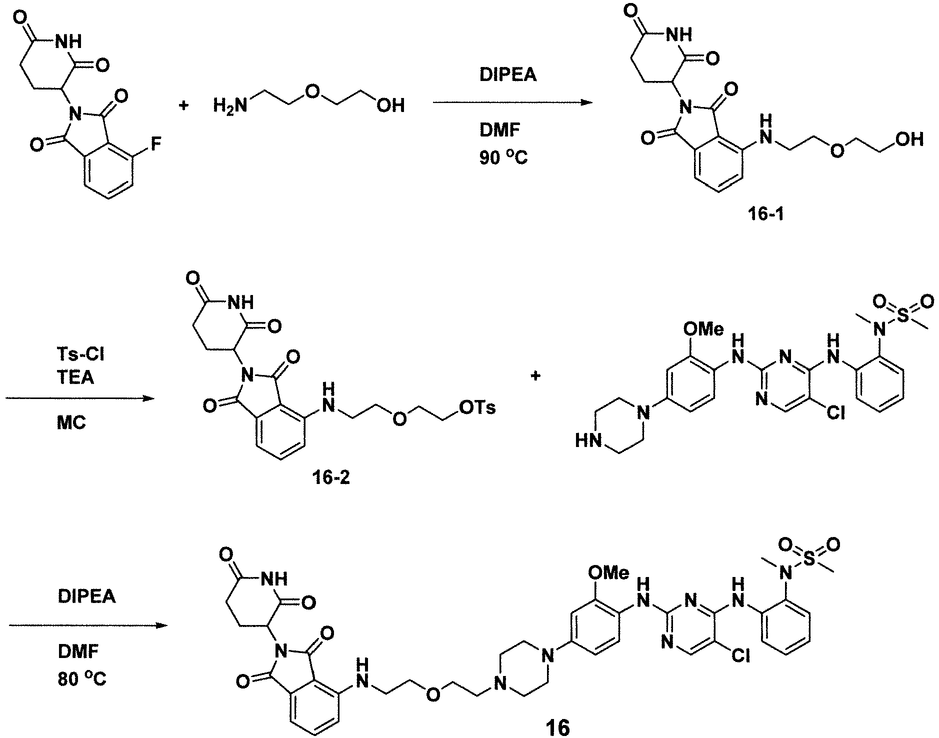

### Step 1: Synthesis of 2-(2,6-dioxopiperidin-3-yl)-4-((2-(2-hydroxyethoxy)ethyl)amino)isoindolin-1,3-dione (16-1)

To a solution of 2-(2,6-dioxopiperidin-3-yl)-4-fluoroisoindolin-1,3-dione (500 mg, 1.81 mmol) in dimethylformamide (1.5 mL), 2-(2-aminoethoxy)ethane-1-ol (209 mg, 1.99 mmol) and N,N-diisopropylethylamine (64 µl, 0.368 mmol) were added and stirred at 90 °C overnight. Extraction was performed by diluting with ethyl acetate and water, and the organic layer was washed with saturated brine. After drying with anhydrous magnesium sulfate, filtration, and concentration, the title compound (251 mg, 39%) was obtained by column purification.

### Step 2: Synthesis of 2-(2-((2-(2,6-dioxopiperidin-3-yl)-1,3-dioxoisoindolin-4-yl)amino)ethoxy)ethyl 4-methylbenzenesulfonate (16-2)

2-(2,6-dioxopiperidin-3-yl)-4-((2-(2-hydroxyethoxy)ethyl)amino)isoindolin-1,3-dione (150 mg, 0.42 mmol) and 4-toluenesulfonyl chloride (95 mg, 0.50 mmol) were dissolved in dichloromethane (4 mL) and triethylamine (0.17 mL, 1.25 mmol) was slowly added at 0 °C. After stirring at room temperature for 24 hours, the mixture was washed with water, and the organic layer was dried over anhydrous magnesium sulfate, filtered, and concentrated. The residue was purified by column to obtain the title compound (155 mg, yield 73%).

### Step 3: Synthesis of Compound 16

The title compound (29 mg, yield 25%) was obtained using a method similar to the Step 6 synthesis method of Example 1 above with 2-(2-((2-(2,6-dioxopiperidin-3-yl)-1,3-dioxoisoindolin-4-yl)amino)ethoxy)ethyl 4-methylbenzenesulfonate (70 mg, 0.14 mmol) and N-(2-((5-chloro-2-((2-methoxy-4-(piperazin-1-yl)phenyl)amino)pyrimidin-4-yl)amino)phenyl)-N-methylmethanesulfonamide (70 mg, 0.14 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 17: Synthesis of (2R,4R)-1-((S)-2-(5-(4-(4-((5-chloro-4-((2-(N-methylmethylsulfonamido)phenyl)amino)pyrimidin-2-yl)amino)-2-fluorophenyl)piperazin-1-yl)pentanamido)3,3-dimethylbutanoyl)4-hydroxy-N-(4-(4-methylthiazol-5-yl)benzyl)pyrrolidin-2-carboxamide (Compound 17)

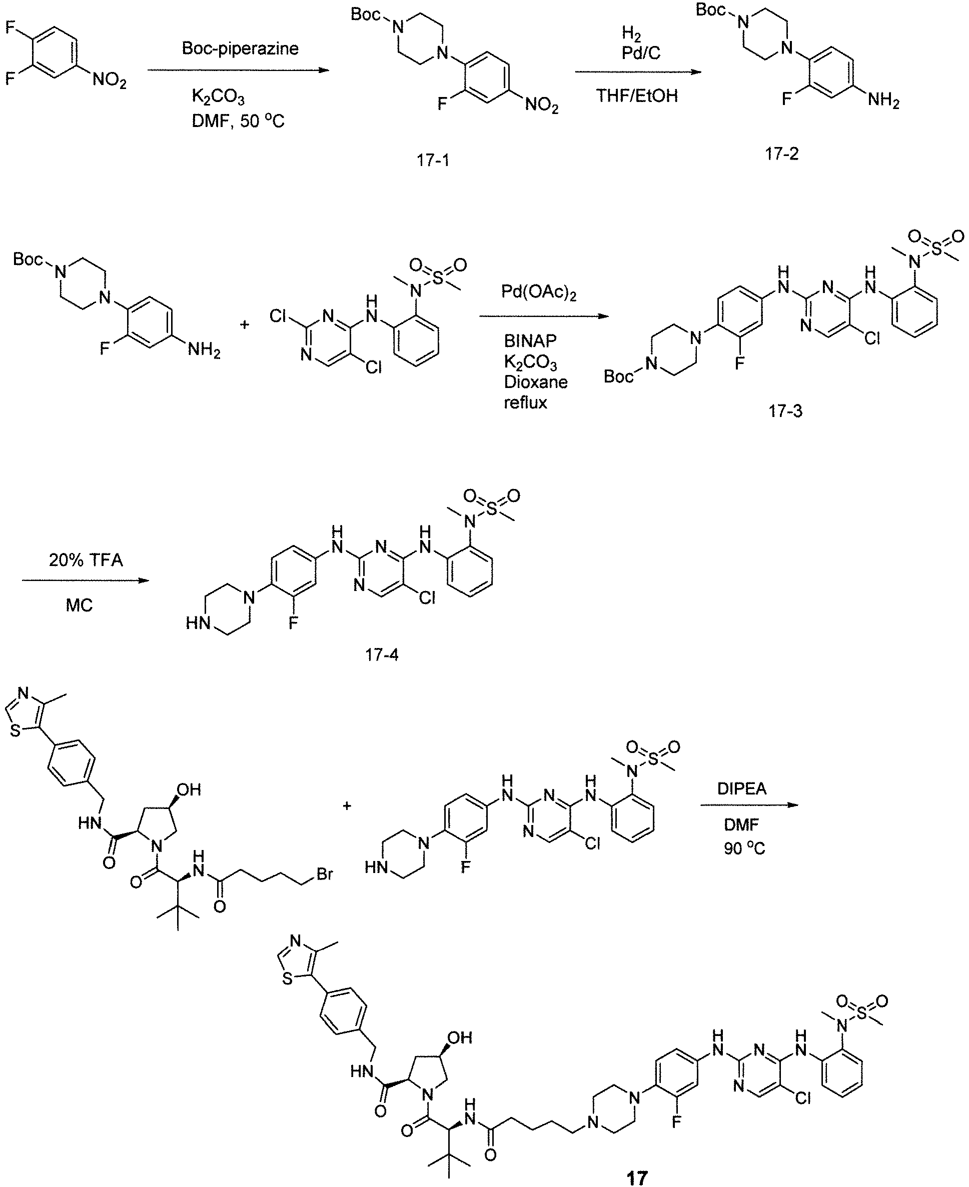

### Step 1: Synthesis of tertiary butyl 4-(2-fluoro-4-nitrophenyl)piperazin-1-carboxylate (17-1)

The title compound (2.0 g, yield 98%) was obtained using a method similar to the Step 2 synthesis method of Example 1 above with 1,2-difluoro-4-nitrobenzene (1.0 g, 6.29 mmol) instead of 4-fluoro-2-methoxy-1-nitrobenzene.

### Step 2: Synthesis of tertiary butyl 4-(4-amino-2-fluorophenyl)piperazin-1-carboxylate (17-2)

The title compound (430 mg, yield 95%) was obtained using a method similar to the Step 3 synthesis method of Example 1 above with tertiary butyl 4-(2-fluoro-4-nitrophenyl)piperazin-1-carboxylate (500 mg, 1.54 mmol) instead of tertiary butyl 4-(3-methoxy-4-nitrophenyl)piperazin-1-carboxylate.

### Step 3: Synthesis of tertiary butyl 4-(4-((5-chloro-4-((2-(N-methylmethylsulfonamido)phenyl)amino)pyrimidin-2-yl)amino)-2-fluorophenyl)piperazin-1-carboxylate (17-3)

The title compound (540 mg, yield 56%) was obtained using a method similar to the Step 4 synthesis method of Example 1 above with tertiary butyl 4-(4-amino-2-fluorophenyl)piperazin-1-carboxylate (450 mg, 1.52 mmol) instead of tertiary butyl 4-(4-amino-3-methoxyphenyl)piperazin-1-carboxylate.

### Step 4: Synthesis of N-(2-((5-chloro-2-((3-fluoro-4-(piperazin-1-yl)phenyl)amino)pyrimidin-4-yl)amino)phenyl)-N-methylmethanesulfonamide (17-4)

The title compound (70 mg, yield 84%) was obtained using a method similar to the Step 5 synthesis method of Example 1 above with tertiary butyl 4-(4-((5-chloro-4-((2-(N-methylmethylsulfonamido)phenyl)amino)pyrimidin-2-yl)amino)-2-fluorophenyl)piperazin-1-carboxylate (100 mg, 0.17 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 5: Synthesis of Compound 17

The title compound (41 mg, yield 34%) was obtained using a method similar to the Step 2 synthesis method of Example 1 above with N-(2-((5-chloro-2-((3-fluoro-4-(piperazin-1-yl)phenyl)amino)pyrimidin-4-yl)amino)phenyl)-N-methylmethanesulfonamide (59 mg, 0.12 mmol) instead of N-(2-((5-chloro-2-((2-methoxy-4-(piperazin-1-yl)phenyl)amino)pyrimidin-4-yl)amino)phenyl)-N-methylmethanesulfonamide.

### Example 18: Synthesis of (2R,4R)-1-((S)-2-(5-(4-(1-(4-((5-chloro-4-((2-(N-methylmethylsulfonamido)phenyl)amino)pyrimidin-2-yl)amino)-2-fluorophenyl)piperidin-4-yl)piperazin-1-yl)pentanamido)-3,3-dimethylbutanoyl)-4-hydroxy-N-(4-(4-methylthiazol-5-yl)benzyl)pyrrolidin-2-carboxamide (Compound 18) (not claimed)

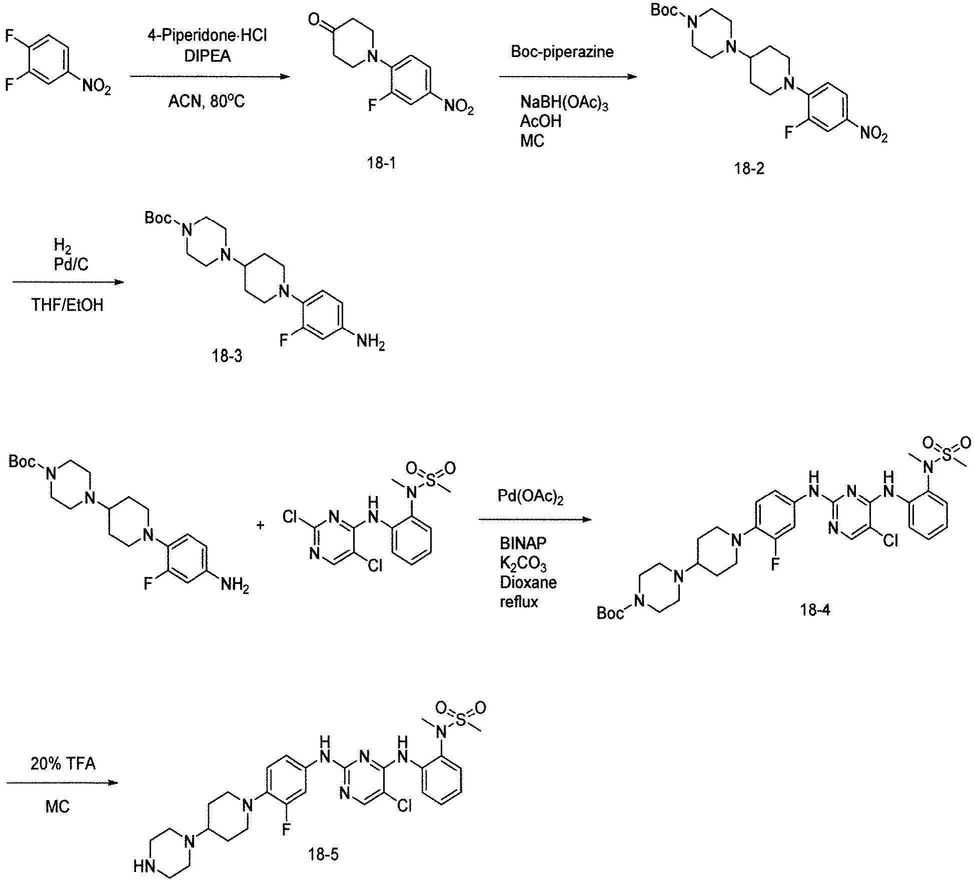

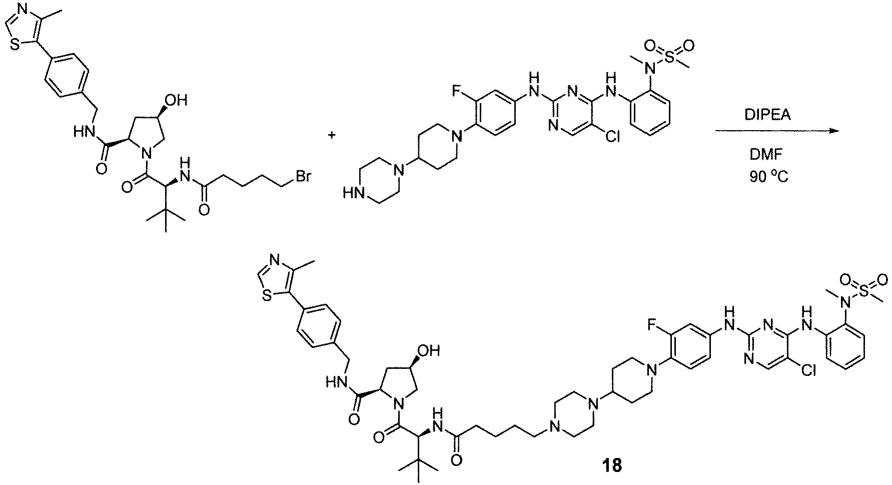

### Step 1: Synthesis of 1-(2-fluoro-4-nitrophenyl)piperidin-4-one (18-1)

The title compound (600 mg, yield 80%) was obtained using a method similar to the Step 1 synthesis method of Example 2 above with 1,2-difluoro-4-nitrobenzene (500 mg, 3.14 mmol) instead of 4-fluoro-2-methoxy-1-nitrobenzene.

### Step 2: Synthesis of tertiary butyl 4-(1-(2-fluoro-4-nitrophenyl)piperidin-4-yl)piperazin-1-carboxylate (18-2)

The title compound (320 mg, yield 62%) was obtained using a method similar to the Step 2 synthesis method of Example 2 above with 1-(2-fluoro-4-nitrophenyl)piperidin-4-one (300 mg, 1.26 mmol) instead of 1-(3-methoxy-4-nitrophenyl)piperidin-4-one.

### Step 3: Synthesis of tertiary butyl 4-(1-(4-amino-2-fluorophenyl)piperidin-4-yl)piperazin-1-carboxylate (18-3)

The title compound (300 mg, yield 100%) was obtained using a method similar to the Step 3 synthesis method of Example 2 above with tertiary butyl 4-(1-(2-fluoro-4-nitrophenyl)piperidin-4-yl)piperazin-1-carboxylate (320 mg, 0.78 mmol) instead of tertiary butyl 4-(1-(3-methoxy-4-nitrophenyl)piperidin-4-yl)piperazin-1-carboxylate.

### Step 4: Synthesis of tertiary butyl 4-(1-(4-((5-chloro-4-((2-(N-methylmethylsulfonamido)phenyl)amino)pyrimidin-2-yl)amino)-2-fluorophenyl)piperidin-4-yl)piperazin-1-carboxylate (18-4)

The title compound (280 mg, yield 53%) was obtained using a method similar to the Step 3 synthesis method of Example 2 above with tertiary butyl 4-(1-(4-amino-2-fluorophenyl)piperidin-4-yl)piperazin-1-carboxylate (290 mg, 0.77 mmol) instead of tertiary butyl 4-(1-(4-amino-3-methoxyphenyl)piperidin-4-yl)piperazin-1-carboxylate.

### Step 5: Synthesis of N-(2-((5-chloro-2-((3-fluoro-4-(4-(piperazin-1-yl)piperidin-1-yl)phenyl)amino)pyrimidin-4-yl)amino)phenyl)-N-methylmethanesulfonamide (18-5)

The title compound (2.5 g, yield 96%) was obtained using a method similar to the Step 3 synthesis method of Example 2 above with tertiary butyl 4-(1-(4-((5-chloro-4-((2-(N-methylmethylsulfonamido)phenyl)amino)pyrimidin-2-yl)amino)-2-fluorophenyl)piperidin-4-yl)piperazin-1-carboxylate (3.1 g, 4.43 mmol) instead of tertiary butyl 4-(1-(4-((5-chloro-4-((2-(N-methylmethylsulfonamido)phenyl)amino)pyrimidin-2-yl)amino)-3-methoxyphenyl)piperidin-4-yl)piperazin-1-carboxylate.

### Step 6: Synthesis of Compound 18

The title compound (42 mg, yield 32%) was obtained using a method similar to the Step 2 synthesis method of Example 6 above with N-(2-((5-chloro-2-((3-fluoro-4-(4-(piperazin-1-yl)piperidin-1-yl)phenyl)amino)pyrimidin-4-yl)amino)phenyl)-N-methylmethanesulfonamide (70 mg, 0.12mmol) instead of N-(2-((5-chloro-2-((2-methoxy-4-(piperazin-1-yl)phenyl)amino)pyrimidin-4-yl)amino)phenyl)-N-methylmethanesulfonamide.

### Example 19: Synthesis of (2R,4R)-1-((S)-2-(2-(4-(1-(4-((5-chloro-4-((2-(N-methylmethylsulfonamido)phenyl)amino)pyrimidin-2-yl)amino)-2-fluorophenyl)piperidin-4-yl)piperazin-1-yl)acetamido)-3,3-dimethylbutanoyl)-4-hydroxy-N-(4-(4-methylthiazol-5-yl)benzyl)pyrrolidin-2-carboxamide (Compound 19)

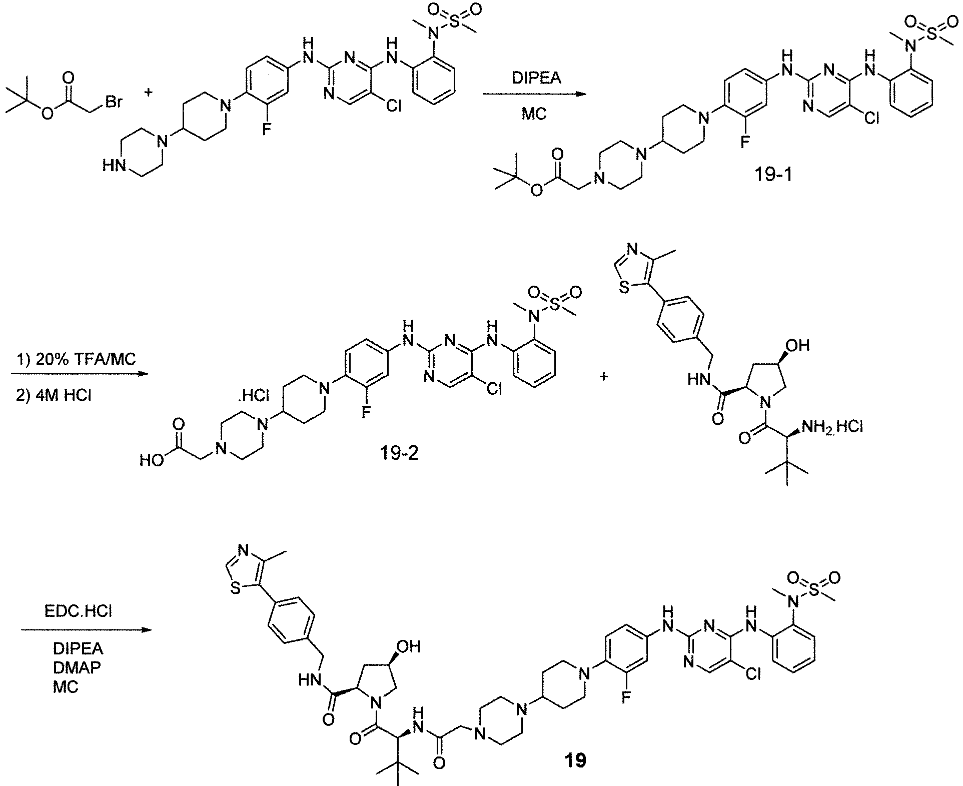

### Step 1: Synthesis of tertiary butyl 2-(4-(1-(4-((5-chloro-4-((2-(N-methylmethylsulfonamido)phenyl)amino)pyrimidin-2-yl)amino)-2-fluorophenyl)piperidin-4-yl)piperazin-1-yl)acetate (19-1)

The title compound was obtained using a method similar to the Step 1 synthesis method of Example 11 above with N-(2-((5-chloro-2-((3-fluoro-4-(4-(piperazin-1-yl)piperidin-1-yl)phenyl)amino)pyrimidin-4-yl)amino)phenyl)-N-methylmethanesulfonamide (100 mg, 0.17 mmol) instead of tertiary butyl 2-bromoacetate (34 mg, 0.17 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide, and was used in the next reaction.

### Step 2: Synthesis of 2-(4-(1-(4-f(5-chloro-4-((2-(N-methylmethylsulfonamido)phenyl)amino)pyrimidin-2-yl)amino)-2-fluorophenyl)piperidin-4-yl)piperazin-1-yl)acetic acid hydrochloride (19-2)

The title compound was obtained using a method similar to the Step 2 synthesis method of Example 7 above with tertiary butyl 2-(4-(1-(4-((5-chloro-4-((2-(N-methylmethylsulfonamido)phenyl)amino)pyrimidin-2-yl)amino)-2-fluorophenyl)piperidin-4-yl)piperazin-1-yl)acetate (119 mg, 0.17 mmol) instead of tertiary butyl-5-(4-(1-(4-((5-chloro-4-((2-(N-methylmethylsulfonamido)phenyl)amino)-3-methoxyphenyl)piperidin-4-yl)piperazin-1-yl)pentanoate, and then was used in the next reaction.

### Step 3: Synthesis of Compound 19

The title compound (80 mg, yield 36%) was obtained using a method similar to the Step 1 synthesis method of Example 6 above with (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride (62 mg, 0.13 mmol) and 2-(4-(1-(4-((5-chloro-4-((2-(N-methylmethylsulfonamido)phenyl)amino)pyrimidin-2-yl)amino)-2-fluorophenyl)piperidin-4-yl)piperazin-1-yl)acetic acid hydrochloride instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentanoic acid, respectively.

### Example 20: Synthesis of (2R,4R)-1-((S)-2-(3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanamido)-3,3-dimethylbutanoyl)-4-hydroxy-N-(4-(4-methylthiazol-5-yl)benzyl)pyrrolidin-2-carboxamide (Compound 20)

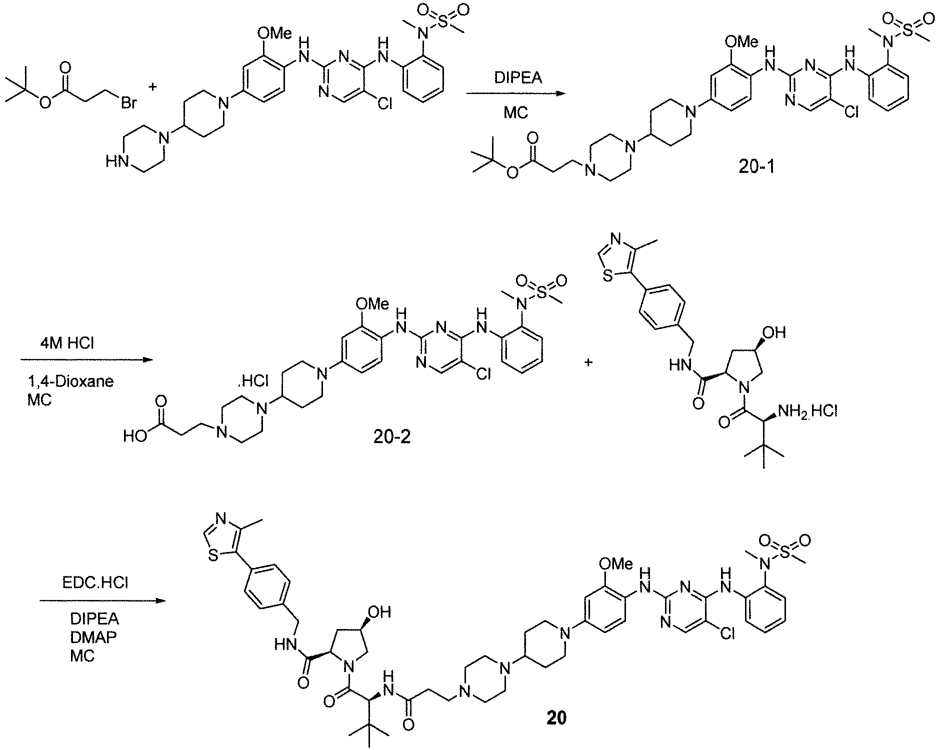

### Step 1: Synthesis of tertiary butyl 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoate (20-1)

To a solution of tertiary butyl 3-bromopropanoate (35 mg, 0.17 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (100 mg, 0.17 mmol) in dichloromethane (1.5 mL), diisopropylethylamine (74 mL, 0.43 mmol) was added and stirred for 4 hours. Extraction was performed by dilution with water and dichloromethane. The organic layer was dried over anhydrous magnesium sulfate, filtered, and concentrated, and used in the next reaction.

### Step 2: Synthesis of 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoic acid hydrochloride (20-2)

To tertiary butyl 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoate obtained in Example 20-1 Step above, 4N HCl 1,4-dioxane solution (2 mL) and dichloromethane (2 mL) were added and stirred at 40 °C for 3 hours. The title compound obtained by concentrating and vacuum drying the reaction mixture was used in the next reaction.

### Step 3: Synthesis of Compound 20

The title compound (57 mg, yield 37%) was obtained using a method similar to the Step 1 synthesis method of Example 6 above with (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride (75 mg, 0.16 mmol) and 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoic acid hydrochloride instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentanoic acid, respectively.

### Example 21: Synthesis of 2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1,3-dioxoisoindolin-4-yl)acetamide (Compound 21) (not claimed)

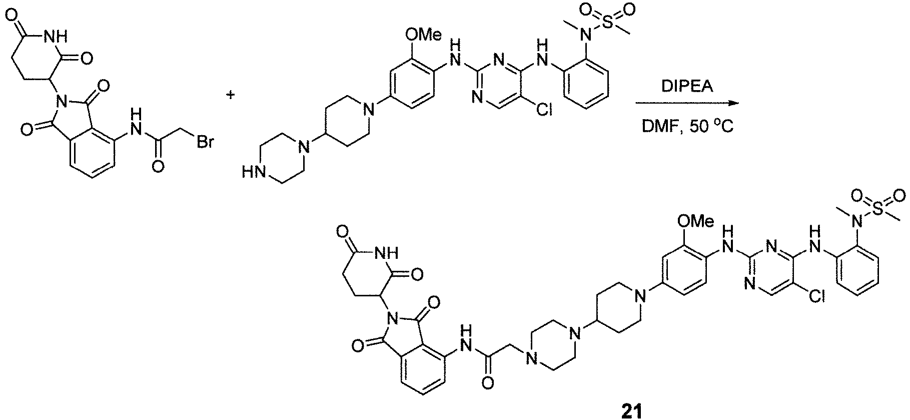

The title compound (66 mg, yield 63%) was obtained using a method similar to the Step 6 synthesis method of Example 1 above with 2-bromo-N-(2-(2,6-dioxopiperidin-3-yl)-1,3-dioxoisoindolin-4-yl)acetamide (46 mg, 0.12 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (70 mg, 0.12 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 22: Synthesis of 2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1-oxoisoindolin-4-yl)acetamide (Compound 22)

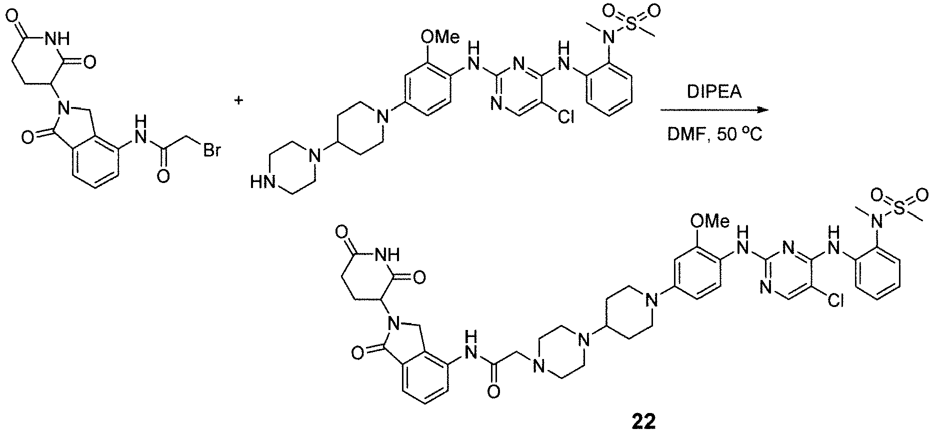

The title compound (72 mg, yield 69%) was obtained using a method similar to the Step 6 synthesis method of Example 1 above with 2-bromo-N-(2-(2,6-dioxopiperidin-3-yl)-1-oxoisoindolin-4-yl)acetamide (44 mg, 0.12 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (70 mg, 0.12 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 23: Synthesis of 2-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)-N-(2-(2,6-dioxopiperidin-3-yl)-1,3-dioxoisoindolin-4-yl)acetamide (Compound 23) (not claimed)

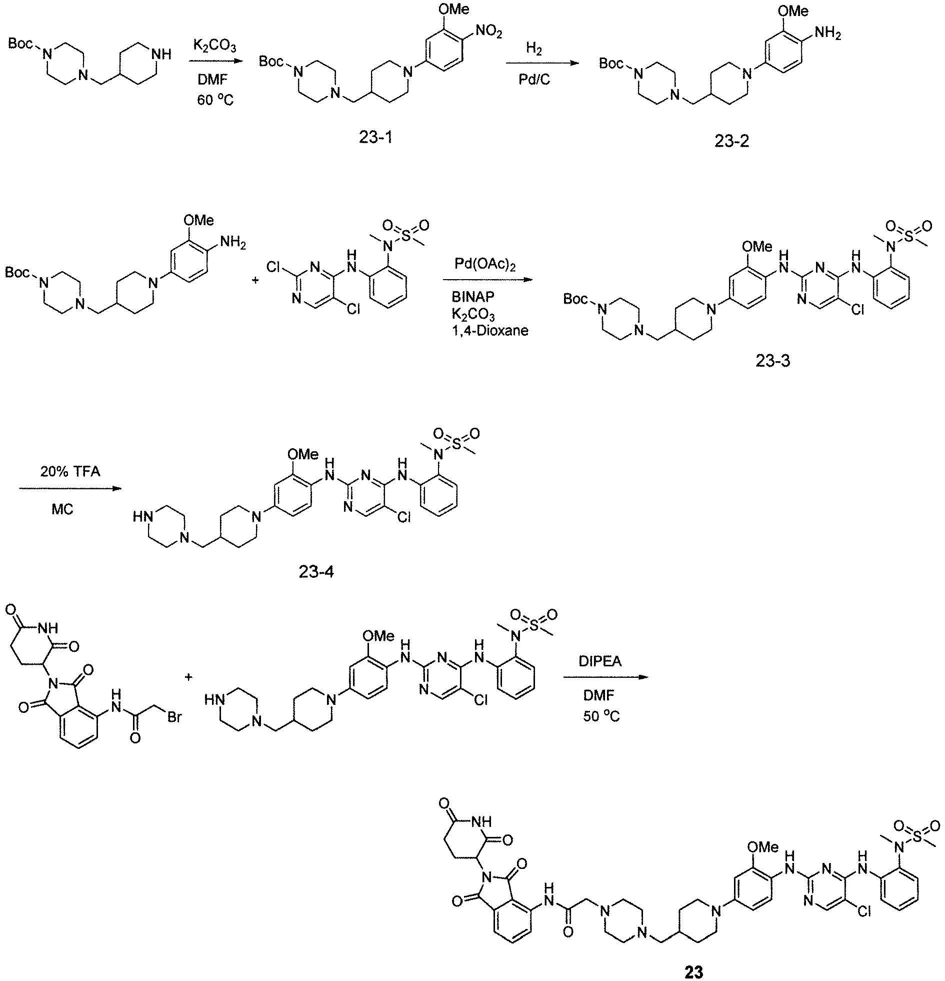

### Step 1: Synthesis of tertiary butyl 4-((1-(3-methoxy-4-nitrophenyl)piperidin-4-yl)methyl)piperazin-1-carboxylate (23-1)

The title compound (647 mg, yield 42%) was obtained using a method similar to the Step 2 synthesis method of Example 1 above with tertiary butyl 4-(piperidin-4-ylmethyl)piperazin-1-carboxylate (1.0 g, 3.54 mmol) instead of Boc-piperazine.

### Step 2: Synthesis of tertiary butyl 4-((1-(4-amino-2-methoxyphenyl)piperidin-4-yl)methyl)piperazin-1-carboxylate (23-2)

The title compound (572 mg, yield 96%) was obtained using a method similar to the Step 3 synthesis method of Example 1 above with tertiary butyl 4-((1-(3-methoxy-4-nitrophenyl)piperidin-4-yl)methyl)piperazin-1-carboxylate (640 mg, 1.47 mmol) instead of tertiary butyl 4-(3-methoxy-4-nitrophenyl)piperazin-1-carboxylate.

### Step 3: Synthesis of tertiary butyl 4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-carboxylate (23-3)

The title compound (625 mg, yield 63%) was obtained using a method similar to the Step 4 synthesis method of Example 1 above with tertiary butyl 4-((1-(4-amino-2-methoxyphenyl)piperidin-4-yl)methyl)piperazin-1-carboxylate (570 mg, 1.41 mmol) instead of tertiary butyl 4-(4-amino-3-methoxyphenyl)piperazin-1-carboxylate.

### Step 4: Synthesis of N-(2-((5-chloro-2-((2-methoxy-4-(4-(piperazin-1-ylmethyl)piperidin-1-yl)phenyl)amino)pyrimidin-4-yl)amino)phenyl)-N-methylmethanesulfonamide (23-4)

The title compound (535 mg, yield 100%) was obtained using a method similar to the Step 6 synthesis method of Example 1 above with tertiary butyl 4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-carboxylate (620 mg, 0.17 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 5: Synthesis of Compound 23

The title compound (70 mg, yield 67%) was obtained using a method similar to the Step 6 synthesis method of Example 1 above with 2-bromo-N-(2-(2,6-dioxopiperidin-3-yl)-1,3-dioxoisoindolin-4-yl)acetamide (45 mg, 0.11 mmol) and N-(2-((5-chloro-2-((2-methoxy-4-(4-(piperazin-1-ylmethyl)piperidin-1-yl)phenyl)amino)pyrimidin-4-yl)amino)phenyl)-N-methylmethanesulfonamide (70 mg, 0.11 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 24: Synthesis of 2-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)-N-(2-(2,6-dioxopiperidin-3-yl)-1-oxoisoindolin-4-yl)acetamide (Compound 24)

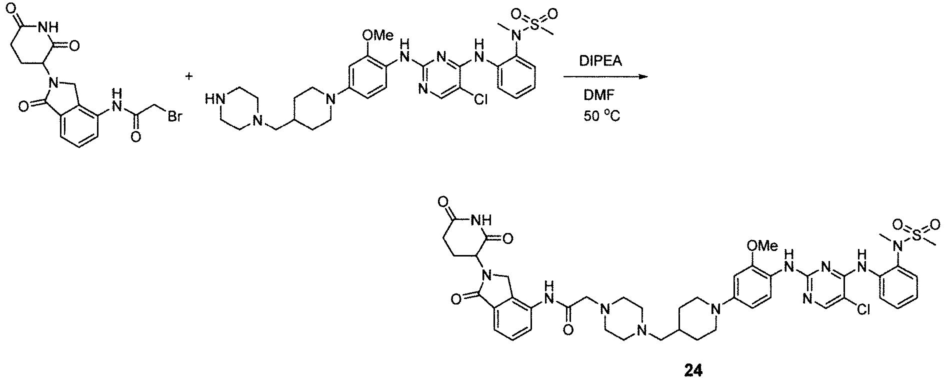

The title compound (78 mg, yield 75%) was obtained using a method similar to the Step 6 synthesis method of Example 1 above with 2-bromo-N-(2-(2,6-dioxopiperidin-3-yl)-1-oxoisoindolin-4-yl)acetamide (43 mg, 0.11 mmol) and N-(2-((5-chloro-2-((2-methoxy-4-(4-(piperazin-1-ylmethyl)piperidin-1-yl)phenyl)amino)pyrimidin-4-yl)amino)phenyl)-N-methylmethanesulfonamide (70 mg, 0.11 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 25: Synthesis of 2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)-N-(2-(2,6-dioxopiperidin-3-yl)-1,3-dioxoisoindolin-4-yl)acetamide (Compound 25) (not claimed)

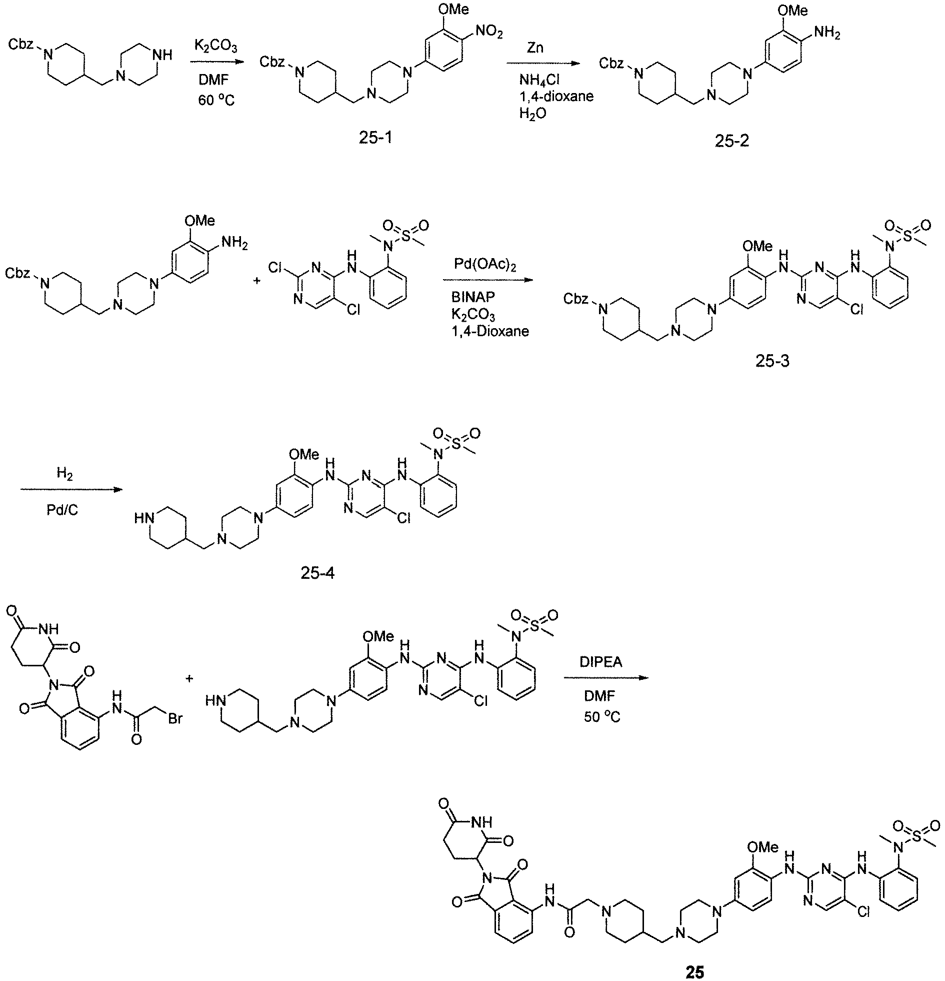

### Step 1: Synthesis of benzyl 4-((4-(3-methoxy-4-nitrophenyl)piperazin-1-yl)methyl)piperidin-l-carboxylate (25-1)

The title compound (903 mg, yield 61%) was obtained using a method similar to the Step 2 synthesis method of Example 1 above with benzyl 4-(piperazin-1-ylmethyl)piperidin-1-carboxylate (1.0 g, 3.16 mmol) instead of Boc-piperazine.

### Step 2: Synthesis of benzyl 4-((4-(4-amino-3-methoxyphenyl)piperazin-1-yl)methyl)piperidin-1-carboxylate (25-2)

To a solution of benzyl 4-((4-(3-methoxy-4-nitrophenyl)piperazin-1-yl)methyl)piperidin-1-carboxylate (900 mg, 1.92 mmol) in 1,4-dioxane (15 mL), NHCl (513 mg, 9.60 mmol) in H₂O (5 mL) was added and purged with argon. Zn (628 mg, 9.60 mmol) was added and stirred at 70 °C. After 19 hours, the mixture was filtered through Celite and concentrated. The residue was diluted with aqueous sodium chloride solution, made basic with aqueous saturated sodium hydrocarbon solution, and extracted with ethyl acetate. The organic layer was dried over sodium sulfate, filtered, concentrated, and purified by column chromatography to obtain the title compound (831 mg, yield 99%).

### Step 3: Synthesis of benzyl 4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-carboxylate (25-3)

The title compound (1.0 g, yield 71%) was obtained using a method similar to the Step 4 synthesis method of Example 1 above with benzyl 4-((4-(4-amino-3-methoxyphenyl)piperazin-1-yl)methyl)piperidin-1-carboxylate (830 mg, 1.89 mmol) instead of tertiary butyl 4-(4-amino-3-methoxyphenyl)piperazin-1-carboxylate.

### Step 4: Synthesis of N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (25-4)

The title compound (617 mg, yield 94%) was obtained using a method similar to the Step 3 synthesis method of Example 1 above with benzyl 4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-carboxylate (800 mg, 1.06 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 5: Synthesis of Compound 25

The title compound (33 mg, yield 31%) was obtained using a method similar to the Step 6 synthesis method of Example 1 above with 2-bromo-N-(2-(2,6-dioxopiperidin-3-yl)-1,3-dioxoisoindolin-4-yl)acetamide (45 mg, 0.11 mmol) and N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (70 mg, 0.11 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 26: Synthesis of 2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)-N-(2-(2,6-dioxopiperidin-3-yl)-1-oxoisoindolin-4-yl)acetamide (Compound 26)

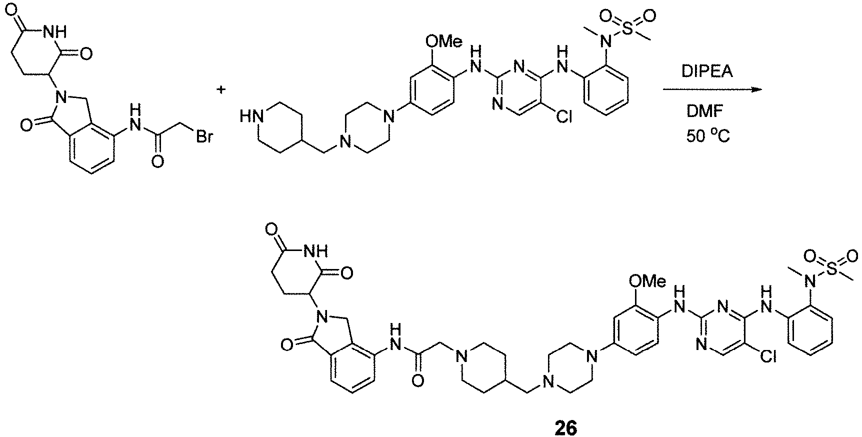

The title compound (78 mg, yield 75%) was obtained using a method similar to the Step 6 synthesis method of Example 1 above with 2-bromo-N-(2-(2,6-dioxopiperidin-3-yl)-1-oxoisoindolin-4-yl)acetamide (43 mg, 0.11 mmol) and N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (70 mg, 0.11 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 27: Synthesis of (2R,4R)-1-((S)-2-(2-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)acetamido)-3,3-dimethylbutanoyl)-4-hydroxy-N-(4-(4-methylthiazol-5-yl)benzyl)pyrrolidin-2-carboxamide (Compound 27)

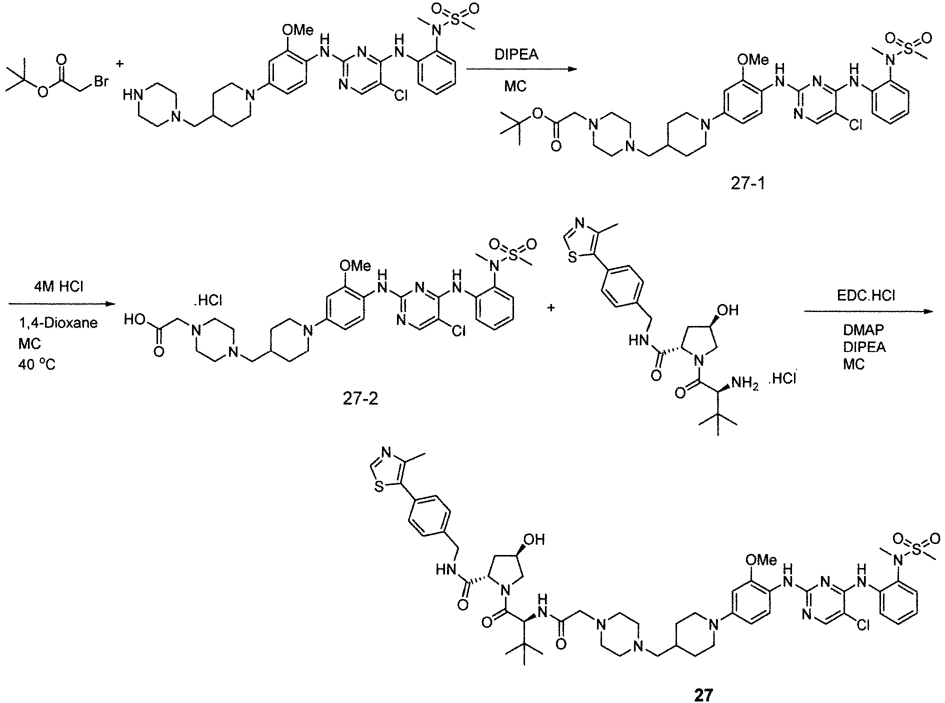

### Step 1: Synthesis of tertiary butyl-2-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)acetate (27-1)

The title compound (119 mg, yield 100%) was obtained using a method similar to the Step 6 synthesis method of Example 1 above with tertiary butyl 2-bromoacetate (32 mg, 0.16 mmol) and N-(2-((5-chloro-2-((2-methoxy-4-(4-(piperazin-1-ylmethyl)piperidin-1-yl)phenyl)amino)pyrimidin-4-yl)amino)phenyl)-N-methylmethanesulfonamide (100 mg, 0.16 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Step 2: Synthesis of 2-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)acetic acid hydrochloride (27-2)

The title compound was obtained using a method similar to the Step 2 synthesis method of Example 20 above with tertiary butyl-2-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)acetate (119 mg, 0.16 mmol) instead of 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoic acid hydrochloride, and then used in the next reaction.

### Step 3: Synthesis of Compound 27

The title compound (104 mg, yield 59%) was obtained using a method similar to the Step 1 synthesis method of Example 6 above with (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 2-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)acetic acid hydrochloride obtained in Step 2 instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentanoic acid, respectively.

### Example 28: Synthesis of (2R,4R)-1-((S)-2-(2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamide (Compound 28)

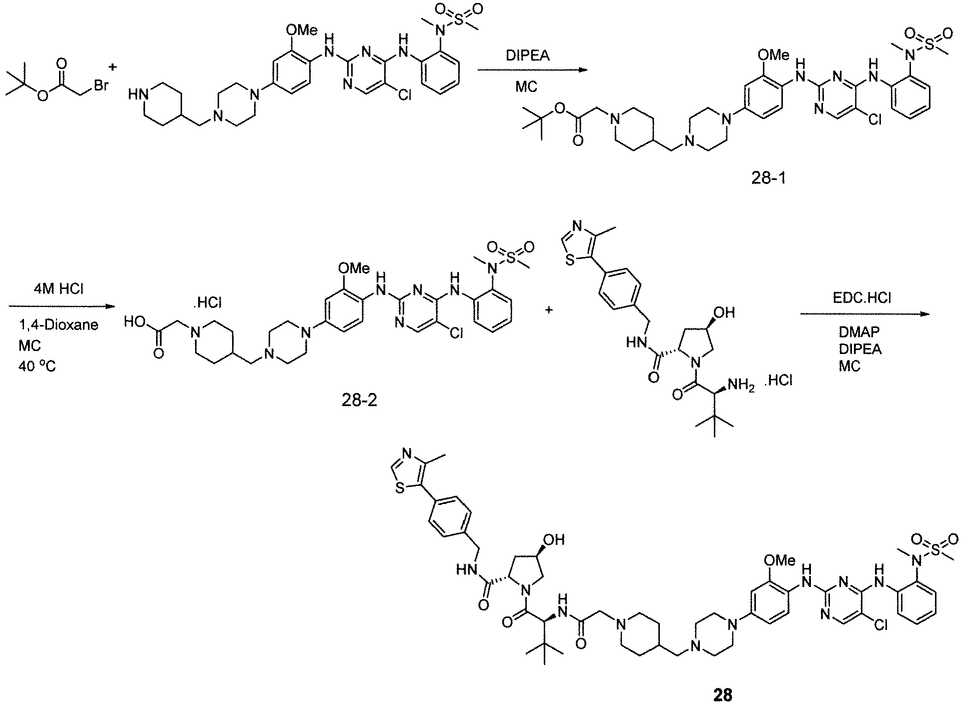

### Step 1: Synthesis of tertiary butyl-2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)acetate (28-1)

The title compound (91 mg, yield 94%) was obtained using a method similar to the Step 6 synthesis method of Example 1 above with tertiary butyl 2-bromoacetate (26 mg, 0.13 mmol) and N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (82 mg, 0.13 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Step 2: Synthesis of 2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)acetic acid hydrochloride (28-2)

The title compound was obtained using a method similar to the Step 2 synthesis method of Example 20 above with tertiary butyl-2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)acetate (91 mg, 0.13 mmol) instead of 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoic acid hydrochloride, and then used in the next reaction.

### Step 3: Synthesis of Compound 28

The title compound (73 mg, yield 51%) was obtained using a method similar to the Step 1 synthesis method of Example 6 above with (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)acetic acid hydrochloride obtained in Step 2 instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentanoic acid, respectively.

### Example 29: Synthesis of 1-((1-(4-((5-chloro-4-((2-(N-methylmethylsulfonamido)phenyl)amino)pyrimidin-2-yl)amino)-3-methoxyphenyl)piperidin-4-yl)methyl)-N-((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)piperidin-4-carboxamide (Compound 29) (not claimed)

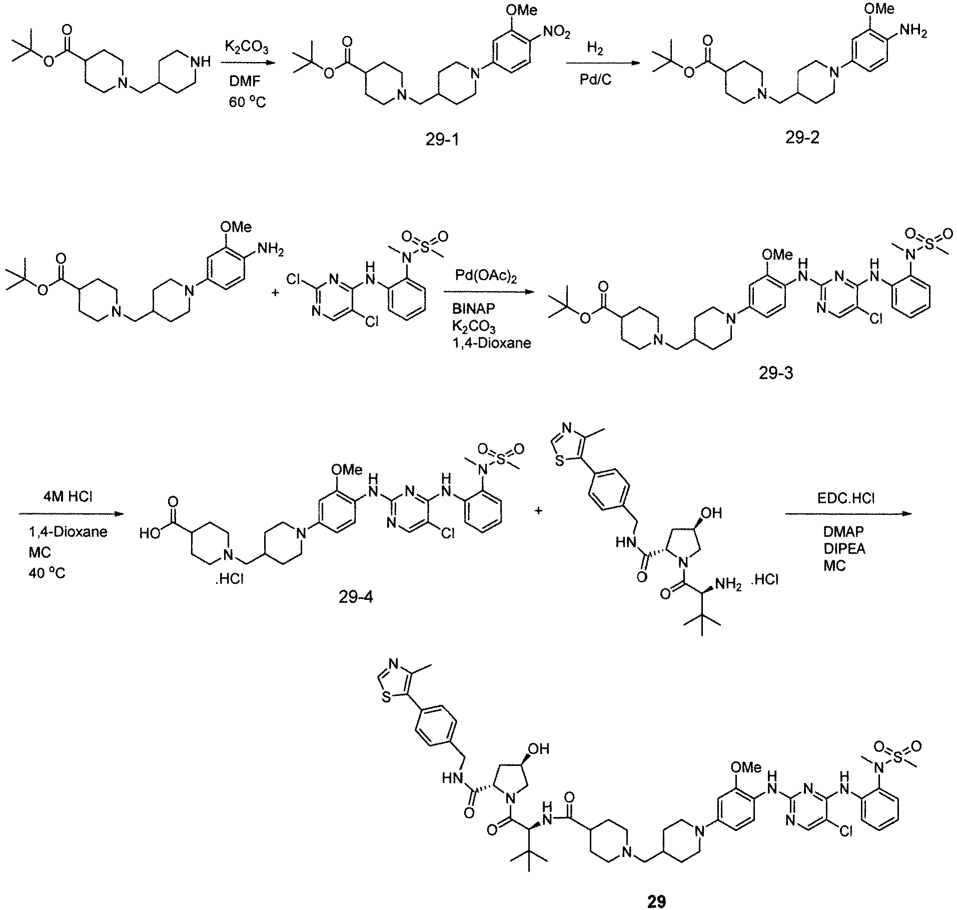

### Step 1: Synthesis of tertiary butyl 1-((1-(3-methoxy-4-nitrophenyl)piperidin-4-yl)methyl)piperidin-4-carboxylate (29-1)

The title compound (462 mg, yield 64%) was obtained using a method similar to the Step 2 synthesis method of Example 1 above with tertiary butyl 1-(piperidin-4-ylmethyl)piperidin-4-carboxylate (470 mg, 1.66 mmol) instead of Boc-piperazine.

### Step 2: Synthesis of tertiary butyl 1-((1-(4-amino-3-methoxyphenyl)piperidin-4-yl)methyl)piperidin-4-carboxylate (29-2)

The title compound (223 mg, yield 99%) was obtained using a method similar to the Step 3 synthesis method of Example 1 above with tertiary butyl 1-((1-(3-methoxy-4-nitrophenyl)piperidin-4-yl)methyl)piperidin-4-carboxylate (240 mg, 0.55 mmol) instead of tertiary butyl 4-(3-methoxy-4-nitrophenyl)piperazin-1-carboxylate.

### Step 3: Synthesis of tertiary butyl 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylate (29-3)

The title compound (95 mg, yield 24%) was obtained using a method similar to the Step 4 synthesis method of Example 1 above with tertiary butyl 1-((1-(4-amino-3-methoxyphenyl)piperidin-4-yl)methyl)piperidin-4-carboxylate (223 mg, 0.55 mmol) instead of tertiary butyl 4-(4-amino-3-methoxyphenyl)piperazin-1-carboxylate.

### Step 4: Synthesis of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride (29-4)

The title compound was obtained using a method similar to the Step 2 synthesis method of Example 20 above with tertiary butyl 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylate (90 mg, 0.13 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate, and then used in the next reaction.

### Step 5: Synthesis of Compound 29

The title compound (27 mg, yield 20%) was obtained using a method similar to the Step 1 synthesis method of Example 6 above with (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride obtained in Step 2 instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentanoic acid, respectively.

### Example 30: Synthesis of 3-(9-(4-((5-chloro-4-((2-(N-methylmethylsulfonamido)phenyl)amino)pyrimidin-2-yl)amino)-3-methoxyphenyl)-3,9-diazaspiro[5,5]undecan-3-yl)-N-(2-(2,6-dioxopiperidin-3-yl)-1,3-dioxoisoindolin-4-yl)propanamide (Compound 30) (not claimed)

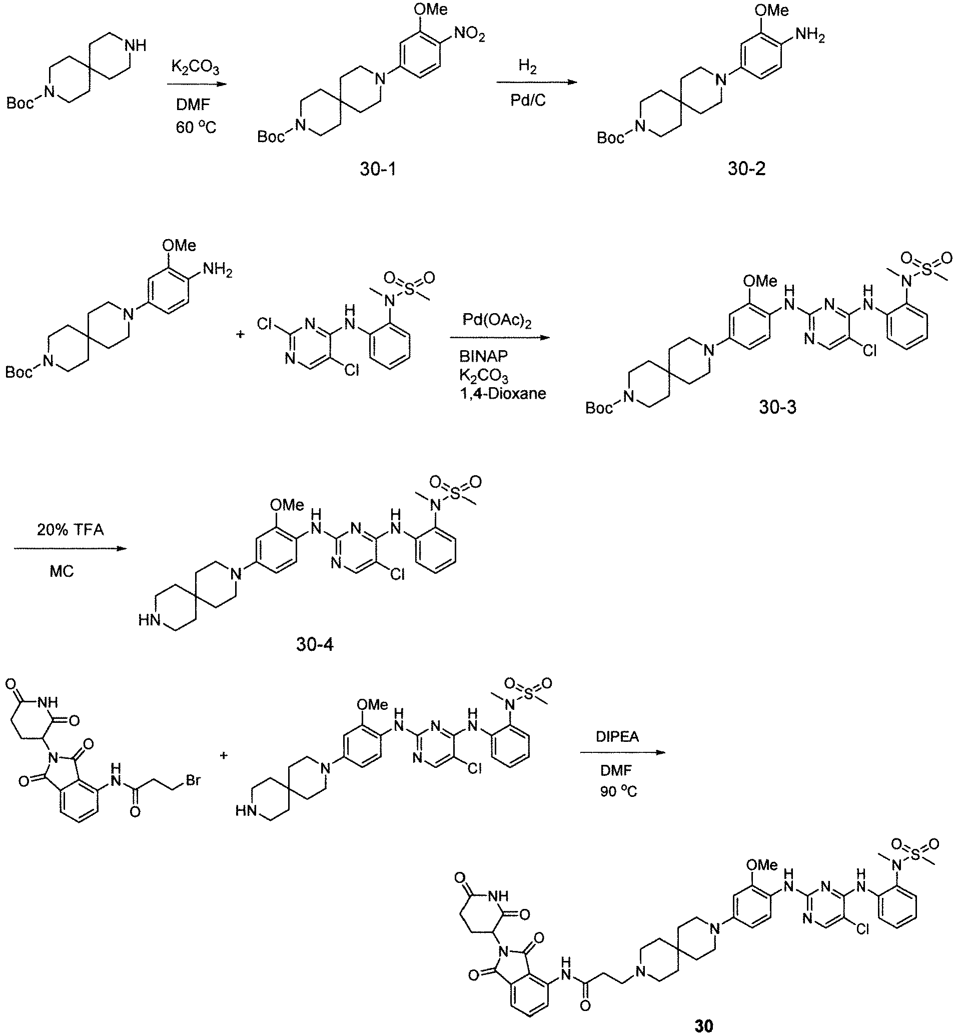

### Step 1: Synthesis of tertiary butyl 9-(3-methoxy-4-nitrophenyl)-3,9-diazaspiro[5,5]undecan-3-carboxylate (30-1)

The title compound (13.2 g, yield 93%) was obtained using a method similar to the Step 2 synthesis method of Example 1 above with tertiary butyl 3,9-diazaspiro[5,5]undecan-3-carboxylate (6.0 g, 35.06 mmol) instead of Boc-piperazine.

### Step 2: Synthesis of tertiary butyl 9-(4-amino-3-methoxyphenyl)-3,9-diazaspiro[5,5]undecan-3-carboxylate (30-2)

The title compound (1.4 g, yield 99%) was obtained using a method similar to the Step 3 synthesis method of Example 1 above with tertiary butyl 9-(3-methoxy-4-nitrophenyl)-3,9-diazaspiro[5,5]undecan-3-carboxylate (1.5 g, 3.70 mmol) instead of tertiary butyl 4-(3-methoxy-4-nitrophenyl)piperazin-1-carboxylate.

### Step 3 : Synthesis of tertiary butyl 9-(4-((5-chloro-4-((2-(N-methylmethylsulfonamido)phenyl)amino)pyrimidin-2-yl)amino)-3-methoxyphenyl)-3,9-diazaspiro[5,5]undecan-3-carboxylate (30-3)

The title compound (1.4 g, yield 54%) was obtained using a method similar to the Step 4 synthesis method of Example 1 above with tertiary butyl 9-(4-amino-3-methoxyphenyl)-3,9-diazaspiro[5,5]undecan-3-carboxylate (1.4 g, 3.60 mmol) instead of tertiary butyl 4-(4-amino-3-methoxyphenyl)piperazin-1-carboxylate.

### Step 4: Synthesis of N-(2-((5-chloro-2-((2-methoxy-4-(3,9-diazaspiro[5,5]undecan-3-yl)phenyl)amino)pyrimidin-4-yl)amino)phenyl)-N-methylmethylsulfonamide (30-4)

The title compound (1.2 g, yield 99%) was obtained using a method similar to the Step 5 synthesis method of Example 1 above with tertiary butyl 9-(4-((5-chloro-4-((2-(N-methylmethylsulfonamido)phenyl)amino)pyrimidin-2-yl)amino)-3-methoxyphenyl)-3,9-diazaspiro[5,5]undecan-3-carboxylate (1.4 g, 1.98 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 5: Synthesis of Compound 30

The title compound (61 mg, yield 56%) was obtained using a method similar to the Step 6 synthesis method of Example 1 above with 3-bromo-N-(2-(2,6-dioxopiperidin-3-yl)-1,3-dioxoisoindolin-4-yl)propanamide (49 mg, 0.12 mmol) and N-(2-((5-chloro-2-((2-methoxy-4-(3,9-diazaspiro[5,5]undecan-3-yl)phenyl)amino)pyrimidin-4-yl)amino)phenyl)-N-methylmethylsulfonamide (70 mg, 0.12 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 31: Synthesis of (2R,4R)-1-((S)-2-(2-(9-(4-((5-chloro-4-((2-(N-methylmethylsulfonamido)phenyl)amino)pyrimidin-2-yl)amino)-3-methoxyphenyl)-3,9-diazaspiro[5,5]undecan-3-yl)acetamido)-3,3-dimethylbutanoyl)-4-hydroxy-N-(4-(4-methylthiazol-5-yl)benzyl)pyrrolidin-2-carboxamide (Compound 31)

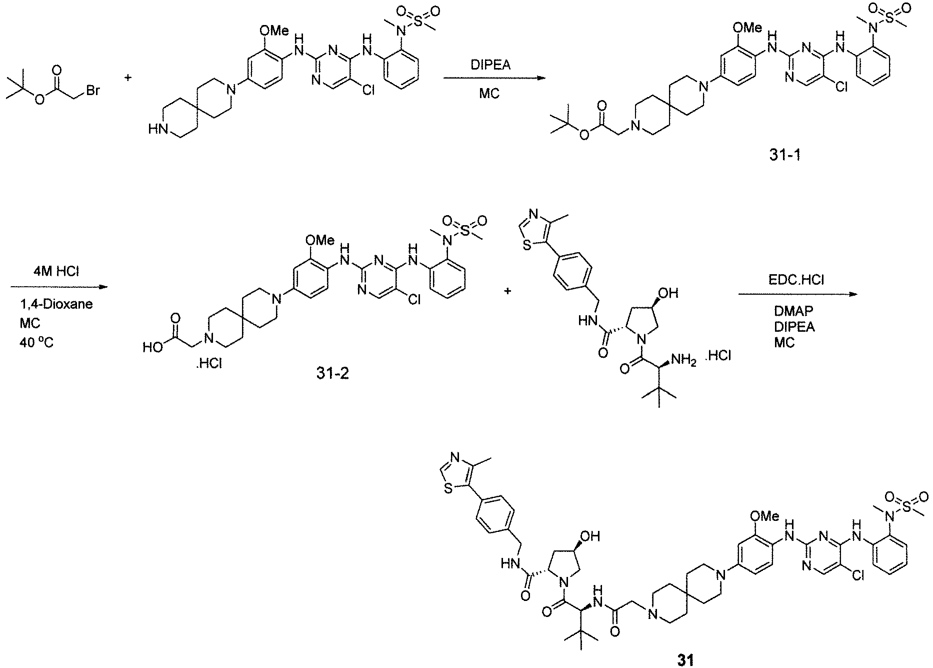

### Step 1: Synthesis of tertiary butyl 2-(9-(4-((5-chloro-4-((2-(N-methylmethylsulfonamido)phenyl)amino)pyrimidin-2-yl)amino)-3-methoxyphenyl)-3,9-diazaspiro[5,5]undecan-3-yl)acetate (31-1)

The title compound (119 mg, yield 100%) was obtained using a method similar to the Step 6 synthesis method of Example 1 above with tertiary butyl 2-bromoacetate (26 mg, 0.13 mmol) and N-(2-((5-chloro-2-((2-methoxy-4-(3,9-diazaspiro[5,5]undecan-3-yl)phenyl)amino)pyrimidin-4-yl)amino)phenyl)-N-methylmethylsulfonamide (100 mg, 0.17 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide.

### Step 2: Synthesis of 2-(9-(4-((5-chloro-4-((2-(N-methylmethylsulfonamido)phenyl)amino)pyrimidin-2-yl)amino)-3-methoxyphenyl)-3,9-diazaspiro[5,5]undecan-3-yl)acetic acid hydrochloride (31-2)

The title compound was obtained using a method similar to the Step 2 synthesis method of Example 20 above with tertiary butyl-2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)acetate (119 mg, 0.17 mmol) instead of 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoic acid hydrochloride, and then used in the next reaction.

### Step 3: Synthesis of Compound 31

The title compound (85 mg, yield 34%) was obtained using a method similar to the Step 1 synthesis method of Example 6 above with (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 2-(9-(4-((5-chloro-4-((2-(N-methylmethylsulfonamido)phenyl)amino)pyrimidin-2-yl)amino)-3-methoxyphenyl)-3,9-diazaspiro[5,5]undecan-3-yl)acetic acid hydrochloride obtained in Step 2 instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentanoic acid, respectively.

### Example 32: Synthesis of 3-(6-(4-((5-chloro-4-((2-(N-methylmethylsulfonamido)phenyl)amino)pyrimidin-2-yl)amino)-3-methoxyphenyl)-2,6-diazaspiro[3,3]heptan-2-yl)-N-(2-(2,6-dioxopiperidin-3-yl)-1,3-dioxoisoindolin-4-yl)propanamide (Compound 32) (not claimed)

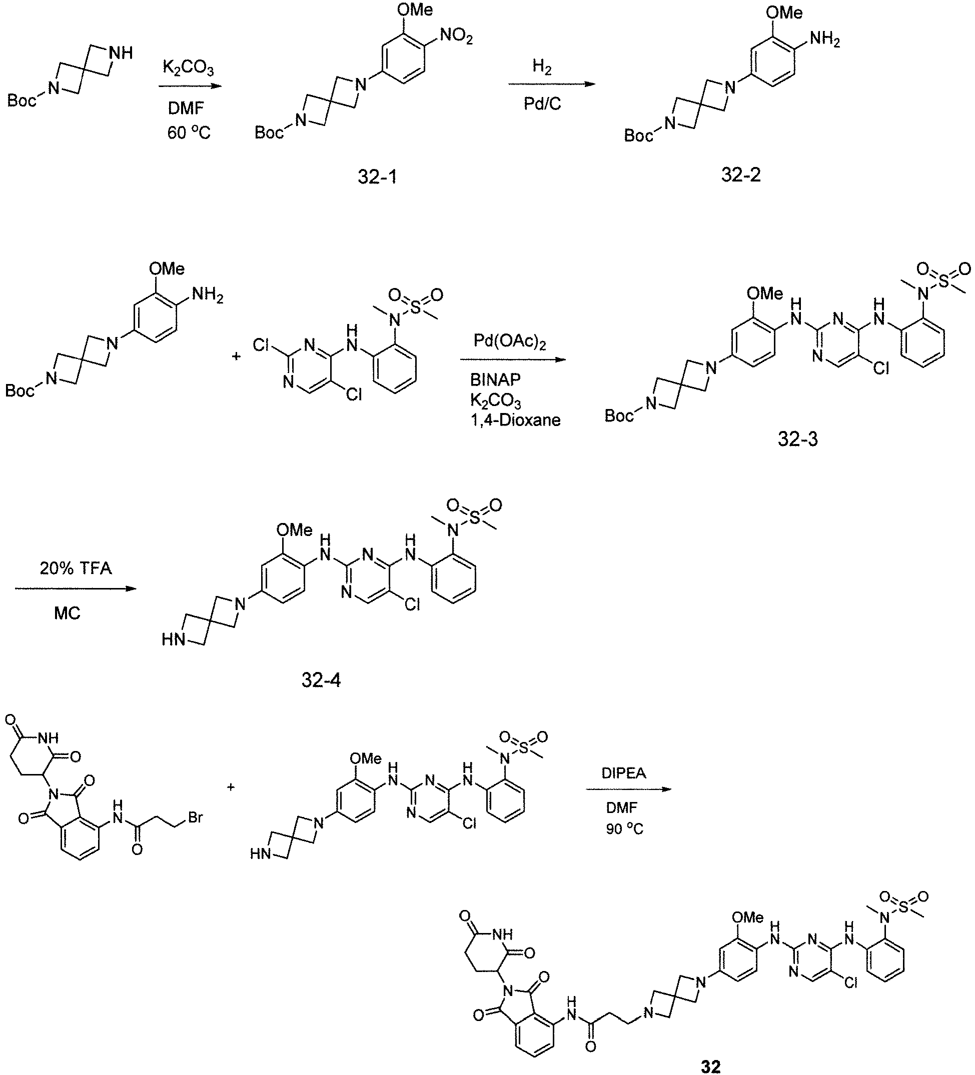

### Step 1: Synthesis of tertiary butyl 6-(3-methoxy-4-nitrophenyl)-2,6-diazaspiro[3,3]heptan-2-carboxylate (32-1)

The title compound (4.8 g, yield 79%) was obtained using a method similar to the Step 2 synthesis method of Example 1 above with tertiary butyl 2,6-diazaspiro[3,3]undecan-2-carboxylate (7.4 g, 21.0 mmol) instead of Boc-piperazine.

### Step 2: Synthesis of tertiary butyl 6-(4-amino-3-methoxyphenyl)-2,6-diazaspiro[3,3]heptan-2-carboxylate (32-2)

The title compound (950 mg, yield 70%) was obtained using a method similar to the Step 3 synthesis method of Example 1 above with tertiary butyl 6-(3-methoxy-4-nitrophenyl)-2,6-diazaspiro[3,3]heptan-2-carboxylate (1.5 g, 4.29 mmol) instead of tertiary butyl 4-(3-methoxy-4-nitrophenyl)piperazin-1-carboxylate.

### Step 3: Synthesis of tertiary butyl 6-(4-((5-chloro-4-((2-(N-methylmethylsulfonamido)phenyl)amino)pyrimidin-2-yl)amino)-3-methoxyphenyl)-2,6-diazaspiro[3,3]undecan-2-carboxylate (32-3)

The title compound (0.7 g, yield 34%) was obtained using a method similar to the Step 4 synthesis method of Example 1 above with tertiary butyl 6-(4-amino-3-methoxyphenyl)-2,6-diazaspiro[3,3]heptan-2-carboxylate (0.9 g, 2.81 mmol) instead of tertiary butyl 4-(4-amino-3-methoxyphenyl)piperazin-1-carboxylate.

### Step 4: Synthesis of N-(2-((5-chloro-2-((2-methoxy-4-(2,6-diazaspiro[3,3]heptan-2-yl)phenyl)amino)pyrimidin-4-yl)amino)phenyl)-N-methylmethylsulfonamide (32-4)

The title compound (580 mg, yield 99%) was obtained using a method similar to the Step 5 synthesis method of Example 1 above with tertiary butyl 6-(4-((5-chloro-4-((2-(N-methylmethylsulfonamido)phenyl)amino)pyrimidin-2-yl)amino)-3-methoxyphenyl)-2,6-diazaspiro[3,3]undecan-2-carboxylate (0.7 g, 1.98 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 5: Synthesis of Compound 32

The title compound (48 mg, yield 41%) was obtained using a method similar to the Step 6 synthesis method of Example 1 above with 3-bromo-N-(2-(2,6-dioxopiperidin-3-yl)-1,3-dioxoisoindolin-4-yl)propanamide (54 mg, 0.13 mmol) and N-(2-((5-chloro-2-((2-methoxy-4-(2,6-diazaspiro[3,3]heptan-2-yl)phenyl)amino)pyrimidin-4-yl)amino)phenyl)-N-methylmethylsulfonamide (70 mg, 0.13 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 33: Synthesis of N-(2-((5-chloro-2-((4-(4-(2-((1-(2-(2,6-dioxopiperidin-3-yl)-1,3-dioxoisoindolin-5-yl)azetidin-3-yl)oxy)ethyl)piperazin-1-yl)-2-methoxyphenyl)amino)pyrimidin-4-yl)amino)phenyl)-N-methylmethanesulfonamide (Compound 33)

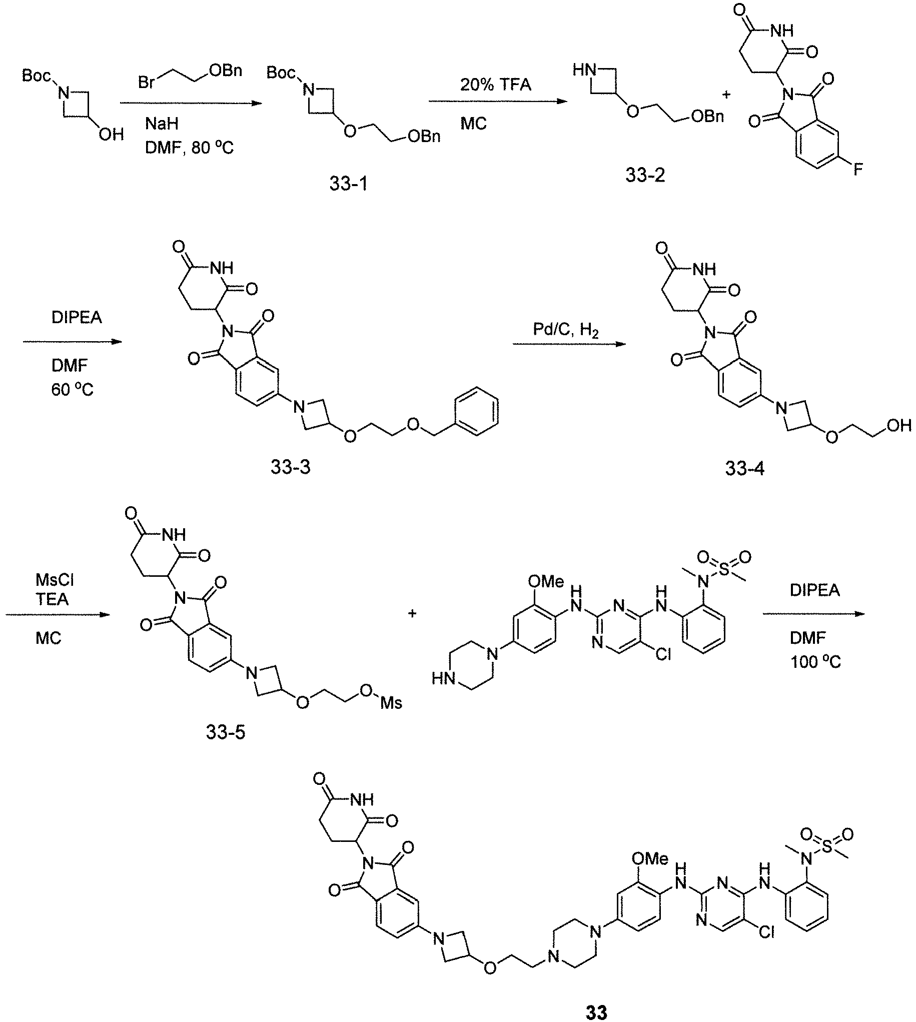

### Step 1: Synthesis of tertiary butyl 3-(2-(benzyloxy)ethoxy)azetidin-1-carboxylate (33-1)

Sodium hydroxide (346 mg, 8.66 mmol) was added to a solution of tertiary butyl 3-hydroxyazetidin-1-carboxylate (1.0 g, 5.77 mmol) in DMF (11 mL) and stirred for 30 minutes. ((2-bromoethoxy)methyl)benzene (1.1 mL, 6.93 mmol) was added slowly at 0 °C. After stirring overnight at 80 °C, extraction was performed with ethyl acetate and water. The organic layer was washed sequentially with water and saturated brine, and dried over anhydrous magnesium sulfate. The title compound (0.95 g, yield 54%) was obtained by filtration, concentration and purification by column chromatography.

### Step 2: Synthesis of 3-(2-(benzyloxy)ethoxy)azetidine (33-2)

A solution of tertiary butyl 3-(2-(benzyloxy)ethoxy)azetidin-1-carboxylate (0.9 g, 2.93 mmol) in 20% trichloroacetic acid in dichloromethane (20 mL) was stirred at room temperature for 5 hours. The reaction mixture was concentrated and extracted with 1N aqueous sodium hydroxide solution and dichloromethane. The organic layer was dried over sodium sulfate, filtered, and concentrated to obtain the title compound (550 mg, yield 92%).

### Step 3: Synthesis of 5-(3-(2-(benzyloxy)ethoxy)azetidin-1-yl)-2-(2,6-dioxopiperidin-3-yl)isoindolin-1,3-dione (33-3)

To a solution of 2-(2,6-dioxopiperidin-3-yl)-5-fluoroisoindolin-1,3-dione (0.6 g, 2.17 mmol) and 3-(2-(benzyloxy)ethoxy)azetidine (0.5 g, 2.39 mmol) in N,N-dimethylformamide (10 mL), diisopropylethylamine (757 mL, 4.34 mmol) was added and stirred overnight at 80 °C. After extraction with ethyl acetate and water, the organic layer was washed with saturated brine. The organic layer was dried over anhydrous sodium sulfate, filtered, concentrated, and purified by column chromatography to obtain the title compound (650 mg, yield 65%).

### Step 4: Synthesis of 2-(2,6-dioxopiperidin-3-yl)-5-(3-(2-hydroxyethoxy)azetidin-1-yl)isoindolin-1,3-dione (33-4)

A solution of 5-(3-(2-(benzyloxy)ethoxy)azetidin-1-yl)-2-(2,6-dioxopiperidin-3-yl)isoindolin-1,3-dione (500 mg, 1.08 mmol) in tetrahydrofuran/methanol (1/1, 20 mL) was purged with argon and 10% Pd/C (0.2 g) was added. The reaction mixture was substituted with hydrogen and stirred overnight. It was diluted with dichloromethane, filtered through celite, and concentrated. The residue was purified by column chromatography to obtain the title compound (130 mg, yield 32%).

### Step 5: Synthesis of 2-((1-(2-(2,6-dioxopiperidin-3-yl)-1,3-dioxoisoindolin-5-yl)azetidin-3-yl)oxy)ethylmethanesulfonate (33-5)

A solution of 2-(2,6-dioxopiperidin-3-yl)-5-(3-(2-hydroxyethoxy)azetidin-1-yl)isoindolin-1,3-dione (130 mg, 0.35 mmol) in dichloromethane (7 mL), triethylamine (146 mL, 1.05 mmol) was added and then methanesulfonyl chloride (54 mL, 0.70 mmol) was slowly added at 0 °C. The reaction mixture was extracted with water and dichloromethane, dried over anhydrous sodium sulfate, filtered, and concentrated. The residue was purified by column chromatography to obtain the title compound (90 mg, yield 57%).

### Step 6: Synthesis of Compound 33

The title compound (40 mg, yield 31%) was obtained using a method similar to the Step 6 synthesis method of Example 1 above with 2-((1-(2-(2,6-dioxopiperidin-3-yl)-1,3-dioxoisoindolin-5-yl)azetidin-3-yl)oxy)ethylmethane sulfonate (70 mg, 0.16 mmol) and N-(2-((5-chloro-2-((2-methoxy-4-(2,6-diazaspiro[3,3]heptan-2-yl)phenyl)amino)pyrimidin-4-yl)amino)phenyl)-N-methylmethylsulfonamide (81 mg, 0.16 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 34: Synthesis of N-(2-((5-chloro-2-((4-(4-(2-((1-(2-(2,6-dioxopiperidin-3-yl)-1,3-dioxoisoindolin-5-yl)piperidin-4-yl)oxy)ethyl)piperazin-1-yl)-2-methoxyphenyl)amino)pyrimidin-4-yl)amino)phenyl)-N-methylmethanesulfonamide (Compound 34) (not claimed)

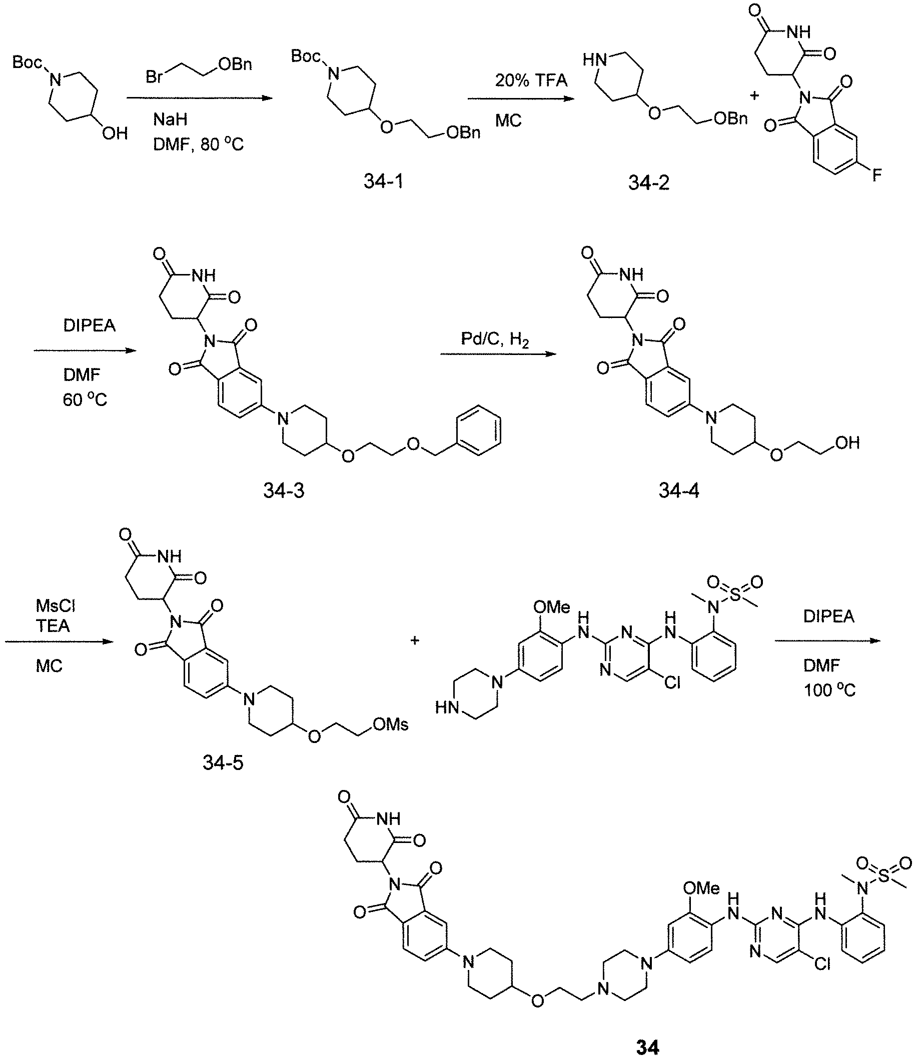

### Step 1: Synthesis of tertiary butyl 4-(2-(benzyloxy)ethoxy)piperidin-1-carboxylate (34-1)

The title compound (0.7 g, yield 42%) was obtained using a method similar to the Step 6 synthesis method of Example 1 above with tertiary butyl 4-hydroxypiperidin-1-carboxylate (1.0 g, 4.97 mmol) instead of tertiary butyl 3-hydroxyazetidin-1-carboxylate.

### Step 2: Synthesis of 4-(2-(benzyloxy)ethoxy)piperidine (34-2)

The title compound (480 mg, yield 98%) was obtained using a method similar to the Step 2 synthesis method of Example 33 above with tertiary butyl 4-(2-(benzyloxy)ethoxy)piperidin-1-carboxylate (0.7 g, 2.09 mmol) instead of tertiary butyl 3-(2-(benzyloxy)ethoxy)azetidin-1-carboxylate.

### Step 3: Synthesis of 5-(4-(2-(benzyloxy)ethoxy)piperidin-1-yl)-2-(2,6-dioxopiperidin-3-yl)isoindolin-1,3-dione (34-3)

The title compound (400 mg, yield 45%) was obtained using a method similar to the Step 3 synthesis method of Example 33 above with 4-(2-(benzyloxy)ethoxy)piperidine (468 mg, 1.99 mmol) instead of 3-(2-(benzyloxy)ethoxy)azetidine.

### Step 4: Synthesis of 2-(2,6-dioxopiperidin-3-yl)-5-(4-(2-hydroxyethoxy)piperidin-1-yl)isoindolin-1,3-dione (34-4)

The title compound (130 mg, yield 40%) was obtained using a method similar to the Step 4 synthesis method of Example 33 above with 5-(4-(2-(benzyloxy)ethoxy)piperidin-1-yl)-2-(2,6-dioxopiperidin-3-yl)isoindolin-1,3-dione (0.4 g, 0.81 mmol) instead of 5-(3-(2-(benzyloxy)ethoxy)azetidin-1-yl)-2-(2,6-dioxopiperidin-3-yl)isoindolin-1,3-dione.

### Step 5: Synthesis of 2-((1-(2-(2,6-dioxopiperidin-3-yl)-1,3-dioxoisoindolin-5-yl)piperidin-4-yl)oxy)ethylmethanesulfonate (34-5)

The title compound (135 mg, yield 90%) was obtained using a method similar to the Step 5 synthesis method of Example 33 above with 2-(2,6-dioxopiperidin-3-yl)-5-(4-(2-hydroxyethoxy)piperidin-1-yl)isoindolin-1,3-dione (125 mg, 0.31 mmol) instead of 2-(2,6-dioxopiperidin-3-yl)-5-(3-(2-hydroxyethoxy)azetidin-1-yl)isoindolin-1,3-dione.

### Step 5: Synthesis of Compound 34

The title compound (70 mg, yield 53%) was obtained using a method similar to the Step 6 synthesis method of Example 1 above with 2-((1-(2-(2,6-dioxopiperidin-3-yl)-1,3-dioxoisoindolin-5-yl)piperidin-4-yl)oxy)ethylmethanesulfonate (70 mg, 0.15 mmol) and N-(2-((5-chloro-2-((2-methoxy-4-(2,6-diazaspiro[3,3]heptan-2-yl)phenyl)amino)pyrimidin-4-yl)amino)phenyl)-N-methylmethylsulfonamide (76 mg, 0.15 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 35: Synthesis of (25,4R)-1-((S)-2-(2-(4-((4-(3-chloro-4-((5-chloro-4-((2-(N-methylmethylsulfonamido)phenyl)amino)pyrimidin-2-yl)amino)phenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamide (Compound 35)

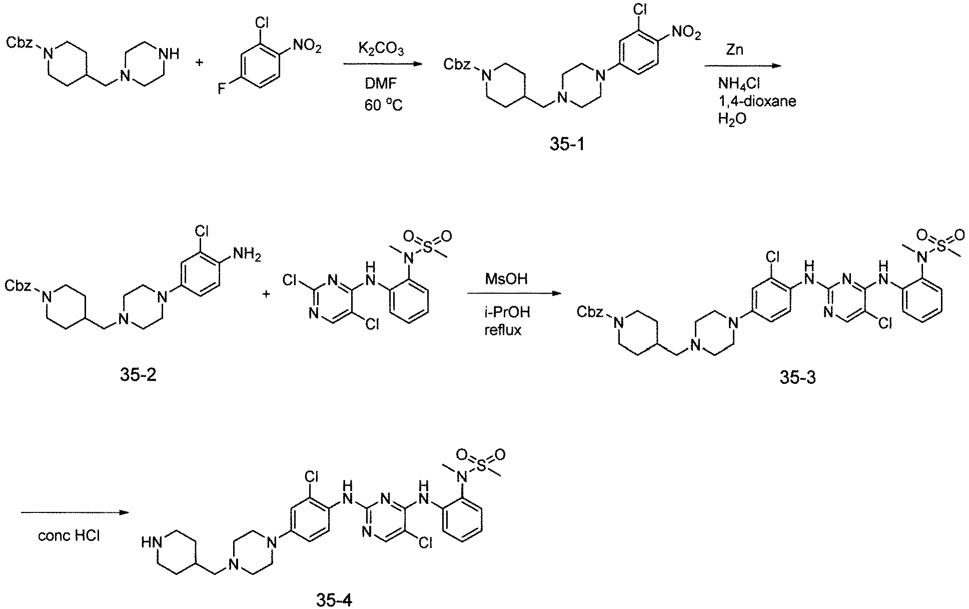

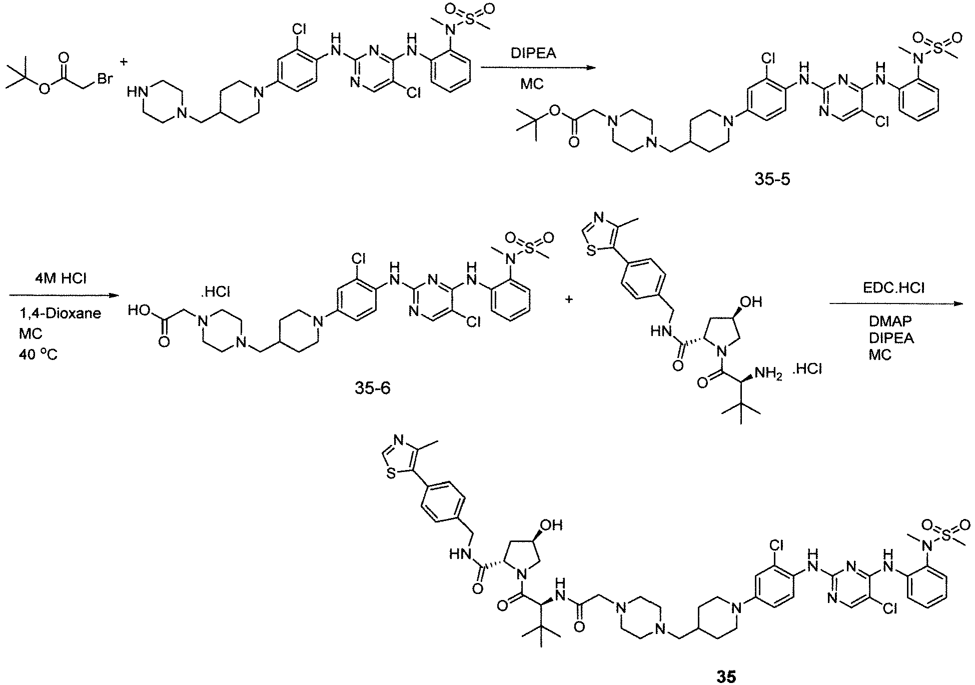

### Step 1: Synthesis of benzyl 4-((4-(3-chloro-4-nitrophenyl)piperazin-1-yl)methyl)piperidin-1-carboxylate (35-1)

The title compound (428 mg, yield 72%) was obtained using a method similar to the Step 2 synthesis method of Example 1 above with 2-chloro-4-fluoro-1-nitrobenzene (221 mg, 1.26 mmol) instead of 4-fluoro-2-methoxy-1-nitrobenzene.

### Step 2: Synthesis of benzyl 4-((4-(4-amino-3-chlorophenyl)piperazin-1-yl)methyl)piperidin-1-carboxylate (35-2)

The title compound (420 mg, yield 99%) was obtained using a method similar to the Step 2 synthesis method of Example 25 above with 4-((4-(3-chloro-4-nitrophenyl)piperazin-1-yl)methyl)piperidin-1-carboxylate (408 mg, 0.91 mmol) instead of benzyl 4-((4-(3-methoxy-4-nitrophenyl)piperazin-1-yl)methyl)piperidin-1-carboxylate.

### Step 3: Synthesis of benzyl 4-((4-(3-chloro-4-((5-chloro-4-((2-(N-methylmethanesulfonamido)phenyl)amino)pyrimidin-2-yl)amino)phenyl)piperazin-1-yl)methyl)piperidin-1-carboxylate (35-3)

To a solution of benzyl 4-((4-(4-amino-3-chlorophenyl)piperazin-1-yl)methyl)piperidin-1-carboxylate (420 mg, 0.95 mmol) and N-(2-((2,5-dichloropyrimidin-4-yl)amino)phenyl)-N-methylmethanesulfonamide (396 mg, 1.14 mmol) in isopropanol (9.5 mL), methanesulfonic acid (0.12 mL, 1.90 mmol) was added and stirred under reflux overnight. The mixture was extracted by diluting with dichloromethane and aqueous sodium hydroxide solution, and dried over anhydrous sodium sulfate. After filtering and concentrating, the title compound (316 mg, 44%) was obtained by purification using a silica gel column.

### Step 4: Synthesis of N-(2-((5-chloro-2-((2-chloro-4-(4-(piperidin-4-ylmethyl)piperazin-1-yl)phenyl)amino)pyrimidin-4-yl)amino)phenyl)-N-methylmethanesulfonamide (35-4)

A saturated aqueous solution of benzyl 4-((4-(3-chloro-4-((5-chloro-4-((2-(N-methylmethanesulfonamido)phenyl)amino)pyrimidin-2-yl)amino)phenyl)piperazin-1-yl)methyl)piperidin-1-carboxylate (50 mg, 0.066 mmol) in hydrochloric acid (1 mL) was stirred overnight at room temperature. The mixture was extracted by diluting with dichloromethane and aqueous sodium hydroxide solution, and dried over anhydrous sodium sulfate. The title compound (21 mg, 52%) was obtained by filtration and concentration.

### Step 5: Synthesis of tertiary butyl 2-(4-((1-(3-chloro-4-((5-chloro-4-((2-(N-methylmethanesulfonamido)phenyl)amino)pyrimidin-2-yl)amino)phenyl)piperidin-4-yl)methyl)piperazin-1-yl)acetate (35-5)

The title compound (50 mg, yield 99%) was obtained using a method similar to the Step 1 synthesis method of Example 11 above with N-(2-((5-chloro-2-((2-chloro-4-(4-(piperidin-4-ylmethyl)piperazin-1-yl)phenyl)amino)pyrimidin-4-yl)amino)phenyl)-N-methylmethanesulfonamide (71 mg, 0.114 mmol) instead of N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide.

### Step 6: Synthesis of 2-(4-((1-(3-chloro-4-((5-chloro-4-((2-(N-methylmethanesulfonamido)phenyl)amino)pyrimidin-2-yl)amino)phenyl)piperidin-4-yl)methyl)piperazin-1-yl)acetic acid hydrochloride (35-6)

The title compound was obtained using a method similar to the Step 2 synthesis method of Example 20 above with tertiary butyl 2-(4-((1-(3-chloro-4-((5-chloro-4-((2-(N-methylmethanesulfonamido)phenyl)amino)pyrimidin-2-yl)amino)phenyl)piperidin-4-yl)methyl)piperazin-1-yl)acetate (50 mg, 0.068 mmol) instead of tertiary butyl 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoate, and then used in the next reaction.

### Step 7: Synthesis of Compound 35

The title compound (51 mg, yield 69%) was obtained using a method similar to the Step 1 synthesis method of Example 6 above with 2-(4-((1-(3-chloro-4-((5-chloro-4-((2-(N-methylmethanesulfonamido)phenyl)amino)pyrimidin-2-yl)amino)phenyl)piperidin-4-yl)methyl)piperazin-1-yl)acetic acid hydrochloride (46 mg, 0.068 mmol) instead of 5-bromopentanoic acid.

### Example 36: Synthesis of (2S,4R)-1-((S)-2-(2-(4-((4-(4-((5-chloro-4-((2-(N-methylmethylsulfonamido)phenyl)amino)pyrimidin-2-yl)amino)2-fluorophenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamide (Compound 36)

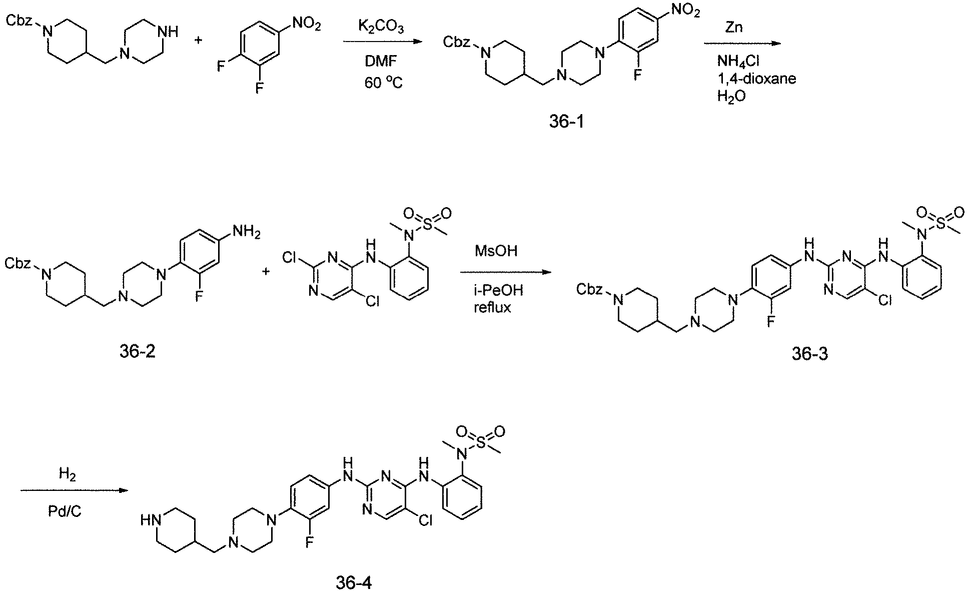

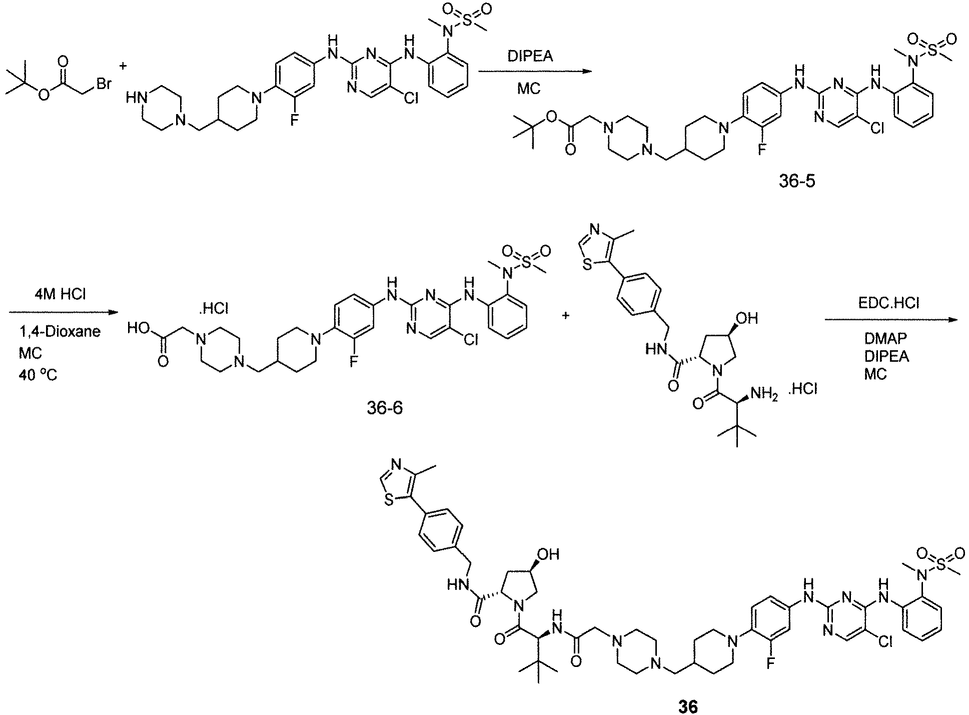

### Step 1: Synthesis of benzyl 4-((4-(2-fluoro-4-nitrophenyl)piperazin-1-yl)methyl)piperidin-1-carboxylate (36-1)

The title compound (765 mg, yield 67%) was obtained using a method similar to the Step 2 synthesis method of Example 1 above with 1,2-difluoro-4-nitrobenzene (400 mg, 2.51 mmol) and benzyl 4-(piperazin-1-ylmethyl)piperidin-1-carboxylate (798 mg, 2.51 mmol) instead of 4-fluoro-2-methoxy-1-nitrobenzene and Boc-piperazine, respectively.

### Step 2: Synthesis of benzyl 4-((4-(4-amino-2-fluorophenyl)piperazin-1-yl)methyl)piperidin-1-carboxylate (36-2)

The title compound (700 mg, yield 99%) was obtained using a method similar to the Step 2 synthesis method of Example 25 above with benzyl 4-((4-(2-fluoro-4-nitrophenyl)piperazin-1-yl)methyl)piperidin-1-carboxylate (750 mg, 1.64 mmol) instead of benzyl 4-((4-(3-methoxy-4-nitrophenyl)piperazin-1-yl)methyl)piperidin-1-carboxylate.

### Step 3 : Synthesis of benzyl 4-((4-(4-((5-chloro-4-((2-(N-methylmethanesulfonamido)phenyl)amino)pyrimidin-2-yl)amino)-2-fluorophenyl)piperazin-1-yl)methyl)piperidin-1-carboxylate (36-3)

The title compound (1.1 g, 91%) was obtained using a method similar to the Step 3 synthesis method of Example 35 above with benzyl 4-((4-(4-amino-2-fluorophenyl)piperazin-1-yl)methyl)piperidin-1-carboxylate (700 mg, 1.64 mmol) instead of benzyl 4-((4-(4-amino-3-chlorophenyl)piperazin-1-yl)methyl)piperidin-1-carboxylate.

### Step 4: Synthesis of N-(2-((5-chloro-2-((3-fluoro-4-(4-(piperidin-4-ylmethyl)piperazin-1-yl)phenyl)amino)pyrimidin-4-yl)amino)phenyl)-N-methylmethanesulfonamide (36-4)

The title compound (900 mg, yield 99%) was obtained using a method similar to the Step 4 synthesis method of Example 33 above with benzyl 4-((4-(4-((5-chloro-4-((2-(N-methylmethanesulfonamido)phenyl)amino)pyrimidin-2-yl)amino)-2-fluorophenyl)piperazin-1-yl)methyl)piperidin-1-carboxylate (1.1 g, 1.49 mmol) instead of 5-(3-(2-(benzyloxy)ethoxy)azetidin-1-yl)-2-(2,6-dioxopiperidin-3-yl)isoindolin-1,3-dione.

### Step 5: Synthesis of tertiary butyl 2-(4-((1-(4-((5-chloro-4-((2-(N-methylmethanesulfonamido)phenyl)amino)pyrimidin-2-yl)amino)-2-fluorophenyl)piperidin-4-yl)methyl)piperazin-1-yl)acetate (36-5)

The title compound (75 mg, yield 63%) was obtained using a method similar to the Step 1 synthesis method of Example 11 above with N-(2-((5-chloro-2-((3-fluoro-4-(4-(piperidin-4-ylmethyl)piperazin-1-yl)phenyl)amino)pyrimidin-4-yl)amino)phenyl)-N-methylmethanesulfonamide (100 mg, 0.166 mmol) instead of N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide.

### Step 6: Synthesis of 2-(4-((1-(4-((5-chloro-4-((2-(N-methylmethanesulfonamido)phenyl)amino)pyrimidin-2-yl)amino)-2-fluorophenyl)piperidin-4-yl)methyl)piperazin-1-yl)acetic acid hydrochloride (36-6)

The title compound (65 mg, 98%) was obtained using a method similar to the Step 2 synthesis method of Example 20 above with tertiary butyl 2-(4-((1-(4-((5-chloro-4-((2-(N-methylmethanesulfonamido)phenyl)amino)pyrimidin-2-yl)amino)-2-fluorophenyl)piperidin-4-yl)methyl)piperazin-1-yl)acetate (70 mg, 0.098 mmol) instead of tertiary butyl 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoate.

### Step 7: Synthesis of Compound 36

The title compound (32 mg, yield 31%) was obtained using a method similar to the Step 1 synthesis method of Example 6 above with 2-(4-((1-(4-((5-chloro-4-((2-(N-methylmethanesulfonamido)phenyl)amino)pyrimidin-2-yl)amino)-2-fluorophenyl)piperidin-4-yl)methyl)piperazin-1-yl)acetic acid hydrochloride (45 mg, 0.096 mmol) instead of 5-bromopentanoic acid.

### Example 37: Synthesis of (2S,4R)-1-((S)-2-(2-(3-((4-(4-((5-chloro-4-((2-(N-methylmethylsulfonamido)phenyl)amino)pyrimidin-2-yl)amino)-3-methoxyphenyl)piperazin-1-yl)methyl)azetidin-1-yl)acetamido)-3,3-dimethylbutanoyl)-4-hydroxy-N-(4-(4-methylthiazol-5-yl)benzyl)pyrrolidin-2-carboxamide (Compound 37)

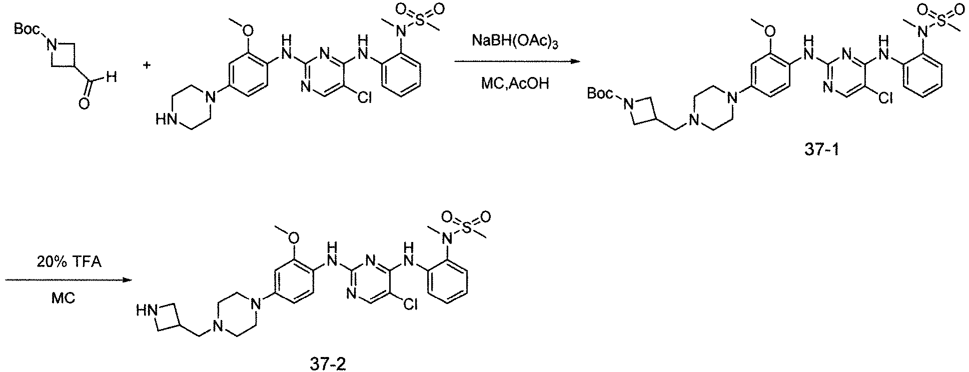

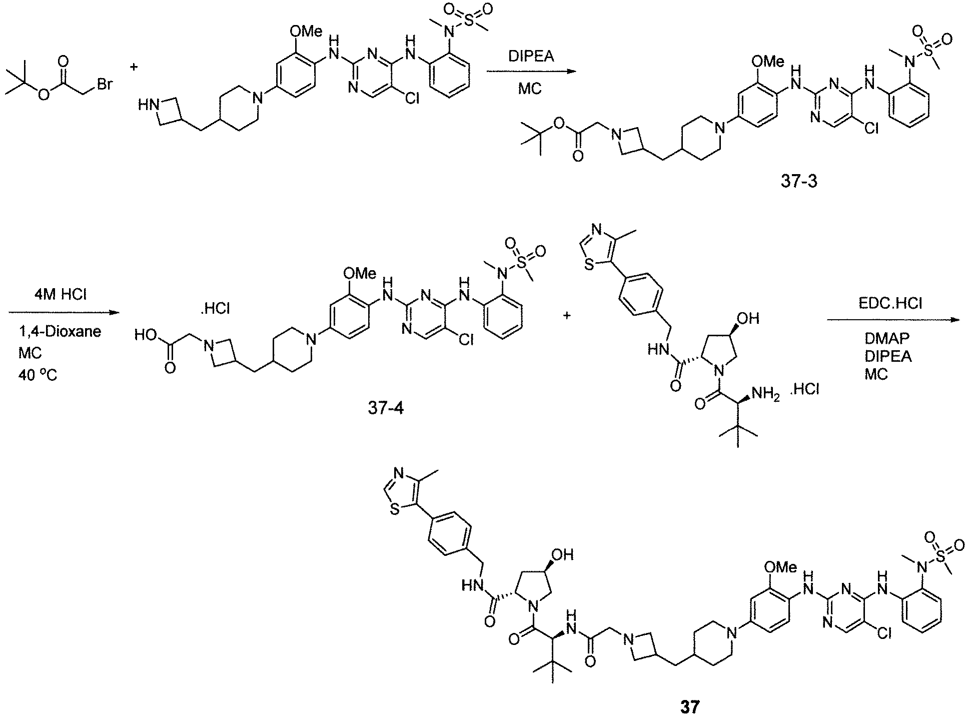

### Step 1: Synthesis of tertiary butyl 3-((4-(4-((5-chloro-4-((2-(N-methylmethanesulfonamido)phenyl)amino)pyrimidin-2-yl)amino)-3-methoxyphenyl)piperazin-1-yl)methyl)azetidin-1-carboxylate (37-1)

The title compound (230 mg, yield 87%) was obtained using a method similar to the Step 2 synthesis method of Example 2 above with tertiary butyl 3-formylazetidin-1-carboxylate (107 mg, 0.58 mmol) and N-(2-((5-chloro-2-((2-methoxy-4-(piperazin-1-yl)phenyl)amino)pyrimidin-4-yl)amino)phenyl)-N-methylmethanesulfonamide (200 mg, 0.39 mmol) instead of 1-(3-methoxy-4-nitrophenyl)piperidin-4-one and N-Boc-piperazine, respectively.

### Step 2: Synthesis of N-(2-((2-((4-(4-(azetidin-3-ylmethyl)piperazin-1-yl)-2-methoxyphenyl)amino)-5-chloropyrimidin-4-yl)amino)phenyl)-N-methylmethanesulfonamide (37-2)

The title compound was obtained using a method similar to the Step 5 synthesis method of Example 1 above with tertiary butyl 3-((4-(4-((5-chloro-4-((2-(N-methylmethanesulfonamido)phenyl)amino)pyrimidin-2-yl)amino)-3-methoxyphenyl)piperazin-1-yl)methyl)azetidin-1-carboxylate (225 mg, 0.33 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 3: Synthesis of tertiary butyl 2-(3-((1-(4-((5-chloro-4-((2-(N-methylmethanesulfonamido)phenyl)amino)pyrimidin-2-yl)amino)-3-methoxyphenyl)piperidin-4-yl)methyl)azetidin-1-yl)acetate (37-3)

The title compound (40 mg, yield 42%) was obtained using a method similar to the Step 1 synthesis method of Example 11 above with N-(2-((2-((4-(4-(azetidin-3-ylmethyl)piperazin-1-yl)-2-methoxyphenyl)amino)-5-chloropyrimidin-4-yl)amino)phenyl)-N-methylmethanesulfonamide (100 mg, 0.166 mmol) instead of N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide.

### Step 4: Synthesis of 2-(3-((1-(4-((5-chloro-4-((2-(N-methylmethanesulfonamido)phenyl)amino)pyrimidin-2-yl)amino)-3-methoxyphenyl)piperidin-4-yl)methyl)azetidin-1-yl)acetic acid hydrochloride (37-4)

The title compound was obtained using a method similar to the Step 2 synthesis method of Example 20 above with tertiary butyl 2-(3-((1-(4-((5-chloro-4-((2-(N-methylmethanesulfonamido)phenyl)amino)pyrimidin-2-yl)amino)-3-methoxyphenyl)piperidin-4-yl)methyl)azetidin-1-yl)acetate (80 mg, 0.117 mmol) instead of tertiary butyl 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoate.

### Step 5: Synthesis of Compound 37

The title compound (45 mg, yield 48%) was obtained using a method similar to the Step 1 synthesis method of Example 6 above with 2-(3-((1-(4-((5-chloro-4-((2-(N-methylmethanesulfonamido)phenyl)amino)pyrimidin-2-yl)amino)-3-methoxyphenyl)piperidin-4-yl)methyl)azetidin-1-yl)acetic acid hydrochloride (85 mg, 0.118 mmol) instead of 5-bromopentanoic acid.

### Example 38: Synthesis of (2S,4R)-1-((S)-2-(1-((1-(4-((5-chloro-4-((2-(N-methylmethylsulfonamido)phenyl)amino)pyrimidin-2-yl)amino)-3-methoxyphenyl)piperidin-4-yl)methyl)azetidin-3-carboxamido)-3,3-dimethylbutanoyl)-4-hydroxy-N-(4-(4-methylthiazol-5-yl)benzyl)pyrrolidin-2-carboxamide (Compound 38) (not claimed)

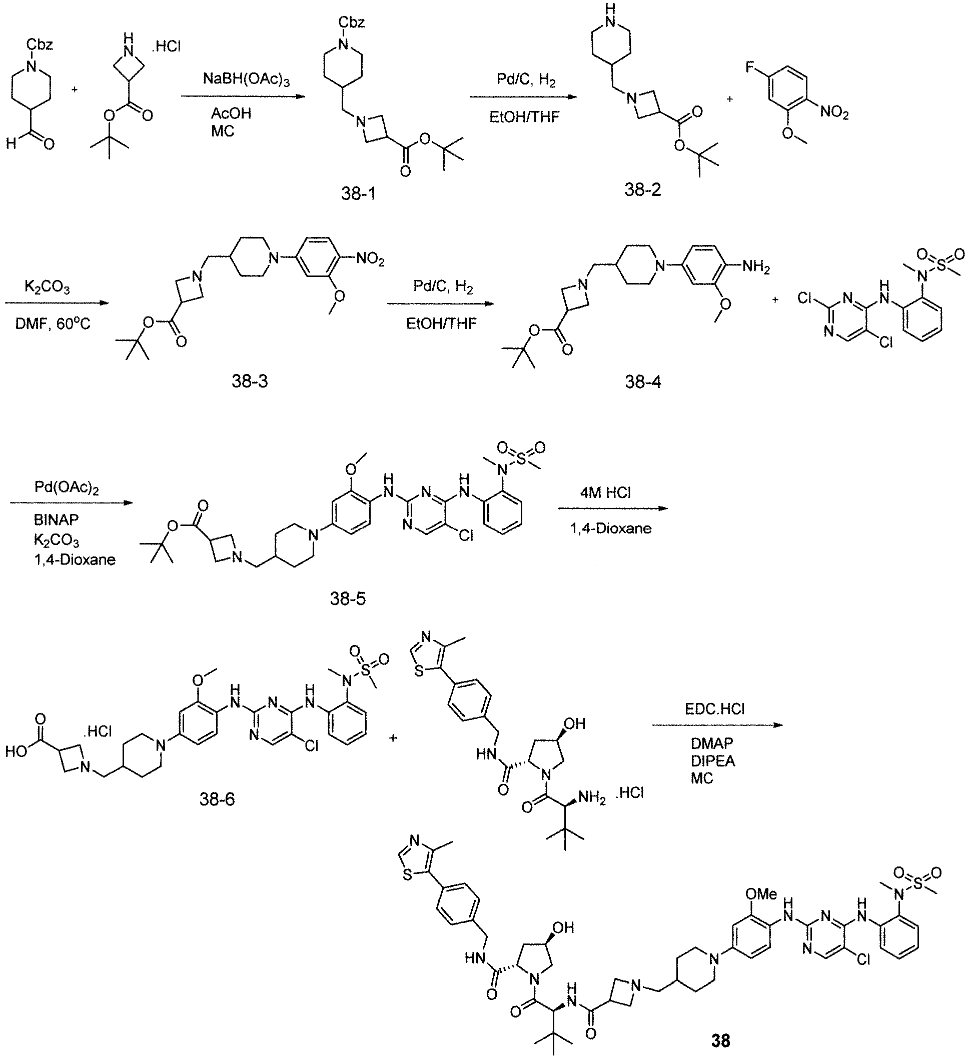

### Step 1: Synthesis of benzyl 4-((3-(tertiary butoxycarbonyl)azetidin-1-yl)methyl)piperidin-1-carboxylate (38-1)

The title compound (640 mg, yield 56%) was obtained using a method similar to the Step 2 synthesis method of Example 2 above with benzyl 4-formylpiperidin-1-carboxylate (732 mg, 2.96 mmol) and tertiary butyl azetidin-3-carboxylate (857 mg, 4.44 mmol) instead of 1-(3-methoxy-4-nitrophenyl)piperidin-4-one and N-Boc-piperazine, respectively.

### Step 2: Synthesis of tertiary butyl 1-(piperidin-4-ylmethyl)azetidin-3-carboxylate (38-2)

The title compound (400 mg, yield 95%) was obtained using a method similar to the Step 4 synthesis method of Example 33 above with benzyl 4-((3-(tertiary butoxycarbonyl)azetidin-1-yl)methyl)piperidin-1-carboxylate (1.3 g, 3.40 mmol) instead of 5-(3-(2-(benzyloxy)ethoxy)azetidin-1-yl)-2-(2,6-dioxopiperidin-3-yl)isoindolin-1,3-dione.

### Step 3: Synthesis of tertiary butyl 1-((1-(3-methoxy-4-nitrophenyl)piperidin-4-yl)methyl)azetidin-3-carboxylate (38-3)

The title compound (510 mg, yield 80%) was obtained using a method similar to the Step 2 synthesis method of Example 1 above with tertiary butyl 1-(piperidin-4-ylmethyl)azetidin-3-carboxylate (400 mg, 1.57 mmol) instead of Boc-piperazine.

### Step 4: Synthesis of tertiary butyl 1-((1-(4-amino-3-methoxyphenyl)piperidin-4-yl)methyl)azetidin-3-carboxylate (38-4)

The title compound (470 mg, yield 99%) was obtained using a method similar to the Step 3 synthesis method of Example 1 above with benzyl tertiary butyl 1-((1-(3-methoxy-4-nitrophenyl)piperidin-4-yl)methyl)azetidin-3-carboxylate (510 mg, 1.26 mmol) instead of tertiary butyl 4-(3-methoxy-4-nitrophenyl)piperazin-1-carboxylate.

### Step 5: Synthesis of tertiary butyl 1-((1-(4-((5-chloro-4-((2-(N-methylmethylsulfonamido)phenyl)amino)pyrimidin-2-yl)amino)-3-methoxyphenyl)piperidin-4-yl)methyl)azetidin-3-carboxylate (38-5)

The title compound (506 mg, yield 59%) was obtained using a method similar to the Step 4 synthesis method of Example 1 above with tertiary butyl 1-((1-(4-amino-3-methoxyphenyl)piperidin-4-yl)methyl)azetidin-3-carboxylate (470 mg, 2.25 mmol) instead of tertiary butyl 4-(4-amino-3-methoxyphenyl)piperazin-1-carboxylate.

### Step 6: Synthesis of 1-((1-(4-((5-chloro-4-((2-(N-methylmethanesulfonamido)phenyl)amino)pyrimidin-2-yl)amino)-3-methoxyphenyl)piperidin-4-yl)methyl)azetidin-3-carboxylic acid hydrochloride (38-6)

The title compound was obtained using a method similar to the Step 2 synthesis method of Example 20 above with tertiary butyl 1-((1-(4-((5-chloro-4-((2-(N-methylmethylsulfonamido)phenyl)amino)pyrimidin-2-yl)amino)-3-methoxyphenyl)piperidin-4-yl)methyl)azetidin-3-carboxylate (100 mg, 0.146 mmol) instead of tertiary butyl 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoate.

### Step 7: Synthesis of Compound 38

The title compound (87 mg, yield 60%) was obtained using a method similar to the Step 1 synthesis method of Example 6 above with 1-((1-(4-((5-chloro-4-((2-(N-methylmethanesulfonamido)phenyl)amino)pyrimidin-2-yl)amino)-3-methoxyphenyl)piperidin-4-yl)methyl)azetidin-3-carboxylic acid hydrochloride (87 mg, 0.14 mmol) instead of 5-bromopentanoic acid.

### Example 39: Synthesis of N-(2-((5-chloro-2-((4-(4-(2-(3-((2-(2,6-dioxopiperidin-3-yl)-1-oxoisoindolin-4-yl)oxy)azetidin-1-yl)ethyl)piperazin-1-yl)-3-fluorophenyl)amino)pyrimidin-4-yl)amino)phenyl)-N-methylmethanesulfonamide (Compound 39)

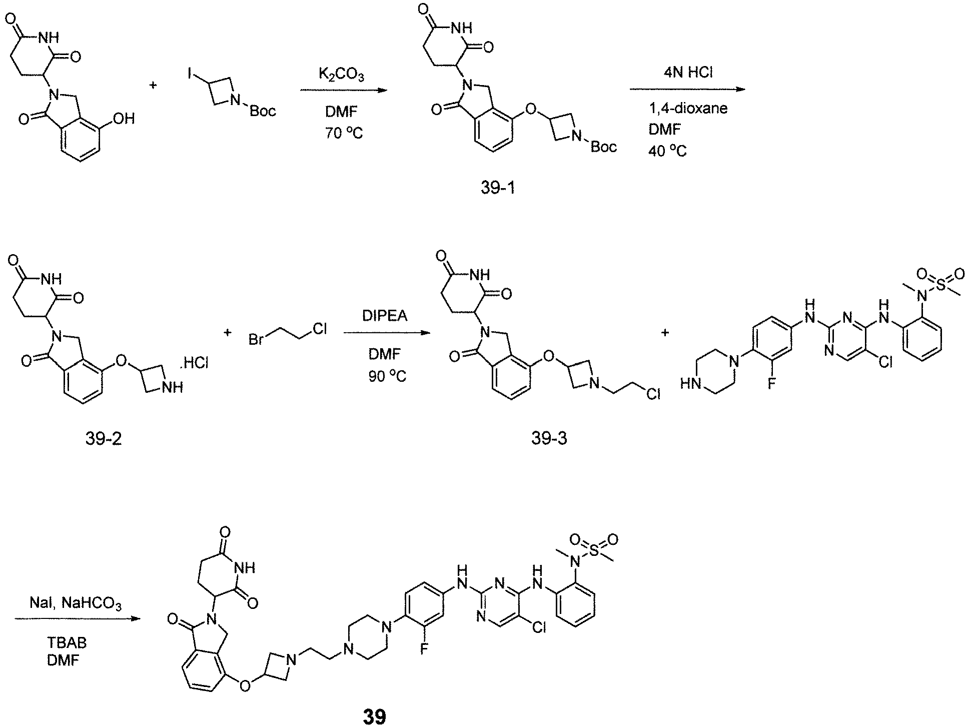

### Step 1: Synthesis of tertiary butyl 3-((2-(2,6-dioxopiperidin-3-yl)-1-oxoisoindolin-4-yl)oxy)azetidin-1-carboxylate (39-1)

A mixture of 3-(4-hydroxy-1-oxoisoindolin-2-yl)piperidin-2,6-dione (297 mg, 1.14 mmol), tertiary butyl 3-iodoazetidin-1-carboxylate (646 mg, 2.28 mmol) and potassium carbonate (315 mg, 2.28 mmol) in N,N-dimethylformamide (3 mL) was stirred at 70 °C. After stirring overnight, tertiary butyl 3-iodoazetidin-1-carboxylate (200 mg) was further added and stirred for 4 hours. A saturated NH₄Cl aqueous solution was added to the reaction mixture, stirred, filtered, washed with water and ethyl acetate, and vacuum dried to obtain the title compound (150 mg, 36%).

### Step 2: Synthesis of 3-(4-(azetidin-3-yloxy)-1-oxoisoindolin-2-yl)piperidin-2,6-dione hydrochloride (39-2)

The title compound was obtained using a method similar to the Step 5 synthesis method of Example 1 above with tertiary butyl 3-((2-(2,6-dioxopiperidin-3-yl)-1-oxoisoindolin-4-yl)oxy)azetidin-1-carboxylate (150 mg, 0.36 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate, and then used in the next reaction.

### Step 3: Synthesis of 3-(4-((1-(2-chloroethyl)azetidin-3-yl)oxy)-1-oxoisoindolin-2-yl)piperidin-2,6-dione (39-3)

To a solution of 3-(4-(azetidin-3-yloxy)-1-oxoisoindolin-2-yl)piperidin-2,6-dione hydrochloride (127 mg, 0.36 mmol) and 1-bromo-2-chloroethane (33 µL, 0.40 mmol) in N,N-dimethylformamide (3.5 mL), diisopropylethylamine (219 µL, 0.29 mmol) was added and stirred at 90 °C. After 4.5 hours, it was concentrated and purified by column to obtain the title compound (70 mg, 51%).

### Step 4: Synthesis of Compound 39

A mixture of 3-(4-((1-(2-chloroethyl)azetidin-3-yl)oxy)-1-oxoisoindolin-2-yl)piperidin-2,6-dione (70 mg, 0.185 mmol), N-(2-((5-chloro-2-((3-fluoro-4-(piperazin-1-yl)phenyl)amino)pyrimidin-4-yl)amino)phenyl)-N-methylmethanesulfonamide (93 mg, 0.185 mmol, sodium iodide (14 mg, 0.093 mmol), sodium hydroxide (31 mg, 0.371 mmol) and tetrabutylammonium bromide (TBAB, 30 mg, 0.093 mmol) in N,N-dimethylformamide (1 mL) was stirred overnight at 80 °C. The mixture was cooled to room temperature, water was added, and a solid was obtained by filtration, and then the title compound (35 mg, 23%) was obtained by column purification.

### Example 40: Synthesis of N-(2-((5-chloro-2-((4-(4-(2-((1-(2-(2,6-dioxopiperidin-3-yl)-1,3-dioxoisoindolin-5-yl)azetidin-3-yl)oxy)ethyl)piperazin-1-yl)-3-fluorophenyl)amino)pyrimidin-4-yl)amino)phenyl)-N-methylmethanesulfonamide (Compound 40)

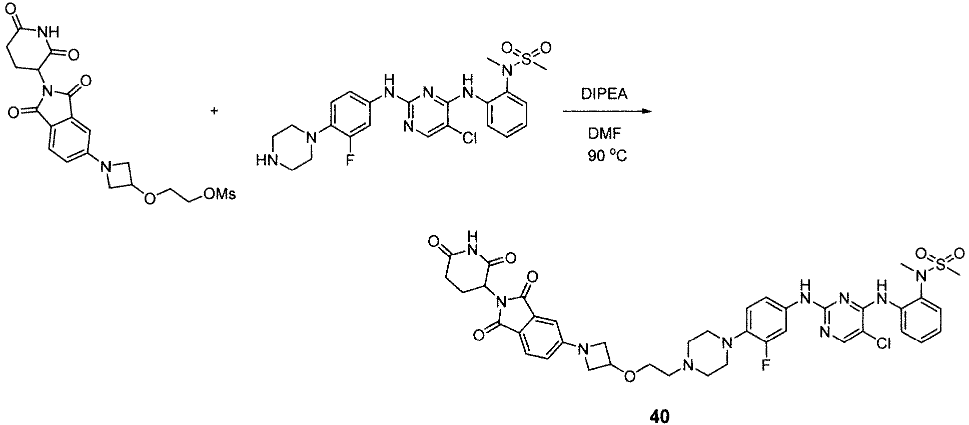

The title compound (57 mg, yield 43%) was obtained using a method similar to the Step 6 synthesis method of Example 1 above with 2-((1-(2-(2,6-dioxopiperidin-3-yl)-1,3-dioxoisoindolin-5yl)azetidin-3-yl)oxy)ethylmethanesulfonate (70 mg, 0.16 mmol) and N-(2-((5-chloro-2-((3-fluoro-4-(piperazin-1-yl)phenyl)amino)pyrimidin-4-yl)amino)phenyl)-N-methylmethylsulfonamide (78 mg, 0.16 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide.

### Example 41: Synthesis of N-(2-((5-chloro-2-((4-(4-((1-((2-(2,6-dioxopiperidin-3-yl)-1,3-dioxoisoindolin-5-yl)methyl)piperidin-4-yl)methyl)piperazin-1-yl)-2-methoxyphenyl)amino)pyrimidin-4-yl)amino)phenyl)-N-methylmethanesulfonamide (Compound 41) (not claimed)

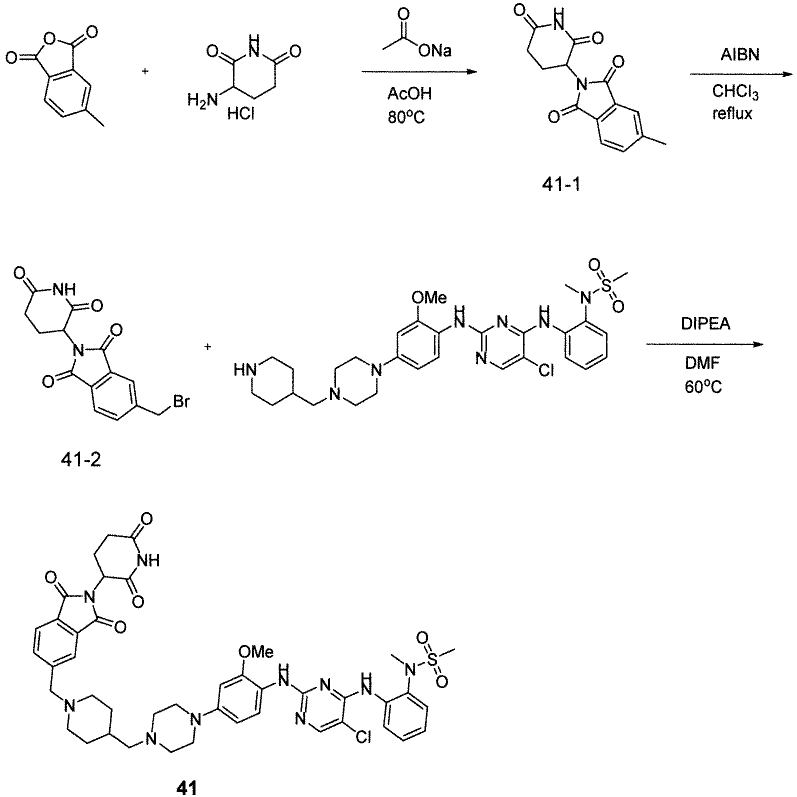

### Step 1: Synthesis of 2-(2,6-dioxopiperidin-3-yl)-5-methylisoindolin-1,3-dione (41-1)

A mixture of 5-methylisofuran-1,3-dione (5.0 g, 30.84 mmol), 3-aminopiperidin-2,6-dione (6.1 g, 37.01 mmol) and sodium acetate (2.5 g, 30.84 mmol) in acetic acid (50 mL) was stirred at 80 °C. After 6 hours, the mixture was cooled to room temperature, water was added, and the mixture was stirred. The precipitated solid was filtered, washed with methyl tertiary butyl ether and dried to obtain the title compound (6.1 g, 72%).

### Step 2: Synthesis of 5-(bromomethyl)-2-(2,6-dioxopiperidin-3-yl)isoindolin-1,3-dione (41-2)

To a solution of 2-(2,6-dioxopiperidin-3-yl)-5-methylisoindolin-1,3-dione (200 mg, 0.74 mmol) and NBS (144 mg, 0.81 mmol) in chloroform (10 mL), AIBN (24 mg, 0.15 mmol) was added and stirred under reflux. After stirring overnight, the mixture was extracted with water, dried over anhydrous magnesium sulfate, and filtered, and the obtained material (140 mg, mixture) was used in the next reaction.

### Step 3: Synthesis of Compound 41

To a solution of 5-(bromomethyl)-2-(2,6-dioxopiperidin-3-yl)isoindolin-1,3-dione (44 mg, 0.125 mmol) and N-(2-((5-chloro-2-((2 N,N of -methoxy-4-(4-(piperidin-4-ylmethyl)piperazin-1-yl)phenyl)amino)pyrimidin-4-yl)amino)phenyl)-N-methylmethanesulfonamide (70 mg, 0.114 mmol) in N,N-dimethylformamide (1 mL), DIPEA (50 µL, 0.285 mmol) was added and stirred at 50°C overnight. After cooling to room temperature, water was added and filtered to obtain a solid, which was purified by column to obtain the title compound (48 mg, 48%).

### Example 42: Synthesis of N-(2-((5-chloro-2-((4-(4-(4-((2-(2,6-dioxopiperidin-3-yl)-1,3-dioxoisoindolin-5-yl)methyl)piperazin-1-yl)piperidin-1-yl)-3-fluorophenyl)amino)pyrimidin-4-yl)amino)phenyl)-N-methylmethanesulfonamide (Compound 42)

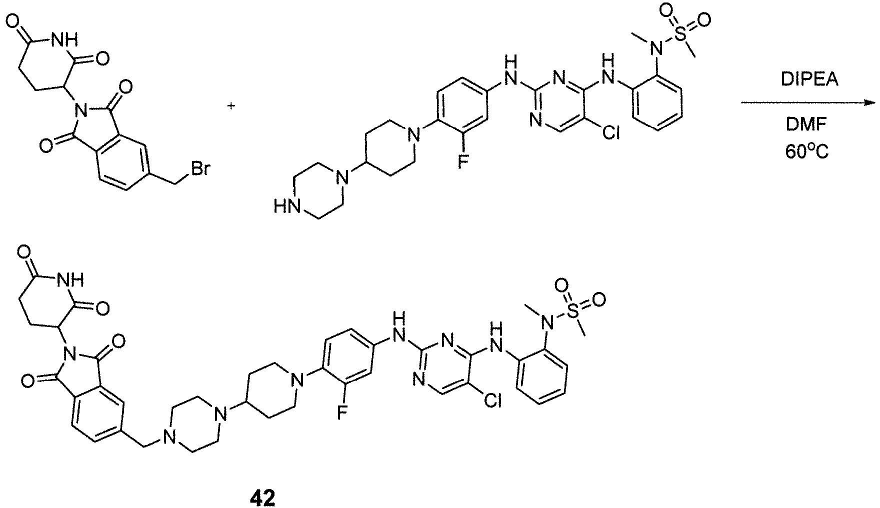

The title compound (56 mg, yield 55%) was obtained using a method similar to the Step 3 synthesis method of Example 41 above with N-(2-((5-chloro-2-((3-fluoro-4-(4-(piperazin-1-yl)piperidin-1-yl)phenyl)amino)pyrimidin-4-yl)amino)phenyl)-N-methylmethanesulfonamide (70 mg, 0.119 mmol) instead of N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide.

### Example 43: Synthesis of N-(2-((5-chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-3-fluorophenyl)amino)pyrimidin-4-yl)amino)phenyl)-N-methylmethanesulfonamide (Compound 43)

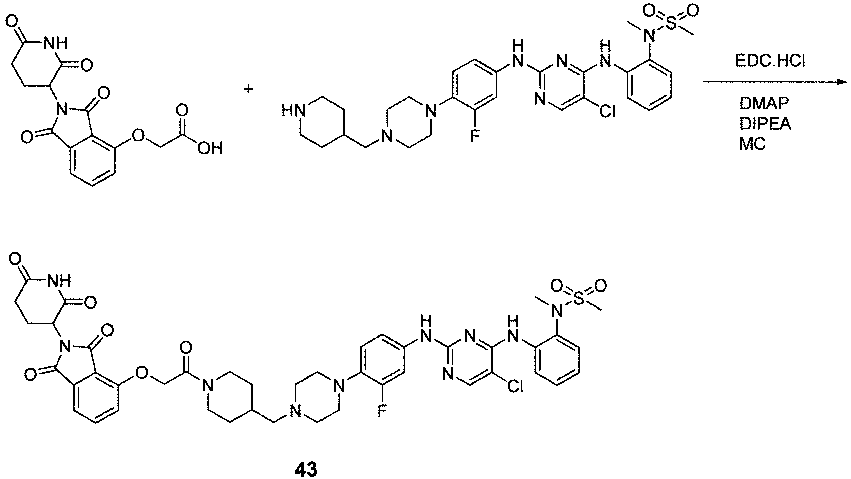

The title compound (30 mg, yield 28%) was obtained using a method similar to the Step 1 synthesis method of Example 6 above with 2-((2-(2,6-dioxopiperidin-3-yl)-1,3-dioxoisoindolin-4-yl)oxy)acetic acid (50 mg, 0.151 mmol) and N-(2-((5-chloro-2-((3-fluoro-4-(4-(piperidin-4-ylmethyl)piperazin-1-yl)phenyl)amino)pyrimidin-4-yl)amino)phenyl)-N-methylmethanesulfonamide (70 mg, 0.116 mmol) instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentanoic acid, respectively.

### Example 44: Synthesis of N-(2-((5-chloro-2-((4-(4-(4-(((2S)-1-(2-(2,6-dioxopiperidin-3-yl)-1,3-dioxoisoindolin-5-yl)pyrrolidin-2-yl)methyl)piperazin-1-yl)piperidin-1-yl)-2-methoxyphenyl)amino)pyrimidin-4-yl)amino)phenyl)-N-methylmethanesulfonamide (Compound 44)

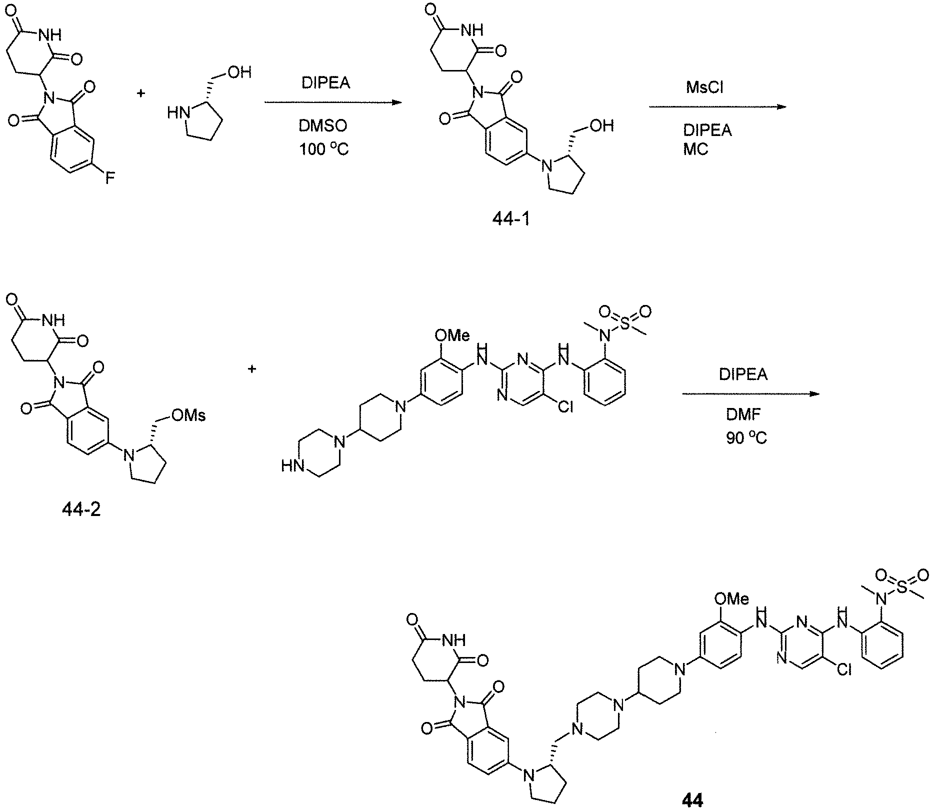

### Step 1: Synthesis of 2-(2,6-dioxopiperidin-3-yl)-5-((S)-2-(hydroxymethyl)pyrrolidin-1-yl)isoindolin-1,3-dione (44-1)

The title compound (120 mg, yield 93%) was obtained using a method similar to the Step 3 synthesis method of Example 33 above with (S)-pyrrolidin-2-ylmethanol (44 mg, 0.43 mmol) instead of 3-(2-(benzyloxy)ethoxy)axetidine.

### Step 2: Synthesis of ((2S)-1-(2-(2,6-dioxopiperidin-3-yl)-1,3-dioxoindolin-5-yl)pyrrolidin-2-yl)methyl methanesulfonate (44-2)

The title compound (416 mg, yield 38%) was obtained using a method similar to the Step 5 synthesis method of Example 33 above with 2-(2,6-dioxopiperidin-3-yl)-5-((S)-2-(hydroxymethyl)pyrrolidin-1-yl)isoindolin-1,3-dione (1.0 g, 2.53 mmol) instead of 2-(2,6-dioxopiperidin-3-yl)-5-(3-(2-hydroxyethoxy)azetidin-1-yl)isoindolin-1,3-dione.

### Step 3: Synthesis of Compound 44

The title compound (18 mg, yield 17%) was obtained using a method similar to the Step 6 synthesis method of Example 1 above with ((2S)-1-(2-(2,6-dioxopiperidin-3-yl)-1,3-dioxoindolin-5-yl)pyrrolidin-2-yl)methyl methanesulfonate (50 mg, 0.115 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (69 mg, 0.115 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide.

### Example 45: Synthesis of N-(2-((5-chloro-2-((4-(4-(4-(2-((2-(2,6-dioxopiperidin-3-yl)-1,3-dioxoisoindolin-4-yl)amino)ethyl)piperazin-1-yl)piperidin-1-yl)-2-methoxyphenyl)amino)pyrimidin-4-yl)amino)phenyl)-N-methylmethanesulfonamide (Compound 45)

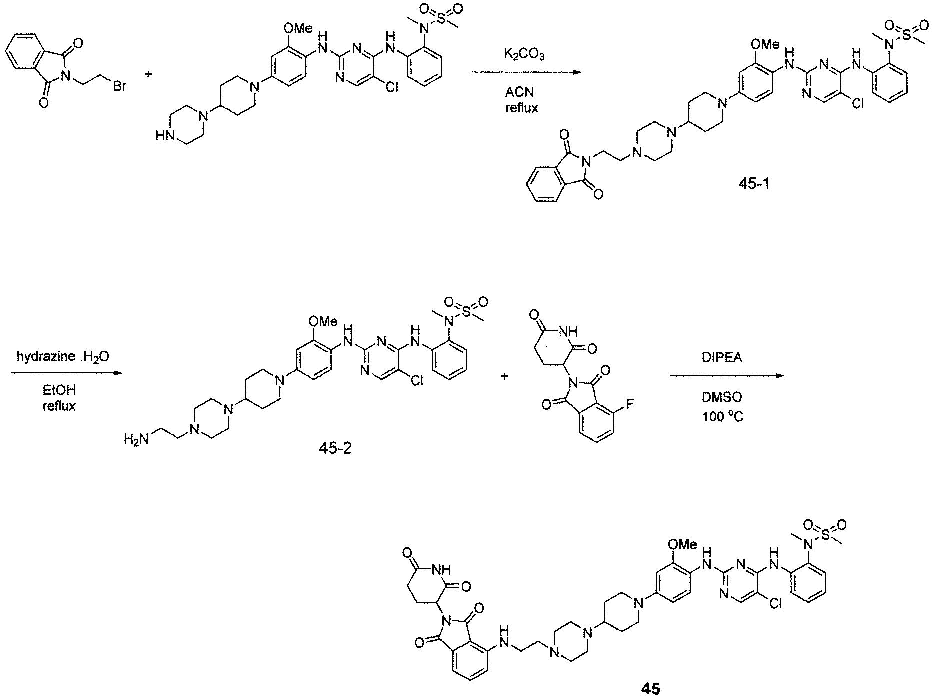

### Step 1: Synthesis of N-(2-((5-chloro-2-((4-(4-(4-(2-(1,3-dioxoindolin-2-yl)ethyl)piperazin-1-yl)piperidin-1-yl)-2-methoxyphenyl)amino)pyrimidin-4-yl)amino)phenyl)-N-methylmethanesulfonamide (45-1)

A mixture of 2-(2-bromoethyl)isoindolin-1,3-dione (205 mg, 0.81 mmol), N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (500 mg, 0.81 mmol) and potassium carbonate (280 mg, 2.03) in acetonitrile (8 mL) was stirred overnight at 70 °C. The reaction mixture was concentrated and extracted with water and dichloromethane. The organic layer was dried over anhydrous sodium sulfate, filtered, and concentrated, and the residue was purified by column chromatography to obtain the title compound (350 mg, yield 57%).

### Step 2: Synthesis of N-(2-((2-((4-(4-(4-(2-aminoethyl)piperazin-1-yl)piperidin-1-yl)-2-methoxyphenyl)amino)-5-chloropyrimidin-4-yl)amino)phenyl)-N-methylmethanesulfonamide (45-2)

To a suspension of N-(2-((5-chloro-2-((4-(4-(4-(2-(1,3-dioxoindolin-2-yl)ethyl)piperazin-1-yl)piperidin-1-yl)-2-methoxyphenyl)amino)pyrimidin-4-yl)amino)phenyl)-N-methylmethanesulfonamide (350 mg, 0.45 mmol) in ethanol (4.5 mL), hydrazine hydrate (57 mg, 1.13 mmol) was added and stirred at 80 °C for 4 hours. The reaction mixture was concentrated and extracted with water and dichloromethane. The organic layer was dried over anhydrous sodium sulfate, filtered, and concentrated to quantitatively obtain the title compound, which was used in the next reaction.

### Step 3: Synthesis of Compound 45

The title compound (35 mg, yield 36%) was obtained using a method similar to the Step 3 synthesis method of Example 33 above with 2-(2,6-dioxopiperidin-3-yl)-4-fluoroisoindolin-1,3-dione (30 mg, 0.109 mmol) and N-(2-((2-((4-(4-(4-(2-aminoethyl)piperazin-1-yl)piperidin-1-yl)-2-methoxyphenyl)amino)-5-chloropyrimidin-4-yl)amino)phenyl)-N-methylmethanesulfonamide (71 mg, 0.109 mmol) instead of 2-(2,6-dioxopiperidin-3-yl)-5-fluoroisoindolin-1,3-dione and 3-(2-(benzyloxy)ethoxy)azetidine.

### Example 46: Synthesis of N-(2-((5-chloro-2-((4-(4-((1-(2-((2-(2,6-dioxopiperidin-3-yl)-1,3-dioxoisoindolin-4-yl)oxy)ethyl)piperidin-4-yl)methyl)piperazin-1-yl)-3-fluorophenyl)amino)pyrimidin-4-yl)amino)phenyl)-N-methylmethanesulfonamide (Compound 46)

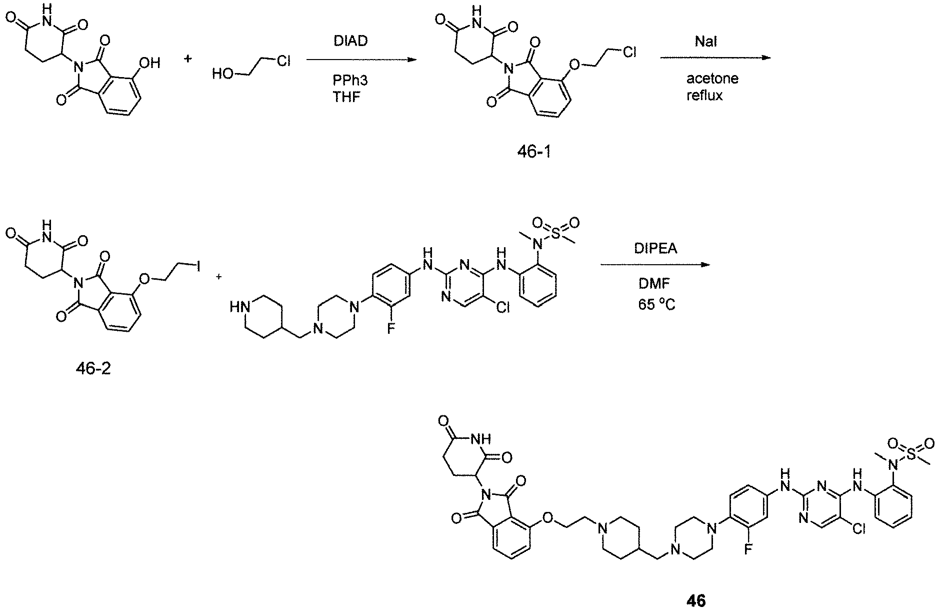

### Step 1: Synthesis of 4-(2-chloroethoxy)-2-(2,6-dioxopiperidin-3-yl)isoindolin-1,3-dione (46-1)

The title compound (323 mg, yield 94%) was obtained using a method similar to the Step 1 synthesis method of Example 3 above with 2-chloroethan-1-ol (110 µL, 1.64 mmol) instead of 4-bromobutan-1-ol.

### Step 2: Synthesis of 2-(2,6-dioxopiperidin-3-yl)-4-(2-iodoethoxy)isoindolin-1,3-dione (46-2)

The title compound (341 mg, yield 78%) was obtained using a method similar to the Step 2 synthesis method of Example 8 above with 4-(2-chloroethoxy)-2-(2,6-dioxopiperidin-3-yl)isoindolin-1,3-dione (320 mg, 1.01 mmol) instead of 2-(2-(2-chloroethoxy)ethoxy)-N-(2-(2,6-dioxopiperidin-3-yl)-1,3-dioxoisoindolin-4-yl)acetamide.

### Step 3: Synthesis of Compound 46

The title compound (36 mg, yield 35%) was obtained using a method similar to the Step 6 synthesis method of Example 1 above with 2-(2,6-dioxopiperidin-3-yl)-4-(2-iodoethoxy)isoindolin-1,3-dione (54 mg, 0.128 mmol) and N-(2-((5-chloro-2-((3-fluoro-4-(4-(piperidin-4-ylmethyl)piperazin-1-yl)phenyl)amino)pyrimidin-4-yl)amino)phenyl)-N-methylmethanesulfonamide (70 mg, 0.116 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 47: Synthesis of 2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1-oxoisoindolin-5-yl)acetamide (Compound 47)

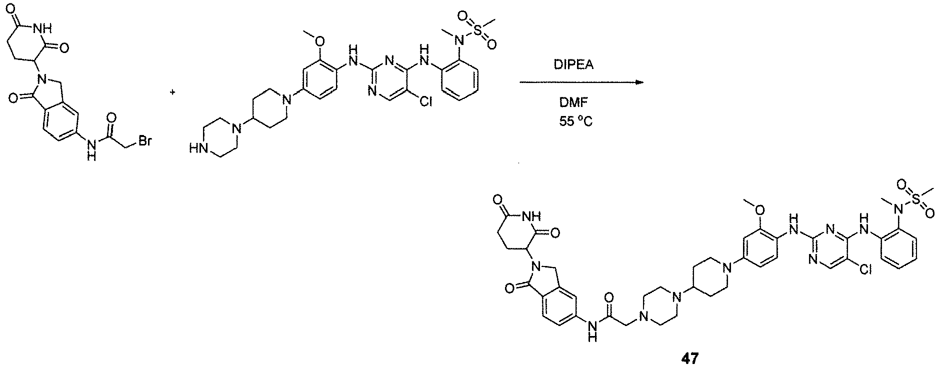

The title compound (63 mg, yield 61%) was obtained using a method similar to the Step 6 synthesis method of Example 1 above with 2-bromo-N-(2-(2,6-dioxopiperidin-3-yl)-1-oxoisoindolin-5-yl)acetamide (44 mg, 0.116 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (70 mg, 0.116 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 48: Synthesis of 2-(4-((4-(4-((5-chloro-4-((2-(N-methylmethylsulfonamido)phenyl)amino)pyrimidin-2-yl)amino)-2-fluorophenyl)piperazin-1-yl)methyl)piperidin-1-yl)-N-(2-(2,6-dioxopiperidin-3-yl)-1-oxoisoindolin-4-yl)acetamide (Compound 48)

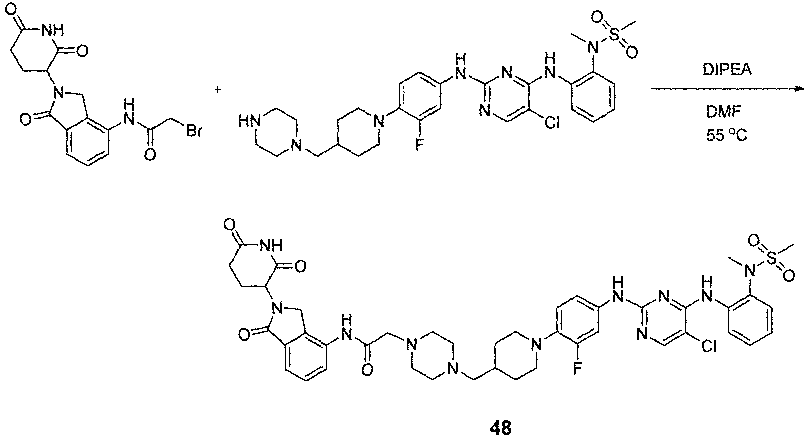

The title compound (63 mg, yield 60%) was obtained using a method similar to the Step 6 synthesis method of Example 1 above with 2-bromo-N-(2-(2,6-dioxopiperidin-3-yl)-1-oxoisoindolin-4-yl)acetamide (44 mg, 0.116 mmol) and N-(2-((5-chloro-2-((3-fluoro-4-(4-(piperazin-1-ylmethyl)piperidin-1-yl)phenyl)amino)pyrimidin-4-yl)amino)phenyl)-N-methylmethanesulfonamide (70 mg, 0.116 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 49: Synthesis of 1-((1-(4-((5-chloro-4-((2-(N-methylmethylsulfonamido)phenyl)amino)pyrimidin-2-yl)amino)-3-methoxyphenyl)piperidin-4-yl)methyl)-N-(2-(2,6-dioxopiperidin-3-yl)-1-oxoisoindolin-4-yl)piperidin-4-carboxamide (Compound 49) (not claimed)

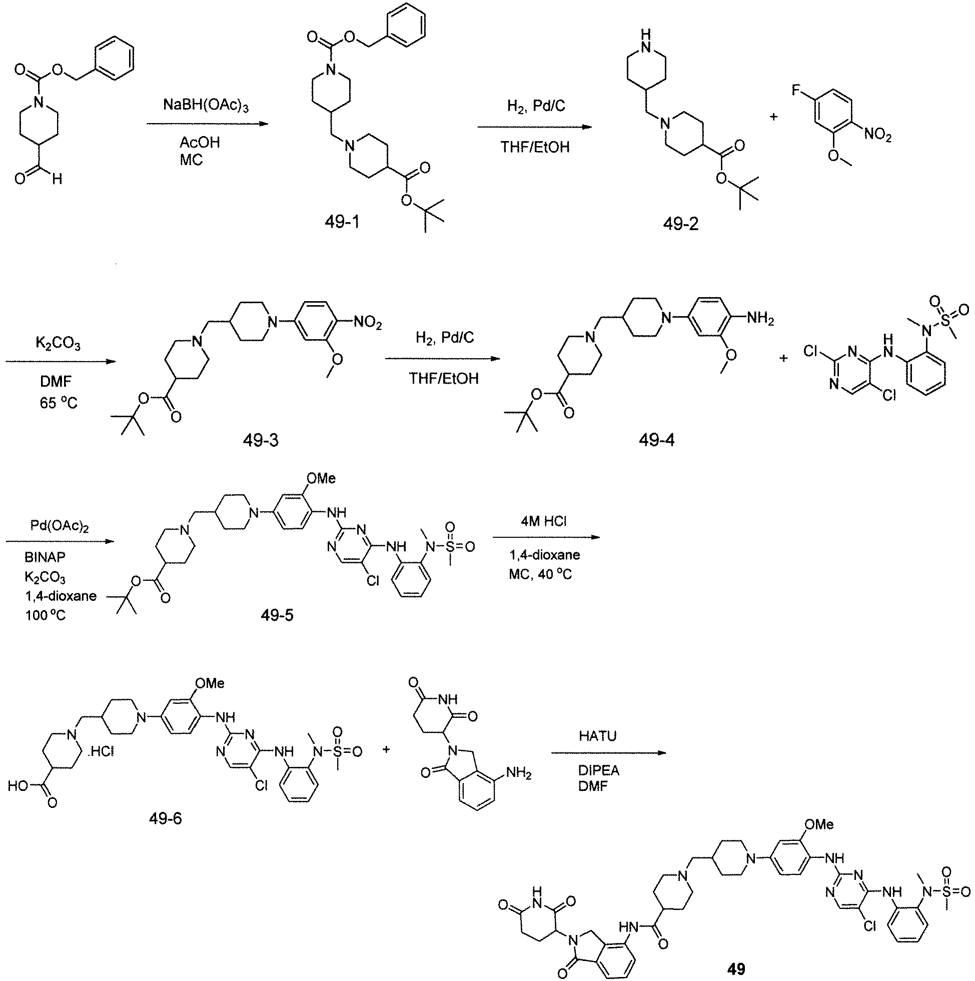

### Step 1: Synthesis of benzyl 4-((4-(tertiary butoxycarbonyl)piperidin-1-yl)methyl)piperidin-1-carboxylate (49-1)

The title compound (700 mg, yield 43%) was obtained using a method similar to the Step 2 synthesis method of Example 2 above with benzyl 4-formylpiperidin-1-carboxylate (1.0 g, 4.04 mmol) and tertiary butyl piperidin-4-carboxylate (1.1 g, 6.07 mmol) instead of 1-(3-methoxy-4-nitrophenyl)piperidin-4-one and N-Boc-piperazine, respectively.

### Step 2: Synthesis of tertiary butyl 1-(piperidin-4-ylmethyl)piperidin-4-carboxylate (49-2)

The title compound (470 mg, yield 99%) was obtained using a method similar to the Step 4 synthesis method of Example 33 above with benzyl 4-((4-(tertiary butoxycarbonyl)piperidin-1-yl)methyl)piperidin-1-carboxylate (700 mg, 1.68 mmol) instead of 5-(3-(2-(benzyloxy)ethoxy)azetidin-1-yl)-2-(2,6-dioxopiperidin-3-yl)isoindolin-1,3-dione.

### Step 3: Synthesis of tertiary butyl 1-((1-(3-methoxy-4-nitrophenyl)piperidin-4-yl)methyl)piperidin-4-carboxylate (49-3)

The title compound (240 mg, yield 12%) was obtained using a method similar to the Step 2 synthesis method of Example 1 above with tertiary butyl 1-(piperidin-4-ylmethyl)piperidin-4-carboxylate (1.3 g, 4.60 mmol) instead of Boc-piperazine.

### Step 4: Synthesis of tertiary butyl 1-((1-(4-amino-3-methoxyphenyl)piperidin-4-yl)methyl)piperidin-4-carboxylate (49-4)

The title compound (223 mg, yield 99%) was obtained using a method similar to the Step 3 synthesis method of Example 1 above with tertiary butyl 1-((1-(3-methoxy-4-nitrophenyl)piperidin-4-yl)methyl)piperidin-4-carboxylate (240 mg, 0.55 mmol) instead of tertiary butyl 4-(3-methoxy-4-nitrophenyl)piperazin-1-carboxylate.

### Step 5: Synthesis of tertiary butyl 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylate (49-5)

The title compound (95 mg, yield 24%) was obtained using a method similar to the Step 4 synthesis method of Example 1 above with tertiary butyl 1-((1-(4-amino-3-methoxyphenyl)piperidin-4-yl)methyl)piperidin-4-carboxylate (223 mg, 0.55 mmol) instead of tertiary butyl 4-(4-amino-3-methoxyphenyl)piperazin-1-carboxylate.

### Step 6: Synthesis of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride (49-6)

The title compound was obtained using a method similar to the Step 2 synthesis method of Example 20 above with tertiary butyl 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylate (100 mg, 0.14 mmol) instead of 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoic acid hydrochloride, and then used in the next reaction.

### Step 7: Synthesis of Compound 49

To a mixture of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride (97 mg, 0.14 mmol), 3-(4-amino-1-oxoisoindolin-2-yl)piperidin-2,6-dione (36 mg, 0.86 mmol) and HATU (107 mg, 0.28 mmol) in N,N-dimethylformamide (1 mL), diisopropylethylamine (98 µL, 0.56 mmol) was added and stirred for 2 days. The reaction mixture was diluted with water and extracted with dichloromethane. The organic layer was dried over anhydrous magnesium sulfate, filtered, concentrated, and then purified with a silica gel column to obtain the title compound (21 mg, 17%).

### Example 50: Synthesis of 2-(9-(4-((5-chloro-4-((2-(N-methylmethylsulfonamido)phenyl)amino)pyrimidin-2-yl)amino)-3-methoxyphenyl)-3,9-diazaspiro[5.5]undecan-3-yl)-N-(2-(2,6-dioxopiperidin-3-yl)-1-oxoisoindolin-4-yl)acetamide (Compound 50)

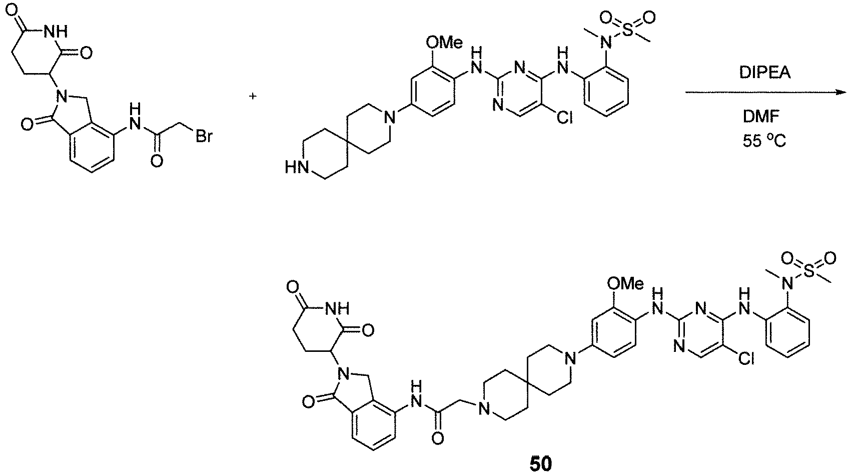

The title compound (90 mg, yield 64%) was obtained using a method similar to the Step 6 synthesis method of Example 1 above with 2-bromo-N-(2-(2,6-dioxopiperidin-3-yl)-1-oxoisoindolin-4-yl)acetamide (60 mg, 0.158 mmol) and N-(2-((5-chloro-2-((2-methoxy-4-(3,9-diazaspiro[5.5]undecan-3-yl)phenyl)amino)pyrimidin-4-yl)amino)phenyl)-N-methylmethanesulfonamide (93 mg, 0.158 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 51: Synthesis of N-(2-((5-chloro-2-((4-(4-(4-(2-(1-(2-(2,6-dioxopiperidin-3-yl)-1,3-dioxoisoindolin-5-yl)piperidin-4-yl)ethyl)piperazin-1-yl)piperidin-1-yl)-2-methoxyphenyl)amino)pyrimidin-4-yl)amino)phenyl)-N-methylmethanesulfonamide (Compound 51)

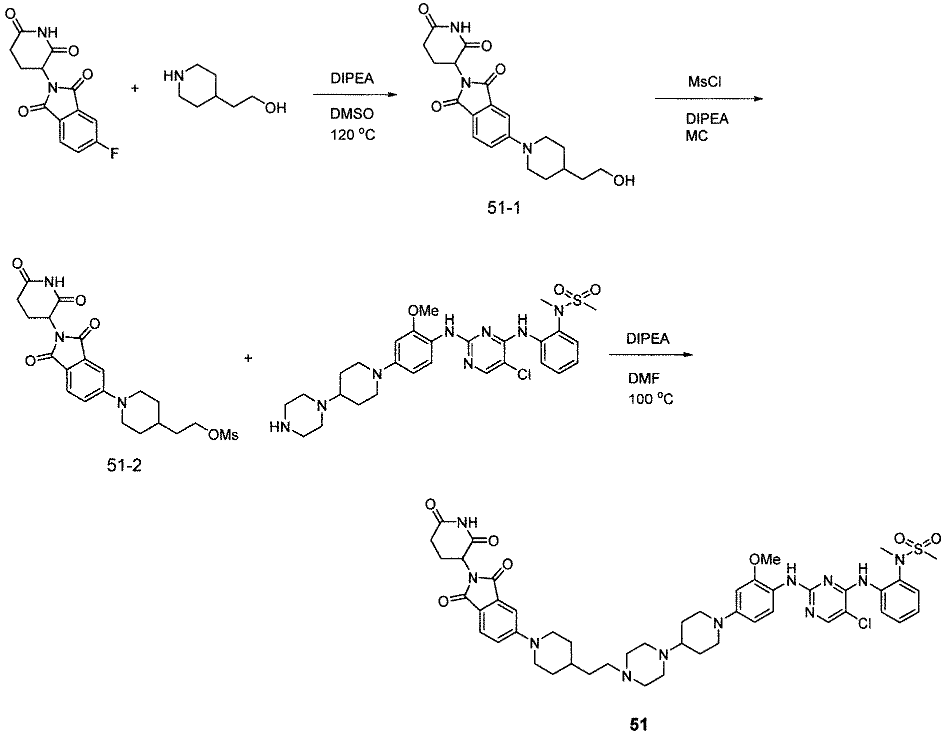

### Step 1: Synthesis of 2-(2,6-dioxopiperidin-3-yl)-5-(4-(2-hydroxyethyl)piperidin-1-yl)isoindolin-1,3-dione (51-1)

The title compound (0.99 g, yield 89%) was obtained using a method similar to the Step 3 synthesis method of Example 33 above with 2-(piperidin-4-yl)ethan-1-ol (412 mg, 3.19 mmol) instead of 3-(2-(benzyloxy)ethoxy)azetidine.

### Step 2: Synthesis of 2-(1-(2-(2,6-dioxopiperidin-3-yl)-1,3-dioxoisoindolin-5-yl)piperidin-4-yl)ethyl methanesulfonate (51-2)

The title compound (510 mg, yield 43%) was obtained using a method similar to the Step 2 synthesis method of Example 33 above with 2-(2,6-dioxopiperidin-3-yl)-5-(4-(2-hydroxyethyl)piperidin-1-yl)isoindolin-1,3-dione (0.99 g, 2.57 mmol) instead of 2-(2,6-dioxopiperidin-3-yl)-5-(3-(2-hydroxyethoxy)azetidin-1-yl)isoindolin-1,3-dione.

### Step 3: Synthesis of Compound 51

The title compound (30 mg, yield 29%) was obtained using a method similar to the Step 6 synthesis method of Example 1 above with 2-(1-(2-(2,6-dioxopiperidin-3-yl)-1,3-dioxoisoindolin-5-yl)piperidin-4-yl)ethyl methanesulfonate (50 mg, 0.108 mmol) and N-(2-((5-chloro-2-((2-methoxy-4-(4-piperazin-1-yl)piperidin-1-yl)phenyl)amino)pyrimidin-4-yl)amino)phenyl)-N-methylmethylsulfonamide (65 mg, 0.108 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 52: Synthesis of N-(2-((5-chloro-2-((4-(4-(4-(2-((2-(2,6-dioxopiperidin-3-yl)-1,3-dioxoisoindolin-4-yl)oxy)ethyl)piperazin-1-yl)piperidin-1-yl)-2-methoxyphenyl)amino)pyrimidin-4-yl)amino)phenyl)-N-methylmethanesulfonamide (Compound 52)

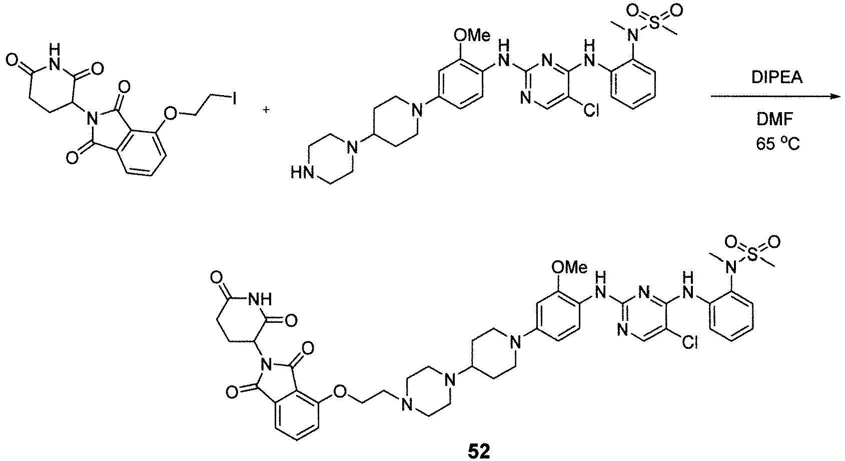

The title compound (54 mg, yield 52%) was obtained using a method similar to the Step 6 synthesis method of Example 1 above with 2-(2,6-dioxopiperidin-3-yl)-4-(2-iodoethoxy)isoindolin-1,3-dione (50 mg, 0.116 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (70 mg, 0.116 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 53: Synthesis of N-(2-((5-chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-2-methoxyphenyl)amino)pyrimidin-4-yl)amino)phenyl)-N-methylmethanesulfonamide (Compound 53)

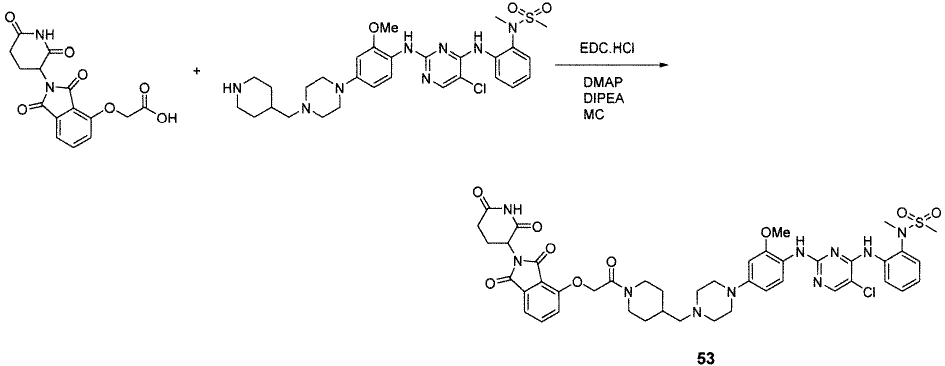

The title compound (42 mg, yield 41%) was obtained using a method similar to the Step 1 synthesis method of Example 6 above with 2-((2-(2,6-dioxopiperidin-3-yl)-1,3-dioxoisoindolin-4-yl)oxy)acetic acid (42 mg, 0.125 mmol) and N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (70 mg, 0.114 mmol) instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentanoic acid, respectively.

### Example 54: Synthesis of N-(2-((5-chloro-2-((4-(4-((1-(3-(2-(2,6-dioxopiperidin-3-yl)-1,3-dioxoisoindolin-4-yl)propanoyl)piperidin-4-yl)methyl)piperazin-1-yl)-2-methoxyphenyl)amino)pyrimidin-4-yl)amino)phenyl)-N-methylmethanesulfonamide (Compound 54)

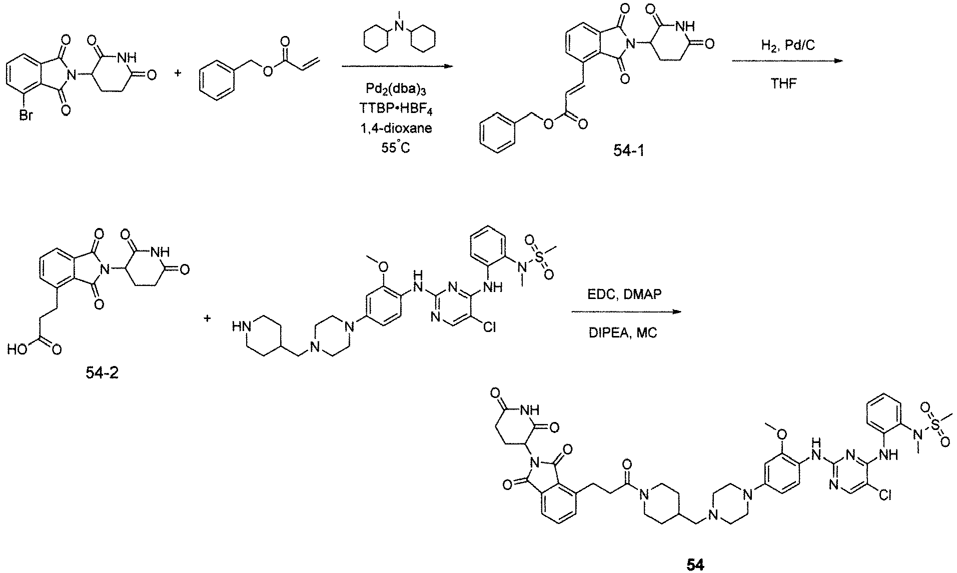

### Step 1: Synthesis of benzyl (E)-3-(2-(2,6-dioxopiperidin-3-yl)-1,3-dioxoisoindolin-4-yl)acrylate (54-1)

A mixture of tri-tertiary butylphosphonium tetrafluoroborate (TTBP.HBF4, 110 mg, 0.38 mmol), Pd₂(dba)₃ (163 mg, 0.18 mmol) and N-cyclohexyl-N-cyclohexanamine (0.27 mL, 1.28 mmol) in 1,4-dioxane (6 mL) was purged with argon gas and stirred for 30 minutes. (4-bromo-2-(2,6-dioxopiperidin-3-yl)isoindolin-1,3-dione (0.3 g, 0.89 mmol) and benzyl acrylate (0.3 mL, 1.98 mmol) were added and stirred at 55 °C for 12 hours. The reaction mixture was filtered through celite, concentrated, and purified by column chromatography to obtain the title compound (368 mg, yield 99%).

### Step 2: Synthesis of 3-(2-(2,6-dioxopiperidin-3-yl)-1,3-dioxoisoindolin-4-yl)propanoic acid (54-2)

To a solution of benzyl (E)-3-(2-(2,6-dioxopiperidin-3-yl)-1,3-dioxoisoindolin-4-yl)acrylate (137 mg, 0.33 mmol) in tetrahydrofuran (5 mL), 10% Pd/C (27 mg) was added and and replaced with hydrogen gas. After stirring overnight, the mixture was filtered through celite and concentrated to obtain the title compound (105 mg, yield 96%).

### Step 3: Synthesis of Compound 54

The title compound (105 mg, yield 75%) was obtained using a method similar to the Step 1 synthesis method of Example 6 above with 3-(2-(2,6-dioxopiperidin-3-yl)-1,3-dioxoisoindolin-4-yl)propanoic acid (50 mg, 0.15 mmol) and N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (92 mg, 0.15 mmol) instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentanoic acid, respectively.

### Example 55: Synthesis of (2R,4R)-1-((S)-2-(2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)acetamido)-2-cyclopropylacetyl)-4-hydroxy-N-(4-(4-methylthiazol-5-yl)benzyl)pyrrolidin-2-carboxamide (Compound 55) (not claimed)

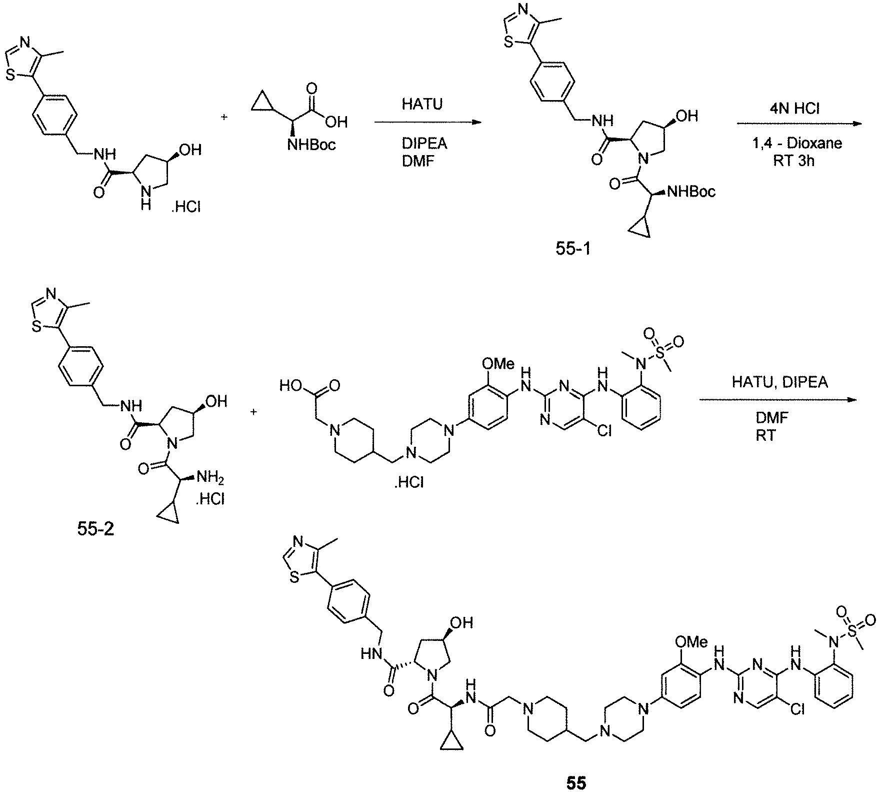

### Step 1: Synthesis of tertiary butyl ((S)-1-cyclopropyl-2-((2R,4R)-4-hydroxy-2-((4-(4-methylthiazol-5-yl)benzyl)carbamoyl)pyrrolidin-1-yl)-2-oxoethyl)carbamate (55-1)

The title compound (330 mg, 57%) was obtained using a method similar to the Step 7 synthesis method of Example 49 above with (S)-2-((tertiary butoxycarbonyl)amino)-2-cyclopropylacetic acid (243 mg, 1.13 mmol) and (2R,4R)-4-hydroxy-N-(4-(4-methylthiazol-5-yl)benzyl)pyrrolidin-2-carboxamide (400 mg, 1.13 mmol) instead of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride and 3-(4-amino-1-oxoisoindolin-2-yl)piperidin-2,6-dione, respectively.

### Step 2: Synthesis of (2R,4R)-1-((S)-2-amino-2-cyclopropylacetyl)-4-hydroxy-N-(4-(4-methylthiazol-5-yl)benzyl)pyrrolidin-2-carboximide hydrochloride (55-2)

The title compound was obtained using a method similar to the Step 2 synthesis method of Example 20 above with tertiary butyl ((S)-1-cyclopropyl-2-((2R,4R)-4-hydroxy-2-((4-(4-methylthiazol-5-yl)benzyl)carbamoyl)pyrrolidin-1-yl)-2-oxoethyl)carbamate (324 mg, 0.63 mmol) instead of 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoic acid hydrochloride, and then used in the next reaction.

### Step 3: Synthesis of Compound 55

The title compound (73 mg, 51%) was obtained using a method similar to the Step 7 synthesis method of Example 49 above with (2R,4R)-1-((S)-2-amino-2-cyclopropylacetyl)-4-hydroxy-N-(4-(4-methylthiazol-5-yl)benzyl)pyrrolidin-2-carboximide hydrochloride (60 mg, 0.134 mmol) and 2-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)acetic acid hydrochloride (100 mg, 0.134 mmol) instead of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride and 3-(4-amino-1-oxoisoindolin-2-yl)piperidin-2,6-dione, respectively.

### Example 56: Synthesis of (2R,4R)-1-((2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)acetyl)-L-valyl)-4-hydroxy-N-(4-(4-methylthiazol-5-yl)benzyl)pyrrolidin-2-carboxamide (Compound 56)

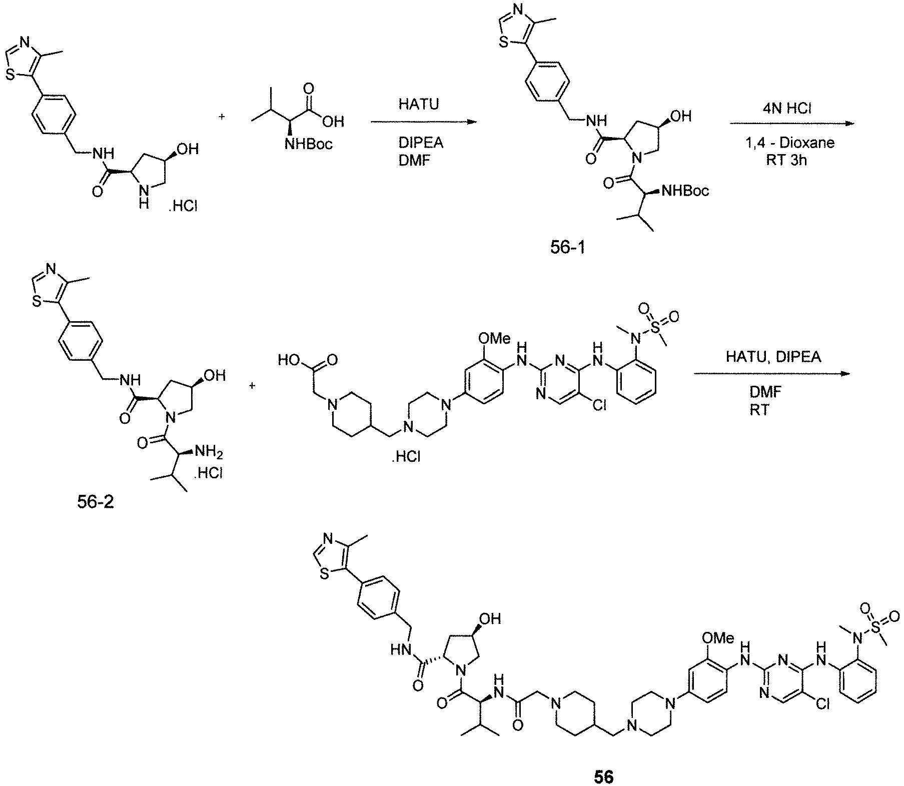

### Step 1: Synthesis of tertiary butyl ((S)-1-((2R,4R)-4-hydroxy-2-((4-(4-methylthiazol-5-yl)benzyl)carbamoyl)pyrrolidin-1-yl)-3-methyl-1-oxobutan-2-yl)carbamate (56-1)

The title compound (232 mg, 79%) was obtained using a method similar to the Step 7 synthesis method of Example 49 above with (tertiary butoxycarbonyl)-L-valine (122 mg, 0.565 mmol) and (2R,4R)-4-hydroxy-N-(4-(4-methylthiazol-5-yl)benzyl)pyrrolidin-2-carboxamide (200 mg, 0.565 mmol) instead of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride and 3-(4-amino-1-oxoisoindolin-2-yl)piperidin-2,6-dione, respectively.

### Step 2: Synthesis of (2R,4R)-1-(L-valyl)-4-hydroxy-N-(4-(4-methylthiazol-5-yl)benzyl)pyrrolidin-2-carboxamide hydrochloride (56-2)

The title compound was obtained using a method similar to the Step 2 synthesis method of Example 20 above with tertiary butyl ((S)-1-((2R,4R)-4-hydroxy-2-((4-(4-methylthiazol-5-yl)benzyl)carbamoyl)pyrrolidin-1-yl)-3-methyl-1-oxobutan-2-yl)carbamate (460 mg, 0.89 mmol) instead of 3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanoic acid hydrochloride, and then used in the next reaction.

### Step 3: Synthesis of Compound 56

The title compound (63 mg, 44%) was obtained using a method similar to the Step 7 synthesis method of Example 49 above with (2R,4R)-1-(L-valyl)-4-hydroxy-N-(4-(4-methylthiazol-5-yl)benzyl)pyrrolidin-2-carboxamide hydrochloride (61 mg, 0.134 mmol) and 2-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)acetic acid hydrochloride (100 mg, 0.134 mmol) instead of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride and 3-(4-amino-1-oxoisoindolin-2-yl)piperidin-2,6-dione, respectively.

### Example 57: Synthesis of N-(2-((5-chloro-2-((4-(4-((1-(2-((2-(2,6-dioxopiperidin-3-yl)-1,3-dioxoisoindolin-4-yl)oxy)ethyl)piperidin-4-yl)methyl)piperazin-1-yl)-2-methoxyphenyl)amino)pyrimidin-4-yl)amino)phenyl)-N-methylmethanesulfonamide (Compound 57) (not claimed)

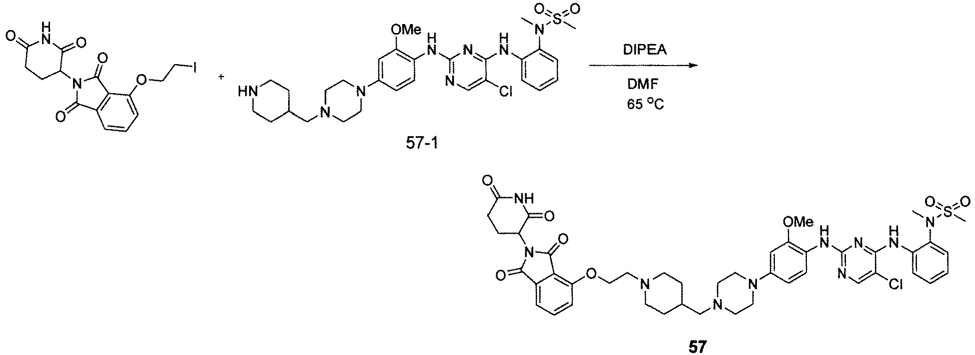

The title compound (30 mg, yield 40%) was obtained using a method similar to the Step 6 synthesis method of Example 1 above with 2-(2,6-dioxopiperidin-3-yl)-4-(2-iodoethoxy)isoindolin-1,3-dione (34 mg, 0.079 mmol) and N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (50 mg, 0.079 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide, respectively.

### Example 58: Synthesis of N-(2-((5-chloro-2-((4-(4-(4-(2-((2-(2,6-dioxopiperidin-3-yl)-1,3-dioxoisoindolin-5-yl)amino)ethyl)piperazin-1-yl)piperidin-1-yl)-2-methoxyphenyl)amino)pyrimidin-4-yl)amino)phenyl)-N-methylmethanesulfonamide (Compound 58)

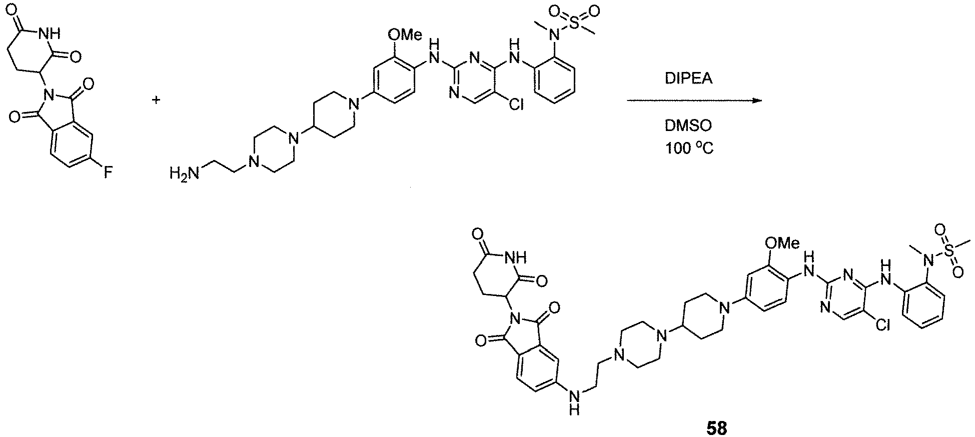

The title compound (20 mg, yield 20%) was obtained using a method similar to the Step 3 synthesis method of Example 33 above with N-(2-((2-((4-(4-(4-(2-aminoethyl)piperazin-1-yl)piperidin-1-yl)-2-methoxyphenyl)amino)-5-chloropyrimidin-4-yl)amino)phenyl)-N-methylmethanesulfonamide (71 mg, 0.109 mmol) instead of 3-(2-(benzyloxy)ethoxy)azetidine.

### Example 59: Synthesis of N-(2-((5-chloro-2-((4-(4-((1-((1-(2-(2,6-dioxopiperidin-3-yl)-1,3-dioxoisoindolin-5-yl)piperidin-4-yl)methyl)piperidin-4-yl)methyl)piperazin-1-yl)-2-methoxyphenyl)amino)pyrimidin-4-yl)amino)phenyl)-N-methylmethanesulfonamide (Compound 59)

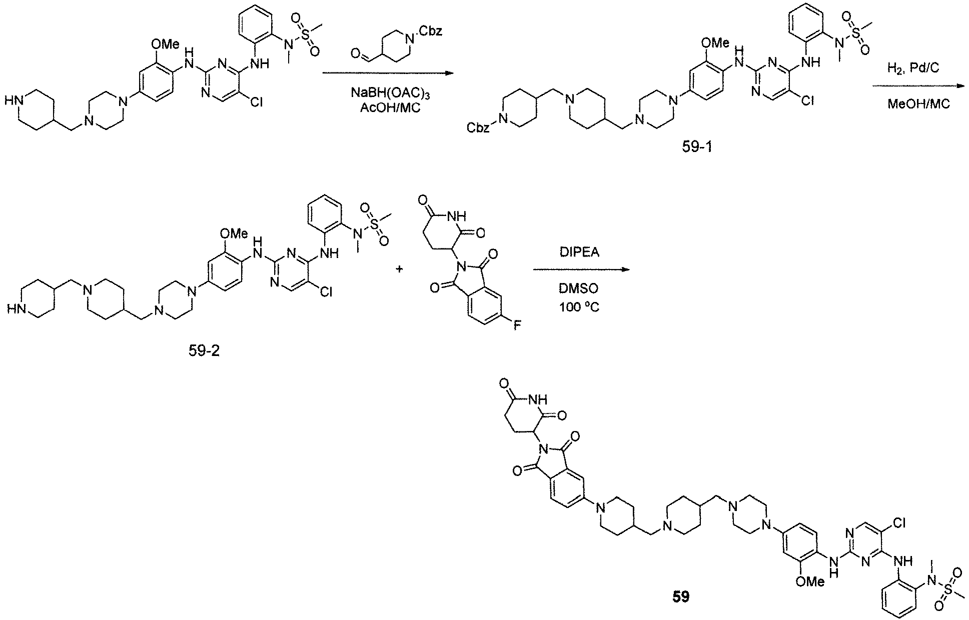

### Step 1: Synthesis of benzyl 4-((4-((4-(4-((5-chloro-4-((2-N-methylmethanesulfonamido)phenyl)amino)pyrimidin-2-yl)amino)-3-methoxyphenyl)piperazin-1-yl)methyl)piperidin-1-yl)methyl)piperidin-1-carboxylate (59-1)

The title compound (340 mg, yield 97%) was obtained using a method similar to the Step 2 synthesis method of Example 2 above with benzyl 4-formylpiperidin-1-carboxylate (121 mg, 0.49 mmol) and N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (250 mg, 0.41 mmol) instead of 1-(3-methoxy-4-nitrophenyl)piperidin-4-one and N-Boc-piperazine, respectively.

### Step 2: Synthesis of N-(2-((5-chloro-2-((2-methoxy-4-(4-((1-(piperidin-4-ylmethyl)piperidin-4-yl)methyl)piperazin-1-yl)phenyl)amino)pyrimidin-4-yl)amino)phenyl)-N-methylmethanesulfonamide (59-2)

The title compound (247 mg, yield 79%) was obtained using a method similar to the Step 4 synthesis method of Example 33 above with benzyl 4-((4-((4-(4-((5-chloro-4-((2-N-methylmethanesulfonamido)phenyl)amino)pyrimidin-2-yl)amino)-3-methoxyphenyl)piperazin-1-yl)methyl)piperidin-1-yl)methyl)piperidin-1-carboxylate (340 mg, 0.40 mmol) instead of 5-(3-(2-(benzyloxy)ethoxy)azetidin-1-yl)-2-(2,6-dioxopiperidin-3-yl)isoindolin-1,3-dione.

### Step 3: Synthesis of Compound 59

The title compound (73 mg, yield 53%) was obtained using a method similar to the Step 3 synthesis method of Example 33 above with N-(2-((5-chloro-2-((2-methoxy-4-(4-((1-(piperidin-4-ylmethyl)piperidin-4-yl)methyl)piperazin-1-yl)phenyl)amino)pyrimidin-4-yl)amino)phenyl)-N-methylmethanesulfonamide (100 mg, 0.14 mmol) instead of 3-(2-(benzyloxy)ethoxy)azetidine.

### Example 60: Synthesis of N-(2-((5-chloro-2-((4-(4-(4-((1-(2-(2,6-dioxopiperidin-3-yl)-1,3-dioxoisoindolin-5-yl)azetidin-3-yl)methyl)piperazin-1-yl)piperidin-1-yl)-2-methoxyphenyl)amino)pyrimidin-4-yl)amino)phenyl)-N-methylmethanesulfonamide (Compound 60)

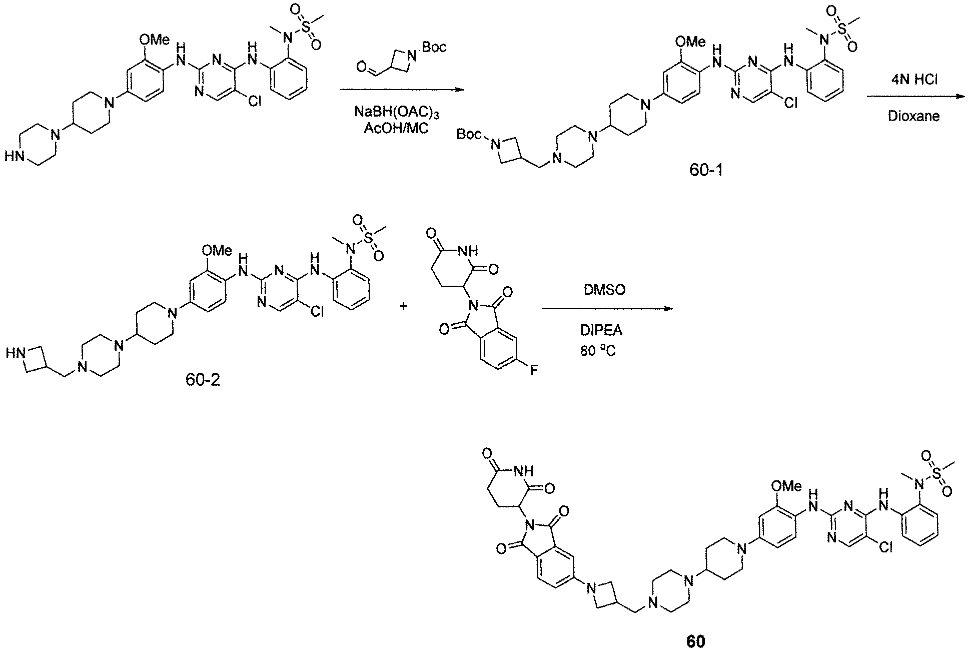

### Step 1: Synthesis of tertiary butyl 3-((4-(1-(4-((5-chloro-4-((2-N-methylmethanesulfonamido)phenyl)amino)pyrimidin-2-yl)amino)-3-methoxyphenyl)piperidin-4-yl)piperazin-1-yl)methyl)azetidin-1-carboxylate (60-1)

The title compound (220 mg, yield >100%) was obtained using a method similar to the Step 2 synthesis method of Example 2 above with tertiary butyl 3-formylazetidin-1-carboxylate (54 mg, 0.293 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (150 mg, 0.244 mmol) instead of 1-(3-methoxy-4-nitrophenyl)piperidin-4-one and N-Boc-piperazine, respectively.

### Step 2: Synthesis of N-(2-((2-((4-(4-(4-(azetidin-3-ylmethyl)piperazin-1-yl)piperidin-1-yl)-2-methoxyphenyl)amino)-5-chloropyrimidin-4-yl)amino)phenyl)-N-methylmethanesulfonamide (60-2)

The title compound (250 mg, yield >100%) was obtained using a method similar to the Step 5 synthesis method of Example 1 above with tertiary butyl 3-((4-(1-(4-((5-chloro-4-((2-N-methylmethanesulfonamido)phenyl)amino)pyrimidin-2-yl)amino)-3-methoxyphenyl)piperidin-4-yl)piperazin-1-yl)methyl)azetidin-1-carboxylate (220 mg, 0.244 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 3: Synthesis of Compound 60

The title compound (28 mg, yield 16%) was obtained using a method similar to the Step 3 synthesis method of Example 33 above with N-(2-((2-((4-(4-(4-(azetidin-3-ylmethyl)piperazin-1-yl)piperidin-1-yl)-2-methoxyphenyl)amino)-5-chloropyrimidin-4-yl)amino)phenyl)-N-methylmethanesulfonamide (150 mg, 0.189 mmol) instead of 3-(2-(benzyloxy)ethoxy)azetidine.

### Example 61: Synthesis of N-(2-((5-chloro-2-((4-(4-((1-((1-(2-(2,6-dioxopiperidin-3-yl)-1,3-dioxoisoindolin-5-yl)azetidin-3-yl)methyl)piperidin-4-yl)methyl)piperazin-1-yl)-2-methoxyphenyl)amino)pyrimidin-4-yl)amino)phenyl)-N-methylmethanesulfonamide (Compound 61)

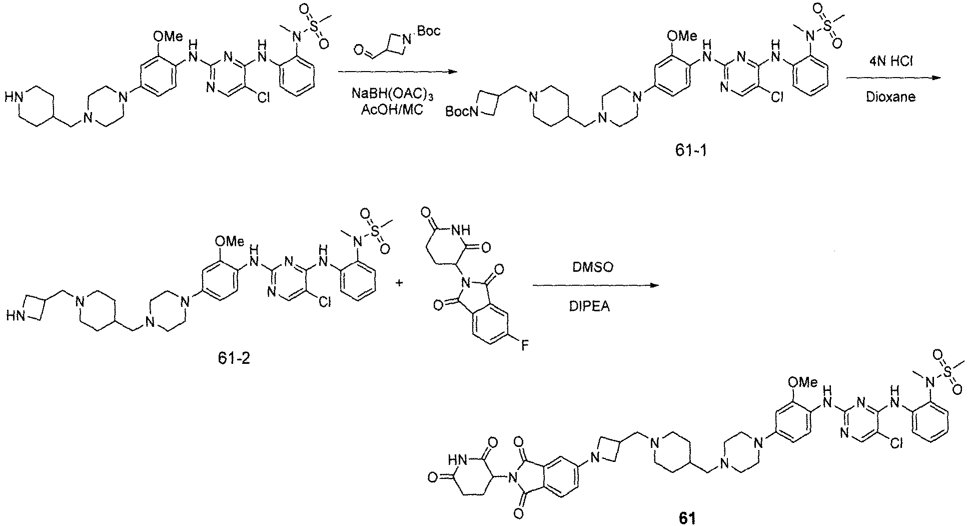

### Step 1: Synthesis of tertiary butyl 3-((4-((4-(4-((5-chloro-4-((2-N-methylmethanesulfonamido)phenyl)amino)pyrimidin-2-yl)amino)-3-methoxyphenyl)piperazin-1-yl)methyl)piperidin-1-yl)methyl)azetidin-1-carboxylate (61-1)

The title compound (220 mg, yield >100%) was obtained using a method similar to the Step 2 synthesis method of Example 2 above with tertiary butyl 3-formylazetidin-1-carboxylate (54 mg, 0.293 mmol) and N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (150 mg, 0.244 mmol) instead of 1-(3-methoxy-4-nitrophenyl)piperidin-4-one and N-Boc-piperazine.

### Step 2: Synthesis of N-(2-((2-((4-(4-((1-(azetidin-3-ylmethyl)piperidin-4-yl)methyl)piperazin-1-yl)-2-methoxyphenyl)amino)-5-chloropyrimidin-4-yl)amino)phenyl)-N-methylmethanesulfonamide (61-2)

The title compound (260 mg, yield > 100%) was obtained using a method similar to the Step 5 synthesis method of Example 1 above with tertiary butyl 3-((4-((4-(4-((5-chloro-4-((2-N-methylmethanesulfonamido)phenyl)amino)pyrimidin-2-yl)amino)-3-methoxyphenyl)piperazin-1-yl)methyl)piperidin-1-yl)methyl)azetidin-1-carboxylate (220 mg, 0.244 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 3: Synthesis of Compound 61

The title compound (30 mg, yield 17%) was obtained using a method similar to the Step 3 synthesis method of Example 33 above with N-(2-((2-((4-(4-((1-(azetidin-3-ylmethyl)piperidin-4-yl)methyl)piperazin-1-yl)-2-methoxyphenyl)amino)-5-chloropyrimidin-4-yl)amino)phenyl)-N-methylmethanesulfonamide (150 mg, 0.189 mmol) instead of 3-(2-(benzyloxy)ethoxy)azetidine.

### Example 62: Synthesis of N-(2-((5-chloro-2-((4-(4-((4-(((2-(2,6-dioxopiperidin-3-yl)-1,3-dioxoisoindolin-4-yl)amino)methyl)piperidin-1-yl)methyl)piperidin-1-yl)-2-methoxyphenyl)amino)pyrimidin-4-yl)amino)phenyl)-N-methylmethanesulfonamide (Compound 62)

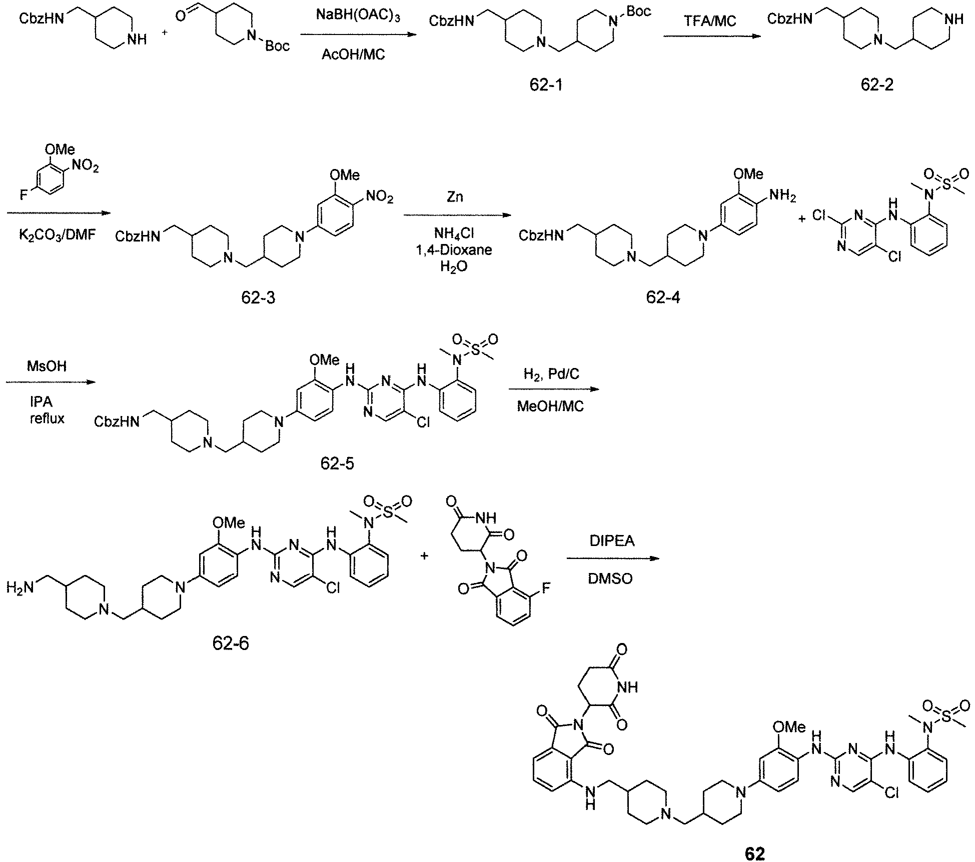

### Step 1: Synthesis of tertiary butyl 4-((4-((((benzyloxy)carbonyl)amino)methyl)piperidin-1-yl)methyl)piperidin-1-carboxylate (62-1)

The title compound (1.5 g, yield 80%) was obtained using a method similar to the Step 2 synthesis method of Example 2 above with tertiary butyl 4-formylpiperidin-1-carboxylate (945 mg, 4.43 mmol) and benzyl (piperidin-4-ylmethyl)carbamate (1.0 g, 4.03 mmol) instead of 1-(3-methoxy-4-nitrophenyl)piperidin-4-one and N-Boc-piperazine, respectively.

### Step 2: Synthesis of benzyl ((1-(piperidin-4-ylmethyl)piperidin-4-yl)methyl)carbamate (62-2)

The title compound (900 mg, yield 81%) was obtained using a method similar to the Step 5 synthesis method of Example 1 above with tertiary butyl 4-((4-((((benzyloxy)carbonyl)amino)methyl)piperidin-1-yl)methyl)piperidin-1-carboxylate (1.5 g, 3.37 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 3: Synthesis of benzyl ((1-((1-(3-methoxy-4-nitrophenyl)piperidin-4-yl)methyl)piperidin-4-yl)methyl)carbamate (62-3)

The title compound (540 mg, yield 94%) was obtained using a method similar to the Step 2 synthesis method of Example 1 above with benzyl ((1-(piperidin-4-ylmethyl)piperidin-4-yl)methyl)carbamate (400 mg, 1.16 mmol) instead of Boc-piperazine.

### Step 4: Synthesis of benzyl ((1-((1-(4-amino-3-methoxyphenyl)piperidin-4-yl)methyl)piperidin-4-yl)methyl)carbamate (62-4)

The title compound (480 mg, yield 95%) was obtained using a method similar to the Step 2 synthesis method of Example 25 above with benzyl ((1-((1-(3-methoxy-4-nitrophenyl)piperidin-4-yl)methyl)piperidin-4-yl)methyl)carbamate (540 mg, 1.09 mmol) instead of benzyl 4-((4-(3-methoxy-4-nitrophenyl)piperazin-1-yl)methyl)piperidin-1-carboxylate.

### Step 5: Synthesis of benzyl ((1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-yl)methyl)carbamate (62-5)

The title compound (480 mg, yield 60%) was obtained using a method similar to the Step 3 synthesis method of Example 35 above with benzyl ((1-((1-(4-amino-3-methoxyphenyl)piperidin-4-yl)methyl)piperidin-4-yl)methyl)carbamate (480 mg, 1.03 mmol) instead of benzyl 4-((4-(4-amino-3-chlorophenyl)piperazin-1-yl)methyl)piperidin-1-carboxylate (420 mg, 0.95 mmol).

### Step 6: Synthesis of N-(2-((2-((4-(4-((4-(aminomethyl)piperidin-1-yl)methyl)piperidin-1-yl)-2-methoxyphenyl)amino)-5-chloropyrimidin-4-yl)amino)phenyl)-N-methylmethanesulfonamide (62-6)

The title compound (395 mg, yield 99%) was obtained using a method similar to the Step 4 synthesis method of Example 33 above with benzyl ((1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-yl)methyl)carbamate (480 mg, 0.62 mmol) instead of 5-(3-(2-(benzyloxy)ethoxy)azetidin-1-yl)-2-(2,6-dioxopiperidin-3-yl)isoindolin-1,3-dione.

### Step 7: Synthesis of Compound 62

The title compound (48 mg, yield 17%) was obtained using a method similar to the Step 3 synthesis method of Example 33 above with 2-(2,6-dioxopiperidin-3-yl)-4-fluoroisoindolin-1,3-dione (129 mg, 0.47 mmol) and N-(2-((2-((4-(4-((4-(aminomethyl)piperidin-1-yl)methyl)piperidin-1-yl)-2-methoxyphenyl)amino)-5-chloropyrimidin-4-yl)amino)phenyl)-N-methylmethanesulfonamide (200 mg, 0.31 mmol) instead of 2-(2,6-dioxopiperidin-3-yl)-5-fluoroisoindolin-1,3-dione and 3-(2-(benzyloxy)ethoxy)azetidine, respectively.

### Example 63: Synthesis of N-(2-((5-chloro-2-((4-(4-((4-(((2-(2,6-dioxopiperidin-3-yl)-1,3-dioxoisoindolin-5-yl)amino)methyl)piperidin-1-yl)methyl)piperidin-1-yl)-2-methoxyphenyl)amino)pyrimidin-4-yl)amino)phenyl)-N-methylmethanesulfonamide (Compound 63)

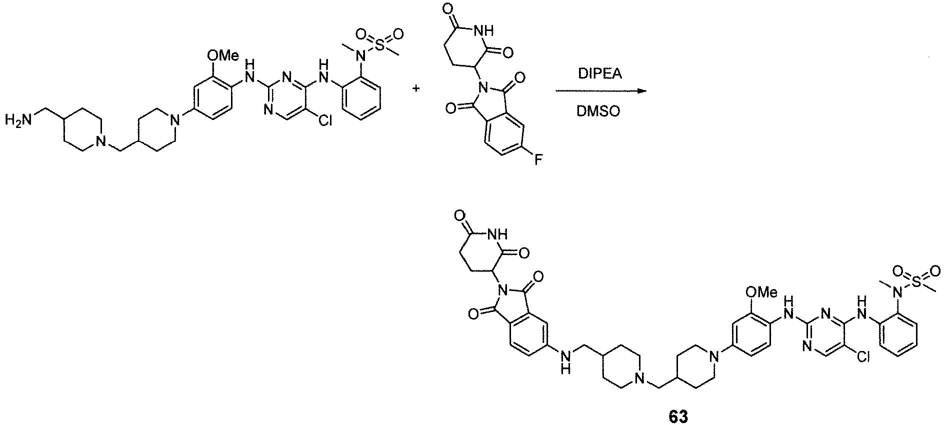

The title compound (65 mg, yield 47%) was obtained using a method similar to the Step 3 synthesis method of Example 33 above with N-(2-((2-((4-(4-((4-(aminomethyl)piperidin-1-yl)methyl)piperidin-1-yl)-2-methoxyphenyl)amino)-5-chloropyrimidin-4-yl)amino)phenyl)-N-methylmethanesulfonamide (100 mg, 0.16 mmol) instead of 3-(2-(benzyloxy)ethoxy)azetidine.

### Example 64: Synthesis of N-(2-((5-chloro-2-((4-(4-((1-(3-(2-(2,6-dioxopiperidin-3-yl)-1,3-dioxoisoindolin-5-yl)propanoyl)piperidin-4-yl)methyl)piperazin-1-yl)-2-methoxyphenyl)amino)pyrimidin-4-yl)amino)phenyl)-N-methylmethanesulfonamide (Compound 64)

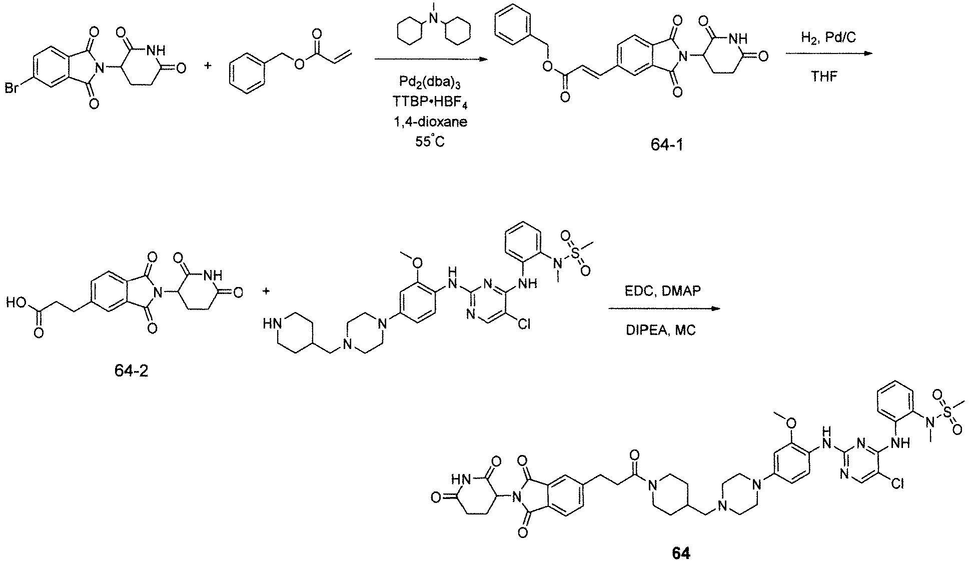

### Step 1: Synthesis of benzyl (E)-3-(2-(2,6-dioxopiperidin-3-yl)-1,3-dioxoisoindolin-5-yl)acrylate (64-1)

The title compound (120 mg, yield 96%) was obtained using a method similar to the Step 1 synthesis method of Example 54 above with (5-bromo-2-(2,6-dioxopiperidin-3-yl)isoindolin-1,3-dione (100 mg, 0.30 mmol) instead of (4-bromo-2-(2,6-dioxopiperidin-3-yl)isoindolin-1,3-dione.

### Step 2: Synthesis of 3-(2-(2,6-dioxopiperidin-3-yl)-1,3-dioxoisoindolin-5-yl)propanoic acid (64-2)

The title compound (60 mg, yield 63%) was obtained using a method similar to the Step 2 synthesis method of Example 54 above with benzyl (E)-3-(2-(2,6-dioxopiperidin-3-yl)-1,3-dioxoisoindolin-5-yl)acrylate (120 mg, 0.29 mmol) instead of benzyl (E)-3-(2-(2,6-dioxopiperidin-3-yl)-1,3-dioxoisoindolin-4-yl)acrylate.

### Step 3: Synthesis of Compound 64

The title compound (75 mg, yield 67%) was obtained using a method similar to the Step 1 synthesis method of Example 6 above with 3-(2-(2,6-dioxopiperidin-3-yl)-1,3-dioxoisoindolin-5-yl)propanoic acid (40 mg, 0.12 mmol) and N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (74 mg, 0.12 mmol) instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentanoic acid.

### Example 65: Synthesis of N-(2-((5-chloro-2-((4-(4-((1-((2-(2,6-dioxopiperidin-3-yl)-1,3-dioxoisoindolin-4-yl)glycyl)piperidin-4-yl)methyl)piperazin-1-yl)-2-methoxyphenyl)amino)pyrimidin-4-yl)amino)phenyl)-N-methylmethanesulfonamide (Compound 65)

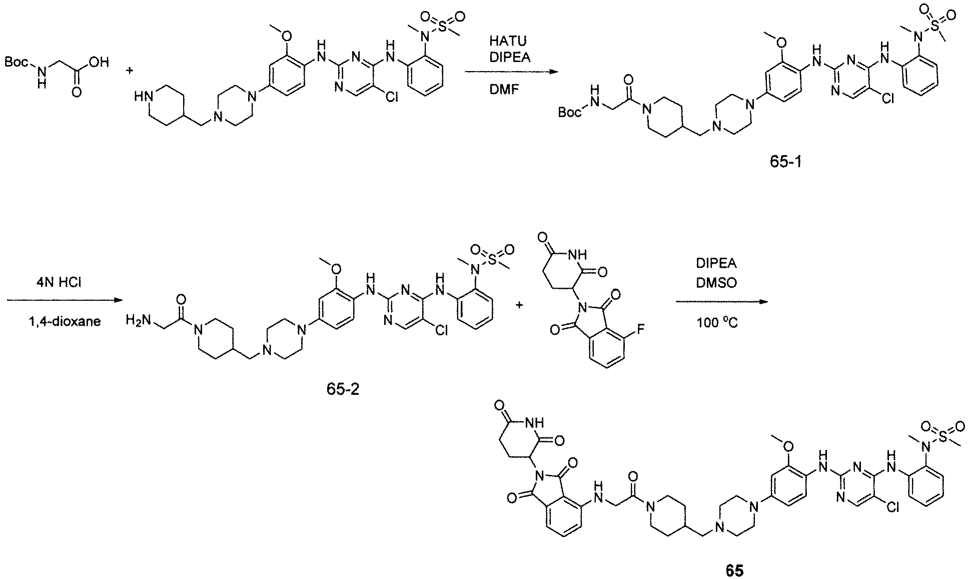

### Step 1: Synthesis of tertiary butyl (2-(4-((4-(4-((5-chloro-4-((2-(N-methylmethanesulfonamido)phenyl)amino)pyrimidin-2-yl)amino)-3-methoxyphenyl)piperazin-1-yl)methyl)piperidin-1-yl)-2-oxoethyl)carbamate (65-1)

The title compound (350 mg, 79%) was obtained using a method similar to the Step 7 synthesis method of Example 49 above with N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (351 mg, 0.57 mmol) and (tertiary butoxycarbonyl)glycine (100 mg, 0.57 mmol) instead of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride and 3-(4-amino-1-oxoisoindolin-2-yl)piperidin-2,6-dione, respectively.

### Step 2: Synthesis of N-(2-((5-chloro-2-((4-(4-((1-glycylpiperidin-4-yl)methyl)piperazin-1-yl)-2-methoxyphenyl)amino)pyrimidin-4-yl)amino)phenyl)-N-methylmethanesulfonamide (65-2)

The title compound (120 mg, yield 92%) was obtained using a method similar to the Step 5 synthesis method of Example 1 above with tertiary butyl (2-(4-((4-(4-((5-chloro-4-((2-(N-methylmethanesulfonamido)phenyl)amino)pyrimidin-2-yl)amino)-3-methoxyphenyl)piperazin-1-yl)methyl)piperidin-1-yl)-2-oxoethyl)carbamate (150 mg, 0.19 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 3: Synthesis of Compound 65

The title compound (80 mg, yield 53%) was obtained using a method similar to the Step 3 synthesis method of Example 33 above with 2-(2,6-dioxopiperidin-3-yl)-4-fluoroisoindolin-1,3-dione (45 mg, 0.16 mmol) and N-(2-((5-chloro-2-((4-(4-((1-glycylpiperidin-4-yl)methyl)piperazin-1-yl)-2-methoxyphenyl)amino)pyrimidin-4-yl)amino)phenyl)-N-methylmethanesulfonamide (110 mg, 0.16 mmol) instead of 2-(2,6-dioxopiperidin-3-yl)-5-fluoroisoindolin-1,3-dione and 3-(2-(benzyloxy)ethoxy)azetidine, respectively.

### Example 66: Synthesis of N-(2-((5-chloro-2-((4-(4-((1-((2-(2,6-dioxopiperidin-3-yl)-1,3-dioxoisoindolin-5-yl)glycyl)piperidin-4-yl)methyl)piperazin-1-yl)-2-methoxyphenyl)amino)pyrimidin-4-yl)amino)phenyl)-N-methylmethanesulfonamide (Compound 66)

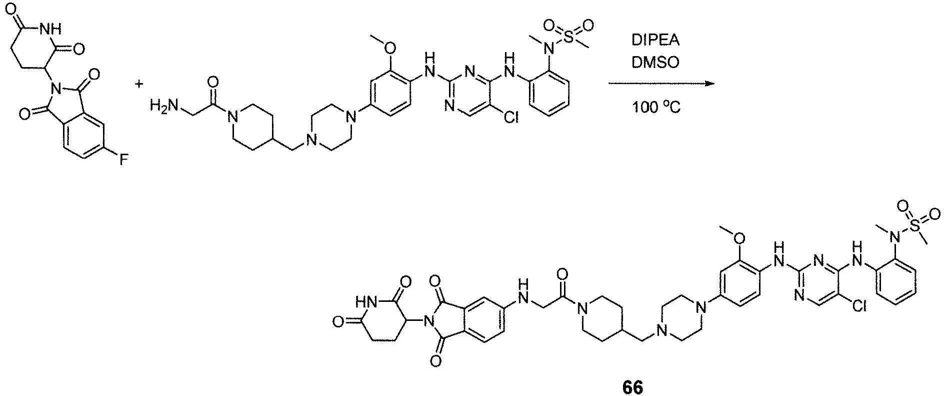

The title compound (28 mg, yield 19%) was obtained using a method similar to the Step 3 synthesis method of Example 33 above with N-(2-((5-chloro-2-((4-(4-((1-glycylpiperidin-4-yl)methyl)piperazin-1-yl)-2-methoxyphenyl)amino)pyrimidin-4-yl)amino)phenyl)-N-methylmethanesulfonamide (110 mg, 0.16 mmol) instead of 3-(2-(benzyloxy)ethoxy)azetidine.

### Example 67: Synthesis of N-(2-((5-chloro-2-((4-(4-((4-(2-(2,6-dioxopiperidin-3-yl)-1,3-dioxoisoindolin-5-yl)piperazin-1-yl)methyl)piperidin-1-yl)-2-methoxyphenyl)amino)pyrimidin-4-yl)amino)phenyl)-N-methylmethanesulfonamide (Compound 67)

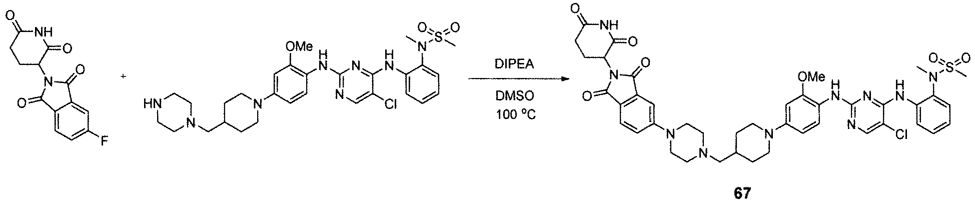

The title compound (90 mg, yield 57%) was obtained using a method similar to the Step 3 synthesis method of Example 33 above with N-(2-((5-chloro-2-((2-methoxy-4-(4-(piperazin-1-ylmethyl)piperidin-1-yl)phenyl)amino)pyrimidin-4-yl)amino)phenyl)-N-methylmethanesulfonamide (111 mg, 0.18 mmol) instead of 3-(2-(benzyloxy)ethoxy)azetidine.

### Example 68: Synthesis of N-(2-((5-chloro-2-((4-(4-((4-(2-(2,6-dioxopiperidin-3-yl)-1,3-dioxoisoindolin-5-yl)piperidin-4-yl)methyl)piperazin-1-yl)-2-methoxyphenyl)amino)pyrimidin-4-yl)amino)phenyl)-N-methylmethanesulfonamide (Compound 68)

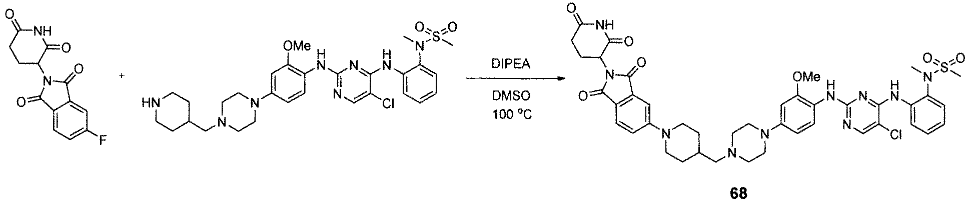

The title compound (110 mg, yield 70%) was obtained using a method similar to the Step 3 synthesis method of Example 33 above with N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (111 mg, 0.18 mmol) instead of 3-(2-(benzyloxy)ethoxy)azetidine.

### Example 69: Synthesis of N-(2-((5-chloro-2-((4-(4-((1-((2-(2,6-dioxopiperidin-3-yl)-1,3-dioxoisoindolin-4-yl)oxy)propanoyl)piperidin-4-yl)methyl)piperazin-1-yl)-2-methoxyphenyl)amino)pyrimidin-4-yl)amino)phenyl)-N-methylmethanesulfonamide (Compound 69)

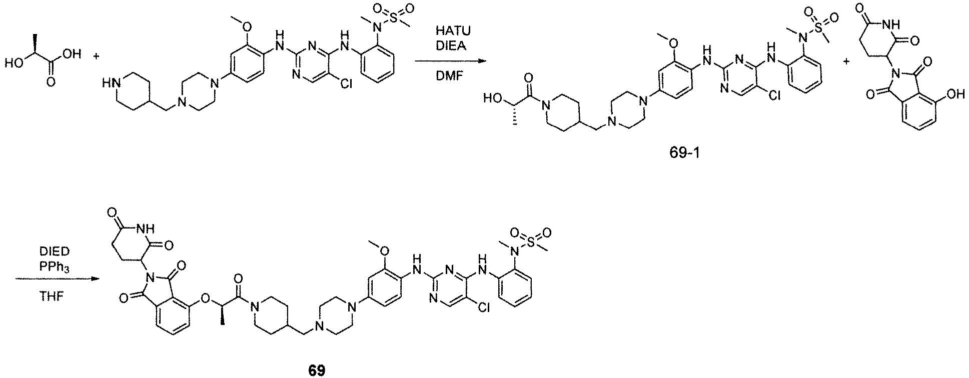

### Step 1: Synthesis of (S)-N-(2-((5-chloro-2-((4-(4-((1-(2-hydroxypropanoyl)piperidin-4-yl)methyl)piperazin-1-yl)-2-methoxyphenyl)amino)pyrimidin-4-yl)amino)phenyl)-N-methylmethanesulfonamide (69-1)

The title compound (80 mg, 53%) was obtained using a method similar to the Step 7 synthesis method of Example 49 above with N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (137 mg, 0.22 mmol) and (S)-hydroxypropanoic acid (20 mg, 0.22 mmol) instead of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride and 3-(4-amino-1-oxoisoindolin-2-yl)piperidin-2,6-dione, respectively.

### Step 2: Synthesis of Compound 69

The title compound (60 mg, yield 55%) was obtained using a method similar to the Step 1 synthesis method of Example 3 above with (S)-N-(2-((5-chloro-2-((4-(4-((1-(2-hydroxypropanoyl)piperidin-4-yl)methyl)piperazin-1-yl)-2-methoxyphenyl)amino)pyrimidin-4-yl)amino)phenyl)-N-methylmethanesulfonamide (80 mg, 0.12 mmol) instead of 4-bromobutan-1-ol.

### Example 70: Synthesis of N-(2-((5-chloro-2-((4-(4-((1-((2-(2,6-dioxopiperidin-3-yl)-1,3-dioxoisoindolin-4-yl)-L-alanyl)piperidin-4-yl)methyl)piperazin-1-yl)-2-methoxyphenyl)amino)pyrimidin-4-yl)amino)phenyl)-N-methylmethanesulfonamide (Compound 70)

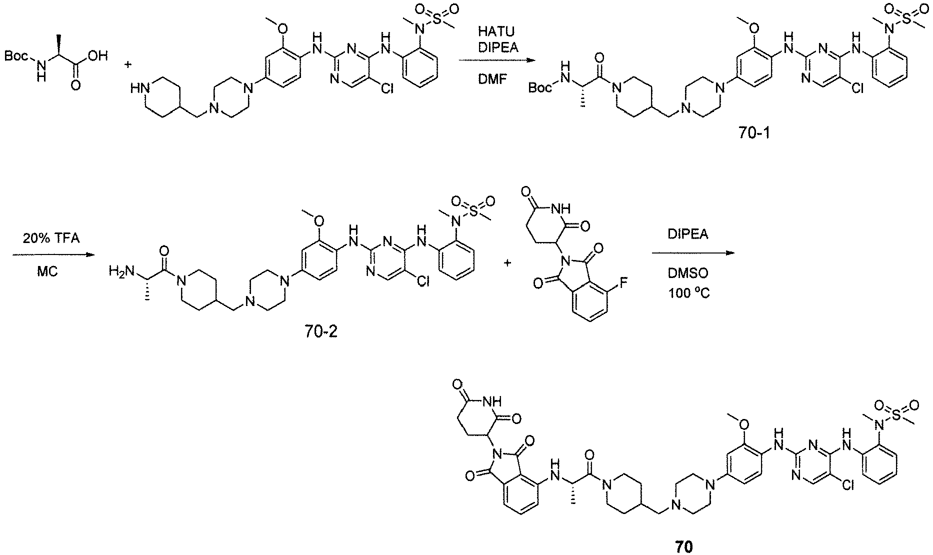

### Step 1: Synthesis of tertiary butyl (S)-(1-(4-((4-(4-((5-chloro-4-((2-(N-methylmethanesulfonamido)phenyl)amino)pyrimidin-2-yl)amino)-3-methoxyphenyl)piperazin-1-yl)methyl)piperidin-1-yl)-1-oxopropan-2-yl))carbamate (70-1)

The title compound (130 mg, 79%) was obtained using a method similar to the Step 7 synthesis method of Example 49 above with N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (130 mg, 0.21 mmol) and (tertiary butoxycarbonyl)-L-alanine (40 mg, 0.21 mmol) instead of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride and 3-(4-amino-1-oxoisoindolin-2-yl)piperidin-2,6-dione, respectively.

### Step 2: Synthesis of N-(2-((2-((4-(4-((1-(L-alanyl)piperidin-4-yl)methyl)piperazin-1-yl)-2-methoxyphenyl)amino)-5-chloropyrimidin-4-yl)amino)phenyl)-N-methylmethanesulfonamide (70-2)

The title compound (105 mg, yield 94%) was obtained using a method similar to the Step 5 synthesis method of Example 1 above with tertiary butyl (S)-(1-(4-((4-(4-((5-chloro-4-((2-(N-methylmethanesulfonamido)phenyl)amino)pyrimidin-2-yl)amino)-3-methoxyphenyl)piperazin-1-yl)methyl)piperidin-1-yl)-1-oxopropan-2-yl))carbamate (128 mg, 0.16 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 3: Synthesis of Compound 70

The title compound (60 mg, yield 60%) was obtained using a method similar to the Step 3 synthesis method of Example 33 above with 2-(2,6-dioxopiperidin-3-yl)-4-fluoroisoindolin-1,3-dione (30 mg, 0.11 mmol) and N-(2-((2-((4-(4-((1-(L-alanyl)piperidin-4-yl)methyl)piperazin-1-yl)-2-methoxyphenyl)amino)-5-chloropyrimidin-4-yl)amino)phenyl)-N-methylmethanesulfonamide (75 mg, 0.11 mmol) instead of 2-(2,6-dioxopiperidin-3-yl)-5-fluoroisoindolin-1,3-dione and 3-(2-(benzyloxy)ethoxy)azetidine, respectively.

### Example 71: Synthesis of N-(2-((5-chloro-2-((4-(4-(4-((1-(2-(2,6-dioxopiperidin-3-yl)-1,3-dioxoisoindolin-5-yl)piperidin-4-yl)methyl)piperazin-1-yl)piperidin-1-yl)-2-methoxyphenyl)amino)pyrimidin-4-yl)amino)phenyl)-N-methylmethanesulfonamide (Compound 71)

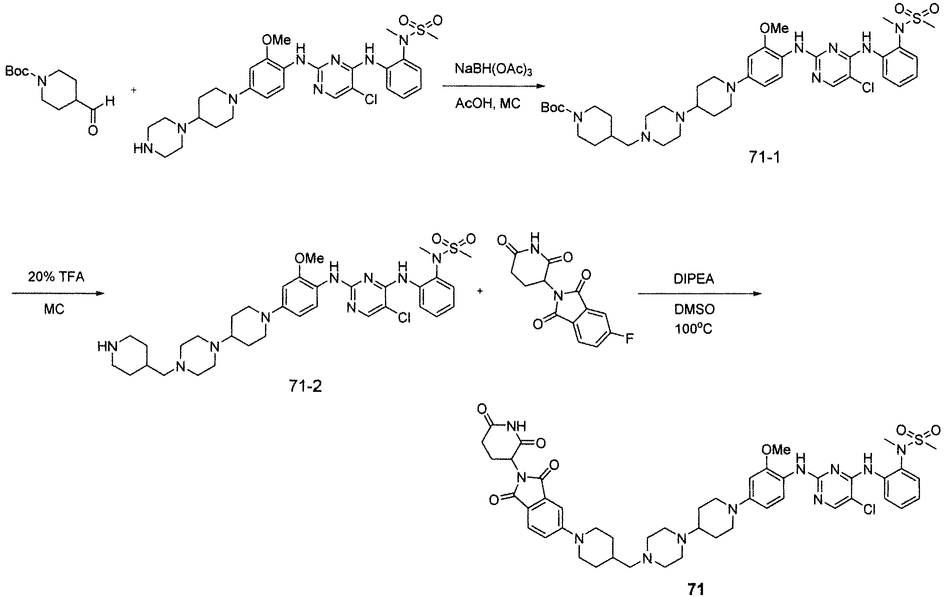

### Step 1: Synthesis of tertiary butyl 4-((4-(1-(4-((5-chloro-4-((2-N-methylmethanesulfonamido)phenyl)amino)pyrimidin-2-yl)amino)-3-methoxyphenyl)piperidin-4-yl)piperazin-1-yl)methyl)piperidin-1-carboxylate (71-1)

The title compound (103 mg, yield 60%) was obtained using a method similar to the Step 2 synthesis method of Example 2 above with tertiary butyl 3-formylazetidin-1-carboxylate (65 mg, 0.31 mmol) and N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide (130 mg, 0.22 mmol) instead of 1-(3-methoxy-4-nitrophenyl)piperidin-4-one and N-Boc-piperazine, respectively.

### Step 2: Synthesis of N-(2-((5-chloro-2-((2-methoxy-4-(4-(4-(piperidin-4-ylmethyl)piperazin-1-yl)piperidin-1-yl)phenyl)amino)pyrimidin-4-yl)amino)phenyl)-N-methylmethanesulfonamide (71-2)

The title compound (90 mg, yield >100%) was obtained using a method similar to the Step 5 synthesis method of Example 1 above with tertiary butyl 4-((4-(1-(4-((5-chloro-4-((2-N-methylmethanesulfonamido)phenyl)amino)pyrimidin-2-yl)amino)-3-methoxyphenyl)piperidin-4-yl)piperazin-1-yl)methyl)piperidin-1-carboxylate (100 mg, 0.125 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 3: Synthesis of Compound 71

The title compound (64 mg, yield 53%) was obtained using a method similar to the Step 3 synthesis method of Example 33 above with N-(2-((5-chloro-2-((2-methoxy-4-(4-(4-(piperidin-4-ylmethyl)piperazin-1-yl)piperidin-1-yl)phenyl)amino)pyrimidin-4-yl)amino)phenyl)-N-methylmethanesulfonamide (88 mg, 0.126 mmol) instead of 3-(2-(benzyloxy)ethoxy)azetidine.

### Example 72: Synthesis of N-(2-((5-chloro-2-((4-(4-(4-(3-(2-(2,6-dioxopiperidin-3-yl)-1,3-dioxoisoindolin-5-yl)prop-2-yn-1-yl)piperazin-1-yl)piperidin-1-yl)-2-methoxyphenyl)amino)pyrimidin-4-yl)amino)phenyl)-N-methylmethanesulfonamide (Compound 72) (not claimed)

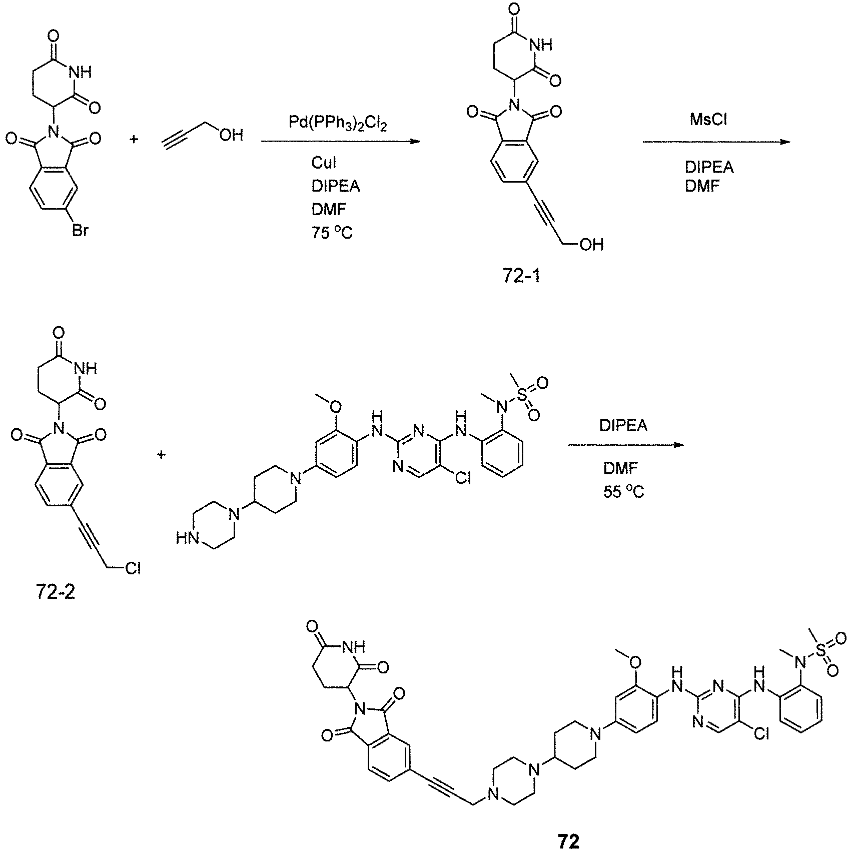

### Step 1: Synthesis of 2-(2,6-dioxopiperidin-3-yl)-5-(3-hydroxyprop-1-yn-1-yl)isoindolin-1,3-dione (72-1)

To a mixture of 5-bromo-2-(2,6-dioxopiperidin-3-yl)isoindolin-1,3-dione (3.0 g, 8.90 mmol), prop-2-yn-1-ol (0.78 mL, 12.82 mmol) and CuI (576 mg, 3.03 mmol) in N,N-dimethylformamide (45 mL), N,N-diisopropyldiamine (3.1 mL, 17.80 mmol) was added and purged with argon gas. Pd(PPh₃)₂Cl₂ (1.0 g, 1.42 mmol) was added thereto and stirred overnight at 75 °C. After extraction with dichloromethane and water, the organic layer was dried over anhydrous magnesium sulfate, filtered, and concentrated. The residue was recrystallized from toluene to obtain the title compound (1.0 g, yield 36%).

### Step 2: Synthesis of 5-(3-chloroprop-1-yn-1-yl)-2-(2,6-dioxopiperidin-3-yl)isoindolin-1,3-dione (72-2)

To a solution of 2-(2,6-dioxopiperidin-3-yl)-5-(3-hydroxyprop-1-yn-1-yl)isoindolin-1,3-dione (170 mg, 0.54 mmol) in N,N-dimethylformamide (5 mL), N,N-diisopropyldiamine (0.13 mL, 1.62 mmol) was added and then methanesulfonyl chloride (0.05 mL, 0.65 mmol) was slowly added at 0 °C. After stirring the reaction mixture overnight, it was extracted with ethyl acetate and water. The organic layer was dried over anhydrous magnesium sulfate, filtered and concentrated. The residue was purified by column chromatography to obtain the title compound (150 mg, yield 84%).

### Step 3: Synthesis of Compound 72

The title compound (180 mg, yield 87%) was obtained using a method similar to the Step 3 synthesis method of Example 41 above with 5-(3-chloroprop-1-yn-1-yl)-2-(2,6-dioxopiperidin-3-yl)isoindolin-1,3-dione (77 mg, 0.23 mmol) and N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide (140 mg, 0.23 mmol) instead of 5-(bromomethyl)-2-(2,6-dioxopiperidin-3-yl)isoindolin-1,3-dione and N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide, respectively.

### Example 73: Synthesis of N-(2-((5-chloro-2-((4-(4-(3-(2-(2,6-dioxopiperidin-3-yl)-1,3-dioxoisoindolin-5-yl)prop-2-yn-1-yl)piperazin-1-yl)-2-methoxyphenyl)amino)pyrimidin-4-yl)amino)phenyl)-N-methylmethanesulfonamide (Compound 73) (not claimed)

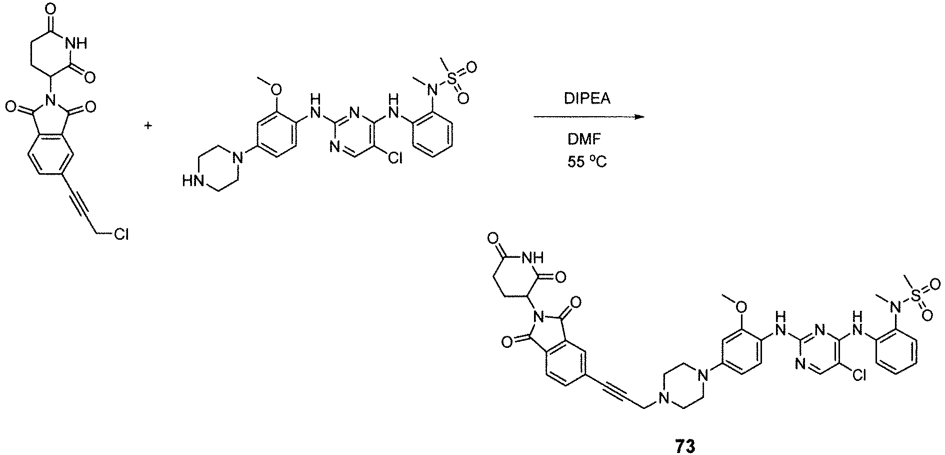

The title compound (68 mg, yield 56%) was obtained using a method similar to the Step 3 synthesis method of Example 41 above with 5-(3-chloroprop-1-yn-1-yl)-2-(2,6-dioxopiperidin-3-yl)isoindolin-1,3-dione (50 mg, 0.15 mmol) and N-(2-((5-chloro-2-((2-methoxy-4-(4-(piperazin-1-yl)phenyl)amino)pyrimidin-4-yl)amino)phenyl)-N-methylmethanesulfonamide (78 mg, 0.15 mmol) instead of 5-(bromomethyl)-2-(2,6-dioxopiperidin-3-yl)isoindolin-1,3-dione and N-(2-((5-chloro-2-((2-methoxy-4-(4-(piperidin-4-ylmethyl)piperazin-1-yl)phenyl)amino)pyrimidin-4-yl)amino)phenyl)-N-methylmethanesulfonamide, respectively.

### Example 74: Synthesis of N-(2-((5-chloro-2-((4-(4-(4-(2-((2-(2,6-dioxopiperidin-3-yl)-1,3-dioxoisoindolin-4-yl)amino)ethyl)piperazin-1-yl)piperidin-1-yl)-2-methoxy-5-methylphenyl)amino)pyrimidin-4-yl)amino)phenyl)-N-methylmethanesulfonamide (Compound 74)

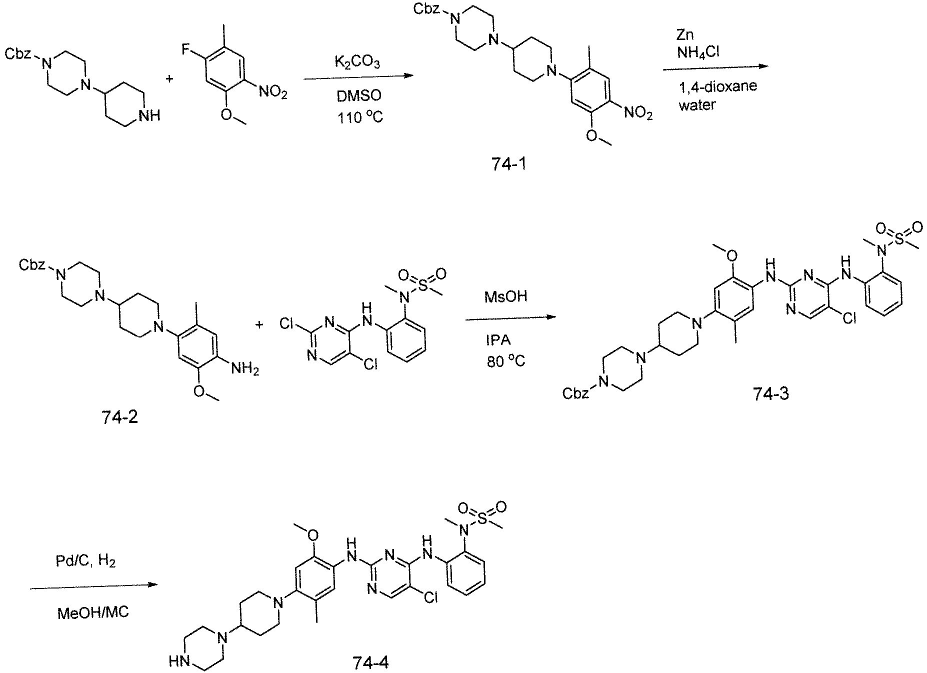

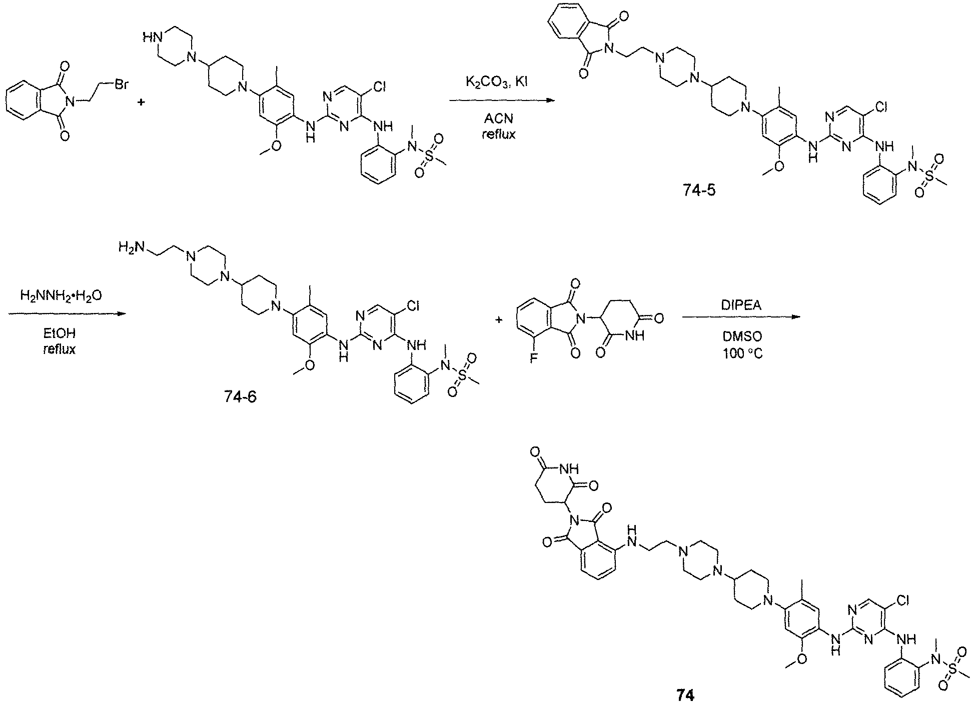

### Step 1: Synthesis of benzyl 4-(1-(5-methoxy-2-methyl-4-nitrophenyl)piperidin-4-yl)piperazin-1-carboxylate (74-1)

The title compound (430 mg, yield 45%) was obtained using a method similar to the Step 2 synthesis method of Example 1 above with benzyl 4-(piperidin-4-yl)piperazin-1-carboxylate (619 mg, 2.04 mmol) instead of Boc-piperazine.

### Step 2: Synthesis of benzyl 4-(1-(4-amino-5-methoxy-2-methylphenyl)piperidin-4-yl)piperazin-1-carboxylate (74-2)

The title compound (873 mg, yield 94%) was obtained using a method similar to the Step 2 synthesis method of Example 25 above with benzyl 4-(1-(5-methoxy-2-methyl-4-nitrophenyl)piperidin-4-yl)piperazin-1-carboxylate (988 mg, 1.09 mmol) instead of benzyl 4-((4-(3-methoxy-4-nitrophenyl)piperazin-1-yl)methyl)piperidin-1-carboxylate.

### Step 3: Synthesis of benzyl 4-(1-(4-((5-chloro-4-((2-(N-methylmethanesulfonamido)phenyl)amino)pyrimidin-2-yl)amino)-5-methoxy-2-methylphenyl)piperidin-4-yl)piperazin-1-carboxylate (74-3)

The title compound (1.1 g, yield 74%) was obtained using a method similar to the Step 3 synthesis method of Example 35 above with benzyl 4-(1-(4-amino-5-methoxy-2-methylphenyl)piperidin-4-yl)piperazin-1-carboxylate (873 mg, 1.99 mmol) instead of benzyl 4-((4-(4-amino-3-chlorophenyl)piperazin-1-yl)methyl)piperidin-1-carboxylate.

### Step 4: Synthesis of N-(2-((5-chloro-2-((2-methoxy-5-methyl-4-(4-(piperazin-1-yl)piperidin-1-yl)phenyl)amino)pyrimidin-4-yl)amino)phenyl)-N-methylmethanesulfonamide (74-4)

The title compound (648 mg, yield 72%) was obtained using a method similar to the Step 4 synthesis method of Example 33 above with benzyl 4-(1-(4-((5-chloro-4-((2-(N-methylmethanesulfonamido)phenyl)amino)pyrimidin-2-yl)amino)-5-methoxy-2-methylphenyl)piperidin-4-yl)piperazin-1-carboxylate (1.1 g, 1.67 mmol) instead of 5-(3-(2-(benzyloxy)ethoxy)azetidin-1-yl)-2-(2,6-dioxopiperidin-3-yl)isoindolin-1,3-dione.

### Step 5: Synthesis of N-(2-((5-chloro-2-((4-(4-(4-(2-(1,3-dioxoindolin-2-yl)ethyl)piperazin-1-yl)piperidin-1-yl)-2-methoxy-5-methylphenyl)amino)pyrimidin-4-yl)amino)phenyl)-N-methylmethanesulfonamide (74-5)

The title compound (80 mg, yield 22%) was obtained using a method similar to the Step 1 synthesis method of Example 45 above with N-(2-((5-chloro-2-((2-methoxy-5-methyl-4-(4-(piperazin-1-yl)piperidin-1-yl)phenyl)amino)pyrimidin-4-yl)amino)phenyl)-N-methylmethanesulfonamide (300 mg, 0.49 mmol) instead of N-(2-((5-chloro-2-((2-methoxy-4-(4-(piperazin-1-yl)piperidin-1-yl)phenyl)amino)pyrimidin-4-yl)amino)phenyl)-N-methylmethanesulfonamide.

### Step 6: Synthesis of N-(2-((2-((4-(4-(4-(2-aminoethyl)piperazin-1-yl)piperidin-1-yl)-2-methoxy-5-methylphenyl)amino)-5-chloropyrimidin-4-yl)amino)phenyl)-N-methylmethanesulfonamide (74-6)

The title compound (49 mg, yield 74%) was obtained using a method similar to the Step 2 synthesis method of Example 45 above with N-(2-((5-chloro-2-((4-(4-(4-(2-(1,3-dioxoindolin-2-yl)ethyl)piperazin-1-yl)piperidin-1-yl)-2-methoxy-5-methylphenyl)amino)pyrimidin-4-yl)amino)phenyl)-N-methylmethanesulfonamide (80 mg, 0.10 mmol) instead of N-(2-((5-chloro-2-((4-(4-(4-(2-(1,3-dioxoindolin-2-yl)ethyl)piperazin-1-yl)piperidin-1-yl)-2-methoxyphenyl)amino)pyrimidin-4-yl)amino)phenyl)-N-methylmethanesulfonamide.

### Step 7: Synthesis of Compound 74

The title compound (21 mg, yield 32%) was obtained using a method similar to the Step 3 synthesis method of Example 33 above with 2-(2,6-dioxopiperidin-3-yl)-4-fluoroisoindolin-1,3-dione (22 mg, 0.079 mmol) and N-(2-((2-((4-(4-(4-(2-aminoethyl)piperazin-1-yl)piperidin-1-yl)-2-methoxy-5-methylphenyl)amino)-5-chloropyrimidin-4-yl)amino)phenyl)-N-methylmethanesulfonamide (47 mg, 0.072 mmol) instead of 2-(2,6-dioxopiperidin-3-yl)-5-fluoroisoindolin-1,3-dione and 3-(2-(benzyloxy)ethoxy)azetidine, respectively.

### Example 75: Synthesis of N-(2-((5-chloro-2-((4-(4-((1-((2-(2,6-dioxopiperidin-3-yl)-1,3-dioxoisoindolin-4-yl)-L-alanyl)piperidin-4-yl)methyl)piperazin-1-yl)-2-methoxy-5-methylphenyl)amino)pyrimidin-4-yl)amino)phenyl)-N-methylmethanesulfonamide (Compound 75)

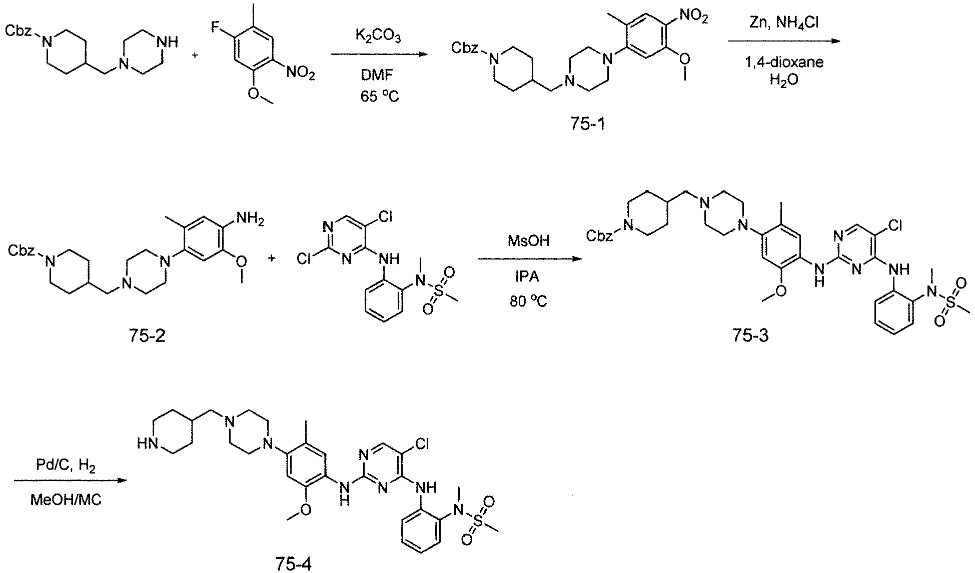

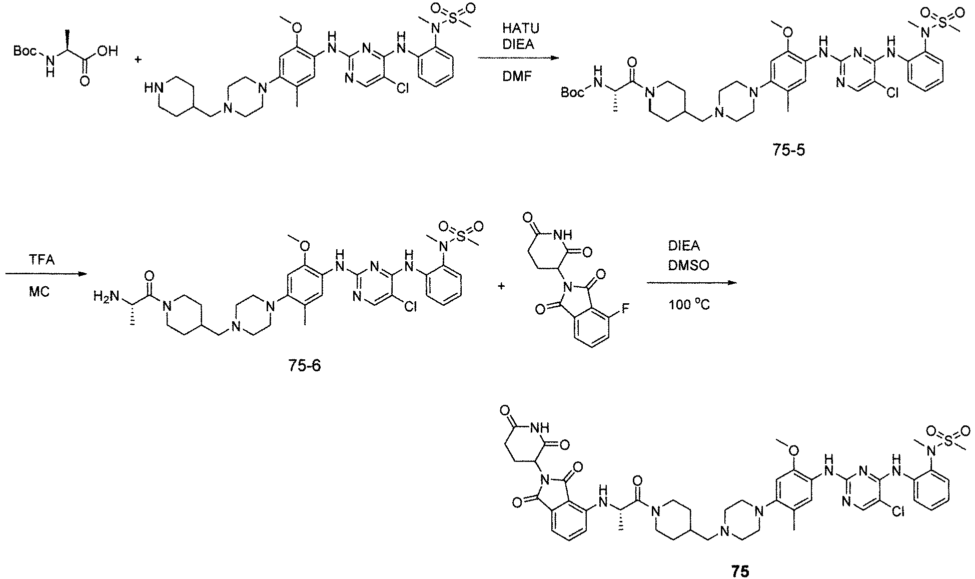

### Step 1: Synthesis of benzyl 4-((4-(5-methoxy-2-methyl-4-nitrophenyl)piperazin-1-yl)methyl)piperidin-1-carboxylate (75-1)

A mixture of 1-fluoro-5-methoxy-2-methyl-4-nitrobenzene (6.5 g, 35.11 mmol), benzyl 4-(piperazin-1-ylmethyl)piperidin-1-carboxylate (14.4 g, 36.86 mmol) and potassium carbonate (17.0 g, 122.87 mmol) in dimethyl sulfoxide (87 mL) was stirred at 110 °C. After stirring overnight, water was added to the reaction mixture, followed by stirring, filtration, and washing with water. The remaining material was washed sequentially with ethyl acetate, followed by water and saturated aqueous sodium chloride solution. The organic layer was dried over anhydrous sodium sulfate, filtered and concentrated. The residue was purified by column chromatography to obtain the title compound (14.0 g, 72%).

### Step 2: Synthesis of benzyl 4-((4-(4-amino-5-methoxy-2-methylphenyl)piperazin-1-yl)methyl)piperidin-1-carboxylate (75-2)

The title compound (718 mg, yield 99%) was obtained using a method similar to the Step 2 synthesis method of Example 25 above with benzyl 4-((4-(5-methoxy-2-methyl-4-nitrophenyl)piperazin-1-yl)methyl)piperidin-1-carboxylate (794 mg, 1.60 mmol) instead of benzyl 4-((4-(3-methoxy-4-nitrophenyl)piperazin-1-yl)methyl)piperidin-1-carboxylate.

### Step 3: Synthesis of benzyl 4-((4-(4-((5-chloro-4-((2-(N-methylmethanesulfonamido)phenyl)amino)pyrimidin-2-yl)amino)-5-methoxy-2-methylphenyl)piperazin-1-yl)methyl)piperidin-1-carboxylate (75-3)

The title compound (1.0 g, yield 83%) was obtained using a method similar to the Step 3 synthesis method of Example 33 above with benzyl 4-((4-(4-amino-5-methoxy-2-methylphenyl)piperazin-1-yl)methyl)piperidin-1-carboxylate (718 mg, 1.58 mmol) instead of benzyl 4-((4-(4-amino-3-chlorophenyl)piperazin-1-yl)methyl)piperidin-1-carboxylate.

### Step 4: Synthesis of N-(2-((5-chloro-2-((2-methoxy-5-methyl-4-(4-(piperidin-4-ylmethyl)piperazin-1-yl)phenyl)amino)pyrimidin-4-yl)amino)phenyl)-N-methylmethanesulfonamide (75-4)

The title compound (800 mg, yield >100%) was obtained using a method similar to the Step 4 synthesis method of Example 33 above with benzyl 4-((4-(4-((5-chloro-4-((2-(N-methylmethanesulfonamido)phenyl)amino)pyrimidin-2-yl)amino)-5-methoxy-2-methylphenyl)piperazin-1-yl)methyl)piperidin-1-carboxylate (1.0 g, 1.31 mmol) instead of 5-(3-(2-(benzyloxy)ethoxy)azetidin-1-yl)-2-(2,6-dioxopiperidin-3-yl)isoindolin-1,3-dione.

### Step 5: Synthesis of tertiary butyl (S)-(1-(4-((4-(4-((5-chloro-4-((2-(N-methylmethanesulfonamido)phenyl)amino)pyrimidin-2-yl)amino)-5-methoxy-2-methylphenyl)piperazin-1-yl)methyl)piperidin-1-yl)-1-oxopropan-2-yl))carbamate (75-5)

The title compound (175 mg, 92%) was obtained using a method similar to the Step 7 synthesis method of Example 49 above with N-(2-((5-chloro-2-((2-methoxy-5-methyl-4-(4-(piperidin-4-ylmethyl)piperazin-1-yl)phenyl)amino)pyrimidin-4-yl)amino)phenyl)-N-methylmethanesulfonamide (150 mg, 0.24 mmol) and (tertiary butoxycarbonyl)-L-alanine (54 mg, 0.29 mmol) instead of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride and 3-(4-amino-1-oxoisoindolin-2-yl)piperidin-2,6-dione, respectively.

### Step 6: Synthesis of N-(2-((2-((4-(4-((1-(L-alanyl)piperidin-4-yl)methyl)piperazin-1-yl)-2-methoxy-5-methylphenyl)amino)-5-chloropyrimidin-4-yl)amino)phenyl)-N-methylmethanesulfonamide (75-6)

The title compound (148 mg, yield 98%) was obtained using a method similar to the Step 5 synthesis method of Example 1 above with tertiary butyl (S)-(1-(4-((4-(4-((5-chloro-4-((2-(N-methylmethanesulfonamido)phenyl)amino)pyrimidin-2-yl)amino)-5-methoxy-2-methylphenyl)piperazin-1-yl)methyl)piperidin-1-yl)-1-oxopropan-2-yl))carbamate (172 mg, 0.22 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 7: Synthesis of Compound 75

The title compound (80 mg, yield 40%) was obtained using a method similar to the Step 3 synthesis method of Example 33 above with 2-(2,6-dioxopiperidin-3-yl)-4-fluoroisoindolin-1,3-dione (57 mg, 0.21 mmol) and N-(2-((2-((4-(4-((1-(L-alanyl)piperidin-4-yl)methyl)piperazin-1-yl)-2-methoxy-5-methylphenyl)amino)-5-chloropyrimidin-4-yl)amino)phenyl)-N-methylmethanesulfonamide (145 mg, 0.21 mmol) instead of 2-(2,6-dioxopiperidin-3-yl)-5-fluoroisoindolin-1,3-dione and 3-(2-(benzyloxy)ethoxy)azetidine, respectively.

### Example 76: Synthesis of N-(2-((5-chloro-2-((4-(4-(4-((1-(2-(2,6-dioxopiperidin-3-yl)-1,3-dioxoisoindolin-5-yl)piperidin-4-yl)methyl)piperazin-1-yl)piperidin-1-yl)-2-methoxy-5-methylphenyl)amino)pyrimidin-4-yl)amino)phenyl)-N-methylmethanesulfonamide (Compound 76)

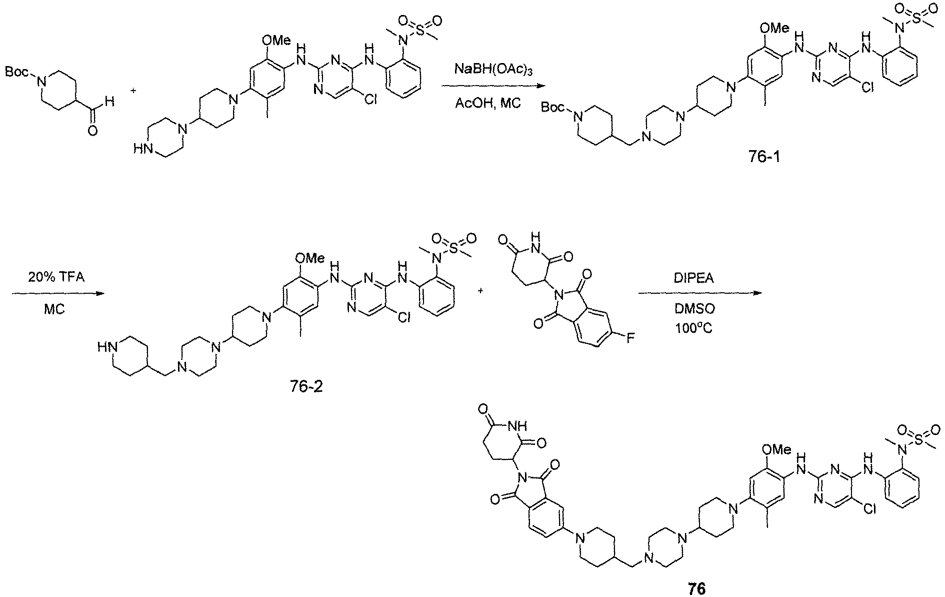

### Step 1: Synthesis of tertiary butyl 4-((4-(1-(4-{(5-chloro-4-((2-N-methylmethanesulfonamido)phenyl)amino)pyrimidin-2-yl)amino)-3-methoxy-5-methylphenyl)piperidin-4-yl)piperazin-1-yl)methyl)piperidin-1-carboxylate (76-1)

The title compound (108 mg, yield 54%) was obtained using a method similar to the Step 2 synthesis method of Example 2 above with tertiary butyl 4-formylpiperidin-1-carboxylate (78 mg, 0.37 mmol) and N-(2-((5-chloro-2-((2-methoxy-5-methyl-4-(4-(piperazin-1-yl)piperidin-1-yl)phenyl)amino)pyrimidin-4-yl)amino)phenyl)-N-methylmethanesulfonamide (150 mg, 0.24 mmol) instead of 1-(3-methoxy-4-nitrophenyl)piperidin-4-one and N-Boc-piperazine, respectively.

### Step 2: Synthesis of N-(2-((5-chloro-2-((2-methoxy-5-methyl-4-(4-(4-(piperidin-4-ylmethyl)piperazin-1-yl)piperidin-1-yl)phenyl)amino)pyrimidin-4-yl)amino)phenyl)-N-methylmethanesulfonamide (76-2)

The title compound (91 mg, yield 99%) was obtained using a method similar to the Step 5 synthesis method of Example 1 above with tertiary butyl 4-((4-(1-(4-((5-chloro-4-((2-N-methylmethanesulfonamido)phenyl)amino)pyrimidin-2-yl)amino)-3-methoxy-5-methylphenyl)piperidin-4-yl)piperazin-1-yl)methyl)piperidin-1-carboxylate (106 mg, 0.13 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 3: Synthesis of Compound 76

The title compound (80 mg, yield 65%) was obtained using a method similar to the Step 3 synthesis method of Example 33 above with N-(2-((5-chloro-2-((2-methoxy-5-methyl-4-(4-(4-(piperidin-4-ylmethyl)piperazin-1-yl)piperidin-1-yl)phenyl)amino)pyrimidin-4-yl)amino)phenyl)-N-methylmethanesulfonamide (90 mg, 0.126 mmol) instead of 3-(2-(benzyloxy)ethoxy)azetidine.

### Example 77: Synthesis of N-(2-((5-chloro-2-((4-(4-(4-(2-(1-(2-(2,6-dioxopiperidin-3-yl)-1,3-dioxoisoindolin-5-yl)piperidin-4-yl)ethyl)piperazin-1-yl)piperidin-1-yl)-2-methoxy5-methylphenyl)amino)pyrimidin-4-yl)amino)phenyl)-N-methylmethanesulfonamide (Compound 77)

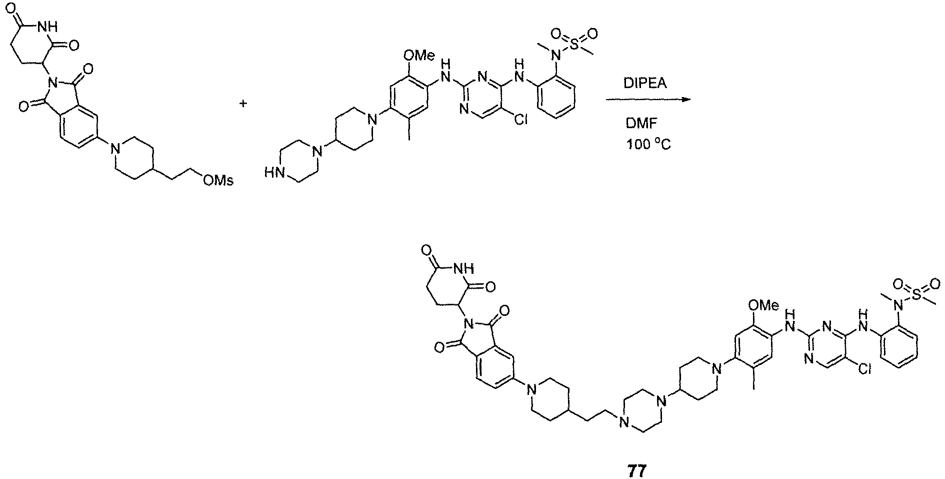

The title compound (65 mg, yield 44%) was obtained using a method similar to the Step 6 synthesis method of Example 1 above with 2-(1-(2-(2,6-dioxopiperidin-3-yl)-1,3-dioxoisoindolin-5-yl)piperidin-4-yl)ethyl methanesulfonate (70 mg, 0.15 mmol) and N-(2-((5-chloro-2-((2-methoxy-5-methyl-4-(4-piperazin-1-yl)piperidin-1-yl)phenyl)amino)pyrimidin-4-yl)amino)phenyl)-N-methylmethylsulfonamide (93 mg, 0.15 mmol) instead of 5-bromo-N-(2-(2,6-dioxopiperidin-3-yl)-1,3-dioxoisoindolin-4-yl)pentanamide and N-(2-((5-chloro-2-((2-methoxy-4-(piperazin-1-yl)phenyl)amino)pyrimidin-4-yl)amino)phenyl)-N-methylmethanesulfonamide.

### Example 78: Synthesis of N-(2-((5-chloro-2-((4-(4-((4-((2-(2,6-dioxopiperidin-3-yl)-1,3-dioxoisoindolin-4-yl)-L-alanyl)piperazin-1-yl)methyl)piperidin-1-yl)-2-methoxy-5-methylphenyl)amino)pyrimidin-4-yl)amino)phenyl)-N-methylmethanesulfonamide (Compound 78)

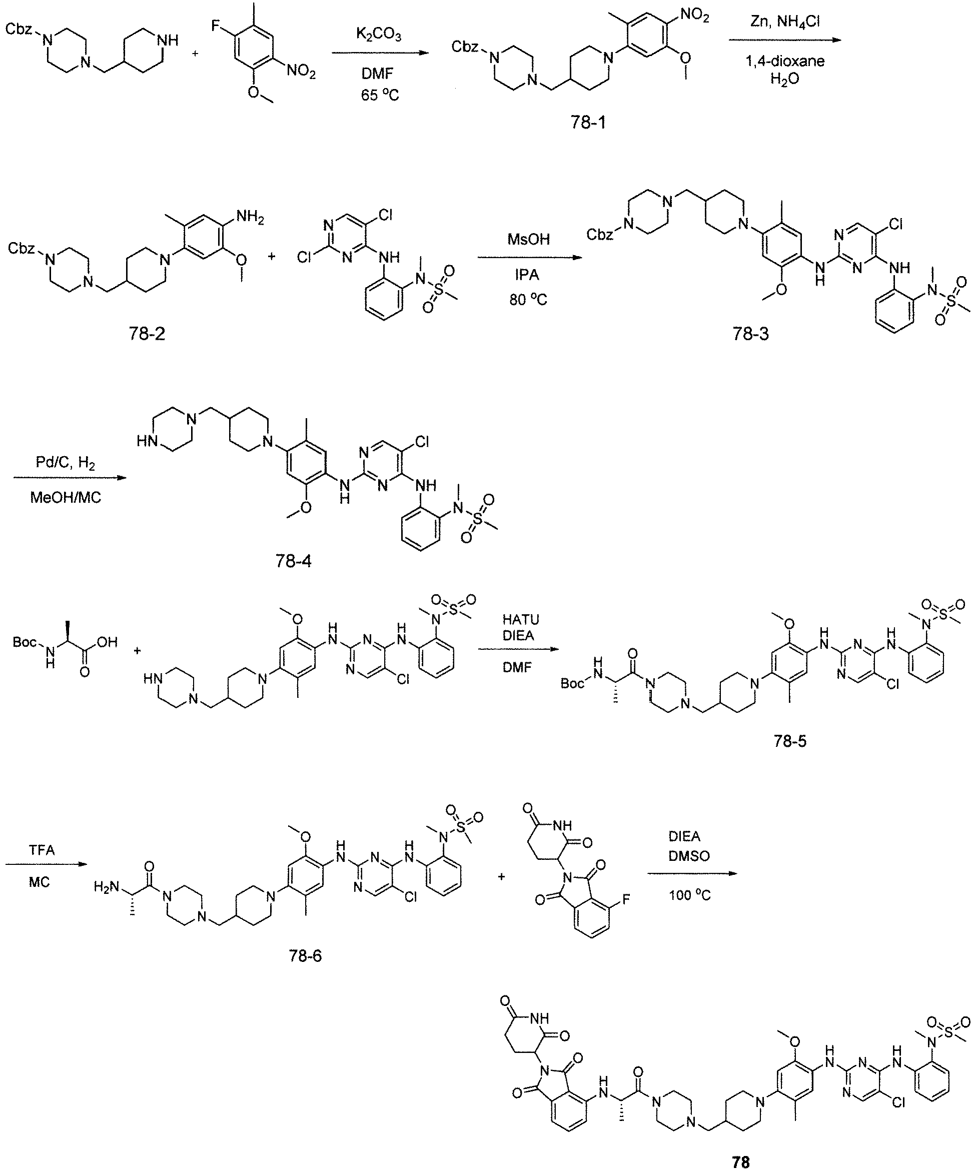

### Step 1: Synthesis of benzyl 4-((1-(5-methoxy-2-methyl-4-nitrophenyl)piperidin-4-yl)methyl)piperazin-1-carboxylate (78-1)

The title compound (2.6 g, yield 97%) was obtained using a method similar to the Step 3 synthesis method of Example 33 above with benzyl 4-(piperidin-4-ylmethyl)piperazin-1-carboxylate (2.3 g, 5.79 mmol) instead of benzyl 4-(piperazin-1-ylmethyl)piperidin-1-carboxylate.

### Step 2: Synthesis of benzyl 4-((1-(4-amino-5-methoxy-2-methylphenyl)piperidin-4-yl)methyl)piperazin-1-carboxylate (78-2)

The title compound (2.38 g, yield 98%) was obtained using a method similar to the Step 2 synthesis method of Example 25 above with benzyl 4-((1-(5-methoxy-2-methyl-4-nitrophenyl)piperidin-4-yl)methyl)piperazin-1-carboxylate (2.6 g, 5.39 mmol) instead of benzyl 4-((4-(3-methoxy-4-nitrophenyl)piperazin-1-yl)methyl)piperidin-1-carboxylate.

### Step 3: Synthesis of benzyl 4-((1-(4-((5-chloro-4-((2-(N-methylmethanesulfonamido)phenyl)amino)pyrimidin-2-yl)amino)-5-methoxy-2-methylphenyl)piperidin-4-yl)methyl)piperazin-1-carboxylate (78-3)

The title compound (4.4 g, yield 83%) was obtained using a method similar to the Step 3 synthesis method of Example 35 above with benzyl 4-((1-(4-amino-5-methoxy-2-methylphenyl)piperidin-4-yl)methyl)piperazin-1-carboxylate (3.2 g, 7.00 mmol) instead of benzyl 4-((4-(4-amino-3-chlorophenyl)piperazin-1-yl)methyl)piperidin-1-carboxylate.

### Step 4: Synthesis of N-(2-((5-chloro-2-((2-methoxy-5-methyl-4-(4-(piperazin-1-ylmethyl)piperidin-1-yl)phenyl)amino)pyrimidin-4-yl)amino)phenyl)-N-methylmethanesulfonamide (78-4)

The title compound (3.5 g, yield >100%) was obtained using a method similar to the Step 4 synthesis method of Example 33 above with benzyl 4-((1-(4-((5-chloro-4-((2-(N-methylmethanesulfonamido)phenyl)amino)pyrimidin-2-yl)amino)-5-methoxy-2-methylphenyl)piperidin-4-yl)methyl)piperazin-1-carboxylate (3.7 g, 4.85 mmol) instead of 5-(3-(2-(benzyloxy)ethoxy)azetidin-1-yl)-2-(2,6-dioxopiperidin-3-yl)isoindolin-1,3-dione.

### Step 5: Synthesis of tertiary butyl (S)-(1-(4-((1-(4-((5-chloro-4-((2-(N-methylmethanesulfonamido)phenyl)amino)pyrimidin-2-yl)amino)-5-methoxy-2-methylphenyl)piperidin-4-yl)methyl)piperazin-1-yl)-1-oxopropan-2-yl)carbamate (78-5)

The title compound (183 mg, 95%) was obtained using a method similar to the Step 7 synthesis method of Example 49 above with N-(2-((5-chloro-2-((2-methoxy-5-methyl-4-(4-(piperazin-1-ylmethyl)piperidin-1-yl)phenyl)amino)pyrimidin-4-yl)amino)phenyl)-N-methylmethanesulfonamide (150 mg, 0.24 mmol) and (tertiary butoxycarbonyl)-L-alanine (45 mg, 0.24 mmol) instead of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride and 3-(4-amino-1-oxoisoindolin-2-yl)piperidin-2,6-dione.

### Step 6: Synthesis of N-(2-((2-((4-(4-((4-(L-alanyl)piperazin-1-yl)methyl)piperidin-1-yl)-2-methoxy-5-methylphenyl)amino)-5-chloropyrimidin-4-yl)amino)phenyl)-N-methylmethanesulfonamide (78-6)

The title compound (110 mg, yield 71%) was obtained using a method similar to the Step 5 synthesis method of Example 1 above with tertiary butyl (S)-(1-(4-((1-(4-((5-chloro-4-((2-(N-methylmethanesulfonamido)phenyl)amino)pyrimidin-2-yl)amino)-5-methoxy-2-methylphenyl)piperidin-4-yl)methyl)piperazin-1-yl)-1-oxopropan-2-yl)carbamate (173 mg, 0.22 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 7: Synthesis of Compound 78

The title compound (67 mg, yield 44%) was obtained using a method similar to the Step 3 synthesis method of Example 33 above with 2-(2,6-dioxopiperidin-3-yl)-4-fluoroisoindolin-1,3-dione (66 mg, 0.24 mmol) and N-(2-((2-((4-(4-((4-(L-alanyl)piperazin-1-yl)methyl)piperidin-1-yl)-2-methoxy-5-methylphenyl)amino)-5-chloropyrimidin-4-yl)amino)phenyl)-N-methylmethanesulfonamide (110 mg, 0.16 mmol) instead of 2-(2,6-dioxopiperidin-3-yl)-5-fluoroisoindolin-1,3-dione and 3-(2-(benzyloxy)ethoxy)azetidine.

### Example 79: Synthesis of N-(2-((5-chloro-2-((4-(4-((4-(2-(2,6-dioxopiperidin-3-yl)-1,3-dioxoisoindolin-5-yl)piperazin-1-yl)methyl)piperidin-1-yl)-2-methoxy-5-methylphenyl)amino)pyrimidin-4-yl)amino)phenyl)-N-methylmethanesulfonamide (Compound 79)

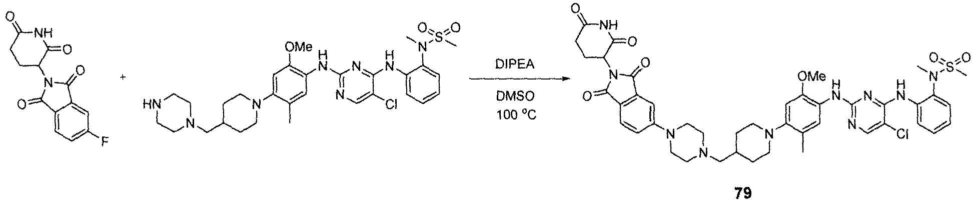

The title compound (51 mg, yield 52%) was obtained using a method similar to the Step 3 synthesis method of Example 33 above with N-(2-((5-chloro-2-((2-methoxy-5-methyl-4-(4-(piperazin-1-ylmethyl)piperidin-1-yl)phenyl)amino)pyrimidin-4-yl)amino)phenyl)-N-methylmethanesulfonamide (71 mg, 0.11 mmol) instead of 3-(2-(benzyloxy)ethoxy)azetidine.

### Example 80: Synthesis of N-(2-((5-chloro-2-((4-(4-((1-((2-(2,6-dioxopiperidin-3-yl)-1,3-dioxoisoindolin-4-yl)glycyl)piperidin-4-yl)methyl)piperazin-1-yl)-2-methoxy-5-methylphenyl)amino)pyrimidin-4-yl)amino)phenyl)-N-methylmethanesulfonamide (Compound 80)

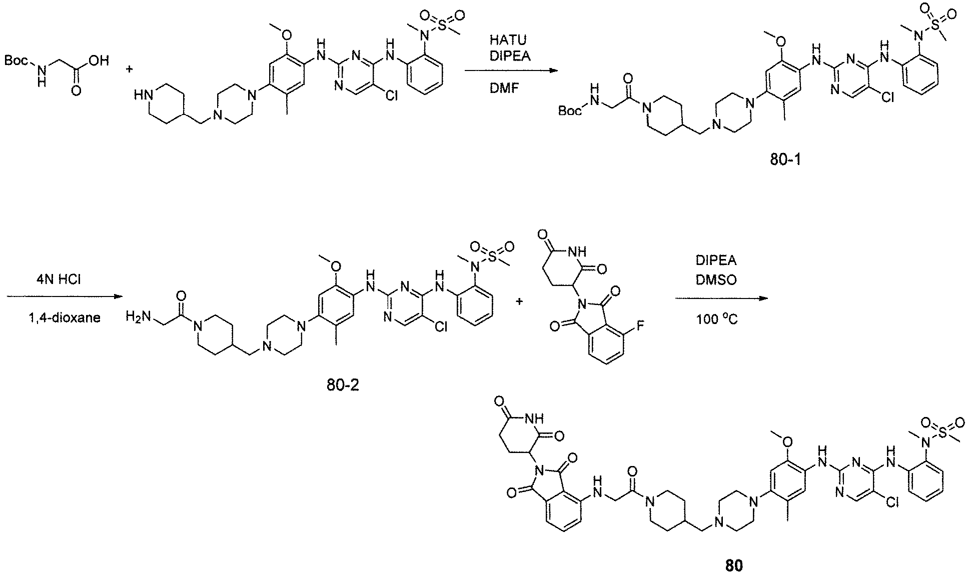

### Step 1: Synthesis of tertiary butyl (2-(4-((4-(4-((5-chloro-4-((2-(N-methylmethanesulfonamido)phenyl)amino)pyrimidin-2-yl)amino)-5-methoxy-2-methylphenyl)piperazin-1-yl)methyl)piperidin-1-yl)-2-oxoethyl)carbamate (80-1)

The title compound (135 mg, 72%) was obtained using a method similar to the Step 7 synthesis method of Example 49 above with N-(2-((5-chloro-2-((2-methoxy-5-methyl-4-(4-(piperidin-4-ylmethyl)piperazin-1-yl)phenyl)amino)pyrimidin-4-yl)amino)phenyl)-N-methylmethanesulfonamide (151 mg, 0.24 mmol) and (tertiary butoxycarbonyl)glycine (42 mg, 0.24 mmol) instead of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride and 3-(4-amino-1-oxoisoindolin-2-yl)piperidin-2,6-dione, respectively.

### Step 2: Synthesis of N-(2-((5-chloro-2-((4-(4-((1-glycylpiperidin-4-yl)methyl)piperazin-1-yl)-2-methoxy-5-methylphenyl)amino)pyrimidin-4-yl)amino)phenyl)-N-methylmethanesulfonamide (80-2)

The title compound (105 mg, yield 93%) was obtained using a method similar to the Step 5 synthesis method of Example 1 above with tertiary butyl (2-(4-((4-(4-((5-chloro-4-((2-(N-methylmethanesulfonamido)phenyl)amino)pyrimidin-2-yl)amino)-5-methoxy-2-methylphenyl)piperazin-1-yl)methyl)piperidin-1-yl)-2-oxoethyl)carbamate (130 mg, 0.17 mmol) instead of tertiary butyl 4-(4-((5-chloro-4-((2-(N-methylmethylsulfonamido)phenyl)amino)pyrimidin-2-yl)amino)-3-methoxyphenyl)piperazin-1-carboxylate.

### Step 3: Synthesis of Compound 80

The title compound (65 mg, yield 48%) was obtained using a method similar to the Step 3 synthesis method of Example 33 above with 2-(2,6-dioxopiperidin-3-yl)-4-fluoroisoindolin-1,3-dione (40 mg, 0.15 mmol) and N-(2-((5-chloro-2-((4-(4-((1-glycylpiperidin-4-yl)methyl)piperazin-1-yl)-2-methoxy-5-methylphenyl)amino)pyrimidin-4-yl)amino)phenyl)-N-methylmethanesulfonamide (99 mg, 0.15 mmol) instead of 2-(2,6-dioxopiperidin-3-yl)-5-fluoroisoindolin-1,3-dione and 3-(2-(benzyloxy)ethoxy)azetidine.

### Example 81: Synthesis of N-(2-((5-chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-2-methoxy-5-methylphenyl)amino)pyrimidin-4-yl)amino)phenyl)-N-methylmethanesulfonamide (Compound 81)

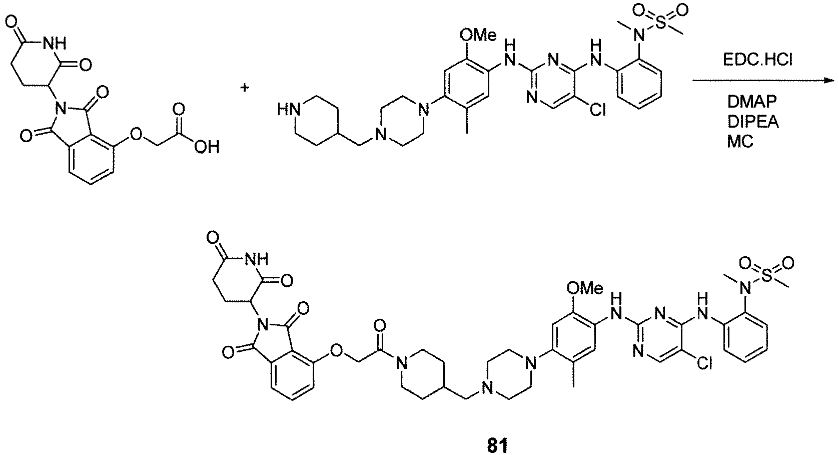

The title compound (105 mg, yield 70%) was obtained using a method similar to the Step 1 synthesis method of Example 6 above with 2-((2-(2,6-dioxopiperidin-3-yl)-1,3-dioxoisoindolin-4-yl)oxy)acetic acid (53 mg, 0.16 mmol) and N-(2-((5-chloro-2-((2-methoxy-5-methyl-4-(4-(piperidin-4-ylmethyl)piperazin-1-yl)phenyl)amino)pyrimidin-4-yl)amino)phenyl)-N-methylmethanesulfonamide (100 mg, 0.16 mmol) instead of (2R, 4R)-1-((S)-2-amino-3,3-dimethylbutanoyl)-4-hydroxy-N-(4-(4-methylthiazol-5-yl)benzyl)pyrrolidin-2-carboxamide hydrochloride and 5-bromopentanoic acid.

### Example 82: Synthesis of N-(2-((5-chloro-2-((4-(4-((1-((2S)-2-((2-(2,6-dioxopiperidin-3-yl)-1,3-dioxoisoindolin-4-yl)oxy)propanoyl)piperidin-4-yl)methyl)piperazin-1-yl)-2-methoxy-5-methylphenyl)amino)pyrimidin-4-yl)amino)phenyl)-N-methylmethanesulfonamide (Compound 82)

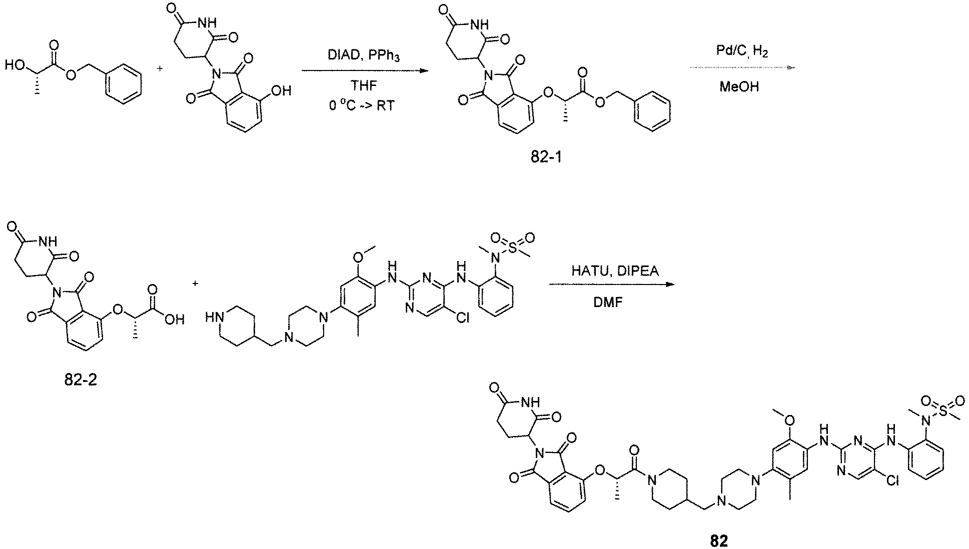

### Step 1: Synthesis of benzyl (2S)-2-((2-(2,6-dioxopiperidin-3-yl)-1,3-dioxoisoindolin-4-yl)oxy)propanoate (82-1)

The title compound (270 mg, yield 32%) was obtained using a method similar to the Step 1 synthesis method of Example 3 above with benzyl (S)-2-hydroxypropanoate (520 mg, 2.89 mmol) instead of 4-bromobutan-1-ol.

### Step 2: Synthesis of (2S)-2-((2-(2,6-dioxopiperidin-3-yl)-1,3-dioxoisoindolin-4-yl)oxy)propanoic acid (82-2)

The title compound (133 mg, yield >100%) was obtained using a method similar to the Step 4 synthesis method of Example 33 above with benzyl (2S)-2-((2-(2,6-dioxopiperidin-3-yl)-1,3-dioxoisoindolin-4-yl)oxy)propanoate (165 mg, 0.38 mmol) instead of 5-(3-(2-(benzyloxy)ethoxy)azetidin-1-yl)-2-(2,6-dioxopiperidin-3-yl)isoindolin-1,3-dione.

### Step 3: Synthesis of Compound 82

The title compound (70 mg, 42%) was obtained using a method similar to the Step 7 synthesis method of Example 49 above with N-(2-((5-chloro-2-((2-methoxy-5-methyl-4-(4-(piperidin-4-ylmethyl)piperazin-1-yl)phenyl)amino)pyrimidin-4-yl)amino)phenyl)-N-methylmethanesulfonamide (109 mg, 0.17 mmol) and (2S)-2-((2-(2,6-dioxopiperidin-3-yl)-1,3-dioxoisoindolin-4-yl)oxy)propanoic acid (60 mg, 0.17 mmol) instead of 1-((1-(4-((5-chloro-4-((2-(N-methylmethylsulfonamido)phenyl)amino)pyrimidin-2-yl)amino)-3-methoxyphenyl)piperidin-4-yl)methyl)piperidin-4-carboxylic acid hydrochloride and 3-(4-amino-1-oxoisoindolin-2-yl)piperidin-2,6-dione, respectively.

The NMR and mass results of the compounds obtained in the Examples are summarized in Table 2 below.

**[Table 2]**

| Compound No. | NMR and MS Data |
|---|---|
| 1 | Step 1: ¹H NMR (400 MHz, CDCl₃): δ 9.40 (br s, 1H), 8.80 (d, 1H), 8.50 (br s, 1H), 7.71 (t, 1H), 7.54 (d, 1H), 4.94 (m, 1H), 3.44 (t, 2H), 2.70-2.94 (m, 3H), 2.50 (t, 2H), 2.11-2.21 (m, 1H), 1.87-2.00 (m, 4H) |
| | Step 2: ¹H NMR (400 MHz, CDCl₃): δ 8.00 (d, 1H), 6.41 (dd, 1H), 6.32 (d, 1H), 3.95 (s, 3H), 3.61 (m, 4H), 3.39 (m, 4H), 1.48 (s, 9H) |
| | Step 3: ¹H NMR (400 MHz, DMSO-*d₆*): δ 6.51 (m, 2H), 6.30 (dd, 1H), 4.27 (br s, 2H), 3.74 (s, 3H), 3.43 (m, 4H), 2.87 (m, 4H), 1.41 (s, 9H) |
| | Step 4: ¹H NMR (400 MHz, DMSO-*d₆*): δ 8.29 (br s, 1H), 8.27 (d, 1H), 8.14 (br s, 1H), 8.11 (s, 1H), 7.59 (dd, 1H), 7.42 (d, 1H), 7.27 (t, 1H), 7.17 (td, 1H), 6.66 (d, 1H), 6.47 (dd, 1H), 3.77 (s, 3H), 3.48 (m, 4H), 3.18 (s, 3H), 3.10 (m, 4H), 3.10 (s, 3H), 1.43 (s, 9H) |
| | Step 5: ¹H NMR (400 MHz, DMSO-*d₆*): δ 8.28 (br s, 1H), 8.27 (d, 1H), 8.13 (br s, 1H), 8.10 (s, 1H), 7.58 (dd, 1H), 7.37 (d, 1H), 7.25 (t, 1H), 7.16 (dt, 1H), 6.61 (d, 1H), 6.43 (dd, 1H), 3.76 (s, 3H), 3.18 (s, 3H), 3.10 (s, 3H), 3.06 (m, 4H), 2.87 (m, 4H) |
| | Compound 1: ¹H NMR (400 MHz, DMSO-*d₆*): δ 11.16 (br s, 1H), 9.73 (br s, 1H), 8.47 (d, 1H), 8.28 (br s, 1H), 8.27 (m, 1H), 8.13 (br s, 1H), 8.10 (s, 1H), 7.84 (t, 1H), 7.62 (d, 1H), 7.58 (dd, 1H), 7.37 (d, 1H), 7.25 (t, 1H), 7.16 (t, 1H), 6.62 (d, 1H), 6.43 (dd, 1H), 5.14 (dd, 1H), 3.76 (s, 3H), 3.18 (s, 3H), 3.13 (m, 4H), 3.10 (s, 3H), 2.88 (m, 1H), 2.46-2.67 (m, 5H), 2.37 (t, 2H), 2.04 (m, 1H), 1.68 (m, 1H), 1.55 (m, 2H), 1.25 (m, 2H), LC/MS (ESI) m/z 873 [M+H]⁺ |
| 2 | Step 1: ¹H NMR (400 MHz, DMSO-*d₆*): δ 7.92 (d, 1H), 6.62 (dd, 1H), 6.53 (d, 1H), 3.93 (s, 3H), 3.84 (t, 4H), 2.51 (m, 4H) |
| | Step 2: ¹H NMR (400 MHz, DMSO-*d₆*): δ 7.87 (d, 1H), 6.58 (dd, 1H), 6.50 (d, 1H), 4.06 (m, 2H), 3.90 (s, 3H), 3.28 (m, 4H), 2.94 (m, 2H), 2.54 (m, 1H), 2.43 (m, 4H), 1.82 (m, 2H), 2.43 (m, 2H), 1.39 (s, 9H) |
| | Step 3: ¹H NMR (400 MHz, DMSO-*d₆*): δ 6.49 (d, 1H), 6.47 (d, 1H), 6.28 (dd, 1H), 4.20 (br s, 2H), 3.73 (s, 3H), 3.43 (m, 2H), 2.30 (m, 4H), 2.44 (m, 4H), 2.26 (m, 1H), 1.78 (m, 4H), 1.51 (m, 2H), 1.39 (s, 9H) |
| | Step 4: ¹H NMR (400 MHz, DMSO-*d₆*): δ 8.28 (br s, 1H), 8.27 (br s, 1H), 8.11 (d, 1H), 8.10 (s, 1H), 7.58 (dd, 1H), 7.37 (d, 1H), 7.25 (t, 1H), 7.16 (td, 1H), 6.62 (d, 1H), 6.44 (dd, 1H), 3.79 (s, 3H), 3.73 (m, 2H), 3.31 (m, 4H), 3.18 (s, 3H), 3.10 (s, 3H), 2.65 (m, 2H), 2.46 (m, 4H), 2.37 (m, 1H), 1.83 (m, 2H), 1.53 (m, 2H), 1.40 (s, 9H) |
| | Step 5: ¹H NMR (400 MHz, DMSO-*d₆*): δ 8.25 (m, 1H), 8.24 (br s, 1H), 8.08 (br s, 1H), 8.06 (s, 1H), 7.54 (dd, 1H), 7.33 (d, 1H), 7.21 (t, 1H), 7.12 (td, 1H), 6.58 (d, 1H), 6.41 (dd, 1H), 3.71 (s, 3H), 3.69 (m, 2H), 3.14 (s, 3H), 3.06 (s, 3H), 3.02 (m, 4H), 2.64 (m, 4H), 2.39 (m, 1H), 1.79 (m, 2H), 1.50 (m, 2H) |
| | Compound 2: LC/MS (ESI) m/z 956 [M+H]⁺ |
| 3 | Step 1: ¹H NMR (400 MHz, CDCl₃): δ 11.11 (s, 1H), 7.82 (m, 1H), 7.52 (d, 1H), 7.45 (d, 1H), 5.09 (m, 1H), 4.26 (t, 2H), 3.67 (t, 2H) |
| | Step 2: ¹H NMR (400 MHz, CDCl₃): δ 7.88 (d, 1H), 6.60 (dd, 1H), 6.52(d, 1H), 4.15 (d, 2H), 3.91 (s, 3H), 2.99 (t, 2H), 2.65 (s, 3H), 2.50 (m, 1H), 1.66 (m, 4H), 1.40 (s, 9H) |
| | Step 3: ¹H NMR (400 MHz, CDCl₃): δ 6.51-6.49 (m, 2H), 6.31(dd, 1H), 4.23 (s, 2H), 3.73 (s, 3H), 3.45 (d, 2H), 2.69 (s, 3H), 2.54 (m, 3H), 1.78 (m, 2H), 1.58 (m, 2H), 1.40 (s, 9H) |
| | Step 4: ¹H NMR (400 MHz, CDCl₃): δ 8.29 (s, 2H), 8.11 (m, 2H), 7.59 (dd, 1H), 7.39 (d, 1H), 7.27 (t, 1H), 7.17 (m, 1H), 6.64 (d, 1H), 6.47 (dd, 1H), 4.76 (m, 5H), 3.18 (s, 3H), 3.10 (s, 3H), 2.70 (m, 5H), 2.50 (m, 1H), 1.80 (m, 2H), 1.64 (m, 2H), 1.42 (s, 9H) |
| | Step 5: ¹H NMR (400 MHz, CDCl₃): δ 8.28 (s, 2H), 8.11 (m, 2H), 7.59 (dd, 1H), 7.36 (d, 1H), 7.26 (t, 1H), 7.16 (m, 1H), 6.62 (d, 1H), 6.45 (dd, 1H), 3.75 (s, 3H), 3.61 (m, 2H), 3.18 (s, 3H), 3.10 (s, 3H), 2.73 (m, 5H), 2.41 (m, 1H), 2.30 (s, 3H), 1.90 (m, 2H), 1.34 (m, 2H) |
| | Compound 3: ¹H NMR (400 MHz, DMSO-*d₆*): δ 11.12 (br s, 1H), 8.30 (m, 1H), 8.28 (br s, 1H), 8.13 (br s, 1H), 8.10 (s, 1H), 7.84 (t, 1H), 7.58 (dd, 1H), 7.54 (d, 1H), 7.47 (d, 1H), 7.40 (d, 1H), 7.27 (t, 1H), 7.16 (td, 1H), 6.64 (d, 1H), 6.47 (dd, 1H), 5.08 (dd, 1H), 4.27 (m, 2H), 3.81 (m, 1H), 3.77 (s, 3H), 3.30-3.41 (m, 4H), 3.19 (s, 3H), 3.10 (s, 3H), 2.86 (m, 1H), 2.44-2.80 (m, 6H), 2.50 (s, 3H), 2.03 (m, 1H), 1.85 (m, 4H). LC/MS (ESI) m/z 874 [M+H]⁺ |
| 4 | Step 1: ¹H NMR (400 MHz, CDCl₃): δ 9.40 (br s, 1H), 8.83 (d, 1H), 8.06 (br s, 1H), 7.70 (t, 1H), 7.53 (d, 1H), 4.94 (m, 1H), 3.39 (t, 2H), 2.70-2.94 (m, 3H), 2.44 (t, 2H), 2.15-2.18 (m, 1H), 1.83 (m, 2H), 1.73 (m, 2H), 1.24-1.43 (m 12H) |
| | Compound 4: ¹H NMR (400 MHz, DMSO-*d₆*): δ 11.17 (br s, 1H), 9.70 (br s, 1H), 8.46 (d, 1H), 8.28 (br s, 1H), 8.27 (m, 1H), 8.13 (br s, 1H), 8.10 (s, 1H), 7.83 (dd, 1H), 7.62 (d, 1H), 7.60 (dd, 1H), 7.37 (d, 1H), 7.25 (t, 1H), 7.18 (td, 1H), 6.62 (d, 1H), 6.44 (dd, 1H), 5.14 (dd, 1H), 3.75 (s, 3H), 3.18 (s, 3H), 3.13 (m, 4H), 3.10 (s, 3H), 2.89 (m, 1H), 2.43-2.67 (m, 8H), 2.30 (t, 2H), 2.06 (m, 1H), 1.62 (m, 1H), 1.45 (m, 2H), 1.28 (m, 12H). LC/MS (ESI) m/z 957 [M+H]⁺ |
| 5 | ¹H NMR (400 MHz, DMSO-*d₆*): δ 11.13 (br s, 1H), 9.65 (br s, 1H), 8.42 (d, 1H), 8.24 (br s, 1H), 8.23 (m, 1H), 8.07 (br s, 1H), 8.06 (s, 1H), 7.79 (dd, 1H), 7.57 (d, 1H), 7.54 (dd, 1H), 7.31 (d, 1H), 7.20 (t, 1H), 7.12 (t, 1H), 6.57 (d, 1H), 6.40 (dd, 1H), 5.10 (dd, 1H), 3.71 (s, 3H), 3.67 (m, 2H), 3.29 (m, 2H), 3.14 (s, 3H), 3.06 (s, 3H), 2.85 (m, 1H), 2.39-2.64 (m, 10H), 2.24 (m, 5H), 2.02 (m, 1H), 1.81 (m, 2H), 1.58 (m, 2H), 1.47 (m, 2H), 1.35 (m, 2H), 1.21 (m, 12H). LC/MS (ESI) m/z 1040 [M+H]⁺ |
| 6 | Step 1: ¹H NMR (400 MHz, CDCl₃): δ 8.69 (br s, 1H), 7.36 (d, 2H), 7.35 (d, 2H), 7.26 (br t, 1H), 7.25 (s, 1H), 6.05 (br d, 1H), 4.72 (t, 1H), 4.57 (dd, 1H), 4.53 (m, 1H), 4.46 (dd, 1H), 4.08 (dd, 1H), 3.58 (dd, 1H), 3.38 (t, 2H), 2.82 (br s, 1H), 2.54-2.60 (m, 1H), 2.51 (s, 3H), 2.23 (t, 2H), 2.12 (m, 1H), 1.83-1.88 (m, 2H), 1.73-1.78 (m, 2H), 0.91 (s, 9H) Compound 6: LC/MS (ESI) m/z 1030 [M+H]⁺ |
| 7 | LC/MS (ESI) m/z 1113 [M+H]⁺ |
| 8 | Step 1: ¹H NMR (400 MHz, CDCl₃): δ 10.46 (br s, 1H), 8.85 (d, 1H), 8.07 (br s, 1H), 7.70 (t, 1H), 7.58 (d, 1H), 4.91-4.98 (m, 1H), 4.20 (s, 2H), 3.78-3.83 (m, 6H), 3.64 (t, 2H), 2.70-2.94 (m, 3H), 2.13-2.17 (m, 1H) |
| | Step 2: ¹H NMR (400 MHz, CDCl₃): δ 10.46 (br s, 1H), 8.86 (d, 1H), 8.01 (br s, 1H), 7.72 (t, 1H), 7.57 (d, 1H), 4.95-4.99 (m, 1H), 4.21 (s, 2H), 3.79-3.85 (m, 6H), 3.28 (t, 2H), 2.72-2.95 (m, 3H), 2.13-2.19 (m, 1H) |
| | Compound 8: ¹H NMR (400 MHz, DMSO-*d₆*): δ 11.19 (br s, 1H), 10.39 (br s, 1H), 8.73 (d, 1H), 8.28 (br s, 1H), 8.27 (m, 1H), 8.13 (br s, 1H), 8.10 (s, 1H), 7.85 (t, 1H), 7.62 (d, 1H), 7.58 (dd, 1H), 7.35 (d, 1H), 7.25 (t, 1H), 7.16 (td, 1H), 6.58 (d, 1H), 6.38 (dd, 1H), 5.17 (dd, 1H), 4.24 (s, 2H), 3.79 (m, 2H), 3.74 (s, 3H), 3.68 (m, 3H), 3.59 (t, 3H), 3.18 (s, 3H), 3.10 (s, 3H), 3.07 (m, 4H), 2.90 (m, 1H), 2.48-2.67 (m, 9H), 2.08 (m, 1H). LC/MS (ESI) m/z 919 [M+H]⁺ |
| 9 | ¹H NMR (400 MHz, DMSO-*d₆*): δ 11.21 (br s, 1H), 10.37 (br s, 1H), 8.74 (d, 1H), 8.28 (br s, 1H), 8.27 (m, 1H), 8.12 (br s, 1H), 8.10 (s, 1H), 7.88 (t, 1H), 7.64 (d, 1H), 7.58 (dd, 1H), 7.36 (d, 1H), 7.25 (t, 1H), 7.16 (td, 1H), 6.61 (d, 1H), 6.44 (dd, 1H), 5.16 (dd, 1H), 4.22 (s, 2H), 3.78 (m, 2H), 3.66 (m, 4H), 3.52 (t, 2H), 3.18 (s, 3H), 3.10 (s, 3H), 2.91 (m, 1H), 2.32-2.66 (m, 14H), 2.22 (m, 1H), 2.08 (m, 1H), 1.78 (m, 2H), 1.47 (m, 2H). LC/MS (ESI) m/z 1002 [M+H]⁺ |
| 10 | Step 1: ¹H NMR (400 MHz, CDCl₃): δ 4.02 (s, 2H), 3.76 (t, 2H), 3.67-3.73 (m, 4H), 3.26 (t, 2H), 1.46 (s, 9H) |
| | Step 2: ¹H NMR (400 MHz, CDCl₃): δ 8.39 (dd, 1H), 8.29 (br s, 1H), 8.06 (s, 1H), 8.02 (d, 1H), 8.00 (br s, 1H), 7.37 (td, 1H), 7.31 (dd, 1H), 7.17 (td, 1H), 6.51 (d, 1H), 6.44 (dd, 1H), 4.01 (s, 2H), 3.84 (s, 3H), 3.27 (s, 3H), 3.21 (br m, 4H), 2.98 (s, 3H), 2.94 (s, 4H), 2.87 (s, 4H), 2.74 (br m, 4H), 1.46 (s, 9H) |
| | Compound 10: ¹H NMR (400 MHz, DMSO-*d₆*): δ 8.97 (br s, 1H), 8.60 (br t, 1H), 8.28 br s, 1H), 8.26 (m, 1H), 8.12 (br s, 1H), 8.10 (s, 1H), 7.58 (dd, 1H), 7.37-7.45 (m, 2H), 7.39 (s, 4H), 7.25 (t, 1H), 7.16 (td, 1H), 6.60 (d, 1H), 6.42 (dd, 1H), 5.16 (d, 1H), 4.57 (d, 1H), 4.45 (d, 1H), 4.42 (dd, 1H), 4.36 (m, 1H), 4.25 (dd, 1H), 3.99 (s, 2H), 3.75 (s, 3H), 3.58-3.69 (m, 8H), 3.18 (s, 3H), 3.10 (s, 3H), 3.09 (m, 1H), 2.55 (m, 5H), 2.44 (s, 3H), 2.06 (m, 1H), 1.91 (m, 1H), 0.96 (s, 9H) |
| | LC/MS (ESI) m/z 1076 [M+H]⁺ |
| 11 | LC/MS (ESI) m/z 1071 [M+H]⁺ |
| 12 | Step 1: ¹H NMR (400 MHz, CDCl₃): δ 7.98 (br s, 1H), 7.67 (t, 1H), 7.47 (d, 1H), 7.27 (d, 1H), 4.94 (dd, 1H), 4.36 (t, 2H), 3.96 (t, 1H), 3.90 (t, 2H), 3.65 (t, 2H), 2.68-2.91 (m, 3H), 2.09-2.14 (m, 1H) |
| | Step 2: ¹H NMR (400 MHz, CDCl₃): δ 7.96 (br s, 1H), 7.68 (t, 1H), 7.47 (d, 1H), 7.28 (d, 1H), 4.94 (dd, 1H), 4.36 (t, 2H), 3.95 (t, 1H), 3.88 (t, 2H), 3.28 (t, 2H), 2.68-2.91 (m, 3H), 2.09-2.16 (m, 1H) |
| | Compound 12: ¹H NMR (400 MHz, DMSO-*d₆*): δ 11.12 (br s, 1H), 8.28 (br s, 1H), 8.27 (m, 1H), 8.13 (br s, 1H), 8.10 (s, 1H), 7.95 (br s, 1H), 7.83 (dd, 1H), 7.57 (t, 1H), 7.46 (d, 1H), 7.36 (d, 1H), 7.24 (t, 1H), 7.15 (t, 1H), 6.60 (d, 1H), 6.42 (dd, 1H), 5.08 (dd, 1H), 4.38 (t, 2H), 3.81 (t, 2H), 3.75 (s, 3H), 3.69 (t, 2H), 3.18 (s, 3H), 3.10 (s, 3H), 3.08 (m, 4H), 2.86 (m, 1H), 2.48-2.60 (m, 9H). LC/MS (ESI) m/z 862 [M+H]⁺ |
| 13 | ¹H NMR (400 MHz, DMSO-*d₆*): δ 11.13 (br s, 1H), 8.28 (br s, 1H), 8.27 (m, 1H), 8.12 (br s, 1H), 8.10 (s, 1H), 7.82 (t, 1H), 7.58 (dd, 1H), 7.54 (d, 1H), 7.47 (d, 1H), 7.37 (d, 1H), 7.26 (t, 1H), 7.16 (td, 1H), 6.64 (s, 1H), 6.65 (d, 1H), 5.09 (dd, 1H), 4.36 (t, 2H), 3.60-3.82 (m, 6H), 3.76 (s, 3H), 3.18 (s, 3H), 3.10 (s, 3H), 2.89 (m, 1H), 2.22-2.70 (m, 15H), 2.04 (m, 1H), 1.83 (m, 2H), 1.51 (m, 2H). LC/MS (ESI) m/z 945 [M+H]⁺ |
| 14 | ¹H NMR (400 MHz, DMSO-*d₆*): δ 11.12 (br s, 1H), 8.28 (br s, 1H), 8.27 (m, 1H), 8.13 (br s, 1H), 8.10 (s, 1H), 7.82 (br s, 1H), 7.58 (dd, 1H), 7.54 (d, 1H), 7.45 (d, 1H), 7.38 (d, 1H), 7.25 (t, 1H), 7.16 (td, 1H), 6.62 (s, 1H), 6.44 (d, 1H), 5.09 (dd, 1H), 4.27 (t, 2H), 3.76 (s, 3H), 3.18 (s, 3H), 3.11 (m, 4H), 3.10 (s, 3H), 2.88 (m, 1H), 2.38-2.62 (m, 8H), 2.03 (m, 1H), 1.82 (m, 2H), 1.70 (m, 2H). LC/MS (ESI) m/z 846 [M+H]⁺ |
| 15 | ¹H NMR (400 MHz, DMSO-*d₆*): δ 11.11 (br s, 1H), 8.28 (br s, 1H), 8.27 (m, 1H), 8.12 (br s, 1H), 8.10 (s, 1H), 7.81 (dd, 1H), 7.58 (dd, 1H), 7.52 (d, 1H), 7.44 (d, 1H), 7.36 (d, 1H), 7.25 (t, 1H), 7.16 (td, 1H), 6.61 (d, 1H), 6.44 (dd, 1H), 5.08 (dd, 1H), 4.24 (t. 2H), 3.75 (s, 3H), 3.70 (m, 2H), 3.18 (s, 3H), 3.10 (s, 3H), 2.88 (m, 1H), 2.25-2.69 (m, 15H), 2.03 (m, 1H), 1.84 (m, 2H), 1.78 (m, 2H), 1.62 (m, 2H), 1.51 (m, 2H). LC/MS (ESI) m/z 929 [M+H]⁺ |
| 16 | Step 1: ¹H NMR (400 MHz, CDCl₃): δ 11.01 (s, 1H), 7.95 (s, 1H), 7.59 (m, 1H), 1.15 (d, 1H), 7.05 (d, 1H), 6.61 (t, 1H), 5.06 (m, 1H), 4.62 (t, 1H), 3.62 (t, 2H), 3.53-3.46 (m, 6H), 2.89 (m, 1H), 2.61-2.53 (m, 2H), 2.03 (m, 1H) |
| | Step 2: ¹H NMR (400 MHz, CDCl₃): δ 7.77 (m, 2H), 7.57 (m, 1H), 7.44 (d, 2H), 7.11 (d, 1H), 7.05 (d, 1H), 6.54 (t, 1H), 5.07 (m, 1H), 4.13 (m, 2H), 3.61 (m, 2H), 3.53 (t, 2H), 3.40 (m, 2H), 2.89 (m, 1H), 2.62-2.54 (m, 2H), 2.37 (s, 3H), 2.03 (m, 1H) |
| | Compound 16: ¹H NMR (400 MHz, DMSO-*d₆*): δ 11.10 (br s, 1H), 8.28 (br s, 1H), 8.27 (m, 1H), 8.13 (br s, 1H), 8.10 (s, 1H), 7.56-7.62 (m, 2H), 7.36 (d, 1H), 7.24 (t, 1H), 7.17 (d, 1H), 7.14 (dd, 1H), 7.04 (d, 1H), 6.64 (br t, 1H), 6.59 (d, 1H), 6.41 (dd, 1H), 5.04 (dd, 1H), 3.75 (s, 3H), 3.63 (t, 2H), 3.61 (t, 2H), 3.50 (m, 2H), 3.18 (s, 3H), 3.10 (s, 3H), 3.08 (m, 4H), 2.84 (m, 1H), 2.47-2.59 (m, 8H), 1.97 (m, 1H). LC/MS (ESI) m/z 861 [M+H].⁺ |
| 17 | Step 1: ¹H NMR (400 MHz, CDCl₃): δ 7.98 (dd, 1H), 7.90 (dd, 1H), 6.91 (t, 1H), 3.61 (t, 4H), 3.24 (t, 4H), 1.47 (s, 9H) |
| | Step 2: ¹H NMR (400 MHz, DMSO-*d₆*): δ 6.77 (dd, 1H), 6.33 (dd, 1H), 6.29 (dd, 1H), 5.02 (br s, 2H), 3.42 (m, 2H), 3.35 (m, 2H), 2.75 (t, 4H), 1.41 (s, 9H) |
| | Step 3: ¹H NMR (400 MHz, DMSO-*d₆*): δ 9.50 (br s, 1H), 8.40 (br s, 1H), 8.26 (d, 1H), 8.23 (s, 1H), 7.65 (dd, 1H), 7.62 (d, 1H), 7.43 (td, 1H), 7.28 (td, 1H), 7.23 (d, 1H), 6.94 (t, 1H), 3.46 (t, 4H), 3.19 (s, 3H), 3.10 (s, 3H), 2.88 (t, 4H), 1.43 (s, 9H) |
| | Step 4: ¹H NMR (400 MHz, DMSO-*d₆*): δ 9.48 (br s, 1H), 8.40 (br s, 1H), 8.27 (d, 1H), 8.22 (s, 1H), 7.64 (dd, 1H), 7.60 (d, 1H), 7.42 (td, 1H), 7.28 (td, 1H), 7.23 (d, 1H), 6.92 (t, 1H), 3.20 (s, 3H), 3.10 (s, 3H), 2.92 (s, 8H) |
| | Compound 17: ¹H NMR (400 MHz, DMSO-*d₆*): δ 9.47 (br s, 1H), 8.98 (s, 1H), 8.58 (br t, 1H), 8.39 (br s, 1H), 8.27 (d, 1H), 8.22 (s, 1H), 7.88 (br d, 1H), 7.64 (dd, 1H), 7.58 (d, 1H), 7.40 (m, 5H), 7.27 (td, 1H), 7.22 (d, 1H), 6.90 (t, 1H), 2.48 (m, 3H), 2.25-2.33 (m, 3H), 2.16 (m, 1H), 2.03 (m, 1H), 1.90 (m, 1H), 1.51 (m, 2H), 1.42 (m, 2H), 0.95 (s, 9H), LC/MS (ESI) m/z 1018 [M+H]⁺ |
| 18 | Step 1: ¹H NMR (400 MHz, CDCl₃): δ 7.93-8.02 (m, 2H), 6.97 (t, 1H), 3.64 (t, 4H), 2.64 (t, 4H) |
| | Step 2: ¹H NMR (400 MHz, DMSO-*d₆*): δ 7.98-8.01 (m, 2H), 7.16 (t, 1H), 3.71 (m, 2H), 3.29 (m, 4H), 2.91 (t, 2H), 2.45 (t, 4H), 1.84 (m, 2H), 1.53 (m, 2H), 1.39 (s, 9H) |
| | Step 3: ¹H NMR (400 MHz, DMSO-*d₆*): δ 6.75 (t, 1H), 6.26-6.33 (m, 2H), 4.95 (br s, 2H), 3.29 (m, 4H), 3.11 (m, 2H), 2.45 (t, 4H), 2.28 (t, 1H), 1.76 (m, 2H), 1.53 (m, 2H), 1.39 (s, 9H) |
| | Step 4: ¹H NMR (400 MHz, DMSO-*d₆*): δ 9.46 (br s, 1H), 8.40 (br s, 1H), 8.27 (d, 1H), 8.22 (s, 1H), 7.64 (dd, 1H), 7.58 (d, 1H), 7.42 (td, 1H), 7.28 (td, 1H), 7.20 (d, 1H), 6.91 (t, 1H), 3.30 (m, 6H), 3.19 (s, 3H), 3.10 (s, 3H), 2.59 (m, 2H), 2.46 (t, 4H), 2.33 (m, 1H), 1.81 (m, 2H), 1.56 (m, 2H), 1.40 (s, 9H) |
| | Step 5: ¹H NMR (400 MHz, DMSO-*d₆*): δ 9.47 (br s, 1H), 8.40 (br s, 1H), 8.27 (m, 1H), 8.22 (s, 1H), 7.64 (dd, 1H), 7.58 (d, 1H), 7.42 (td, 1H), 7.28 (td, 1H), 7.21 (d, 1H), 6.92 (d, 1H), 3.30 (m, 2H), 3.19 (s, 3H), 3.10 (s, 3H), 3.04 (m, 4H), 2.67 (m, 4H), 2.60 (t, 2H), 2.37 (m, 1H), 1.82 (m, 2H), 1.57 (m, 2H) |
| | Compound 18: LC/MS (ESI) m/z 1101 [M+H]⁺ |
| 19 | LC/MS (ESI) m/z 1058 [M+H]⁺ |
| 20 | LC/MS (ESI) m/z 1085 [M+H]⁺ |
| 21 | ¹H NMR (400 MHz, DMSO-*d₆*): δ 11.23 (br s, 1H), 8.78 (d, 1H), 8.28 (m, 2H), 8.11 (s, 2H), 7.86 (t, 1H), 7.61 (d, 1H), 7.58 (dd, 1H), 7.38 (d, 1H), 7.29 (t, 1H), 7.16 (t, 1H), 6.67 (d, 1H), 6.46 (dd, 1H), 5.17 (dd, 1H), 3.78 (s, 3H), 3.73 (m, 2H), 3.38 (m, 2H), 3.21 (m, 2H), 3.19 (s, 3H), 3.10 (s, 3H), 2.95 (m, 1H), 2.54-2.71 (m, 11H), 2.40 (m, 1H), 2.12 (m, 1H), 1.87 (m, 2H), 1.59 (m, 2H). LC/MS (ESI) m/z 914 [M+H]⁺ |
| 22 | ¹H NMR (400 MHz, DMSO-*d₆*): δ 11.05 (br s, 1H), 10.07 (br s, 1H), 8.78 (d, 1H), 8.28 (m, 2H), 8.11 (s, 1H), 8.10 (s, 1H), 7.83 (dd, 1H), 7.52-7.59 (m, 3H), 7.36 (d, 1H), 7.25 (t, 1H), 7.18 (t, 1H), 6.63 (d, 1H), 6.45 (dd, 1H), 5.17 (dd, 1H), 4.37 (q, 1H), 3.80 (s, 3H), 3.72 (m, 2H), 3.34 (m, 2H), 3.18 (s, 3H), 3.17 (m, 2H), 3.10 (s, 3H), 2.92 (m, 1H), 2.54-2.71 (m, 11H), 2.36 (m, 1H), 2.06 (m, 1H), 1.85 (m, 2H), 1.57 (m, 2H). LC/MS (ESI) m/z 900 [M+H]⁺ |
| 23 | Step 1: ¹H NMR (400 MHz, CDCl₃): δ 7.97 (d, 1H), 6.39 (dd, 1H), 6.28 (d, 1H), 3.93 (s, 3H), 3.88 (m, 2H), 3.40 (m, 4H), 2.93 (m, 2H), 2.34 (m, 4H), 2.19 (d, 2H), 1.86 (m, 2H), 1.77 (m, 1H), 1.45 (s, 9H), 1.26 (m, 2H) |
| | Step 2: ¹H NMR (400 MHz, DMSO-*d₆*): δ 6.49 (d, 1H), 6.47 (d, 1H), 6.28 (dd, 1H), 4.19 (br s, 2H), 3.73 (s, 3H), 3.35 (m, 2H), 3.30 (m, 4H), 2.47 (m, 2H), 2.28 (m, 4H), 2.15 (d, 2H), 1.75 (m, 2H), 1.56 (m, 1H), 1.39 (s, 9H), 1.23 (m, 2H) Step 3: ¹H NMR (400 MHz, DMSO-*d₆*): δ 8.28 (m, 2H), 8.11 (br s, 1H), 7.57 (d, 1H), 7.35 (d, 1H), 7.25 (t, 1H), 7.16 (td, 1H), 6.61 (d, 1H), 6.44 (dd, 1H), 3.75 (s, 3H), 3.66 (m, 2H), 3.30 (m, 4H), 3.18 (s, 3H), 3.10 (s, 1H), 2.64 (m, 2H), 2.30 (m, 4H), 2.17 (d, 2H), 1.79 (m, 2H), 1.67 (m, 1H), 1.40 (s, 9H), 1.24 (m, 2H) |
| | Step 4: ¹H NMR (400 MHz, DMSO-*d₆*): δ 8.29 (m, 2H), 8.13 (br s, 1H), 7.58 (dd, 1H), 7.36 (d, 1H), 7.26 (t, 1H), 7.16 (td, 1H), 6.61 (d, 1H), 6.45 (dd, 1H), 3.76 (s, 3H), 3.67 (m, 2H), 3.34 (m, 4H), 3.19 (s, 3H), 3.11 (s, 1H), 3.01 (m, 4H), 2.66 (m, 2H), 2.21 (d, 2H), 1.80 (m, 2H), 1.67 (m, 1H), 1.23 (m, 2H) |
| | Compound 23: ¹H NMR (400 MHz, DMSO-*d₆*): δ 11.16 (br s, 1H), 10.99 (br s, 1H), 8.79 (d, 1H), 8.28 (m, 2H), 8.11 (s, 1H), 8.10 (s, 1H), 7.86 (t, 1H), 7.60 (d, 1H), 7.59 (d, 1H), 7.36 (d, 1H), 7.25 (t, 1H), 7.16 (t, 1H), 6.61 (d, 1H), 6.44 (dd, 1H), 5.16 (dd, 1H), 3.75 (s, 3H), 3.23 (q, 1H), 3.18 (s, 3H), 3.10 (s, 3H), 2.92 (m, 1H), 2.50-2.70 (m, 12H), 2.23 (d, 2H), 2.10 (m, 1H), 1.82 (m, 2H), 1.68 (m, 1H), 1.23 (m, 2H). LC/MS (ESI) m/z 928 [M+H]⁺ |
| 24 | ¹H NMR (400 MHz, DMSO-*d₆*): δ 11.02 (br s, 1H), 9.72 (br s, 1H), 8.28 (m, 2H), 8.11 (s, 1H), 8.10 (s, 1H), 7.80 (dd, 1H), 7.51-7.60 (m, 3H), 7.36 (d, 1H), 7.25 (t, 1H), 7.16 (t, 1H), 6.61 (d, 1H), 6.44 (dd, 1H), 5.13 (dd, 1H), 4.38 (q, 1H), 3.75 (s, 3H), 3.67 (m, 2H), 3.18 (s, 3H), 3.17 (m, 1H), 3.10 (s, 3H), 2.92 (m, 1H), 2.34-2.68 (m, 12H), 2.19 (d, 2H), 2.03 (m, 1H), 1.80 (m, 2H), 1.68 (m, 1H), 1.24 (m, 2H). LC/MS (ESI) m/z 914 [M+H]⁺ |
| 25 | Step 1: ¹H NMR (400 MHz, CDCl₃): δ 7.98 (d, 1H), 7.33 (m, 5H), 6.40 (dd, 1H), 6.29 (d, 1H), 5.12 (s, 2H), 4.18 (m, 2H), 3.94 (s, 3H), 3.37 (m, 4H), 2.78 (m, 2H), 2.53 (m, 4H), 2.21 (d, 2H), 1.75 (m, 3H), 1.10 (m, 2H) |
| | Step 2: ¹H NMR (400 MHz, DMSO-d6): δ 7.34 (m, 5H), 6.50 (d, 1H), 6.48 (d, 1H), 6.27 (dd, 1H), 5.06 (s, 2H), 4.20 (br s, 2H), 4.01 (m, 2H), 3.73 (s, 3H), 2.93 (m, 4H), 2.81 (m, 2H), 2.46 (m, 4H), 2.15 (d, 2H), 1.70 (m, 3H), 0.98 (m, 2H) |
| | Step 3: ¹H NMR (400 MHz, DMSO-*d₆*): δ 8.28 (br s, 1H), 8.27 (m, 1H), 8.12 (br s, 1H), 7.58 (dd, 1H), 7.32-7.40 (m, 6H), 7.25 (t, 1H), 7.16 (dd, 1H), 6.62 (d, 1H), 6.44 (dd, 1H), 5.07 (s, 2H), 4.01 (m, 2H), 3.76 (s, 3H), 3.18 (s, 3H), 3.13 (m, 4H), 3.10 (s, 3H), 2.84 (m, 2H), 2.50 (m, 4H), 2.18 (d, 2H), 1.73 (m, 3H), 1.00 (m, 2H) |
| | Step 4: ¹H NMR (400 MHz, DMSO-*d₆*): δ 8.28 (br s, 1H), 8.27 (m, 1H), 8.12 (br s, 1H), 7.58 (dd, 1H), 7.38 (d, 1H), 7.25 (t, 1H), 7.16 (dd, 1H), 6.62 (d, 1H), 6.43 (dd, 1H), 3.75 (s, 3H), 3.18 (s, 3H), 3.13 (m, 4H), 3.10 (s, 3H), 2.90 (m, 2H), 2.49 (m, 4H), 2.44 (m, 2H), 2.15 (d, 2H), 1.63 (m, 3H), 0.97 (m, 2H) |
| | Compound 25: ¹H NMR (400 MHz, DMSO-*d₆*): δ 11.17 (br s, 1H), 11.10 (br s, 1H), 8.79 (d, 1H), 8.28 (m, 2H), 8.12 (s, 1H), 8.10 (s, 1H), 7.86 (t, 1H), 7.60 (d, 1H), 7.59 (d, 1H), 7.39 (d, 1H), 7.26 (t, 1H), 7.17 (t, 1H), 6.63 (d, 1H), 6.44 (dd, 1H), 5.16 (dd, 1H), 3.76 (s, 3H), 3.33 (m, 2H), 3.20 (q, 1H), 3.18 (s, 3H), 3.15 (m, 4H), 3.10 (s, 3H), 2.89 (m, 3H), 2.50-2.65 (m, 4H), 2.23-2.32 (m, 4H), 2.11 (m, 1H), 1.74 (m, 2H), 1.61 (m, 1H), 1.40 (m, 2H). LC/MS (ESI) m/z 928 [M+H]⁺ |
| 26 | ¹H NMR (400 MHz, DMSO-*d₆*): δ 11.03 (br s, 1H), 9.73 (br s, 1H), 8.28 (m, 2H), 8.12 (s, 1H), 8.10 (s, 1H), 7.80 (dd, 1H), 7.51-7.59 (m, 3H), 7.38 (d, 1H), 7.25 (t, 1H), 7.17 (t, 1H), 6.63 (d, 1H), 6.44 (dd, 1H), 5.13 (dd, 1H), 4.38 (q, 1H), 3.76 (s, 3H), 3.33 (m, 2H), 3.18 (s, 3H), 3.15 (m, 6H), 3.10 (s, 3H), 2.92 (m, 3H), 2.60 (m, 1H), 2.50 (m, 2H), 2.38 (m, 1H), 2.14-2.22 (m, 4H), 2.04 (m, 1H), 1.73 (m, 2H), 1.56 (m, 1H), 1.25 (m, 2H). LC/MS (ESI) m/z 914 [M+H]⁺ |
| 27 | ¹H NMR (400 MHz, DMSO-*d₆*): δ 9.00 (s, 1H), 8.63 (t, 1H), 8.28 (m, 2H), 8.13 (s, 1H), 8.10 (s, 1H), 7.81 (d, 1H), 7.57 (dd, 1H), 7.37-7.44 (m, 5H), 7.26 (t, 1H), 7.17 (t, 1H), 6.62 (d, 1H), 6.44 (dd, 1H), 5.16 (d, 1H), 4.50 (d, 1H), 4.26-4.46 (m, 4H), 3.76 (s, 3H), 3.63 (m, 2H), 3.18 (s, 3H), 3.13 (m, 4H), 3.10 (s, 3H), 2.81-3.04 (m, 4H), 2.47 (m, 4H), 2.45 (s, 3H), 2.03-2.19 (m, 5H), 1.90 (m, 1H), 1.74 (m, 2H), 1.57 (m, 1H), 1.16 (m, 2H), 0.95 (s, 9H). LC/MS (ESI) m/z 1085 [M+H]⁺ |
| 28 | LC/MS (ESI) m/z 1085 [M+H]⁺ |
| 29 | Step 1: ¹H NMR (400 MHz, DMSO-*d₆*): δ 7.86 (d, 1H), 6.56 (dd, 1H), 6.47 (d, 1H), 4.02 (m, 2H), 3.88 (s, 3H), 2.95 (m, 2H), 2.74 (m, 2H), 2.14 (m, 1H), 2.10 (m, 2H), 1.91 (m, 2H), 1.75 (m, 5H), 1.50 (m, 2H), 1.39 (s, 9H), 1.09 (m, 2H) |
| | Step 2: ¹H NMR (400 MHz, DMSO-*d₆*): δ 6.49 (d, 1H), 6.47 (d, 1H), 6.28 (dd, 1H), 4.19 (br s, 2H), 3.73 (s, 3H), 3.36 (m, 2H), 2.72 (m, 2H), 2.45 (m, 2H), 2.16 (m, 1H), 2.11 (m, 2H), 1.91 (m, 2H), 1.73 (m, 4H), 1.53 (m, 3H), 1.39 (s, 9H), 1.20 (m, 2H) |
| | Step 3: ¹H NMR (400 MHz, DMSO-*d₆*): δ 8.28 (m, 2H), 8.11 (br s, 1H), 7.58 (d, 1H), 7.35 (d, 1H), 7.24 (t, 1H), 7.16 (td, 1H), 6.60 (d, 1H), 6.45 (dd, 1H), 3.75 (s, 3H), 3.65 (m, 2H), 3.18 (s, 3H), 3.10 (s, 3H), 2.75 (m, 2H), 2.64 (m, 2H), 2.16 (m, 1H), 2.13 (m, 2H), 1.92 (m, 2H), 1.76 (m, 4H), 1.54 (m, 3H), 1.40 (s, 9H), 1.20 (m, 2H) |
| | Compound 29: LC/MS (ESI) m/z 1070 [M+H]⁺ |
| 30 | Step 1: ¹H NMR (400 MHz, DMSO-*d₆*): δ 7.87 (d, 1H), 6.58 (dd, 1H), 6.47 (d, 1H), 3.90 (s, 3H), 3.46 (t, 4H), 3.35 (t, 4H), 1.54 (t, 4H), 1.42 (t, 4H), 1.40 (s, 9H) |
| | Step 2: ¹H NMR (400 MHz, DMSO-*d₆*): δ 6.50 (d, 1H), 6.48 (d, 1H), 6.29 (dd, 1H), 4.18 (br s, 2H), 3.73 (s, 3H), 3.31 (t, 4H), 2.91 (t, 4H), 1.56 (t, 4H), 1.39 (s, 9H), 1.38 (t, 4H) |
| | Step 3: ¹H NMR (400 MHz, DMSO-*d₆*): δ 8.28 (m, 2H), 8.10 (m, 2H), 7.58 (dd, 1H), 7.38 (d, 1H), 7.26 (t, 1H), 7.16 (td, 1H), 6.61 (d, 1H), 6.45 (dd, 1H), 3.74 (s, 3H), 3.35 (m, 4H), 3.18 (s, 3H), 3.14 (m, 4H), 3.07 (s, 3H), 1.59 (m, 4H), 1.43 (m, 4H), 1.40 (s, 9H) |
| | Step 4: ¹H NMR (400 MHz, DMSO-*d₆*): δ 8.29 (m, 2H), 8.10 (m, 2H), 7.58 (dd, 1H), 7.39 (d, 1H), 7.26 (t, 1H), 7.16 (td, 1H), 6.62 (d, 1H), 6.46 (dd, 1H), 3.76 (s, 3H), 3.19 (s, 3H), 3.14 (m, 4H), 3.11 (s, 3H), 3.08 (m, 4H), 1.62 (m, 8H) |
| | Compound 30: ¹H NMR (400 MHz, DMSO-*d₆*): δ 11.16 (br s, 1H), 11.66 (br s, 1H), 8.61 (d, 1H), 8.28 (m, 2H), 8.10 (s, 2H), 7.83 (t, 1H), 7.61 (d, 1H), 7.58 (d, 1H), 7.37 (d, 1H), 7.27 (t, 1H), 7.16 (t, 1H), 6.61 (d, 1H), 6.45 (dd, 1H), 5.15 (dd, 1H), 3.76 (s, 3H), 3.18 (s, 3H), 3.13 (m, 4H), 3.10 (s, 3H), 2.93 (m, 1H), 2.64 (m, 6H), 2.48 (m, 4H), 2.09 (m, 1H), 1.57 (m, 8H). LC/MS (ESI) m/z 913 [M+H]⁺ |
| 31 | ¹H NMR (400 MHz, DMSO-*d₆*): δ 8.99 (br s, 1H), 8.62 (br t, 1H), 8.29 (m, 1H), 8.28 br s, 1H), 8.10 (s, 1H), 8.09 (br s, 1H), 7.83 (d, 1H), 7.58 (dd, 1H), 7.37-7.45 (m, 5H), 7.26 (t, 1H), 7.16 (td, 1H), 6.60 (d, 1H), 6.44 (dd, 1H), 5.16 (d, 1H), 4.50 (d, 1H), 4.36-4.46 (m, 3H), 4.25 (dd, 1H), 3.76 (s, 3H), 3.63 (m, 2H), 3.35 (m, 4H), 3.18 (s, 3H), 3.11 (m, 4H), 3.10 (s, 3H), 2.98 (dd, 2H), 2.45 (s, 3H), 2.06 (m, 1H), 1.91 (m, 1H), 0.95 (s, 9H). LC/MS (ESI) m/z 1056 [M+H]⁺ |
| 32 | Step 1: ¹H NMR (400 MHz, DMSO-*d₆*): δ 7.88 (d, 1H), 6.01 (dd, 1H), 5.98 (d, 1H), 4.16 (s, 4H), 4.05 (s, 4H), 3.87 (s, 3H), 1.38 (s, 9H) |
| | Step 2: ¹H NMR (400 MHz, DMSO-*d₆*): δ 6.49 (d, 1H), 5.99 (d, 1H), 5.81 (dd, 1H), 4.06 (br, s, 2H), 3.98 (s, 4H), 3.76 (s, 4H), 3.72 (s, 3H), 1.38 (s, 9H) |
| | Step 3: ¹H NMR (400 MHz, DMSO-*d₆*): δ 8.28 (m, 1H), 8.26 (br s, 1H), 8.10 (br s, 1H), 8.08 (s, 1H), 7.58 (dd, 1H), 7.29 (d, 1H), 7.23 (t, 1H), 7.16 (td, 1H), 6.11 (d, 1H), 5.97 (dd, 1H), 4.04 (s, 4H), 3.93 (s, 4H), 3.72 (s, 3H), 3.18 (s, 3H), 3.10 (s, 3H), 1.39 (s, 9H) |
| | Step 4: ¹H NMR (400 MHz, DMSO-*d₆*): δ 8.28 (m, 1H), 8.27 (br s, 1H), 8.11 (br s, 1H), 8.08 (s, 1H), 7.58 (dd, 1H), 7.29 (d, 1H), 7.24 (t, 1H), 7.16 (td, 1H), 6.12 (d, 1H), 5.99 (dd, 1H), 4.04 (s, 4H), 3.95 (s, 4H), 3.72 (s, 3H), 3.19 (s, 3H), 3.11 (s, 3H) |
| | Compound 32: ¹H NMR (400 MHz, DMSO-*d₆*): δ 11.31 (br s, 1H), 11.20 (br s, 1H), 8.68 (d, 1H), 8.26 (m, 1H), 8.25 (br s, 1H), 8.12 (br s, 1H), 8.08 (s, 1H), 7.83 (t, 1H), 7.60 (d, 1H), 7.57 (d, 1H), 7.12-7.27 (m, 3H), 6.09 (d, 1H), 5.94 (dd, 1H), 5.19 (dd, 1H), 3.91 (s, 4H), 3.72 (s, 3H), 3.46 (s, 4H), 3.18 (s, 3H), 3.10 (s, 3H), 2.92 (m, 1H), 2.74 (m, 2H), 2.60 (m, 2H), 2.43 (m, 2H), 2.14 (m, 1H). LC/MS (ESI) m/z 857 [M+H]⁺ |
| 33 | Step 1: ¹H NMR (400 MHz, DMSO-*d₆*): δ 7.33 (m, 5H), 4.48 (s, 2H), 4.25 (m, 1H), 3.98 (m, 2H), 3.64 (m, 2H), 3.53 (m, 4H) |
| | Step 2: ¹H NMR (400 MHz, CDCl₃): δ 7.28 (m, 5H), 4.54 (s, 2H), 4.35 (m, 1H), 3.65 (m, 4H), 3.56 (m, 4H) |
| | Step 3: ¹H NMR (400 MHz, CDCl₃): δ 7.97 (br s, 1H), 7.61 (d, 1H), 7.28 (m, 5H), 6.75 (d, 1H), 6.48 (dd, 1H), 4.90 (dd, 1H), 4.56 (s, 2H), 4.51 (m, 1H), 4.19 (dd, 2H), 3.90 (dd, 2H), 3.63 (m, 4H), 2.78 (m, 3H), 2.10 (m, 1H) |
| | Step 4: ¹H NMR (400 MHz, CDCl₃): δ 8.08 (br s, 1H), 7.63 (d, 1H), 6.78 (d, 1H), 6.51 (dd, 1H), 4.91 (dd, 1H), 4.52 (m, 1H), 4.23 (dd, 2H), 3.91 (dd, 2H), 3.78 (dd, 2H), 3.57 (dd, 2H), 2.78 (m, 3H), 2.10 (m, 1H) |
| | Step 5: ¹H NMR (400 MHz, CDCl₃): δ 7.98 (br s, 1H), 7.63 (d, 1H), 6.78 (d, 1H), 6.52 (dd, 1H), 4.90 (dd, 1H), 4.52 (m, 1H), 4.37 (m, 2H), 4.24 (dd, 2H), 3.91 (dd, 2H), 3.74 (m, 2H), 3.05 (s, 3H), 2.79 (m, 3H), 2.11 (m, 1H) |
| | Compound 33: ¹H NMR (400 MHz, DMSO-*d₆*): δ 11.08 (br s, 1H), 8.28 (br s, 1H), 8.27 (m, 1H), 8.12 (br s, 1H), 8.10 (s, 1H), 7.65 (d, 1H), 7.57 (dd, 1H), 7.39 (d, 1H), 7.25 (t, 1H), 7.15 (td, 1H), 6.82 (d, 1H), 6.67 (dd, 1H), 6.63 (d, 1H), 6.44 (dd, 1H), 5.05 (dd, 1H), 4.50 (m, 1H), 4.27 (dd, 2H), 3.88 (dd, 2H), 3.75 (s, 3H), 3.60 (t, 2H), 3.18 (s, 3H), 3.15 (m, 4H), 3.10 (s, 3H), 2.87 (m, 1H), 2.58 (m, 8H), 1.99 (m, 1H). LC/MS (ESI) m/z 873 [M+H]⁺ |
| 34 | Step 1: ¹H NMR (400 MHz, CDCl₃): δ 7.28 (m, 5H), 4.56 (s, 2H), 3.75 (m, 2H), 3.62 (m, 4H), 3.47 (m, 1H), 3.04 (m, 2H), 1.81 (m, 2H), 1.50 (m, 2H), 1.43 (s, 9H) |
| | Step 3: ¹H NMR (400 MHz, CDCl₃): δ 8.00 (br s, 1H), 7.65 (d, 1H), 7.25-7.33 (m, 6H), 7.03 (dd, 1H), 4.91 (dd, 1H), 4.56 (s, 2H), 3.61-3.74 (m, 7H), 3.24 (m, 2H), 2.79 (m, 3H), 2.11 (m, 1H), 1.95 (m, 2H), 1.72 (m, 2H) |
| | Step 4: ¹H NMR (400 MHz, CDCl₃): δ 8.07 (br s, 1H), 7.65 (d, 1H), 7.27 (d, 1H), 7.04 (dd, 1H), 4.92 (dd, 1H), 3.67-3.75 (m, 4H), 3.59-3.65 (m, 3H), 3.25 (m, 2H), 2.79 (m, 3H), 2.11 (m, 1H), 1.98 (m, 2H), 1.72 (m, 2H) |
| | Step 5: ¹H NMR (400 MHz, CDCl₃): δ 7.96 (br s, 1H), 7.66 (d, 1H), 7.28 (d, 1H), 7.06 (dd, 1H), 4.92 (dd, 1H), 4.36 (m, 2H), 3.76 (m, 2H), 3.67 (m, 3H), 3.26 (m, 2H), 3.04 (s, 3H), 2.79 (m, 3H), 2.11 (m, 1H), 1.98 (m, 2H), 1.72 (m, 2H) |
| | Compound 34: ¹H NMR (400 MHz, DMSO-*d₆*): δ 11.04 (br s, 1H), 8.24 (s, 1H), 8.23 (m, 1H), 8.08 (br s, 1H), 8.06 (s, 1H), 7.61 (dd, 1H), 7.53 (dd, 1H), 7.33 (d, 1H), 7.30 (m, 1H), 7.21 (m, 2H), 7.11 (td, 1H), 6.58 (d, 1H), 6.40 (dd, 1H), 5.02 (dd, 1H), 3.75 (m, 2H), 3.71 (s, 3H), 3.58 (t, 2H), 3.44 (m, 1H), 3.23 (m, 2H), 3.14 (s, 3H), 3.10 (m, 4H), 3.06 (s, 3H), 2.81 (m, 1H), 2.57 (m, 5H), 2.52 (m, 2H), 1.98 (m, 1H), 1.90 (m, 2H), 1.47 (m, 2H). LC/MS (ESI) m/z 901 [M+H]⁺ |
| 35 | Step 1: ¹H NMR (400 MHz, CDCl₃): δ 8.01 (d, 1H), 7.29-7.35 (m, 5H), 6.83 (d, 1H), 6.70 (dd, 1H), 5.12 (s, 2H), 4.19 (br s, 2H), 3.36 (m, 4H), 2.78 (m, 2H), 2.52 (m, 4H), 2.21 (d, 2H), 1.75 (m, 2H), 1.68 (m, 1H), 1.11 (m, 2H) |
| | Step 3: ¹H NMR (400 MHz, DMSO-*d₆*): δ 8.71 (br s, 1H), 8.25 (br s, 1H), 8.18 (m, 1H), 8.07 (s, 1H), 7.53 (dd, 1H), 7.26-7.36 (m, 6H), 7.09 (m, 2H), 6.99 (d, 1H), 6.87 (dd, 1H), 5.03 (s, 2H), 3.96 (m, 2H), 3.14 (s, 3H), 3.12 (m, 4H), 3.06 (s, 3H), 2.79 (m, 1H), 2.45 (m, 5H), 2.14 (d, 2H), 1.71 (m, 3H), 0.96 (m, 2H) |
| | Step 4: ¹H NMR (400 MHz, DMSO-*d₆*): δ 8.76 (br s, 1H), 8.29 (br s, 1H), 8.22 (m, 1H), 8.11 (s, 1H), 7.57 (dd, 1H), 7.31 (d, 1H), 7.11-7.18 (m, 2H), 7.03 (d, 1H), 6.91 (dd, 1H), 3.18 (s, 3H), 3.16 (m, 4H), 3.10 (s, 3H), 2.92 (m, 2H), 2.44-2.54 (m, 6H), 2.15 (d, 2H), 1.64 (m, 3H), 0.99 (m, 2H) |
| | Step 5: ¹H NMR (400 MHz, DMSO-*d₆*): δ 8.72 (br s, 1H), 8.25 (br s, 1H), 8.18 (m, 1H), 8.07 (s, 1H), 7.53 (dd, 1H), 7.27 (d, 1H), 7.07-7.14 (m, 2H), 6.98 (d, 1H), 6.87 (dd, 1H), 3.14 (s, 3H), 3.12 (m, 4H), 3.06 (s, 3H), 3.02 (s, 2H), 2.76 (m, 2H), 2.43-2.46 (m, 6H), 2.13 (d, 2H), 2.09 (m, 2H), 1.63 (m, 2H), 1.45 (m, 1H), 1.37 (s, 9H), 1.07 (m, 2H) |
| | Compound 35: ¹H NMR (400 MHz, DMSO-*d₆*): δ 9.00 (s, 1H), 8.77 s, 1H), 8.63 (t, 1H), 8.29 (s, 1H), 8.22 (m, 1H), 8.12 (s, 1H), 7.81 (d, 1H), 7.57 (dd, 1H), 7.39-7.44 (m, 4H), 7.31 (d, 1H), 7.11-7.18 (m, 2H), 7.03 (d, 1H), 6.91 (dd, 1H), 5.16 (d, 1H), 4.50 (d, 1H), 4.26-4.46 (m, 4H), 3.18 (s, 3H), 3.15 (m, 4H), 3.10 (s, 3H), 2.80-3.04 (m, 4H), 2.46 (m, 4H), 2.45 (s, 3H), 2.03-2.19 (m, 5H), 1.90 (m, 1H), 1.74 (m, 2H), 1.55 (m, 1H), 1.16 (m, 2H), 0.95 (s, 9H). LC/MS (ESI) m/z 1089 [M+H]⁺ |
| 36 | ¹H NMR (400 MHz, DMSO-*d₆*): δ 9.47 (s, 1H), 8.99 (s, 1H), 8.62 (t, 1H), 8.39 (s, 1H), 7.27 (m, 1H), 8.22 (s, 1H), 7.80 (d, 1H), 7.64 (dd, 1H),7.59 (d, 1H), 7.39-7.46 (m, 5H), 7.28 (t, 1H), 7.22 (d, 1H), 6.90 (t, 1H), 5.15 (d, 1H), 4.50 (d, 1H), 4.25-4.46 (m, 4H), 3.63 (m, 2H), 3.19 (s, 3H), 3.10 (s, 3H), 2.80-3.03 (m, 8H), 2.46 (m, 4H), 2.45 (s, 3H), 2.03-2.18 (m, 5H), 1.90 (m, 1H), 1.74 (m, 2H), 1.53 (m, 1H), 1.14 (m, 2H), 0.95 (s, 9H). LC/MS (ESI) m/z 1073 [M+H]⁺ |
| 37 | Step 1: ¹H NMR (400 MHz, CDCl₃): δ 8.40 (d, 1H), 8.32 (br s, 1H), 8.07 (s, 1H), 8.06 (m, 1H), 7.39 (td, 1H), 7.33 (dd, 1H), 7.27 (m, 1H), 7.18 (td, 1H), 6.52 (m, 1H), 6.44 (dd, 1H), 4.09 (m, 2H), 3.86 (s, 3H), 3.64 (m, 2H), 3.28 (s, 3H), 3.19 (m, 4H), 2.99 (s, 3H), 2.69 (m, 6H), 1.44 (s, 9H), 1.28 (m, 1H) |
| | Step 2: ¹H NMR (400 MHz, CDCl₃): δ 8.41 (d, 1H), 8.31 (br s, 1H), 8.07 (s, 1H), 8.04 (dd, 1H), 7.38 (td, 1H), 7.32 (dd, 1H), 7.25 (m, 1H), 7.17 (td, 1H), 6.52 (m, 1H), 6.42 (m, 1H), 3.99 (m, 1H), 3.85 (s, 3H), 3.69 (m, 1H), 3.28 (s, 3H), 3.12 (m, 4H), 2.99 (s, 3H), 2.71 (m, 1H), 2.60 (m, 5H), 2.17 (m, 2H), 1.25 (m, 1H) |
| | Step 3: ¹H NMR (400 MHz, DMSO-*d₆*): δ 8.28 (br s, 1H), 8.27 (m, 1H), 8.12 (br s, 1H), 8.10 (s, 1H), 7.58 (dd, 1H), 7.37 (d, 1H), 7.25 (t, 1H), 7.16 (td, 1H), 6.61 (d, 1H), 6.43 (dd, 1H), 3.75 (s, 3H), 3.42 (t, 2H), 3.18 (s, 3H), 3.12 (m, 5H), 3.10 (s, 3H), 2.86 (t, 2H), 2.63 (m, 1H), 2.47-2.53 (m, 6H), 1.41 (m, 1H), 1.40 (s, 9H) |
| | Compound 37: ¹H NMR (400 MHz, DMSO-*d₆*): δ 8.98 (s, 1H), 8.62 (t, 1H), 8.28 (m, 2H), 8.13 (s, 1H), 8.10 (s, 1H), 7.56-7.59 (m, 2H), 7.37-7.41 (m, 5H), 7.25 (t, 1H), 7.16 (td, 1H), 6.61 (d, 1H), 6.43 (dd, 1H), 5.16 (d, 1H), 4.51 (d, 1H), 4.35-4.46 (m, 3H), 4.26 (dd, 1H), 3.75 (s, 3H), 3.65 (dd, 1H), 3.58 (m, 1H), 3.51 (t, 1H), 3.45 (t, 1H), 3.18 (s, 3H), 3.10 (s, 3H), 3.09 (m, 4H), 2.94 (m, 2H), 2.68 (m, 1H), 2.56 (m, 2H), 2.48 (m, 6H), 2.05 (m, 1H), 1.90 (m, 1H), 0.94 (s, 9H). LC/MS (ESI) m/z 1057 [M+H]⁺ |
| 38 | Step 1: ¹H NMR (400 MHz, DMSO-*d₆*): δ 7.31-7.39 (m, 5H), 5.05 (s, 2H), 3.95 (m, 2H), 3.34 (m, 2H), 3.14 (m, 1H), 3.05 (m, 2H), 2.75 (m, 2H), 2.20 (d, 2H), 1.62 (m, 2H), 1.42 (m, 1H), 1.40 (s, 9H), 0.95 (m, 2H) |
| | Step 2: ¹H NMR (400 MHz, DMSO-*d₆*): δ 3.33 (t, 2H), 3.13 (m, 1H), 3.03 (m, 2H), 2.85 (m, 2H), 2.37 (m, 2H), 2.16 (d, 2H), 1.52 (m, 2H), 1.40 (s, 9H), 1.27 (m, 1H), 0.92 (m, 2H) |
| | Step 3: ¹H NMR (400 MHz, DMSO-*d₆*): δ 7.86 (d, 1H), 6.55 (dd, 1H), 6.46 (d, 1H), 4.00 (m, 2H), 3.89 (s, 3H), 3.36 (m, 2H), 3.16 (m, 1H), 3.08 (m, 2H), 2.91 (m, 2H), 2.23 (d, 2H), 1.72 (m, 2H), 1.55 (m, 1H), 1.40 (s, 9H), 1.11 (m, 2H) |
| | Step 4: ¹H NMR (400 MHz, DMSO-*d₆*): δ 6.49 (d, 1H), 6.46 (d, 1H), 6.27 (dd, 1H), 4.19 (br s, 2H), 3.72 (s, 3H), 3.35 (m, 4H), 3.16 (m, 1H), 3.07 (m, 2H), 2.44 (m, 2H), 2.24 (d, 2H), 1.69 (m, 2H), 1.40 (s, 9H), 1.27 (m, 1H), 1.22 (m, 2H) |
| | Step 5: ¹H NMR (400 MHz, DMSO-*d₆*): δ 8.28 (br s, 1H), 8.27 (m, 1H), 8.11 (br s, 1H), 8.10 (s, 1H), 7.58 (dd, 1H), 7.35 (d, 1H), 7.25 (t, 1H), 7.16 (td, 1H), 6.60 (d, 1H), 6.43 (dd, 1H), 3.75 (s, 3H), 3.64 (m, 2H), 3.38 (t, 2H), 3.20 (s, 3H), 3.16 (m, 1H), 3.10 (s, 3H), 3.09 (m, 2H), 2.61 (m, 2H), 2.26 (d, 2H), 1.73 (m, 2H), 1.55 (m, 1H), 1.41 (s, 9H), 1.22 (m, 3H) |
| | Compound 38: ¹H NMR (400 MHz, DMSO-*d₆*): δ 8.99 (s, 1H), 8.59 (t, 1H), 8.29 (m, 2H), 8.11 (s, 2H), 7.96 (d, 1H), 7.59 (dd, 1H), 7.35-7.44 (m, 5H), 7.25 (t, 1H), 7.17 (td, 1H), 6.60 (d, 1H), 6.43 (dd, 1H), 5.16 (d, 1H), 4.56 (d, 1H), 4.42-4.48 (m, 2H), 4.37 (m, 1H), 4.23 (dd, 1H), 3.75 (s, 3H), 3.65 (m, 4H), 3.38 (m, 4H), 3.19 (t, 1H), 3.11 (s, 3H), 3.03 (t, 1H), 2.62 (m, 2H), 2.27 (m, 2H), 2.05 (m, 1H), 1.91 (m, 1H), 1.75 (m, 2H), 1.40 (m, 1H), 1.23 (m, 2H), 0.94 (s, 9H). LC/MS (ESI) m/z 1042 [M+H]⁺ |
| 39 | Step 1: ¹H NMR (400 MHz, DMSO-*d₆*): δ 7.47 (t, 1H), 7.36 (d, 1H), 6.99 (d, 1H), 5.10-5.17 (m, 2H), 4.42 (d, 1H), 4.34 (m, 2H), 4.26 (d, 1H), 3.83 (m, 2H), 2.90 (m, 1H), 2.59 (m, 1H), 2.45 (m, 1H), 1.99 (m, 1H), 1.39 (s, 9H) |
| | Step 3: ¹H NMR (400 MHz, DMSO-*d₆*): δ 10.99 (br s, 1H), 7.46 (t, 1H), 7.33 (d, 1H), 7.02 (d, 1H), 5.10 (dd, 1H), 4.97 (m, 1H), 4.40 (d, 1H), 4.24 (d, 1H), 3.83 (dd, 2H), 3.57 (t, 2H), 3.12 (dd, 2H), 2.91 (m, 1H), 2.80 (t, 2H), 2.58 (m, 1H), 2.46 (m, 1H), 1.99 (m, 1H) |
| | Compound 39: ¹H NMR (400 MHz, DMSO-*d₆*): δ 10.98 (br s, 1H), 9.46 (br s, 1H), 8.39 (br s, 1H), 8.26 (m, 1H), 8.21 (s, 1H), 7.63 (d, 1H), 7.60 (d, 1H), 7.39-7.47 (m, 2H), 7.32 (d, 1H), 7.27 (t, 1H), 7.20 (d, 1H), 7.03 (d, 1H), 6.90 (t, 1H), 5.10 (dd, 1H), 4.93 (m, 1H), 4.39 (d, 1H), 4.23 (d, 1H), 3.80 (m, 2H), 3.18 (s, 3H), 3.09 (s, 3H), 3.02 (m, 2H), 2.91 (m, 4H), 2.41-2.60 (m, 9H), 2.34 (m, 2H), 1.99 (m, 1H). LC/MS (ESI) m/z 847 [M+H]⁺ |
| 40 | ¹H NMR (400 MHz, DMSO-*d₆*): δ 11.06 (br s, 1H), 9.45 (br s, 1H), 8.39 (br s, 1H), 8.27 (m, 1H), 8.22 (s, 1H), 7.64 (m, 2H), 7.58 (d, 1H), 7.42 (td, 1H), 7.27 (td, 1H), 7.22 (d, 1H), 6.91 (t, 1H), 6.81 (d, 1H), 6.67 (dd, 1H), 5.05 (dd, 1H), 4.51 (m, 1H), 4.27 (dd, 2H), 3.87 (dd, 2H), 3.58 (t, 2H), 3.19 (s, 3H), 3.10 (s, 3H), 2.95 (m, 4H), 2.88 (m, 1H), 2.52-2.59 (m, 8H), 2.01 (m, 1H). LC/MS (ESI) m/z 861 [M+H]⁺ |
| 41 | Step 1: ¹H NMR (400 MHz, DMSO-*d₆*): δ 11.11 (br s, 1H), 7.81 (d, 1H), 7.76 (s, 1H), 6.69 (d, 1H), 5.13 (dd, 1H), 2.89 (m, 1H), 2.59 (m, 1H), 2.55 (m, 1H), 2.06 (m, 1H) |
| | Step 2: ¹H NMR (400 MHz, DMSO-*d₆*): δ 11.13 (br s, 1H), 8.02 (s, 1H), 7.94 (m, 2H), 5.16 (dd, 1H), 4.90 (s, 2H), 2.89 (m, 1H), 2.62 (m, 1H), 2.53 (m, 1H), 2.06 (m, 1H) |
| | Compound 41: ¹H NMR (400 MHz, DMSO-*d₆*): δ 11.14 (br s, 1H), 8.28 (m, 2H), 8.12 (br s, 1H), 8.10 (s, 1H), 7.88 (d, 1H), 7.84 (s, 1H), 7.80 (d, 1H), 7.58 (dd, 1H), 7.38 (d, 1H), 7.25 (t, 1H), 7.16 (td, 1H), 6.62 (d, 1H), 6.44 (dd, 1H), 5.15 (dd, 1H), 3.75 (s, 3H), 3.65 (s, 2H), 3.18 (s, 3H), 3.13 (m, 4H), 3.10 (s, 3H), 2.89 (m, 1H), 2.80 (m, 2H), 2.49-2.62 (m, 6H), 2.19 (m, 2H), 1.98-2.09 (m, 3H), 1.70(m, 2H), 1.55 (m, 1H), 1.16 (m, 2H). LC/MS (ESI) m/z 885 [M+H]⁺ |
| 42 | ¹H NMR (400 MHz, DMSO-*d₆*): δ 11.11 (br s, 1H), 9.43 (br s, 1H), 8.38 (br s, 1H), 8.26 (m, 1H), 8.20 (s, 1H), 7.87 (d, 1H), 7.83 (s, 1H), 7.80 (d, 1H), 7.63 (dd, 1H), 7.57 (d, 1H), 7.39 (td, 1H), 7.27 (td, 1H), 7.19 (d, 1H), 6.90 (t, 1H), 5.14 (dd, 1H), 3.64 (s, 2H), 3.28 (m, 4H), 3.18 (s, 3H), 3.09 (s, 3H), 2.48-2.63 (m, 7H), 2.42 (m, 3H), 2.28 (m, 1H), 2.06 (m, 1H), 1.81 (m, 2H), 1.54 (m, 2H). LC/MS (ESI) m/z 859 [M+H]⁺ |
| 43 | ¹H NMR (400 MHz, DMSO-*d₆*): δ 11.12 (br s, 1H), 9.47 (br s, 1H), 8.39 (br s, 1H), 8.27 (m, 1H), 8.22 (s, 1H), 7.77 (dd, 1H), 7.64 (dd, 1H), 7.58 (d, 1H), 7.44 (d, 1H), 7.42 (td, 1H), 7.31 (d, 1H), 7.28 (td, 1H), 7.23 (d, 1H), 6.92 (t, 1H), 5.18 (m, 2H), 5.10 (dd, 1H), 4.29 (m, 1H), 3.80 (m, 1H), 3.76 (s, 3H), 3.18 (s, 3H), 3.14 (m, 4H), 3.08 (m, 1H), 2.89 (m, 1H), 2.50-2.68 (m, 8H), 2.20 (m, 2H), 2.04 (m, 1H), 1.80 (m, 3H), 0.97 (m, 1H). LC/MS (ESI) m/z 917 [M+H]⁺ |
| 44 | Step 1: ¹H NMR (400 MHz, DMSO-*d₆*): δ 11.06 (br s, 1H), 7.64 (d, 1H), 7.02 (d, 1H), 6.89 (dd, 1H), 5.05 (dd, 1H), 4.91 (dd, 1H), 3.93 (m, 1H), 3.49 (m, 2H), 3.26 (m, 2H), 2.88 (m, 1H), 2.56 (m, 2H), 1.91-2.09 (m, 5H) |
| | Step 2: ¹H NMR (400 MHz, DMSO-*d₆*): δ 11.07 (br s, 1H), 7.67 (d, 1H), 7.07 (d, 1H), 6.98 (dd, 1H), 5.06 (dd, 1H), 4.34 (m, 1H), 4.21 (m, 2H), 3.59 (m, 1H), 3.29 (m, 1H), 3.18 (s, 3H), 2.89 (m, 1H), 2.56 (m, 2H), 1.99-2.14 (m, 5H) |
| | Compound 44: ¹H NMR (400 MHz, DMSO-*d₆*): δ 11.08 (br s, 1H), 8.28 (br s, 1H), 8.27 (m, 1H), 8.12 (br s, 1H), 8.10 (s, 1H), 7.64 (d, 1H), 7.58 (dd, 1H), 7.36 (d, 1H), 7.26 (t, 1H), 7.16 (td, 1H), 7.00 (d, 1H), 6.86 (d, 1H), 6.62 (d, 1H), 6.45 (dd, 1H), 5.02 (dd, 1H), 4.13 (m, 1H), 3.76 (s, 3H), 3.71 (m, 2H), 3.52 (m, 1H), 3.25 (m, 1H), 3.18 (s, 3H), 3.10 (s, 3H), 2.88 (m, 1H), 2.42-2.70 (m, 10H), 2.33 (m, 3H), 1.95-2.09 (m, 5H), 1.85 (m, 2H), 1.53 (m, 2H). LC/MS (ESI) m/z 940 [M+H]⁺ |
| 45 | Step 1: ¹H NMR (400 MHz, CDCl₃): δ 8.42 (dd, 1H), 8.31 (br s, 1H), 8.07 (s, 1H), 8.02 (d, 1H), 7.85 (m, 1H), 7.72 (m, 2H), 7.39 (td, 1H), 7.32 (dd, 1H), 7.25 (m, 1H), 7.18 (td, 1H), 6.53 (d, 1H), 6.44 (dd, 1H), 3.86 (s, 3H), 3.82 (d, 2H), 3.63 (m, 2H), 3.29 (s, 3H), 3.00 (s, 3H), 2.60-2.70 (m, 12H), 2.34 (m, 1H), 1.93 (m, 2H), 1.68 (m, 2H) |
| | Step 2: ¹H NMR (400 MHz, CDCl₃): δ 8.42 (dd, 1H), 8.31 (br s, 1H), 8.07 (s, 1H), 8.02 (d, 1H), 7.40 (td, 1H), 7.33 (dd, 1H), 7.26 (m, 1H), 7.18 (td, 1H), 6.55 (d, 1H), 6.46 (dd, 1H), 3.86 (s, 3H), 3.64 (m, 2H), 3.29 (s, 3H), 3.00 (s, 3H), 2.81 (t, 2H), 2.52-2.72 (m, 10H), 2.44 (t, 2H), 2.38 (m, 1H), 1.95 (m, 2H), 1.73 (m, 2H) |
| | Compound 45: ¹H NMR (400 MHz, DMSO-*d₆*): δ 11.11 (br s, 1H), 8.28 (br s, 1H), 8.27 (m, 1H), 8.11 (br s, 1H), 8.10 (s, 1H), 7.57-7.62 (m, 2H), 7.36 (d, 1H), 7.25 (t, 1H), 7.16 (td, 1H), 7.10 (d, 1H), 7.03 (d, 1H), 6.76 (t, 1H), 6.62 (d, 1H), 6.45 (dd, 1H), 5.07 (dd, 1H), 3.76 (s, 3H), 3.72 (m, 2H), 3.36 (m, 2H), 3.18 (s, 3H), 3.10 (s, 3H), 2.89 (m, 1H), 2.42-2.72 (m, 14H), 2.33 (m, 1H), 2.01 (m, 1H), 1.87 (m, 2H), 1.53 (m, 2H). LC/MS (ESI) m/z 900 [M+H]⁺ |
| 46 | Step 2: ¹H NMR (400 MHz, DMSO-*d₆*): δ 11.11 (br s, 1H), 7.82 (t, 1H), 7.53 (d, 1H), 7.49 (d, 1H), 5.09 (dd, 1H), 4.51 (d, 1H), 3.55 (t, 2H), 2.88 (m, 1H), 2.56 (m, 2H), 2.04 (m, 1H) |
| | Compound 46: ¹H NMR (400 MHz, DMSO-*d₆*): δ 11.11 (br s, 1H), 9.47 (br s, 1H), 8.39 (br s, 1H), 8.27 (m, 1H), 8.21 (s, 1H), 7.82 (dd, 1H), 7.64 (dd, 1H), 7.58 (d, 1H), 7.42 (td, 1H), 7.28 (td, 1H), 7.22 (d, 1H), 6.91 (t, 1H), 5.08 (dd, 1H), 3.19 (s, 3H), 3.10 (s, 3H), 2.97 (m, 2H), 2.93 (m, 4H), 2.87 (m, 1H), 2.74 (m, 2H), 2.48-2.62 (m, 6H), 2.16 (m, 2H), 2.03 (m, 3H), 1.67 (m, 2H), 1.51 (m, 1H), 1.12 (m, 2H). LC/MS (ESI) m/z 903 [M+H]⁺ |
| 47 | ¹H NMR (400 MHz, DMSO-*d₆*): δ 10.99 (br s, 1H), 10.06 (br s, 1H), 8.28 (m, 2H), 8.12 (br s, 2H), 8.10 (s, 1H), 8.01 (s, 1H), 7.67 (s, 2H), 7.57 (d, 1H), 7.36 (d, 1H), 7.25 (t, 1H), 7.16 (t, 1H), 6.62 (d, 1H), 6.46 (dd, 1H), 5.08 (dd, 1H), 4.43 (d, 1H), 4.28 (d, 1H), 3.75 (s, 3H), 3.73 (m, 2H), 3.18 (s, 1H), 3.15 (m, 4H), 3.10 (s, 3H), 2.91 (m, 1H), 2.68 (m, 2H), 2.50-2.61 (m, 7H), 2.36 (m, 2H), 2.00 (m, 1H), 1.87 (m, 2H), 1.56 (m, 2H). LC/MS (ESI) m/z 900 [M+H]⁺ |
| 48 | ¹H NMR (400 MHz, DMSO-*d₆*): δ 11.01 (br s, 1H), 9.72 (br s, 1H), 9.45 (br s, 1H), 8.39 (br s, 1H), 8.27 (m, 1H), 8.22 (s, 1H), 7.81 (dd, 1H), 7.64 (dd, 1H), 7.49-7.60 (m, 2H), 7.42 (td, 1H), 7.26 (td, 1H), 7.22 (d, 1H), 6.91 (t, 1H), 5.12 (dd, 1H), 4.42 (d, 1H), 4.35 (d, 1H), 3.19 (s, 3H), 3.14 (m, 2H), 3.10 (s, 3H), 2.94 (m, 7H), 2.61 (m, 1H), 2.48-2.52 (m, 4H), 2.38 (m, 1H), 2.19 (m, 4H), 2.06 (m, 1H), 1.72 (m, 2H), 1.54 (m, 1H), 1.26 (m, 2H). LC/MS (ESI) m/z 902 [M+H]⁺ |
| 49 | Step 1: ¹H NMR (400 MHz, DMSO-*d₆*): δ 7.31-7.39 (m, 5H), 5.04 (s, 2H), 3.97 (m, 2H), 3.70-2.80 (m, 4H), 2.13 (m, 1H), 2.06 (d, 2H), 1.89 (m, 2H), 1.68 (m, 4H), 1.51 (m, 2H), 1.38 (s, 9H), 0.94 (m, 2H) |
| | Step 2: ¹H NMR (400 MHz, DMSO-*d₆*): δ 2.89 (m, 2H), 2.70 (m, 2H), 2.41 (td, 2H), 2.13 (m, 1H), 2.03 (d, 2H), 1.87 (td, 2H), 1.72 (m, 2H), 1.45-1.59 (m, 5H), 1.38 (s, 9H), 0.92 (m, 2H) |
| | Step 3: ¹H NMR (400 MHz, DMSO-*d₆*): δ 7.86 (d, 1H), 6.57 (dd, 1H), 6.47 (d, 1H), 4.02 (m, 2H), 3.90 (s, 3H), 2.95 (m, 2H), 2.73 (m, 2H), 2.15 (m, 1H), 2.10 (d, 2H), 1.91 (m, 2H), 1.75 (m, 5H), 1.51 (m, 2H), 1.39 (s, 9H), 1.09 (m, 2H) |
| | Step 4: ¹H NMR (400 MHz, DMSO-*d₆*): δ 6.50 (d, 1H), 6.47 (d, 1H), 6.28 (dd, 1H), 4.19 (br s, 2H), 3.73 (s, 3H), 3.36 (m, 2H), 2.73 (m, 2H), 2.47 (m, 2H), 2.15 (m, 1H), 2.11 (d, 2H), 1.91 (m, 2H), 1.73 (m, 4H), 1.53 (m, 3H), 1.39 (s, 9H), 1.19 (m, 2H) |
| | Step 5: ¹H NMR (400 MHz, DMSO-*d₆*): δ 8.28 (m, 2H), 8.11 (br s, 1H), 8.10 (s, 1H), 7.58 (d, 1H), 7.35 (d, 1H), 7.25 (t, 1H), 7.16 (td, 1H), 6.60 (d, 1H), 6.45 (dd, 1H), 3.75 (s, 3H), 3.66 (m, 2H), 2.76 (m, 2H), 2.64 (td, 2H), 2.15 (m, 1H), 2.13 (d, 2H), 1.93 (td, 2H), 1.76 (m, 4H), 1.64 (m, 1H), 1.54 (m, 2H), 1.40 (s, 9H), 1.25 (m, 2H) |
| | Compound 49: ¹H NMR (400 MHz, DMSO-*d₆*): δ 11.03 (br s, 1H), 9.74 (br s, 1H), 8.28 (m, 2H), 8.12 (br s, 2H), 8.10 (s, 1H), 7.82 (dd, 1H), 7.59 (dd, 1H), 7.50 (m, 2H), 7.36 (d, 1H), 7.25 (t, 1H), 7.16 (td, 1H), 6.61 (d, 1H), 6.46 (dd, 1H), 5.15 (dd, 1H), 4.39 (d, 1H), 4.32 (d, 1H), 3.76 (s, 3H), 3.67 (m, 2H), 3.18 (s, 1H), 3.10 (s, 3H), 2.91 (m, 3H), 2.64 (m, 3H), 2.40 (m, 1H), 2.17 (m, 2H), 2.01 (m, 1H), 1.91 (m, 3H), 1.80 (m, 4H), 1.70 (m, 3H), 1.24 (2H). LC/MS (ESI) m/z 900 [M+H]⁺ |
| 50 | ¹H NMR (400 MHz, DMSO-*d₆*): δ 11.01 (br s, 1H), 9.74 (br s, 1H), 8.28 (m, 2H), 8.09 (s, 1H), 8.07 (br s, 1H), 7.79 (dd, 1H), 7.51-7.59 (m, 3H), 7.38 (d, 1H), 7.26 (t, 1H), 7.16 (td, 1H), 6.61 (d, 1H), 6.45 (dd, 1H), 5.12 (dd, 1H), 4.42 (d, 3H), 3.35 (d, 2H), 3.75 (s, 3H), 3.18 (s, 3H), 3.14 (m, 4H), 3.09 (s, 3H), 2.91 (m, 1H), 2.58 (m, 1H), 2.55 (m, 4H), 2.36 (m, 1H), 2.03 (m, 1H), 1.57 (m, 8H). LC/MS (ESI) m/z 885 [M+H]⁺ |
| 51 | ¹H NMR (400 MHz, DMSO-*d₆*): δ 11.07 (br s, 1H), 8.28 (m, 2H), 8.10 (s, 1H), 8.09 (br s, 1H), 7.64 (d, 1H), 7.58 (dd, 1H), 7.36 (d, 1H), 7.30 (d, 1H), 7.26 (m, 1H), 7.22 (dd, 1H), 7.16 (td, 1H), 6.61 (d, 1H), 6.45 (dd, 1H), 5.05 (dd, 1H), 4.02 (m, 2H), 3.75 (s, 3H), 3.70 (m, 2H), 3.18 (s, 3H), 3.10 (s, 3H), 2.95 (m, 2H), 2.88 (m, 1H), 2.67 (m, 2H), 2.26-2.61 (m, 12H), 2.01 (m, 1H), 1.87 (m, 2H), 1.75 (m, 2H), 1.54 (m, 4H), 1.37 (m, 2H), 1.18 (m, 2H). LC/MS (ESI) m/z 968 [M+H]⁺ |
| 52 | ¹H NMR (400 MHz, DMSO-*d₆*): δ 11.11 (br s, 1H), 8.28 (br s, 1H), 8.27 (m, 1H), 8.11 (br s, 1H), 8.10 (s, 1H), 7.81 (dd, 1H), 7.57 (dd, 1H), 7.54 (d, 1H), 7.45 (d, 1H), 7.36 (d, 1H), 7.25 (t, 1H), 7.16 (td, 1H), 6.62 (d, 1H), 6.44 (dd, 1H), 5.08 (dd, 1H), 4.32 (t, 2H), 3.75 (s, 3H), 3.70 (m, 2H), 3.18 (s, 3H), 3.10 (s, 3H), 2.89 (m, 1H), 2.74 (t, 2H), 2.48-2.68 (m, 10H), 2.30 (m, 1H), 2.03 (m, 1H), 1.85 (m, 2H), 1.52 (m, 2H). LC/MS (ESI) m/z 901 [M+H]⁺ |
| 53 | ¹H NMR (400 MHz, DMSO-*d₆*): δ 11.13 (br s, 1H), 8.29 (br s, 1H), 8.28 (m, 1H), 8.14 (br s, 1H), 8.11 (s, 1H), 7.78 (dd, 1H), 7.59 (dd, 1H), 7.44 (d, 1H), 7.38 (d, 1H), 7.31 (d, 1H), 7.26 (t, 1H), 7.17 (td, 1H), 6.63 (d, 1H), 6.45 (dd, 1H), 5.19 (m, 1H), 5.10 (dd, 1H), 4.29 (m, 1H), 3.80 (m, 1H), 3.76 (s, 3H), 3.18 (s, 3H), 3.14 (m, 4H), 3.08 (m, 1H), 2.89 (m, 1H), 2.50-2.68 (m, 8H), 2.21 (m, 2H), 2.04 (m, 1H), 1.80 (m, 3H), 1.90 (m, 2H), 0.97 (m, 1H). LC/MS (ESI) m/z 929 [M+H]⁺ |
| 54 | Step 1: ¹H NMR (400 MHz, CDCl₃): δ 8.66 (d, 1H), 7.96 (d, 1H), 7.95 (br s, 1H), 7.90 (d, 1H), 7.76 (t, 1H), 7.34-7.44 (m, 5H), 6.70 (d, 1H), 5.29 (s, 2H), 5.00 (dd, 1H), 2.73-2.96 (m, 3H), 2.16 (m, 1H) |
| | Step 2: ¹H NMR (400 MHz, DMSO-*d₆*): δ 12.27 (br s, 1H), 11.15 (s, 1H), 7.72-7.81 (m, 3H), 5.13 (dd, 2H), 3.25 (t, 2H), 2.89 (m, 1H), 2.62 (t, 2H), 2.55 (m, 2H), 2.06 (m, 1H). LC/MS (ESI) m/z 331 [M+H]⁺ |
| | Compound 54: ¹H NMR (400 MHz, DMSO-*d₆*): δ 11.14 (br s, 1H), 8.28 (br s, 1H), 8.27 (m, 1H), 8.14 (br s, 1H), 8.10 (s, 1H), 7.77 (m, 3H), 7.59 (dd, 1H), 7.38 (d, 1H), 7.25 (t, 1H), 7.17 (t, 1H), 6.62 (d, 1H), 6.44 (dd, 1H), 5.14 (dd, 1H), 4.37 (m, 1H), 3.86 (m, 1H), 3.76 (s, 3H), 3.36 (m, 2H), 3.25 (t, 2H), 3.18 (s, 3H), 3.13 (m, 4H), 3.10 (s, 3H), 2.85-3.00 (m, 2H), 2.50-2.72 (m, 8H), 2.17 (m, 2H), 2.06 (m, 1H), 1.79 (m, 1H), 1.72 (m, 2H), 0.97 (m, 1H). LC/MS (ESI) m/z 927 [M+H]⁺ |
| 55 | ¹H NMR (400 MHz, DMSO-*d₆*): δ 9.00 (s, 1H), 8.52 (t, 1H), 8.28 (m, 2H), 8.14 (s, 1H), 8.10 (s, 1H), 7.84 (d, 1H), 7.58 (dd, 1H), 7.35-7.45 (m, 5H), 7.26 (t, 1H), 7.16 (t, 1H), 6.62 (d, 1H), 6.44 (dd, 1H), 5.15 (d, 1H), 4.54 (dd, 1H), 4.30-4.43 (m, 4H), 3.76 (s, 3H), 3.68 (dd, 1H), 3.57 (m, 1H), 3.18 (s, 3H), 3.13 (m, 4H), 3.10 (s, 3H), 2.93 (dd, 2H), 2.82 (m, 2H), 2.47 (m, 4H), 2.46 (s, 3H), 2.03-2.17 (m, 5H), 1.91 (m, 1H), 1.73 (m, 2H), 1.53 (m, 1H), 1.18 (m, 3H), 0.30-0.50 (m, 4H). LC/MS (ESI) m/z 1069 [M+H]⁺ |
| 56 | ¹H NMR (400 MHz, DMSO-*d₆*): δ 9.00 (s, 1H), 8.58 (t, 1H), 8.28 (m, 2H), 8.14 (s, 1H), 8.10 (s, 1H), 7.80 (d, 1H), 7.58 (dd, 1H), 7.37-7.44 (m, 5H), 7.25 (t, 1H), 7.17 (t, 1H), 6.62 (d, 1H), 6.44 (dd, 1H), 5.14 (d, 1H), 4.26-4.53 (m, 5H), 3.76 (s, 3H), 3.57-3.67 (m, 4H), 3.18 (s, 3H), 3.13 (m, 4H), 3.10 (s, 3H), 2.95 (s, 2H), 2.83 (m, 2H), 2.45 (s, 3H), 2.44 (m, 4H), 2.03-2.16 (m, 5H), 1.90 (m, 1H), 1.74 (m, 2H), 1.54 (m, 1H), 1.17 (m, 2H), 0.90 (d, 3H), 0.83 (m, 1H), 0.78 (d, 3H). LC/MS (ESI) m/z 1071 [M+H]⁺ |
| 57 | ¹H NMR (400 MHz, DMSO-*d₆*): δ 11.12 (br s, 1H), 8.28 (br s, 1H), 8.27 (m, 1H), 8.14 (br s, 1H), 8.10 (s, 1H), 7.81 (dd, 1H), 7.58 (d, 1H), 7.54 (d, 1H), 7.45 (d, 1H), 7.37 (d, 1H), 7.25 (t, 1H), 7.18 (t, 1H), 6.62 (d, 1H), 6.45 (dd, 1H), 5.09 (dd, 1H), 4.32 (t, 2H), 3.75 (s, 3H), 3.18 (s, 3H), 3.13 (m, 4H), 3.10 (s, 3H), 2.98 (m, 2H), 2.88 (m, 1H), 2.74 (m, 2H), 2.48-2.68 (m, 7H), 2.17 (m, 2H), 1.98-2.10 (m, 3H), 1.68 (m, 2H), 1.55 (m, 1H), 1.10 (m, 2H). LC/MS (ESI) m/z 915 [M+H]⁺ |
| 58 | ¹H NMR (400 MHz, DMSO-*d₆*): δ 11.08 (br s, 1H), 8.28 (br s, 1H), 8.27 (m, 1H), 8.13 (br s, 1H), 8.10 (s, 1H), 7.56-7.60 (m, 2H), 7.36 (d, 1H), 7.25 (t, 1H), 7.16 (td, 1H), 7.00-7.03 (m, 2H), 6.89 (dd, 1H), 6.62 (d, 1H), 6.45 (dd, 1H), 5.03 (dd, 1H), 3.75 (s, 3H), 3.72 (m, 2H), 3.29 (m, 2H), 3.18 (s, 3H), 3.10 (s, 3H), 2.88 (m, 1H), 2.44-2.70 (m, 14H), 2.33 (m, 1H), 2.00 (m, 1H), 1.86 (m, 2H), 1.53 (m, 2H). LC/MS (ESI) m/z 900 [M+H]⁺ |
| 59 | ¹H NMR (400 MHz, DMSO-*d₆*): δ 11.09 (br s, 1H), 8.28 (m, 2H), 8.14 (brs, 1H), 8.10 (s, 1H), 7.65 (d, 1H), 7.59 (dd, 1H), 7.38 (d, 1H), 7.30 (d, 1H), 7.22-7.28 (m, 2H), 7.17 (td, 1H), 6.62 (d, 1H), 6.44 (dd, 1H), 5.06 (dd, 1H), 3.76 (s, 3H), 3.18 (s, 3H), 3.14 (m, 4H), 3.11 (s, 3H), 2.81-2.99 (m, 5H), 2.47-2.61 (m, 8H), 2.18 (m, 2H), 2.11 (m, 2H), 2.01 (m, 1H), 1.77-1.88 (m, 4H), 1.68 (m, 2H), 1.52 (m, 1H), 1.14 (m, 3H). LC/MS (ESI) m/z 968 [M+H]⁺ |
| 60 | ¹H NMR (400 MHz, DMSO-*d₆*): δ 11.09 (br s, 1H), 8.28 (m, 2H), 8.13 (brs, 1H), 8.10 (s, 1H), 7.63 (d, 1H), 7.58 (d, 1H), 7.36 (d, 1H), 7.25 (t, 1H), 7.16 (t, 1H), 6.77 (d, 1H), 6.62-6.65 (m, 2H), 6.46 (dd, 1H), 5.05 (dd, 1H), 4.13 (t, 2H), 3.76 (s, 3H), 3.70 (m, 4H), 3.18 (s, 3H), 3.10 (s, 3H), 2.99 (m, 1H), 2.88 (m, 1H), 2.29-2.69 (m, 15H), 2.00 (m, 1H), 1.85 (m, 2H), 1.53 (m, 2H). LC/MS (ESI) m/z 926 [M+H]⁺ |
| 61 | ¹H NMR (400 MHz, DMSO-*d₆*): δ 11.08 (br s, 1H), 8.28 (m, 2H), 8.14 (brs, 1H), 8.10 (s, 1H), 7.63 (d, 1H), 7.58 (d, 1H), 7.38 (d, 1H), 7.24 (t, 1H), 7.17 (t, 1H), 6.77 (d, 1H), 6.63-6.65 (m, 2H), 6.46 (d, 1H), 5.05 (dd, 1H), 4.12 (t, 2H), 3.76 (s, 3H), 3.67 (m, 2H), 3.18 (s, 3H), 3.14 (m, 4H), 3.10 (s, 3H), 2.82-33.05 (m, 3H), 2.46-2.60 (m, 9H), 2.18 (m, 2H), 1.90-2.00 (m, 3H), 1.69 (m, 2H), 1.55 (m, 1H), 1.13 (m, 2H). LC/MS (ESI) m/z 940 [M+H]⁺ |
| 62 | Step 2: ¹H NMR (400 MHz, DMSO-*d₆*): δ 7.24-7.34 (m, 5H), 4.95 (s, 2H), 2.96 (m, 2H), 2.82 (m, 2H), 2.71 (m, 2H), 2.52 (t, 2H), 2.00 (d, 2H), 1.71 (t, 2H), 1.50-1.72 (m, 5H), 1.29 (m, 1H), 0.95-1.05 (m, 4H) |
| | Step 3: ¹H NMR (400 MHz, DMSO-*d₆*): δ 7.87 (d, 1H), 7.29-7.37 (m, 5H), 6.57 (dd, 1H), 6.47 (d, 1H), 5.00 (s, 2H), 4.02 (m, 2H), 3.90 (s, 3H), 2.95 (m, 2H), 2.88 (m, 2H), 2.79 (m, 2H), 2.09 (d, 2H), 1.78 (m, 5H), 1.58 (m, 2H), 1.35 (m, 1H), 1.09 (m, 4H) |
| | Step 5: ¹H NMR (400 MHz, DMSO-*d₆*): δ 8.28 (m, 2H), 8.13 (br s, 1H), 8.10 (s, 1H), 7.58 (dd, 1H), 7.62 (dd, 1H), 7.29-7.38 (m, 6H), 7.24 (t, 1H), 7.18 (td, 1H), 6.60 (d, 1H), 6.44 (dd, 1H), 5.01 (s, 2H), 3.75 (s, 3H), 3.66 (m, 2H), 3.18 (s, 3H), 3.10 (s, 3H), 2.89 (t, 4H), 2.82 (m 2H), 2.64 (m, 2H), 2.13 (d, 2H), 1.80 (m, 4H), 1.65 (m, 1H), 1.59 (m, 2H), 1.36 (m, 1H), 1.22 (m, 2H), 1.10 (m, 2H) |
| | Compound 62: ¹H NMR (400 MHz, DMSO-*d₆*): δ 11.11 (br s, 1H), 8.28 (br s, 1H), 8.27 (m, 1H), 8.13 (br s, 1H), 8.10 (s, 1H), 7.56-7.60 (m, 2H), 7.35 (d, 1H), 7.25 (t, 1H), 7.15 (m, 2H), 7.02 (d, 1H), 6.60 (m, 2H), 6.45 (dd, 1H), 5.05 (dd, 1H), 3.75 (s, 3H), 3.66 (m, 2H), 3.22 (m, 2H), 3.18 (s, 3H), 3.10 (s, 3H), 2.88 (m, 3H), 2.48-2.68 (m, 6H), 2.14 (m, 2H), 2.03 (m, 1H), 1.81 (m, 3H), 1.67 (m, 2H), 1.58 (m, 1H), 1.23 (m, 4H). LC/MS (ESI) m/z 899 [M+H]⁺ |
| 63 | ¹H NMR (400 MHz, DMSO-*d₆*): δ 11.18 (br s, 1H), 8.29 (br s, 1H), 8.28 (m, 1H), 8.13 (br s, 1H), 8.10 (s, 1H), 7.55-7.60 (m, 2H), 7.36 (d, 1H), 7.25 (t, 1H), 7.16-7.19 (m, 2H), 7.00 (s, 1H), 6.88 (d, 1H), 6.61(d, 1H), 6.45 (dd, 1H), 5.03 (dd, 1H), 3.75 (s, 3H), 3.66 (m, 2H), 3.18 (s, 3H), 3.10 (s, 3H), 3.08 (m, 2H), 2.88 (m, 3H), 2.48-2.68 (m, 6H), 2.15 (m, 2H), 1.99 (m, 1H), 1.66-1.88 (m, 5H), 1.56 (m, 1H), 1.23 (m, 4H). LC/MS (ESI) m/z 899 [M+H]⁺ |
| 64 | Step 1: ¹H NMR (400 MHz, CDCl₃): δ 8.03 (s, 1H), 7.99 (br s, 1H), 7.90 (d, 1H), 7.86 (dd, 1H), 7.79 (d, 1H), 7.36-7.44 (m, 5H), 6.66 (d, 1H), 5.28 (s, 2H), 5.00 (dd, 1H), 2.72-2.96 (m, 3H), 2.17 (m, 1H) |
| | Step 2: ¹H NMR (400 MHz, DMSO-*d₆*): δ 12.24 (br s, 1H), 11.14 (s, 1H), 7.80-7.85 (m, 2H), 7.75 (dd, 1H), 5.15 (dd, 2H), 3.01 (t, 2H), 2.87 (m, 1H), 2.64 (t, 2H), 2.55 (m, 2H), 2.05 (m, 1H) |
| | Compound 64: ¹H NMR (400 MHz, DMSO-*d₆*): δ 11.14 (br s, 1H), 8.28 (br s, 1H), 8.27 (m, 1H), 8.14 (br s, 1H), 8.10 (s, 1H), 7.83 (m, 2H), 7.76 (d, 1H), 7.58 (dd, 1H), 7.37 (d, 1H), 7.25 (t, 1H), 7.17 (td, 1H), 6.62 (d, 1H), 6.44 (dd, 1H), 5.14 (dd, 1H), 4.37 (m, 1H), 3.86 (m, 1H), 3.75 (s, 3H), 3.18 (s, 3H), 3.13 (m, 4H), 3.10 (s, 3H), 2.85-3.03 (m, 5H), 2.50-2.80 (m, 8H), 2.16 (m, 2H), 2.06 (m, 1H), 1.80 (m, 1H), 1.72 (m, 2H), 0.90 (m, 2H). LC/MS (ESI) m/z 927 [M+H]⁺ |
| 65 | Step 1: ¹H NMR (400 MHz, CDCl₃): δ 8.37 (br s, 1H), 8.34 (br s, 1H), 8.07 (s, 2H), 8.00 (d, 1H), 7.44 (br s, 1H), 7.38 (td, 1H), 7.33 (dd, 1H), 7.28 (dd, 1H), 7.22 (td, 1H), 6.50 (d, 1H), 6.41 (dd, 1H), 5.52 (m, 1H), 4.52 (m, 1H), 3.90 (m, 2H), 3.84 (s, 3H), 3.64 (m, 1H), 3.30 (m, 4H), 3.28 (s, 3H), 2.99 (s, 3H), 2.96 (m, 4H), 2.58 (m, 3H), 1.83-1.98 (m, 3H), 1.44 (s, 9H), 1.14 (m, 2H) |
| | Compound 65: ¹H NMR (400 MHz, DMSO-*d₆*): δ 11.12 (br s, H), 8.29 (br s, 1H), 8.28 (m, 1H), 8.14 (br s, 1H), 8.11 (s, 1H), 7.58-7.63 (m, 2H), 7.38 (d, 1H), 7.26 (t, 1H), 7.17 (td, 1H), 7.11-7.13 (m, 2H),7.07 (d, 1H), 6.63 (d, 1H), 6.45 (dd, 1H), 5.07 (dd, 1H), 4.39 (m, 1H), 4.18 (m, 2H), 3.90 (m, 1H), 3.76 (s, 3H), 3.19 (s, 3H), 3.15 (m, 4H), 3.11 (s, 3H), 3.05 (m, 1H), 2.89 (m, 1H), 2.68 (m, 1H), 2.50-2.62 (m, 6H), 2.21 (m, 2H), 2.05 (m, 1H), 1.86 (m, 1H), 1.80 (m, 2H), 1.15 (m, 1H), 1.00 (m, 1H). LC/MS (ESI) m/z 928 [M+H]⁺ |
| 66 | ¹H NMR (400 MHz, DMSO-*d₆*): δ 11.09 (br s, H), 8.29 (br s, 1H), 8.28 (m, 1H), 8.15 (br s, 1H), 8.11 (s, 1H), 7.57-7.60 (m, 2H), 7.38 (d, 1H), 7.26 (t, 1H), 7.17 (td, 1H), 7.10-7.13 (m, 3H),7.02 (d, 1H), 6.63 (d, 1H), 6.45 (dd, 1H), 5.04 (dd, 1H), 4.38 (m, 1H), 4.13 (t, 2H), 3.95 (m, 1H), 3.76 (s, 3H), 3.19 (s, 3H), 3.15 (m, 4H), 3.11 (s, 3H), 3.05 (m, 1H), 2.88 (m, 1H), 2.48-2.70 (m, 7H), 2.21 (m, 2H), 2.00 (m, 1H), 1.86 (m, 1H), 1.80 (m, 2H), 1.15 (m, 1H), 0.96 (m, 1H). LC/MS (ESI) m/z 928 [M+H]⁺ |
| 67 | ¹H NMR (400 MHz, DMSO-*d₆*): δ 11.07 (br s, 1H), 8.28 (m, 2H), 8.10 (s, 2H), 7.68 (d, 1H), 7.58 (dd, 1H), 7.36 (m, 2H), 7.26 (m, 2H), 7.17 (td, 1H), 6.62 (d, 1H), 6.46 (dd, 1H), 5.07 (dd, 1H), 3.76 (s, 3H), 3.68 (m, 2H), 3.46 (m, 4H), 3.19 (s, 3H), 3.10 (s, 3H), 2.84-2.93 (m, 1H), 2.48-2.72 (m, 8H), 2.24 (m, 2H), 2.01 (m, 1H), 1.83 (m, 2H), 1.73 (m, 1H), 1.27 (m, 2H). LC/MS (ESI) m/z 871 [M+H]⁺ |
| 68 | ¹H NMR (400 MHz, DMSO-*d₆*): δ 11.07 (br s, 1H), 8.28 (m, 2H), 8.10 (s, 2H), 7.65 (d, 1H), 7.58 (d, 1H), 7.38 (d, 1H), 7.32 (d, 1H), 7.24-7.28 (m, 2H), 7.17 (t, 1H), 6.63 (d, 1H), 6.45 (dd, 1H), 5.06 (dd, 1H), 4.06 (m, 2H), 3.76 (s, 3H), 3.18 (s, 3H), 3.15 (m, 4H), 3.10 (s, 3H), 2.84-3.02 (m, 3H), 2.48-2.61 (m, 6H), 2.22 (m, 2H), 2.00 (m, 1H), 1.89 (m, 1H), 1.83 (m, 2H), 1.18 (m, 2H). LC/MS (ESI) m/z 871 [M+H]⁺ |
| 69 | Step 1: ¹H NMR (400 MHz, CDCl₃): δ 8.42 (dd, 1H), 8.31 (br s, 1H), 8.08 (s, 2H), 8.04 (d, 1H), 7.39 (td, 1H), 7.33 (dd, 1H), 7.25 (d, 1H), 7.17 (td, 1H), 6.53 (d, 1H), 6.45 (dd, 1H), 6.40 (m, 1H), 4.46 (quin, 1H), 3.93 (m, 1H), 3.86 (s, 3H), 3.72 (m, 1H), 3.28 (s, 3H), 3.14 (m, 4H), 3.02 (m, 1H), 3.00 (s, 3H), 2.70 (m, 1H), 2.59 (m, 4H), 2.27 (m, 2H), 1.87 (m, 3H), 1.32 (m, 3H), 1.14 (m, 1H) |
| | Compound 69: ¹H NMR (400 MHz, DMSO-*d₆*): δ 11.07 (br s, 1H), 8.24 (br s, 1H), 8.23 (m, 1H), 8.06 (s, 2H), 7.76 (m, 1H), 7.54 (d, 1H), 7.42 (d, 1H), 7.35 (d, 1H), 7.22 (t, 1H), 7.10-7.17 (m, 2H), 6.58 (d, 1H), 6.41 (dd, 1H), 5.50 (m, 1H), 5.05 (dd, 1H), 4.27 (m, 1H), 4.00 (m, 1H), 3.72 (s, 3H), 3.14 (s, 3H), 3.09 (m, 4H), 3.06 (s, 3H), 2.58 (m, 1H), 2.46-2.65 (m, 8H), 2.16 (m, 2H), 2.00 (m, 1H), 1.77 (m, 3H), 1.46 (m, 3H), 1.15 (m, 1H), 0.91 (m, 1H). LC/MS (ESI) m/z 943 [M+H]⁺ |
| 70 | Step 1: ¹H NMR (400 MHz, CDCl₃): δ 8.42 (dd, 1H), 8.30 (br s, 1H), 8.08 (s, 2H), 8.04 (d, 1H), 7.39 (td, 1H), 7.33 (dd, 1H), 7.25 (d, 1H), 7.18 (td, 1H), 6.53 (d, 1H), 6.45 (dd, 1H), 5.59 (m, 1H), 4.61 (m, 1H), 3.90 (m, 1H), 3.86 (s, 3H), 3.28 (s, 3H), 3.14 (m, 4H), 3.04 (m, 1H), 3.00 (s, 3H), 2.60 (m, 5H), 2.26 (m, 2H), 1.78-1.96 (m, 3H), 1.30 (m, 3H), 1.12 (m, 1H) |
| | Step 2: ¹H NMR (400 MHz, DMSO-*d₆*): δ 8.28 (s, 1H), 8.27 (m, 1H), 8.10 (s, 2H), 7.58 (dd, 1H), 7.39 (d, 1H), 7.26 (t, 1H), 7.17 (td, 1H), 6.62 (d, 1H), 6.45 (dd, 1H), 5.59 (m, 1H), 4.38 (m, 1H), 3.90 (m, 1H), 3.76 (s, 3H), 3.72 (m, 1H), 3.18 (s, 3H), 3.14 (m, 4H), 3.10 (s, 3H), 2.99 (m, 1H), 2.49-2.60 (m, 5H), 2.19 (d, 2H), 1.73-1.82 (m, 3H), 1.07 (m, 1H), 1.05 (m, 3H), 0.93 (m, 1H) |
| | Compound 70: ¹H NMR (400 MHz, DMSO-*d₆*): δ 11.10 (br s, H), 8.28 (br s, 1H), 8.27 (m, 1H), 8.10 (s, 2H), 7.57-7.64 (m, 2H), 7.39 (d, 1H), 7.16-7.28 (m, 4H), 7.06 (d, 1H), 6.63 (d, 1H), 6.45 (dd, 1H), 5.06 (dd, 1H), 4.87 (m, 1H), 4.40 (m, 1H), 4.09 (m, 1H), 3.76 (s, 3H), 3.18 (s, 3H), 3.14 (m, 4H), 3.10 (s, 3H), 2.89 (m, 1H), 2.50-2.72 (m, 8H), 2.22 (m, 2H), 2.03 (m, 1H), 1.78-1.88 (m, 3H), 1.29 (m, 3H), 1.10 (m, 1H), 0.99 (m, 1H). LC/MS (ESI) m/z 942 [M+H]⁺ |
| 71 | Step 1: ¹H NMR (400 MHz, DMSO-*d₆*): δ 8.28 (br s, 1H), 8.27 (m, 1H), 8.10 (s, 1H), 8.09 (br s, 1H), 7.58 (dd, 1H), 7.36 (d, 1H), 7.25 (t, 1H), 7.16 (td, 1H), 6.61 (d, 1H), 6.45 (dd, 1H), 3.90 (m, 2H), 3.75 (s, 3H), 3.70 (m, 2H), 3.18 (s, 3H), 3.10 (s, 3H), 2.66 (m, 4H), 2.51 (m, 5H), 2.33 (m, 4H), 2.09 (d, 2H), 1.84 (m, 2H), 1.64 (m, 3H), 1.52 (m, 2H), 1.39 (s, 9H), 0.91 (m, 2H) |
| | Step 2: ¹H NMR (400 MHz, DMSO-*d₆*): δ 8.28 (br s, 1H), 8.27 (m, 1H), 8.10 (s, 1H), 8.09 (br s, 1H), 7.58 (dd, 1H), 7.37 (d, 1H), 7.25 (t, 1H), 7.16 (td, 1H), 6.61 (d, 1H), 6.45 (dd, 1H), 3.75 (s, 3H), 3.70 (m, 2H), 3.18 (s, 3H), 3.10 (s, 3H), 3.08 (m, 2H), 2.64 (m, 4H), 2.51 (m, 5H), 2.34 (m, 4H), 2.09 (d, 2H), 1.84 (m, 2H), 1.70 (m, 3H), 1.52 (m, 2H), 1.09 (m, 2H) |
| | Compound 71: ¹H NMR (400 MHz, DMSO-*d₆*): δ 11.06 (br s, 1H), 8.28 (m, 2H), 8.10 (s, 1H), 8.09 (br s, 1H), 7.64 (d, 1H), 7.58 (dd, 1H), 7.37 (d, 1H), 7.30 (d, 1H), 7.26 (d, 1H), 7.22 (dd, 1H), 7.16 (td, 1H), 6.62 (d, 1H), 6.45 (dd, 1H), 5.05 (dd, 1H), 4.03 (m, 2H), 3.75 (s, 3H), 3.71 (m, 2H), 3.18 (s, 3H), 3.10 (s, 3H), 2.96 (m, 2H), 2.88 (m, 1H), 2.67 (m, 2H), 2.29-2.57 (m, 11H), 2.12 (d, 2H), 2.01 (m, 1H), 1.77-1.87 (m, 5H), 1.54 (m, 2H), 1.14 (m, 2H). LC/MS (ESI) m/z 954 [M+H]⁺ |
| 72 | Step 1: ¹H NMR (400 MHz, DMSO-*d₆*): δ 11.14 (br s, 1H), 7.86-7.92 (m, 3H), 5.49 (t, 1H), 5.14 (dd, 1H), 4.39 (d, 2H), 2.80 (m, 1H), 2.60 (m, 2H), 2.06 (m, 1H) |
| | Step 2: ¹H NMR(400 MHz, DMSO-*d₆*): δ 11.16 (br s, 1H), 7.94-7.98 (m, 3H), 5.17(dd, 1H), 4.79 (d, 2H), 2.89 (m, 1H), 2.60 (m, 2H), 2.08 (m, 1H) |
| | Compound 72: ¹H NMR (400 MHz, DMSO-*d₆*): δ 11.15 (br s, 1H), 8.28 (m, 2H), 8.11 (br s, 1H), 8.10 (s, 1H), 7.92 (m, 3H), 7.57 (dd, 1H), 7.36 (d, 1H), 7.25 (t, 1H), 7.16 (t, 1H), 6.62 (d, 1H), 6.45 (dd, 1H), 5.16 (dd, 1H), 3.75 (s, 3H), 3.72 (m, 2H), 3.59 (s, 2H), 3.18 (s, 3H), 3.10 (s, 3H), 2.89 (m, 2H), 2.48-2.70 (m, 11H), 2.33 (m, 1H), 2.06 (m, 1H), 1.87 (m, 2H), 1.54 (m, 2H). LC/MS (ESI) m/z 895 [M+H]⁺ |
| 73 | ¹H NMR (400 MHz, DMSO-*d₆*): δ 11.15 (br s, 1H), 8.28 (m, 2H), 8.14 (br s, 1H), 8.11 (s, 1H), 7.95 (m, 3H), 7.58 (dd, 1H), 7.40 (d, 1H), 7.26 (t, 1H), 7.16 (t, 1H), 6.66 (d, 1H), 6.47 (dd, 1H), 5.16 (dd, 1H), 3.76 (s, 3H), 3.70 (s, 2H), 3.21 (m, 4H), 3.18 (s, 3H), 3.10 (s, 3H), 2.89 (m, 1H), 2.75 (m, 4H), 2.46-2.67 (m, 2H), 2.06 (m, 1H). LC/MS (ESI) m/z 812 [M+H]⁺ |
| 74 | Step 1: ¹H NMR (400 MHz, CDCl₃): δ 7.81 (s, 1H), 7.30-7.39 (m, 5H), 6.54 (s, 1H), 5.14 (s, 2H), 4.02 (m, 2H), 3.93 (s, 3H), 3.55 (t, 4H), 3.33 (m, 2H), 2.71 (m, 2H), 2.58 (m, 4H), 2.43 (m, 1H), 2.23 (s, 3H), 1.94 (m, 2H), 1.70 (m, 2H) |
| | Step 2: ¹H NMR (400 MHz, CDCl₃): δ 7.30-7.37 (m, 5H), 6.56 (s, 2H), 5.14 (s, 2H), 3.81 (s, 3H), 3.54 (t, 4H), 3.06 (m, 2H), 2.59 (m, 6H), 2.36 (m, 1H), 2.16 (s, 3H), 1.86 (m, 2H), 1.67 (m, 2H) |
| | Step 3: ¹H NMR (400 MHz, DMSO-*d₆*): δ 8.32 (br s, 1H), 8.22 (m, 2H), 8.13 (s, 1H), 8.06 (br s, 1H), 7.59 (dd, 1H), 7.43 (s, 1H), 7.30-7.40 (m, 5H), 7.26 (t, 1H), 7.19 (td, 1H), 6.70 (s, 1H), 5.10 (s, 2H), 3.75 (s, 3H), 3.41 (m, 4H), 3.18 (s, 3H), 3.10 (s, 3H), 3.08 (m, 1H), 2.63 (m 2H), 2.50 (m, 6H), 2.37 (m, 1H), 2.11 (s, 3H), 1.84 (m, 2H), 1.58 (m, 2H). LC/MS (ESI) m/z 749 [M+H]⁺ |
| | Step 4: ¹H NMR (400 MHz, DMSO-*d₆*): δ 8.32 (br s, 1H), 8.22 (m, 2H), 8.13 (s, 1H), 8.06 (br s, 1H), 7.60 (dd, 1H), 7.43 (s, 1H), 7.27 (t, 1H), 7.19 (td, 1H), 6.70 (s, 1H), 3.76 (s, 3H), 3.18 (s, 3H), 3.10 (s, 3H), 3.08 (m, 2H), 2.71 (m 4H), 2.62 (m, 2H), 2.46 (m, 4H), 2.27 (m, 1H), 2.11 (s, 3H), 1.84 (m, 2H), 1.57 (m, 2H). LC/MS (ESI) m/z 615 [M+H]⁺ Step 5: ¹H NMR (400 MHz, CDCl₃): δ 8.31 (br s, 1H), 8.21 (m, 1H), 8.13 (s, 1H), 8.05 (br s, 1H), 7.85-7.90 (m, 4H), 7.59 (dd, 1H), 7.42 (s, 1H), 7.26 (m, 1H), 7.18 (td, 1H), 6.69 (s, 1H), 3.75 (s, 3H), 3.70 (t, 2H), 3.18 (s, 3H), 3.10 (s, 3H), 3.06 (m, 2H), 2.60 (m, 2H), 2.52 (t, 2H), 2.46 (m, 8H), 2.25 (m, 1H), 2.10 (s, 3H), 1.82 (m, 2H), 1.53 (m, 2H). LC/MS (ESI) m/z 788 [M+H]⁺ |
| | Step 6: ¹H NMR (400 MHz, CDCl₃): δ 8.31 (br s, 1H), 8.22 (m, 1H), 8.13 (s, 1H), 8.06 (br s, 1H), 7.59 (d, 1H), 7.43 (s, 1H), 7.27 (t, 1H), 7.19 (t, 1H), 6.70 (s, 1H), 3.76 (s, 3H), 3.18 (s, 3H), 3.10 (s, 3H), 3.07 (m, 2H), 2.64 (m, 2H), 2.60 (t, 2H), 2.53 (m, 4H), 2.38 (m, 4H), 2.33 (m, 1H), 2.28 (t, 2H), 2.11 (s, 3H), 1.86 (m, 2H), 1.57 (m, 2H) |
| | Compound 74: ¹H NMR (400 MHz, DMSO-*d₆*): δ 11.08 (br s, 1H), 8.32 (br s, 1H), 8.22 (m, 1H), 8.13 (s, 2H), 8.06 (br s, 1H), 7.58-7.62 (m, 2H), 7.43 (s, 1H), 7.27 (t, 1H), 7.19 (t, 1H), 7.10 (d, 1H), 7.03 (d, 1H), 6.75 (br t, 1H), 6.70 (s, 1H), 5.07 (dd, 1H), 3.76 (s, 3H), 3.37 (m, 2H), 3.18 (s, 3H), 3.10 (s, 3H), 3.08 (m, 2H), 2.89 (m, 1H), 2.46-2.67 (m, 14H), 2.32 (m, 1H), 2.11 (s, 3H), 2.03 (m, 1H), 1.87 (m, 2H), 1.58 (m, 2H). LC/MS (ESI) m/z 914 [M+H]⁺ |
| 75 | Step 1: ¹H NMR (400 MHz, DMSO-*d₆*): δ 7.82 (s, 1H), 7.30-7.37 (m, 5H), 6.55 (s, 1H), 5.13 (s, 2H), 4.20 (m, 2H), 3.93 (s, 3H), 3.02 (t, 4H), 2.79 (m, 2H), 2.58 (m, 4H), 2.25 (d, 2H), 2.24 (s, 3H), 1.78 (m, 2H), 1.71 (m, 1H), 1.13 (m, 2H) |
| | Step 2: ¹H NMR (400 MHz, DMSO-*d₆*): δ 7.30-7.40 (m, 5H), 6.58 (s, 1H), 6.43 (s, 1H), 5.06 (s, 2H), 4.30 (br s, 2H), 4.00 (m, 2H), 3.71 (s, 3H), 2.80 (m, 2H), 2.73 (t, 4H), 2.46 (m, 4H), 2.17 (d, 2H), 2.06 (s, 3H), 1.71 (m, 3H), 0.98 (m, 2H) |
| | Step 3: ¹H NMR (400 MHz, DMSO-*d₆*): δ 8.32 (br s, 1H), 8.22 (m, 1H), 8.13 (s, 1H), 8.07 (br s, 1H), 7.59 (dd, 1H), 7.43 (s, 1H), 7.32-7.40 (m, 5H), 7.26 (t, 1H), 7.18 (td, 1H), 6.73 (s, 1H), 5.07 (s, 2H), 4.01 (m, 2H), 3.76 (s, 3H), 3.18 (s, 3H), 3.10 (s, 3H), 2.85 (t, 4H), 2.82 (m 2H), 2.52 (m, 4H), 2.20 (d, 2H), 2.11 (s, 3H), 1.73 (m, 3H), 1.01 (m, 2H) |
| | Step 4: ¹H NMR (400 MHz, DMSO-*d₆*): δ 8.79 (br s, 2H), 8.33 (br s, 1H), 8.23 (m, 1H), 8.14 (s, 1H), 8.07 (br s, 1H), 7.60 (dd, 1H), 7.45 (s, 1H), 7.27 (t, 1H), 7.19 (td, 1H), 6.72 (s, 1H), 3.77 (s, 3H), 3.24 (m, 2H), 3.18 (s, 3H), 3.10 (s, 3H), 2.87 (m, 2H), 2.85 (m 4H), 2.53 (m, 4H), 2.22 (m, 2H), 2.12 (s, 3H), 1.86 (m, 3H), 1.30 (m, 2H) |
| | Step 5: ¹H NMR (400 MHz, DMSO-*d₆*): δ 8.32 (br s, 1H), 8.21 (m, 1H), 8.13 (s, 1H), 8.06 (br s, 1H), 7.59 (dd, 1H), 7.43 (s, 1H), 7.26 (t, 1H), 7.18 (td, 1H), 6.85 (m, 1H), 6.73 (s, 1H), 4.42 (m, 1H), 4.32 (m, 1H), 3.89 (m, 1H), 3.76 (s, 3H), 3.18 (s, 3H), 3.09 (s, 3H), 2.99 (m, 1H), 2.86 (m, 4H), 2.54 (m, 5H), 2.20 (m, 2H), 2.11 (s, 3H), 1.72-1.81 (m, 3H), 1.36 (s, 9H), 1.12 (m, 3H), 0.95 (m, 2H) |
| | Step 6: ¹H NMR (400 MHz, DMSO-*d₆*): δ 8.32 (br s, 1H), 8.22 (m, 1H), 8.13 (s, 1H), 8.07 (br s, 1H), 7.59 (d, 1H), 7.43 (s, 1H), 7.27 (t, 1H), 7.19 (t, 1H), 6.73 (s, 1H), 4.37 (m, 1H), 3.90 (m, 1H), 3.77 (s, 3H), 3.72 (m, 1H), 3.18 (s, 3H), 3.10 (s, 3H), 2.99 (m, 1H), 2.86 (m, 4H), 2.54 (m, 5H), 2.21 (d, 2H), 2.11 (s, 3H), 1.73-1.81 (m, 3H), 1.05 (m, 3H), 0.95 (m, 2H) |
| | Compound 75: ¹H NMR (400 MHz, DMSO-*d₆*): δ 11.10 (br s, H), 8.33 (br s, 1H), 8.22 (m, 1H), 8.14 (s, 1H), 8.07 (br s, 1H), 7.59-7.63 (m, 2H), 7.44 (s, 1H), 7.18-7.28 (m, 4H), 7.06 (d, 1H), 6.74 (s, 1H), 6.45 (dd, 1H), 5.06 (dd, 1H), 4.87 (m, 1H), 4.40 (m, 1H), 4.09 (m, 1H), 3.77 (s, 3H), 3.18 (s, 3H), 3.11 (m, 1H), 3.10 (s, 3H), 2.87-2.93 (m, 5H), 2.50-2.70 (m, 8H), 2.23 (m, 2H), 2.17 (s, 3H), 2.03 (m, 1H), 1.78-1.87 (m, 3H), 1.29 (m, 3H), 1.11 (m, 1H), 0.98 (m, 1H). LC/MS (ESI) m/z 956 [M+H]⁺ |
| 76 | Step 1: ¹H NMR (400 MHz, DMSO-*d₆*): δ 8.33 (br s, 1H), 8.22 (m, 1H), 8.13 (s, 1H), 8.08 (br s, 1H), 7.59 (dd, 1H), 7.42 (s, 1H), 7.26 (t, 1H), 7.19 (td, 1H), 6.70 (d, 1H), 3.91 (m, 2H), 3.75 (s, 3H), 3.18 (s, 3H), 3.10 (s, 3H), 3.08 (m, 2H), 2.65 (m, 4H), 2.51 (m, 5H), 2.30 (m, 4H), 2.11 (s, 3H), 2.09 (d, 2H), 1.85 (m, 2H), 1.65 (m, 3H), 1.55 (m, 2H), 1.39 (s, 9H), 0.91 (m, 2H) |
| | Step 2: ¹H NMR (400 MHz, DMSO-*d₆*): δ 8.33 (br s, 1H), 8.22 (m, 1H), 8.14 (s, 1H), 8.09 (br s, 1H), 7.60 (dd, 1H), 7.42 (s, 1H), 7.27 (t, 1H), 7.19 (td, 1H), 6.70 (s, 1H), 3.75 (s, 3H), 3.18 (s, 3H), 3.10 (s, 3H), 3.08 (m, 4H), 2.64 (m, 4H), 2.51 (m, 5H), 2.30 (m, 4H), 2.11 (s, 3H), 2.09 (d, 2H), 1.85 (m, 2H), 1.71 (m, 3H), 1.56 (m, 2H), 1.11 (m, 2H) |
| | Compound 76: ¹H NMR (400 MHz, DMSO-*d₆*): δ 11.09 (br s, 1H), 8.33 (br s, 1H), 8.22 (m, 1H), 8.14 (s, 1H), 8.08 (br s, 1H), 7.65 (d, 1H), 7.60 (dd, 1H), 7.42 (s, 1H), 7.31 (d, 1H), 7.17-7.28 (m, 3H), 6.70 (s, 1H), 5.06 (dd, 1H), 4.02 (m, 2H), 3.76 (s, 3H), 3.18 (s, 3H), 3.10 (s, 3H), 3.08 (m, 2H), 2.96 (m, 2H), 2.88 (m, 1H), 2.49-2.66 (m, 9H), 2.26-2.37 (m, 4H), 2.13 (d, 2H), 2.11 (s, 3H), 2.01 (m, 1H), 1.77-1.88 (m, 5H), 1.58 (m, 2H), 1.14 (m, 2H). LC/MS (ESI) m/z 968 [M+H]⁺ |
| 77 | ¹H NMR (400 MHz, DMSO-*d₆*): δ 11.09 1H), 8.33 (br s, 8.22 (m, 1H), 8.14 (s, 1H), 8.08 (br s, 1H), 7.65 (d, 1H), 7.60 (dd, 1H), 7.42 (s, 1H), 7.31 (d, 1H), 7.17-7.28 (m, 3H), 6.70 (s, 1H), 5.06 (dd, 1H), 4.02 (m, 2H), 3.76 (s, 3H), 3.18 (s, 3H), 3.10 (s, 3H), 3.08 (m, 2H), 2.96 (m, 2H), 2.88 (m, 1H), 2.63 (m, 2H), 2.48-2.60 (m, 7H), 2.26-2.37 (m, 4H), 2.13 (m, 2H), 2.11 (s, 3H), 2.01 (m, 1H), 1.77-1.88 (m, 5H), 1.57 (m, 2H), 1.14 (m, 2H), LC/MS (ESI) m/z 968 [M+H]⁺ |
| 78 | Step 1: ¹H NMR (400 MHz, DMSO-*d₆*): δ 7.77 (s, 1H), 7.30-7.40 (m, 5H), 6.68 (s, 1H), 5.07 (s, 2H), 3.90 (s, 3H), 3.40 (m, 4H), 3.28 (m, 2H), 2.71 (m, 2H), 2.34 (m, 4H), 2.21 (d, 2H), 2.19 (s, 3H), 1.82 (m, 2H), 1.71 (m, 1H), 1.26 (m, 2H) |
| | Step2: ¹H NMR (400 MHz, DMSO-*d₆*): δ 7.31-7.40 (m, 5H), 6.55 (s, 1H), 6.43 (s, 1H), 5.07 (s, 2H), 4.29 (br s, 2H), 3.70 (s, 3H), 3.39 (m, 4H), 2.87 (m, 2H), 2.53 (m, 2H), 2.32 (m, 4H), 2.18 (d, 2H), 2.05 (s, 3H), 1.75 (m, 2H), 1.58 (m, 1H), 1.22 (m, 2H) |
| | Step 3: ¹H NMR (400 MHz, CDCl₃): δ 8.43 (dd, 1H), 8.33 (br s, 1H), 8.08 (s, 1H), 7.98 (br s, 1H), 7.30-7.41 (m, 8H), 7.17 (td, 1H), 6.61 (s, 1H), 5.14 (s, 2H), 3.85 (s, 3H), 3.52 (m, 4H), 3.29 (s, 3H), 3.08 (m, 2H), 3.00 (s, 3H), 2.59 (t, 2H), 2.39 (m, 4H), 2.25 (d, 2H), 2.17 (s, 3H), 1.83 (m, 2H), 1.60 (m, 1H), 1.37 (m, 2H) |
| | Step 4: ¹H NMR (400 MHz, CDCl₃): δ 8.43 (dd, 1H), 8.33 (br s, 1H), 8.08 (s, 1H), 7.98 (br s, 1H), 7.39 (td, 1H), 7.34 (m, 2H), 7.17 (td, 1H), 6.61 (s, 1H), 3.85 (s, 3H), 3.29 (s, 3H), 3.23 (m, 4H), 3.09 (m, 2H), 3.00 (s, 3H), 2.75 (m, 4H), 2.59 (m, 2H), 2.34 (d, 2H), 2.18 (s, 3H), 1.83 (m, 2H), 1.60 (m, 1H), 1.36 (m, 2H) |
| | Step 5: ¹H NMR (400 MHz, CDCl₃): δ 8.44 (dd, 1H), 8.33 (br s, 1H), 8.08 (s, 1H), 7.98 (br s, 1H), 7.39 (td, 1H), 7.35 (s, 1H), 7.33 (dd, 1H), 7.17 (td, 1H), 6.62 (s, 1H), 5.56 (br d, 1H), 4.61 (m, 1H), 3.85 (s, 3H), 3.47-3.71 (m, 4H), 3.29 (s, 3H), 3.09 (m, 1H), 3.00 (s, 3H), 2.61 (t, 2H), 2.43 (m, 4H), 2.27 (d, 2H), 2.17 (s, 3H), 1.83 (m, 2H), 1.64 (m, 1H), 1.44 (s, 9H), 1.39 (m, 2H), 1.25 (d, 3H) |
| | Step 6: ¹H NMR (400 MHz, CDCl₃): δ 8.43 (dd, 1H), 8.33 (br s, 1H), 8.08 (s, 1H), 7.98 (br s, 1H), 7.39 (td, 1H), 7.34 (dd, 1H), 7.33 (s, 1H), 7.17 (td, 1H), 6.62 (s, 1H), 3.85 (s, 3H), 3.78 (q, 1H), 3.65 (m, 2H), 3.49 (m, 2H), 3.29 (s, 3H), 3.09 (m, 1H), 3.00 (s, 3H), 2.60 (t, 2H), 2.42 (m, 4H), 2.27 (d, 2H), 2.17 (s, 3H), 1.83 (m, 2H), 1.62 (m, 1H), 1.37 (m, 2H), 1.25 (d, 3H) |
| | Compound 78: ¹H NMR (400 MHz, DMSO-*d₆*): δ 11.11 (br s, H), 8.33 (br s, 1H), 8.23 (m, 1H), 8.14 (s, 1H), 8.09 (br s, 1H), 7.59-7.64 (m, 2H), 7.42 (s, 1H), 7.14-7.34 (m, 4H),7.07 (d, 1H), 6.71 (s, 1H), 5.06 (dd, 1H), 4.90 (m, 1H), 3.76 (s, 3H), 3.45-3.70 (m, 4H), 3.18 (s, 3H), 3.10 (s, 3H), 3.05 (m, 2H), 2.89 (m, 1H), 2.33-2.66 (m, 8H), 2.25 (d, 2H), 2.12 (s, 3H), 2.03 (m, 1H), 1.83 (m, 2H), 1.68 (m, 1H), 1.30 (m, 2H), 1.29 (m, 3H). LC/MS (ESI) m/z 956 [M+H]⁺ |
| 79 | ¹H NMR (400 MHz, DMSO-*d₆*): δ 11.09 (br s, 1H), 8.32 (br s, 1H), 8.23 (m, 1H), 8.14 (s, 1H), 8.09 (br s, 1H), 7.69 (d, 1H), 7.60 (dd, 1H), 7.42 (s, 1H), 7.35 (d, 1H), 7.24-7.32 (m, 2H), 7.19 (td, 1H), 6.72 (d, 1H), 5.07 (dd, 1H), 3.76 (s, 3H), 3.46 (m, 4H), 3.18 (s, 3H), 3.10 (s, 3H), 3.06 (m, 2H), 2.88 (m, 1H), 2.48-2.68 (m, 8H), 2.27 (d, 2H), 2.12 (s, 3H), 2.01 (m, 1H), 1.85 (m, 2H), 1.70 (m, 1H), 1.30 (m, 2H). LC/MS (ESI) m/z 885 [M+H]⁺ |
| 80 | Step 1: ¹H NMR (400 MHz, CDCl₃): δ 8.40 (dd, 1H), 8.33 (br s, 1H), 8.07 (s, 1H), 8.00 (br s, 1H), 7.38 (td, 1H), 7.35 (s, 1H), 7.32 (dd, 1H), 7.16 (td, 1H), 6.64 (s, 1H), 5.52 (m, 1H), 4.57 (m, 1H), 3.94 (m, 2H), 3.83 (s, 3H), 3.70 (m, 1H), 3.28 (s, 3H), 3.01 (m, 5H), 2.99 (s, 3H), 2.78 (m, 3H), 2.64 (m, 2H), 2.45 (m, 2H), 2.16 (s, 3H), 1.86 (m, 3H), 1.43 (s, 9H), 1.16 (m, 2H) |
| | Step 2: ¹H NMR (400 MHz, CDCl₃): δ 8.43 (dd, 1H), 8.34 (br s, 1H), 8.09 (s, 1H), 8.00 (br s, 1H), 7.40 (td, 1H), 7.34 (s, 1H), 7.33 (dd, 1H), 7.17 (td, 1H), 6.65 (s, 1H), 4.61 (m, 1H), 3.84 (s, 3H), 3.71 (m, 1H), 3.45 (s, 2H), 3.29 (s, 3H), 3.00 (s, 3H), 2.97 (m, 1H), 2.90 (m, 4H), 2.63 (m, 2H), 2.57 (m, 3H), 2.26 (d, 2H), 2.18 (s, 3H), 1.86 (m, 2H), 1.79 (m, 1H), 1.58 (s, 9H), 1.12 (m, 2H) |
| | Compound 80: ¹H NMR (400 MHz, DMSO-*d₆*): δ 11.11 (br s, H), 8.33 (br s, 1H), 8.23 (m, 1H), 8.14 (s, 1H), 8.09 (br s, 1H), 7.60-7.64 (m, 2H), 7.43 (s, 1H), 7.27 (t, 1H), 7.19 (td, 1H), 7.12 (m, 2H), 7.07 (d, 1H), 6.74 (s, 1H), 5.06 (dd, 1H), 4.40 (m, 1H), 4.18 (m, 2H), 3.90 (m, 1H), 3.77 (s, 3H), 3.18 (s, 3H), 3.10 (s, 3H), 3.05 (m, 1H), 2.87-2.94 (m, 5H), 2.50-2.72 (m, 7H), 2.23 (d, 2H), 2.12 (s, 3H), 2.04 (m, 1H), 1.78-1.85 (m, 3H), 1.14 (m, 1H), 0.99 (m, 1H). LC/MS (ESI) m/z 942 [M+H]⁺ |
| 81 | ¹H NMR (400 MHz, DMSO-*d₆*): δ 11.11 (br s, 1H), 8.32 (br s, 1H), 8.22 (m, 1H), 8.13 (s, 1H), 8.09 (s, 1H), 7.77 (dd, 1H), 7.59 (dd, 1H), 7.44 (d, 1H), 7.43 (s, 1H), 7.31 (d, 1H), 7.26 (t, 1H), 7.18 (td, 1H), 6.73 (s, 1H), 5.18 (m, 1H), 5.10 (dd, 1H), 4.28 (m, 1H), 3.79 (m, 1H), 3.76 (s, 3H), 3.17 (s, 3H), 3.09 (m, 1H), 3.05 (m, 1H), 2.86-2.94 (m, 5H), 2.50-2.67 (m, 7H), 2.22 (d, 2H), 2.11 (s, 3H), 2.03 (m, 1H), 1.75-1.83 (m, 3H), 1.23 (m, 1H), 0.97 (m, 1H) LC/MS (ESI) m/z 943 [M+H]⁺ |
| 82 | Step 1: ¹H NMR (400 MHz, DMSO-*d₆*): δ 11.12 (br s, 1H), 7.75 (m, 1H), 7.49 (d, 1H), 7.32-7.38 (m, 6H), 5.40 (m, 1H), 5.19 (m, 2H), 5.09 (dd, 1H), 2.89 (m, 1H), 2.57 (m, 2H), 2.03 (m, 1H), 1.60 (d, 3H) |
| | Step 2: ¹H NMR (400 MHz, DMSO-*d₆*): δ 11.12 (br s, 1H), 7.78 (dd, 1H), 7.45 (d, 1H), 7.30 (d, 1H), 5.08 (m, 2H), 2.89 (m, 1H), 2.57 (m, 2H), 2.04 (m, 1H), 1.55 (d, 3H) |
| | Compound 82: ¹H NMR (400 MHz, DMSO-*d₆*): δ 11.11 (br s, H), 8.32 (br s, 1H), 8.22 (m, 1H), 8.13 (s, 1H), 8.08 (br s, 1H), 7.80 (m, 1H), 7.59 (dd, 1H), 7.45 (dd, 1H), 7.43 (s, 1H), 7.26 (t, 1H), 7.19 (m, 1H), 7.18 (td, 1H), 6.73 (s, 1H), 5.54 (m, 1H), 5.09 (dd, 1H), 4.31 (m, 1H), 4.03 (m, 1H), 3.76 (s, 3H), 3.17 (s, 3H), 3.09 (s, 3H), 3.08 (m, 1H), 2.86-2.93 (m, 5H), 2.48-2.66 (m, 7H), 2.20 (m, 2H), 2.11 (s, 3H), 2.04 (m, 1H), 1.78 (m, 3H), 1.50 (m, 3H), 1.16 (m, 1H), 0.95 (m, 1H). LC/MS (ESI) m/z 957 [M+H]⁺ |

### Evaluation of the compounds of the present invention

### Experimental Example 1: EGFR protein degradation test in Ba/F3 cells expressing normal EGFR or C797S mutant EGFR

The ability of the compounds of the present invention to degrade the mutant EGFR protein was confirmed in Ba/F3 cells expressing normal EGFR, mutant EGFR^{del19+T790M+C797S}, or mutant EGFR^{L858R+T790M+C797S}. A Western blot test using the EGFR antibody was performed.

Specifically, Ba/F3 cells expressing EGFR were cultured in RPMI 1640 (Gibco, NY, USA) containing 10% fetal bovine serum (FBS, Gibco, NY, USA). Cells expressing normal EGFR, mutant EGFR^{del19+T790M+C797S} or mutant EGFR^{L858R+T790M+C797S} were treated with the compounds of the present invention at concentrations of 0.1 µM and 1 µM, respectively, for 24 hours. After that, it was washed twice with phosphate buffered saline (PBS) and SDS-PAGE was performed with 30 µg of cell lysate. After the protein separated on the gel was transferred to a nitrocellulose membrane, the expression level of the EGFR protein was confirmed using an EGFR antibody (Santa Cruz biotechnology, Santacruz, CA, USA). EGFR bands separated by Western blotting were quantitatively analyzed with Fluo-S MultiImager (Bio-Rad, Hercules, CA, USA).

The experimental results are shown in Table 3 below.

**[Table 3]**

| **Compound** | **DTC** | **LTC** | **Compound** | **DTC** | **LTC** |
|---|---|---|---|---|---|
| **1** | **-** | **-** | **24** | **++** | **++** |
| **2** | **+** | **-** | **25** | **-** | **-** |
| **3** | **-** | **-** | **26** | **++** | **++** |
| **4** | **-** | **-** | **27** | **++** | **++** |
| **5** | **-** | **-** | **28** | **++** | **++** |
| **6** | **+** | **-** | **29** | **-** | **-** |
| **7** | **+** | **-** | **30** | **-** | **-** |
| **8** | **-** | **-** | **31** | **++** | **+** |
| **9** | **-** | **-** | **32** | **-** | **-** |
| **10** | **-** | **-** | **33** | **+** | **-** |
| **11** | **++** | **++** | **34** | **-** | **-** |
| **12** | **-** | **-** | **35** | **++** | **+** |
| **13** | **+** | **+** | **36** | **++** | **+** |
| **14** | **-** | **+** | **37** | **+** | **-** |
| **15** | **+** | **+** | **38** | **-** | **-** |
| **16** | **-** | **+** | **39** | **+** | **+** |
| **17** | **+** | **-** | **40** | **+** | **+** |
| **18** | **-** | **-** | **41** | **-** | **-** |
| **19** | **++** | **+** | **42** | **+** | **-** |
| **20** | **++** | **++** | **43** | **++** | **++** |
| **21** | - | **-** | **44** | **-** | **+** |
| **22** | **++** | **++** | **45** | **++** | **++** |
| **23** | **-** | **-** | **46** | **+** | **+** |

| **Compound** | **DTC** | **LTC** | **Compound** | **DTC** | **LTC** |
|---|---|---|---|---|---|
| **47** | **++** | **++** | **65** | **++** | **++** |
| **48** | **+** | **-** | **66** | **++** | **++** |
| **49** | **-** | **-** | **67** | **-** | **++** |
| **50** | **+** | **-** | **68** | **+** | **++** |
| **51** | **++** | **++** | **69** | **++** | **++** |
| **52** | **+** | **-** | **70** | **++** | **++** |
| **53** | **++** | **++** | **71** | **+** | **++** |
| **54** | **++** | **++** | **72** | **-** | **-** |
| **55** | **-** | **-** | **73** | **-** | **-** |
| **56** | **+** | **-** | **74** | **-** | **++** |
| **57** | **-** | **-** | **75** | **++** | **++** |
| **58** | **-** | **+** | **76** | **+** | **++** |
| **59** | **++** | **+** | **77** | **+** | **++** |
| **60** | **-** | **++** | **78** | **++** | **++** |
| **61** | **-** | **+** | **79** | **-** | **+** |
| **62** | **+** | **++** | **80** | **++** | **++** |
| **63** | **+** | **++** | **81** | **++** | **++** |
| **64** | **-** | **+** | **82** | **++** | **++** |

| | | | | | |
|---|---|---|---|---|---|
| DTC : EGFR^{del19+T790M+C797S} LTC: EGFR^{L858R+T790M+C797S} ++ : Protein degradation >80% + : Protein degradation 20~80% - : Protein degradation <20% | | | | | |

### Experimental Example 2: Pharmacokinetic evaluation

Pharmacokinetic tests for the compounds of the present invention were conducted as below. After a single oral administration of the compound of the present invention to ICR mice, the concentration of the compound over time was followed up and analyzed.

The compound of the present invention was suspended in 10% DMSO / 90% (30% HPbCD aqueous solution) and orally administered to mice at a dose of 5 mg/kg. Blood was collected at a fixed time and plasma was separated. Drug analysis was performed using HPLC (XBridge column C18, Waters, mobile phase 0.1% formic acid:acetonitrile (30:70, %/%)) and MS/MS (ESI positive, MRM). Mouse blood plasma and each commercially available standard solution were mixed at a ratio of 9:1 to prepare and calibrate at concentrations of 5, 50, 100, 500, 100 and 5,000 ng/mL. In addition, the preparation of QC samples was prepared by mixing the mouse blood plasma and the standard solution for QC at a ratio of 9:1, and prepared to be concentrations of 100, 750 and 2,500 ng/mL. 100 µL of the plasma sample was transferred to a centrifugation tube, and 10 µL of the internal standard solution and 300 µL of methanol were added, followed by mixing for about 30 seconds for pretreatment. The tube was centrifuged at 3,000 x g (4 °C) for about 5 minutes, and the supernatant was taken and transferred to an LC vial and injected into the instrument. Then, the concentration of the compound in mouse plasma was quantified by applying a previously validated assay. For pharmacokinetic parameters, the program WinNonlin 5.2 (Pharsight, USA) was used. AUC₀₋ₜ, AUC_{0-∞}, Cmax, Tmax, and t_{1/2} were calculated by Noncompartment modeling (best fit). The pharmacokinetic parameter results were expressed as the mean (Mean) and standard deviation (SD), and were statistically processed using the SPSS program (Statistical Package for the Social Sciences, 10.0K, USA).

The test results are shown in Table 4 below.

**[Table 4]**

| **Compound** | **AUC₀₋₂₄ (ng.h/mL)** | ***t*_{*1*/*2*} (h)** | **Compound** | **AUC₀₋₂₄ (ng.h/mL)** | ***t*_{*1*/*2*} (h)** |
|---|---|---|---|---|---|
| **11** | 553 | 6.54 | **65** | 607 | 4.01 |
| **20** | 42 | 8.99 | **69** | 280 | 3.22 |
| **22** | 47 | 12.40 | **70** | 598 | 3.07 |
| **24** | 17 | 12.40 | **71** | 1,228 | 7.77 |
| **26** | 32 | 9.29 | **74** | 5,522 | 4.44 |
| **27** | 68 | 8.89 | **75** | 2,043 | 2.82 |
| **28** | 1168 | 6.23 | **76** | 5,237 | 6.66 |
| **43** | 346 | 5.07 | **77** | 1,168 | 2.96 |
| **45** | 992 | 3.45 | **78** | 5,739 | 3.75 |
| **47** | 134 | 4.13 | **79** | 1,128 | 3.47 |
| **51** | 635 | 2.81 | **80** | 1,038 | 3.55 |
| **53** | 44 | 3.65 | **81** | 502 | 4.73 |
| **54** | 840 | 6.12 | **82** | 366 | 6.03 |

When introducing elements of the present invention or preferred embodiment(s) thereof, the articles "a", "an", "the" and "said" refer to one or more elements is intended to mean that there is. The terms "comprising", "including" and "having" are intended to be inclusive and mean that there may be additional elements other than those listed. Although the invention has been described in terms of a particular embodiment or embodiments, it should not be construed as limiting the details of these embodiments.

## Claims

1. A compound or a pharmaceutically acceptable salt thereof,
wherein the compound is any one of the following compounds:
| Compound No. | IUPAC Name |
|---|---|
| | |
| 2 | 5-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1,3-dioxoisoindolin-4-yl)pentanamide |
| 6 | (2R,4R)-1-((S)-2-(5-(4-(4-((5-chloro-4-((2-(N-methylmethylsulfonamido)phenyl)amino)pyrimidin-2-yl)amino)-3-methoxyphenyl)piperazin-1-yl)pentanamido)3,3-dimethylbutanoyl)4-hydroxy-N-(4-(4-methylthiazol-5-yl)benzyl)pyrrolidin-2-carboxamide |
| 7 | (2R,4R)-1-((S)-2-(5-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)pentanamido)-3,3-dimethylbutanoyl)-4-hydroxy-N-(4-(4-methylthiazol-5-yl)benzyl)pyrrolidin-2-carboxamide |
| 11 | (2R,4R)-1-((S)-2-(2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)acetamido)-3,3-dimethylbutanoyl)-4-hydroxy-N-(4-(4-methylthiazol-5-yl)benzyl)pyrrolidin-2-carboxamide |
| 13 | N-(2-((5-chloro-2-((4-(4-(4-(2-(2-((2-(2,6-dioxopiperidin-3-yl)-1,3-dioxoisoindolin-4-yl)oxy)ethoxy)ethyl)piperazin-1-yl)piperidin-1-yl)-2-methoxyphenyl)amino)pyrimidin-4-yl)amino)phenyl)N-methylmethanesulfonamide |
| 14 | N-(2-((5-chloro-2-((4-(4-(4-(2-(2-((2-(2,6-dioxopiperidin-3-yl)-1,3-dioxoisoindolin-4-yl)oxy)ethoxy)ethyl)piperazin-1-yl)piperidin-1-yl)-2-methoxyphenyl)amino)pyrimidin-4-yl)amino)phenyl)N-methylmethanesulfonamide |
| 15 | N-(2-((5-chloro-2-((4-(4-(4-(4-((2-(2,6-dioxopiperidin-3-yl)-1,3-dioxoisoindolin-4-yl)oxy)butyl)piperazin-1-yl)piperidin-1-yl)-2-methoxyphenyl)amino)pyrimidin-4-yl)amino)phenyl)-N-methylmethanesulfonamide |
| 16 | N-(2-((5-chloro-2-((4-(4-(2-(2-((2-(2,6-dioxopiperidin-3-yl)-1,3-dioxoisoindolin-4-yl)amino)ethoxy)ethyl)piperazin-1-yl)-2-methoxyphenyl)amino)piperidin-4-yl)amino)phenyl)-N-methylmethanesulfonamide |
| 17 | (2R,4R)-1-((S)-2-(5-(4-(4-((5-chloro-4-((2-(N-methylmethylsulfonamido)phenyl)amino)pyrimidin-2-yl)amino)-2-fluorophenyl)piperazin-1-yl)pentanamido)3,3-dimethylbutanoyl)4-hydroxy-N-(4-(4-methylthiazol-5-yl)benzyl)pyrrolidin-2-carboxamide |
| 19 | (2R,4R)-1-((S)-2-(2-(4-(1-(4-((5-chloro-4-((2-(N-methylmethylsulfonamido)phenyl)amino)pyrimidin-2-yl)amino)-2-fluorophenyl)piperidin-4-yl)piperazin-1-yl)acetamido)-3,3-dimethylbutanoyl)-4-hydroxy-N-(4-(4-methylthiazol-5-yl)benzyl)pyrrolidin-2-carboxamide |
| 20 | (2R,4R)-1-((S)-2-(3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanamido)-3,3-dimethylbutanoyl)-4-hydroxy-N-(4-(4-methylthiazol-5-yl)benzyl)pyrrolidin-2-carboxamide |
| 22 | 2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1-oxoisoindolin-4-yl)acetamide |
| 24 | 2-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)-N-(2-(2,6-dioxopiperidin-3-yl)-1-oxoisoindolin-4-yl)acetamide |
| 26 | 2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)-N-(2-(2,6-dioxopiperidin-3-yl)-1-oxoisoindolin-4-yl)acetamide |
| 27 | (2R,4R)-1-((S)-2-(5-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)acetamido)-3,3-dimethylbutanoyl)-4-hydroxy-N-(4-(4-methylthiazol-5-yl)benzyl)pyrrolidin-2-carboxamide |
| 28 | (2R,4R)-1-((S)-2-(2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamide |
| 31 | (2R,4R)-1-((S)-2-(2-(9-(4-((5-chloro-4-((2-(N-methylmethylsulfonamido)phenyl)amino)pyrimidin-2-yl)amino)-3-methoxyphenyl)-3,9-diazaspiro[5,5]undecan-3-yl)acetamido)-3,3-dimethylbutanoyl)-4-hydroxy-N-(4-(4-methylthiazol-5-yl)benzyl)pyrrolidin-2-carboxamide |
| 33 | N-(2-((5-chloro-2-((4-(4-(2-((1-(2-(2,6-dioxopiperidin-3-yl)-1,3-dioxoisoindolin-5-yl)azetidin-3-yl)oxy)ethyl)piperazin-1-yl)-2-methoxyphenyl)amino)pyrimidin-4-yl)amino)phenyl)-N-methylmethanesulfonamide |
| 35 | (2S,4R)-1-((S)-2-(2-(4-((4-(3-chloro-4-((5-chloro-4-((2-(N-methylmethylsulfonamido)phenyl)amino)pyrimidin-2-yl)amino)phenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamide |
| 36 | (2S,4R)-1-((S)-2-(2-(4-((4-(4-((5-chloro-4-((2-(N-methylmethylsulfonamido)phenyl)amino)pyrimidin-2-yl)amino)2-fluorophenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamide |
| 37 | (2S,4R)-1-((S)-2-(2-(3-((4-(4-((5-chloro-4-((2-(N-methylmethylsulfonamido)phenyl)amino)pyrimidin-2-yl)amino)-3-methoxyphenyl)piperazin-1-yl)methyl)azetidin-1-yl)acetamido)-3,3-dimethylbutanoyl)-4-hydroxy-N-(4-(4-methylthiazol-5-yl)benzyl)pyrrolidin-2-carboxamide |
| 39 | N-(2-((5-chloro-2-((4-(4-(2-(3-((2-(2,6-dioxopiperidin-3-yl)-1-oxoisoindolin-4-yl)oxy)azetidin-1-yl)ethyl)piperazin-1-yl)-3-fluorophenyl)amino)pyrimidin-4-yl)amino)phenyl)-N-methylmethanesulfonamide |
| 40 | N-(2-((5-chloro-2-((4-(4-(2-((1-(2-(2,6-dioxopiperidin-3-yl)-1,3-dioxoisoindolin-5-yl)azetidin-3-yl)oxy)ethyl)piperazin-1-yl)-3-fluorophenyl)amino)pyrimidin-4-yl)amino)phenyl)-N-methylmethanesulfonamide |
| 42 | N-(2-((5-chloro-2-((4-(4-(4-((2-(2,6-dioxopiperidin-3-yl)-1,3-dioxoisoindolin-5-yl)methyl)piperazin-1-yl)piperidin-1-yl)-3-fluorophenyl)amino)pyrimidin-4-yl)amino)phenyl)-N-methylmethanesulfonamide |
| 43 | N-(2-((5-chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-3-fluorophenyl)amino)pyrimidin-4-yl)amino)phenyl)-N-methylmethanesulfonamide |
| 44 | N-(2-((5-chloro-2-((4-(4-(4-(((25)-1-(2-(2,6-dioxopiperidin-3-yl)-1,3-dioxoisoindolin-5-yl)pyrrolidin-2-yl)methyl)piperazin-1-yl)piperidin-1-yl)-2-methoxyphenyl)amino)pyrimidin-4-yl)amino)phenyl)-N-methylmethanesulfonamide |
| 45 | N-(2-((5-chloro-2-((4-(4-(4-(2-((2-(2,6-dioxopiperidin-3-yl)-1,3-dioxoisoindolin-4-yl)amino)ethyl)piperazin-1-yl)piperidin-1-yl)-2-methoxyphenyl)amino)pyrimidin-4-yl)amino)phenyl)-N-methylmethanesulfonamide |
| 46 | N-(2-((5-chloro-2-((4-(4-((1-(2-((2-(2,6-dioxopiperidin-3-yl)-1,3-dioxoisoindolin-4-yl)oxy)ethyl)piperidin-4-yl)methyl)piperazin-1-yl)-3-fluorophenyl)amino)pyrimidin-4-yl)amino)phenyl)-N-methylmethanesulfonamide |
| 47 | 2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1-oxoisoindolin-5-yl)acetamide |
| 48 | 2-(4-((4-(4-((5-chloro-4-((2-(N-methylmethylsulfonamido)phenyl)amino)pyrimidin-2-yl)amino)-2-fluorophenyl)piperazin-1-yl)methyl)piperidin-1-yl)-N-(2-(2,6-dioxopiperidin-3-yl)-1-oxoisoindolin-4-yl)acetamide |
| 50 | 2-(9-(4-((5-chloro-4-((2-(N-methylmethylsulfonamido)phenyl)amino)pyrimidin-2-yl)amino)-3-methoxyphenyl)-3,9-diazaspiro[5.5]undecan-3-yl)-N-(2-(2,6-dioxopiperidin-3-yl)-1-oxoisoindolin-4-yl)acetamide |
| 51 | N-(2-((5-chloro-2-((4-(4-(4-(2-(1-(2-(2,6-dioxopiperidin-3-yl)-1,3-dioxoisoindolin-5-yl)piperidin-4-yl)ethyl)piperazin-1-yl)piperidin-1-yl)-2-methoxyphenyl)amino)pyrimidin-4-yl)amino)phenyl)-N-methylmethanesulfonamide |
| 52 | N-(2-((5-chloro-2-((4-(4-(4-(2-((2-(2,6-dioxopiperidin-3-yl)-1,3-dioxoisoindolin-4-yl)oxy)ethyl)piperazin-1-yl)piperidin-1-yl)-2-methoxyphenyl)amino)pyrimidin-4-yl)amino)phenyl)-N-methylmethanesulfonamide |
| 53 | N-(2-((5-chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-2-methoxyphenyl)amino)pyrimidin-4-yl)amino)phenyl)-N-methylmethanesulfonamide |
| 54 | N-(2-((5-chloro-2-((4-(4-((1-(3-(2-(2,6-dioxopiperidin-3-yl)-1,3-dioxoisoindolin-4-yl)propanoyl)piperidin-4-yl)methyl)piperazin-1-yl)-2-methoxyphenyl)amino)pyrimidin-4-yl)amino)phenyl)-N-methylmethanesulfonamide |
| 56 | (2R,4R)-1-((2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)acetyl)-L-valyl)-4-hydroxy-N-(4-(4-methylthiazol-5-yl)benzyl)pyrrolidin-2-carboxamide |
| 58 | N-(2-((5-chloro-2-((4-(4-(4-(2-((2-(2,6-dioxopiperidin-3-yl)-1,3-dioxoisoindolin-5-yl)amino)ethyl)piperazin-1-yl)piperidin-1-yl)-2-methoxyphenyl)amino)pyrimidin-4-yl)amino)phenyl)-N-methylmethanesulfonamide |
| 59 | N-(2-((5-chloro-2-((4-(4-((1-((1-(2-(2,6-dioxopiperidin-3-yl)-1,3-dioxoisoindolin-5-yl)piperidin-4-yl)methyl)piperidin-4-yl)methyl)piperazin-1-yl)-2-methoxyphenyl)amino)pyrimidin-4-yl)amino)phenyl)-N-methylmethanesulfonamide |
| 60 | N-(2-((5-chloro-2-((4-(4-(4-((1-(2-(2,6-dioxopiperidin-3-yl)-1,3-dioxoisoindolin-5-yl)azetidin-3-yl)methyl)piperazin-1-yl)piperidin-1-yl)-2-methoxyphenyl)amino)pyrimidin-4-yl)amino)phenyl)-N-methylmethanesulfonamide |
| 61 | N-(2-((5-chloro-2-((4-(4-((1-((1-(2-(2,6-dioxopiperidin-3-yl)-1,3-dioxoisoindolin-5-yl)azetidin-3-yl)methyl)piperidin-4-yl)methyl)piperazin-1-yl)-2-methoxyphenyl)amino)pyrimidin-4-yl)amino)phenyl)-N-methylmethanesulfonamide |
| 62 | N-(2-((5-chloro-2-((4-(4-((4-(((2-(2,6-dioxopiperidin-3-yl)-1,3-dioxoisoindolin-4-yl)amino)methyl)piperidin-1-yl)methyl)piperidin-1-yl)-2-methoxyphenyl)amino)pyrimidin-4-yl)amino)phenyl)-N-methylmethanesulfonamide |
| 63 | N-(2-((5-chloro-2-((4-(4-((4-(((2-(2,6-dioxopiperidin-3-yl)-1,3-dioxoisoindolin-5-yl)amino)methyl)piperidin-1-yl)methyl)piperidin-1-yl)-2-methoxyphenyl)amino)pyrimidin-4-yl)amino)phenyl)-N-methylmethanesulfonamide |
| 64 | N-(2-((5-chloro-2-((4-(4-((1-(3-(2-(2,6-dioxopiperidin-3-yl)-1,3-dioxoisoindolin-5-yl)propanoyl)piperidin-4-yl)methyl)piperazin-1-yl)-2-methoxyphenyl)amino)pyrimidin-4-yl)amino)phenyl)-N-methylmethanesulfonamide |
| 65 | N-(2-((5-chloro-2-((4-(4-((1-((2-(2,6-dioxopiperidin-3-yl)-1,3-dioxoisoindolin-4-yl)glycyl)piperidin-4-yl)methyl)piperazin-1-yl)-2-methoxyphenyl)amino)pyrimidin-4-yl)amino)phenyl)-N-methylmethanesulfonamide |
| 66 | N-(2-((5-chloro-2-((4-(4-((1-((2-(2,6-dioxopiperidin-3-yl)-1,3-dioxoisoindolin-5-yl)glycyl)piperidin-4-yl)methyl)piperazin-1-yl)-2-methoxyphenyl)amino)pyrimidin-4-yl)amino)phenyl)-N-methylmethanesulfonamide |
| 67 | N-(2-((5-chloro-2-((4-(4-((4-(2-(2,6-dioxopiperidin-3-yl)-1,3-dioxoisoindolin-5-yl)piperazin-1-yl)methyl)piperidin-1-yl)-2-methoxyphenyl)amino)pyrimidin-4-yl)amino)phenyl)-N-methylmethanesulfonamide |
| 68 | N-(2-((5-chloro-2-((4-(4-((4-(2-(2,6-dioxopiperidin-3-yl)-1,3-dioxoisoindolin-5-yl)piperidin-4-yl)methyl)piperazin-1-yl)-2-methoxyphenyl)amino)pyrimidin-4-yl)amino)phenyl)-N-methylmethanesulfonamide |
| 69 | N-(2-((5-chloro-2-((4-(4-((1-((2-(2,6-dioxopiperidin-3-yl)-1,3-dioxoisoindolin-4-yl)oxy)propanoyl)piperidin-4-yl)methyl)piperazin-1-yl)-2-methoxyphenyl)amino)pyrimidin-4-yl)amino)phenyl)-N-methylmethanesulfonamide |
| 70 | N-(2-((5-chloro-2-((4-(4-((1-((2-(2,6-dioxopiperidin-3-yl)-1,3-dioxoisoindolin-4-yl)-L-alanyl)piperidin-4-yl)methyl)piperazin-1-yl)-2-methoxyphenyl)amino)pyrimidin-4-yl)amino)phenyl)-N-methylmethanesulfonamide |
| 71 | N-(2-((5-chloro-2-((4-(4-(4-((1-(2-(2,6-dioxopiperidin-3-yl)-1,3-dioxoisoindolin-5-yl)piperidin-4-yl)methyl)piperazin-1-yl)piperidin-1-yl)-2-methoxyphenyl)amino)pyrimidin-4-yl)amino)phenyl)-N-methylmethanesulfonamide |
| 74 | N-(2-((5-chloro-2-((4-(4-(4-(2-((2-(2,6-dioxopiperidin-3-yl)-1,3-dioxoisoindolin-4-yl)amino)ethyl)piperazin-1-yl)piperidin-1-yl)-2-methoxy-5-methylphenyl)amino)pyrimidin-4-yl)amino)phenyl)-N-methylmethanesulfonamide |
| 75 | N-(2-((5-chloro-2-((4-(4-((1-((2-(2,6-dioxopiperidin-3-yl)-1,3-dioxoisoindolin-4-yl)-L-alanyl)piperidin-4-yl)methyl)piperazin-1-yl)-2-methoxy-5-methylphenyl)amino)pyrimidin-4-yl)amino)phenyl)-N-methylmethanesulfonamide |
| 76 | N-(2-((5-chloro-2-((4-(4-(4-((1-(2-(2,6-dioxopiperidin-3-yl)-1,3-dioxoisoindolin-5-yl)piperidin-4-yl)methyl)piperazin-1-yl)piperidin-1-yl)-2-methoxy-5-methylphenyl)amino)pyrimidin-4-yl)amino)phenyl)-N-methylmethanesulfonamide |
| 77 | N-(2-((5-chloro-2-((4-(4-(4-(2-(1-(2-(2,6-dioxopiperidin-3-yl)-1,3-dioxoisoindolin-5-yl)piperidin-4-yl)ethyl)piperazin-1-yl)piperidin-1-yl)-2-methoxy5-methylphenyl)amino)pyrimidin-4-yl)amino)phenyl)-N-methylmethanesulfonamide |
| 78 | N-(2-((5-chloro-2-((4-(4-((4-((2-(2,6-dioxopiperidin-3-yl)-1,3-dioxoisoindolin-4-yl)-L-alanyl)piperazin-1-yl)methyl)piperidin-1-yl)-2-methoxy-5-methylphenyl)amino)pyrimidin-4-yl)amino)phenyl)-N-methylmethanesulfonamide |
| 79 | N-(2-((5-chloro-2-((4-(4-((4-(2-(2,6-dioxopiperidin-3-yl)-1,3-dioxoisoindolin-5-yl)piperazin-1-yl)methyl)piperidin-1-yl)-2-methoxy-5-methylphenyl)amino)pyrimidin-4-yl)amino)phenyl)-N-methylmethanesulfonamide |
| 80 | N-(2-((5-chloro-2-((4-(4-((1-((2-(2,6-dioxopiperidin-3-yl)-1,3-dioxoisoindolin-4-yl)glycyl)piperidin-4-yl)methyl)piperazin-1-yl)-2-methoxy-5-methylphenyl)amino)pyrimidin-4-yl)amino)phenyl)-N-methylmethanesulfonamide |
| 81 | N-(2-((5-chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-2-methoxy-5-methylphenyl)amino)pyrimidin-4-yl)amino)phenyl)-N-methylmethanesulfonamide |
| 82 | N-(2-((5-chloro-2-((4-(4-((1-((2S)-2-((2-(2,6-dioxopiperidin-3-yl)-1,3-dioxoisoindolin-4-yl)oxy)propanoyl)piperidin-4-yl)methyl)piperazin-1-yl)-2-methoxy-5-methylphenyl)amino)pyrimidin-4-yl)amino)phenyl)-N-methylmethanesulfonamide |

2. The compound of Claim 1 or a pharmaceutically acceptable salt thereof, wherein the compound is any one of the following compounds:
| Compound No. | IUPAC Name |
|---|---|
| 11 | (2R,4R)-1-((S)-2-(2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)acetamido)-3,3-dimethylbutanoyl)-4-hydroxy-N-(4-(4-methylthiazol-5-yl)benzyl)pyrrolidin-2-carboxamide |
| 19 | (2R,4R)-1-((S)-2-(2-(4-(1-(4-((5-chloro-4-((2-(N-methylmethylsulfonamido)phenyl)amino)pyrimidin-2-yl)amino)-2-fluorophenyl)piperidin-4-yl)piperazin-1-yl)acetamido)-3,3-dimethylbutanoyl)-4-hydroxy-N-(4-(4-methylthiazol-5-yl)benzyl)pyrrolidin-2-carboxamide |
| 20 | (2R,4R)-1-((S)-2-(3-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)propanamido)-3,3-dimethylbutanoyl)-4-hydroxy-N-(4-(4-methylthiazol-5-yl)benzyl)pyrrolidin-2-carboxamide |
| 22 | 2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1-oxoisoindolin-4-yl)acetamide |
| 24 | 2-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)-N-(2-(2,6-dioxopiperidin-3-yl)-1-oxoisoindolin-4-yl)acetamide |
| 26 | 2-(4-((4-(4-((5-chloro-4-((2-(N-methylmethylsulfonamido)phenyl)amino)pyrimidin-2-yl)amino)-3-methoxyphenyl)piperazin-1-yl)methyl)piperidin-1-yl)-N-(2-(2,6-dioxopiperidin-3-yl)-1-oxoisoindolin-4-yl)acetamide |
| 27 | (2R,4R)-1-((S)-2-(5-(4-((1-(4-((5-chloro-4-((2-(N-methylmethylsulfonamido)phenyl)amino)pyrimidin-2-yl)amino)-3-methoxyphenyl)piperidin-4-yl)methyl)piperazin-1-yl)acetamido)-3,3-dimethylbutanoyl)-4-hydroxy-N-(4-(4-methylthiazol-5-yl)benzyl)pyrrolidin-2-carboxamide |
| 28 | (2R,4R)-1-((S)-2-(2-(4-((4-(4-((S-chloro-4-((2-(N-methylmethylsulfonamido)phenyl)amino)pyrimidin-2-yl)amino)-3-methoxyphenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamide |
| 31 | (2R,4R)-1-((S)-2-(2-(9-(4-((5-chloro-4-((2-(N-methylmethylsulfonamido)phenyl)amino)pyrimidin-2-yl)amino)-3-methoxyphenyl)-3,9-diazaspiro[5,5]undecan-3-yl)acetamido)-3,3-dimethylbutanoyl)-4-hydroxy-N-(4-(4-methylthiazol-5-yl)benzyl)pyrrolidin-2-carboxamide |
| 35 | (2S,4R)-1-((S)-2-(2-(4-((4-(3-chloro-4-((5-chloro-4-((2-(N-methylmethylsulfonamido)phenyl)amino)pyrimidin-2-yl)amino)phenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamide |
| 36 | (2S,4R)-1-((S)-2-(2-(4-((4-(4-((5-chloro-4-((2-(N-methylmethylsulfonamido)phenyl)amino)pyrimidin-2-yl)amino)2-fluorophenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamide |
| 39 | N-(2-((5-chloro-2-((4-(4-(2-(3-((2-(2,6-dioxopiperidin-3-yl)-1-oxoisoindolin-4-yl)oxy)azetidin-1-yl)ethyl)piperazin-1-yl)-3-fluorophenyl)amino)pyrimidin-4-yl)amino)phenyl)-N-methylmethanesulfonamide |
| 40 | N-(2-((5-chloro-2-((4-(4-(2-((1-(2-(2,6-dioxopiperidin-3-yl)-1,3-dioxoisoindolin-5-yl)azetidin-3-yl)oxy)ethyl)piperazin-1-yl)-3-fluorophenyl)amino)pyrimidin-4-yl)amino)phenyl)-N-methylmethanesulfonamide |
| 43 | N-(2-((5-chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-3-fluorophenyl)amino)pyrimidin-4-yl)amino)phenyl)-N-methylmethanesulfonamide |
| 45 | N-(2-((5-chloro-2-((4-(4-(4-(2-((2-(2,6-dioxopiperidin-3-yl)-1,3-dioxoisoindolin-4-yl)amino)ethyl)piperazin-1-yl)piperidin-1-yl)-2-methoxyphenyl)amino)pyrimidin-4-yl)amino)phenyl)-N-methylmethanesulfonamide |
| 46 | N-(2-((5-chloro-2-((4-(4-((1-(2-((2-(2,6-dioxopiperidin-3-yl)-1,3-dioxoisoindolin-4-yl)oxy)ethyl)piperidin-4-yl)methyl)piperazin-1-yl)-3-fluorophenyl)amino)pyrimidin-4-yl)amino)phenyl)-N-methylmethanesulfonamide |
| 47 | 2-(4-(1-(4-((5-chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1-oxoisoindolin-5-yl)acetamide |
| 51 | N-(2-((5-chloro-2-((4-(4-(4-(2-(1-(2-(2,6-dioxopiperidin-3-yl)-1,3-dioxoisoindolin-5-yl)piperidin-4-yl)ethyl)piperazin-1-yl)piperidin-1-yl)-2-methoxyphenyl)amino)pyrimidin-4-yl)amino)phenyl)-N-methylmethanesulfonamide |
| 53 | N-(2-((5-chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-2-methoxyphenyl)amino)pyrimidin-4-yl)amino)phenyl)-N-methylmethanesulfonamide |
| 54 | N-(2-((5-chloro-2-((4-(4-((1-(3-(2-(2,6-dioxopiperidin-3-yl)-1,3-dioxoisoindolin-4-yl)propanoyl)piperidin-4-yl)methyl)piperazin-1-yl)-2-methoxyphenyl)amino)pyrimidin-4-yl)amino)phenyl)-N-methylmethanesulfonamide |
| 59 | N-(2-((5-chloro-2-((4-(4-((1-((1-(2-(2,6-dioxopiperidin-3-yl)-1,3-dioxoisoindolin-5-yl)piperidin-4-yl)methyl)piperidin-4-yl)methyl)piperazin-1-yl)-2-methoxyphenyl)amino)pyrimidin-4-yl)amino)phenyl)-N-methylmethanesulfonamide |
| 62 | N-(2-((5-chloro-2-((4-(4-((4-(((2-(2,6-dioxopiperidin-3-yl)-1,3-dioxoisoindolin-4-yl)amino)methyl)piperidin-1-yl)methyl)piperidin-1-yl)-2-methoxyphenyl)amino)pyrimidin-4-yl)amino)phenyl)-N-methylmethanesulfonamide |
| 63 | N-(2-((5-chloro-2-((4-(4-((4-(((2-(2,6-dioxopiperidin-3-yl)-1,3-dioxoisoindolin-5-yl)amino)methyl)piperidin-1-yl)methyl)piperidin-1-yl)-2-methoxyphenyl)amino)pyrimidin-4-yl)amino)phenyl)-N-methylmethanesulfonamide |
| 65 | N-(2-((5-chloro-2-((4-(4-((1-((2-(2,6-dioxopiperidin-3-yl)-1,3-dioxoisoindolin-4-yl)glycyl)piperidin-4-yl)methyl)piperazin-1-yl)-2-methoxyphenyl)amino)pyrimidin-4-yl)amino)phenyl)-N-methylmethanesulfonamide |
| 66 | N-(2-((5-chloro-2-((4-(4-((1-((2-(2,6-dioxopiperidin-3-yl)-1,3-dioxoisoindolin-5-yl)glycyl)piperidin-4-yl)methyl)piperazin-1-yl)-2-methoxyphenyl)amino)pyrimidin-4-yl)amino)phenyl)-N-methylmethanesulfonamide |
| 68 | N-(2-((5-chloro-2-((4-(4-((4-(2-(2,6-dioxopiperidin-3-yl)-1,3-dioxoisoindolin-5-yl)piperidin-4-yl)methyl)piperazin-1-yl)-2-methoxyphenyl)amino)pyrimidin-4-yl)amino)phenyl)-N-methylmethanesulfonamide |
| 69 | N-(2-((5-chloro-2-((4-(4-((1-((2-(2,6-dioxopiperidin-3-yl)-1,3-dioxoisoindolin-4-yl)oxy)propanoyl)piperidin-4-yl)methyl)piperazin-1-yl)-2-methoxyphenyl)amino)pyrimidin-4-yl)amino)phenyl)-N-methylmethanesulfonamide |
| 70 | N-(2-((5-chloro-2-((4-(4-((1-((2-(2,6-dioxopiperidin-3-yl)-1,3-dioxoisoindolin-4-yl)-L-alanyl)piperidin-4-yl)methyl)piperazin-1-yl)-2-methoxyphenyl)amino)pyrimidin-4-yl)amino)phenyl)-N-methylmethanesulfonamide |
| 71 | N-(2-((5-chloro-2-((4-(4-(4-((1-(2-(2,6-dioxopiperidin-3-yl)-1,3-dioxoisoindolin-5-yl)piperidin-4-yl)methyl)piperazin-1-yl)piperidin-1-yl)-2-methoxyphenyl)amino)pyrimidin-4-yl)amino)phenyl)-N-methylmethanesulfonamide |
| 74 | N-(2-((5-chloro-2-((4-(4-(4-(2-((2-(2,6-dioxopiperidin-3-yl)-1,3-dioxoisoindolin-4-yl)amino)ethyl)piperazin-1-yl)piperidin-1-yl)-2-methoxy-5-methylphenyl)amino)pyrimidin-4-yl)amino)phenyl)-N-methylmethanesulfonamide |
| 75 | N-(2-((5-chloro-2-((4-(4-((1-((2-(2,6-dioxopiperidin-3-yl)-1,3-dioxoisoindolin-4-yl)-L-alanyl)piperidin-4-yl)methyl)piperazin-1-yl)-2-methoxy-5-methylphenyl)amino)pyrimidin-4-yl)amino)phenyl)-N-methylmethanesulfonamide |
| 76 | N-(2-((5-chloro-2-((4-(4-(4-((1-(2-(2,6-dioxopiperidin-3-yl)-1,3-dioxoisoindolin-5-yl)piperidin-4-yl)methyl)piperazin-1-yl)piperidin-1-yl)-2-methoxy-5-methylphenyl)amino)pyrimidin-4-yl)amino)phenyl)-N-methylmethanesulfonamide |
| 77 | N-(2-((5-chloro-2-((4-(4-(4-(2-(1-(2-(2,6-dioxopiperidin-3-yl)-1,3-dioxoisoindolin-5-yl)piperidin-4-yl)ethyl)piperazin-1-yl)piperidin-1-yl)-2-methoxy5-methylphenyl)amino)pyrimidin-4-yl)amino)phenyl)-N-methylmethanesulfonamide |
| 78 | N-(2-((5-chloro-2-((4-(4-((4-((2-(2,6-dioxopiperidin-3-yl)-1,3-dioxoisoindolin-4-yl)-L-alanyl)piperazin-1-yl)methyl)piperidin-1-yl)-2-methoxy-5-methylphenyl)amino)pyrimidin-4-yl)amino)phenyl)-N-methylmethanesulfonamide |
| 80 | N-(2-((5-chloro-2-((4-(4-((1-((2-(2,6-dioxopiperidin-3-yl)-1,3-dioxoisoindolin-4-yl)glycyl)piperidin-4-yl)methyl)piperazin-1-yl)-2-methoxy-5-methylphenyl)amino)pyrimidin-4-yl)amino)phenyl)-N-methylmethanesulfonamide |
| 81 | N-(2-((5-chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-2-methoxy-5-methylphenyl)amino)pyrimidin-4-yl)amino)phenyl)-N-methylmethanesulfonamide |
| 82 | N-(2-((5-chloro-2-((4-(4-((1-((2S)-2-((2-(2,6-dioxopiperidin-3-yl)-1,3-dioxoisoindolin-4-yl)oxy)propanoyl)piperidin-4-yl)methyl)piperazin-1-yl)-2-methoxy-5-methylphenyl)amino)pyrimidin-4-yl)amino)phenyl)-N-methylmethanesulfonamide |

3. A composition comprising a compound of Claim 1 or Claim 2 or a pharmaceutically acceptable salt thereof, and a pharmaceutically acceptable carrier.

4. A pharmaceutical composition for use in the treatment or prevention of cancer having an EGFR mutation, comprising the compound of Claim 1 or Claim 2 or a pharmaceutically acceptable salt thereof as an active ingredient.

5. The pharmaceutical composition for use of Claim 4, wherein the cancer is lung cancer, liver cancer, esophageal cancer, stomach cancer, colon cancer, small intestine cancer, pancreatic cancer, melanoma, breast cancer, oral cancer, brain tumor, thyroid cancer, parathyroid cancer, kidney cancer, cervical cancer, sarcoma, prostate cancer, urethral cancer, bladder cancer, testicular cancer, blood cancer, lymphoma, skin cancer or fibroadenoma.

6. The pharmaceutical composition for use of Claim 5, wherein the cancer is lung cancer.

7. The pharmaceutical composition for use of Claim 6, wherein the lung cancer is non-small cell lung cancer.

## Patentansprüche

1. Verbindung oder ein pharmazeutisch akzeptables Salz davon,
wobei es sich bei der Verbindung um eine der folgenden Verbindungen handelt:
| Verbindung Nr. | IUPAC-Benennung |
|---|---|
| | |
| 2 | 5-(4-(1-(4-((5-Chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1,3-dioxoisoindolin-4-yl)pentanamid |
| 6 | (2R,4R)-1-((5)-2-(5-(4-(4-((5-Chloro-4-((2-(N-methylmethylsulfonamido)phenyl)amino)pyrimidin-2-yl)amino)-3-methoxyphenyl)piperazin-1-yl)pentanamido)3,3-dimethylbutanoyl)4-hydroxy-N-(4-(4-methylthiazol-5-yl)benzyl)pyrrolidin-2-carboxamid |
| 7 | (2R,4R)-1-((S)-2-(5-(4-(1-(4-((5-Chloro-4-((2-(N-methylmethylsulfonamido)phenyl)amino)pyrimidin-2-yl)amino)-3-methoxyphenyl)piperidin-4-yl)piperazin-1-yl)pentanamido)-3,3-dimethylbutanoyl)-4-hydroxy-N-(4-(4-methylthiazol-5-yl)benzyl)pyrrolidin-2-carboxamid |
| 11 | (2R,4R)-1-((S)-2-(2-(4-(1-(4-((5-Chloro-4-((2-(N-methylmethylsulfonamido)phenyl)amino)pyrimidin-2-yl)amino)-3-methoxyphenyl)piperidin-4-yl)piperazin-1-yl)acetamido)-3,3-dimethylbutanoyl)-4-hydroxy-N-(4-(4-methylthiazol-5-yl)benzyl)pyrrolidin-2-carboxamid |
| 13 | N-(2-((5-Chloro-2-((4-(4-(4-(2-(2-((2-(2,6-dioxopiperidin-3-yl)-1,3-dioxoisoindolin-4-yl)oxy)ethoxy)ethyl)piperazin-1-yl)piperidin-1-yl)-2-methoxyphenyl)amino)pyrimidin-4-yl)amino)phenyl)N-methylmethansulfonamid |
| 14 | N-(2-((5-Chloro-2-((4-(4-(4-(2-(2-((2-(2,6-dioxopiperidin-3-yl)-1,3-dioxoisoindolin-4-yl)oxy)ethoxy)ethyl)piperazin-1-yl)piperidin-1-yl)-2-methoxyphenyl)amino)pyrimidin-4-yl)amino)phenyl)N-methylmethansulfonamid |
| 15 | N-(2-((5-Chloro-2-((4-(4-(4-(4-((2-(2,6-dioxopiperidin-3-yl)-1,3-dioxoisoindolin-4-yl)oxy)butyl)piperazin-1-yl)piperidin-1-yl)-2-methoxyphenyl)amino)pyrimidin-4-yl)amino)phenyl)-N-methylmethansulfonamid |
| 16 | N-(2-((5-Chloro-2-((4-(4-(2-(2-((2-(2,6-dioxopiperidin-3-yl)-1,3-dioxoisoindolin-4-yl)amino)ethoxy)ethyl)piperazin-1-yl)-2-methoxyphenyl)amino)piperidin-4-yl)amino)phenyl)-N-methylmethansulfonamid |
| 17 | (2R,4R)-1-((S)-2-(5-(4-(4-((5-Chloro-4-((2-(N-methylmethylsulfonamido)phenyl)amino)pyrimidin-2-yl)amino)-2-fluorophenyl)piperazin-1-yl)pentanamido)3,3-dimethylbutanoyl)4-hydroxy-N-(4-(4-methylthiazol-5-yl)benzyl)pyrrolidin-2-carboxamid |
| 19 | (2R,4R)-1-((S)-2-(2-(4-(1-(4-((5-Chloro-4-((2-(N-methylmethylsulfonamido)phenyl)amino)pyrimidin-2-yl)amino)-2-fluorophenyl)piperidin-4-yl)piperazin-1-yl)acetamido)-3,3-dimethylbutanoyl)-4-hydroxy-N-(4-(4-methylthiazol-5-yl)benzyl)pyrrolidin-2-carboxamid |
| 20 | (2R,4R)-1-((S)-2-(3-(4-(1-(4-((5-Chloro-4-((2-(N-methylmethylsulfonamido)phenyl)amino)pyrimidin-2-yl)amino)-3-methoxyphenyl)piperidin-4-yl)piperazin-1-yl)propanamido)-3,3-dimethylbutanoyl)-4-hydroxy-N-(4-(4-methylthiazol-5-yl)benzyl)pyrrolidin-2-carboxamid |
| 22 | 2-(4-(1-(4-((5-Chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1-oxoisoindolin-4-yl)acetamid |
| 24 | 2-(4-((1-(4-((5-Chloro-4-((2-(N-methylmethylsulfonamido)phenyl)amino)pyrimidin-2-yl)amino)-3-methoxyphenyl)piperidin-4-yl)methyl)piperazin-1-yl)-N-(2-(2,6-dioxopiperidin-3-yl)-1-oxoisoindolin-4-yl)acetamid |
| 26 | 2-(4-((4-(4-((5-Chloro-4-((2-(N-methylmethylsulfonamido)phenyl)amino)pyrimidin-2-yl)amino)-3-methoxyphenyl)piperazin-1-yl)methyl)piperidin-1-yl)-N-(2-(2,6-dioxopiperidin-3-yl)-1-oxoisoindolin-4-yl)acetamid |
| 27 | (2R,4R)-1-((S)-2-(5-(4-((1-(4-((5-Chloro-4-((2-(N-methylmethylsulfonamido)phenyl)amino)pyrimidin-2-yl)amino)-3-methoxyphenyl)piperidin-4-yl)methyl)piperazin-1-yl)acetamido)-3,3-dimethylbutanoyl)-4-hydroxy-N-(4-(4-methylthiazol-5-yl)benzyl)pyrrolidin-2-carboxamid |
| 28 | (2R,4R)-1-((S)-2-(2-(4-((4-(4-((5-Chloro-4-((2-(N-methylmethylsulfonamido)phenyl)amino)pyrimidin-2-yl)amino)-3-methoxyphenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamid |
| 31 | (2R,4R)-1-((S)-2-(2-(9-(4-((5-Chloro-4-((2-(N-methylmethylsulfonamido)phenyl)amino)pyrimidin-2-yl)amino)-3-methoxyphenyl)-3,9-diazaspiro[5,5]undecan-3-yl)acetamido)-3,3-dimethylbutanoyl)-4-hydroxy-N-(4-(4-methylthiazol-5-yl)benzyl)pyrrolidin-2-carboxamid |
| 33 | N-(2-((5-Chloro-2-((4-(4-(2-((1-(2-(2,6-dioxopiperidin-3-yl)-1,3-dioxoisoindolin-5-yl)azetidin-3-yl)oxy)ethyl)piperazin-1-yl)-2-methoxyphenyl)amino)pyrimidin-4-yl)amino)phenyl)-N-methylmethansulfonamid |
| 35 | (2S,4R)-1-((S)-2-(2-(4-((4-(3-Chloro-4-((5-chloro-4-((2-(N-methylmethylsulfonamido)phenyl)amino)pyrimidin-2-yl)amino)phenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamid |
| 36 | (2S,4R)-1-((S)-2-(2-(4-((4-(4-((5-Chloro-4-((2-(N-methylmethylsulfonamido)phenyl)amino)pyrimidin-2-yl)amino)2-fluorophenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamid |
| 37 | (2S,4R)-1-((S)-2-(2-(3-((4-(4-((5-Chloro-4-((2-(N-methylmethylsulfonamido)phenyl)amino)pyrimidin-2-yl)amino)-3-methoxyphenyl)piperazin-1-yl)methyl)azetidin-1-yl)acetamido)-3,3-dimethylbutanoyl)-4-hydroxy-N-(4-(4-methylthiazol-5-yl)benzyl)pyrrolidin-2-carboxamid |
| 39 | N-(2-((5-Chloro-2-((4-(4-(2-(3-((2-(2,6-dioxopiperidin-3-yl)-1-oxoisoindolin-4-yl)oxy)azetidin-1-yl)ethyl)piperazin-1-yl)-3-fluorophenyl)amino)pyrimidin-4-yl)amino)phenyl)-N-methylmethansulfonamid |
| 40 | N-(2-((5-Chloro-2-((4-(4-(2-((1-(2-(2,6-dioxopiperidin-3-yl)-1,3-dioxoisoindolin-5-yl)azetidin-3-yl)oxy)ethyl)piperazin-1-yl)-3-fluorophenyl)amino)pyrimidin-4-yl)amino)phenyl)-N-methylmethansulfonamid |
| 42 | N-(2-((5-Chloro-2-((4-(4-(4-((2-(2,6-dioxopiperidin-3-yl)-1,3-dioxoisoindolin-5-yl)methyl)piperazin-1-yl)piperidin-1-yl)-3-fluorophenyl)amino)pyrimidin-4-yl)amino)phenyl)-N-methylmethansulfonamid |
| 43 | N-(2-((5-Chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-3-fluorophenyl)amino)pyrimidin-4-yl)amino)phenyl)-N-methylmethansulfonamid |
| 44 | N-(2-((5-Chloro-2-((4-(4-(4-(((2S)-1-(2-(2,6-dioxopiperidin-3-yl)-1,3-dioxoisoindolin-5-yl)pyrrolidin-2-yl)methyl)piperazin-1-yl)piperidin-1-yl)-2-methoxyphenyl)amino)pyrimidin-4-yl)amino)phenyl)-N-methylmethansulfonamid |
| 45 | N-(2-((5-Chloro-2-((4-(4-(4-(2-((2-(2,6-dioxopiperidin-3-yl)-1,3-dioxoisoindolin-4-yl)amino)ethyl)piperazin-1-yl)piperidin-1-yl)-2-methoxyphenyl)amino)pyrimidin-4-yl)amino)phenyl)-N-methylmethansulfonamid |
| 46 | N-(2-((5-Chloro-2-((4-(4-((1-(2-((2-(2,6-dioxopiperidin-3-yl)-1,3-dioxoisoindolin-4-yl)oxy)ethyl)piperidin-4-yl)methyl)piperazin-1-yl)-3-fluorophenyl)amino)pyrimidin-4-yl)amino)phenyl)-N-methylmethansulfonamid |
| 47 | 2-(4-(1-(4-((5-Chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1-oxoisoindolin-5-yl)acetamid |
| 48 | 2-(4-((4-(4-((5-Chloro-4-((2-(N-methylmethylsulfonamido)phenyl)amino)pyrimidin-2-yl)amino)-2-fluorophenyl)piperazin-1-yl)methyl)piperidin-1-yl)-N-(2-(2,6-dioxopiperidin-3-yl)-1-oxoisoindolin-4-yl)acetamid |
| 50 | 2-(9-(4-((5-Chloro-4-((2-(N-methylmethylsulfonamido)phenyl)amino)pyrimidin-2-yl)amino)-3-methoxyphenyl)-3,9-diazaspiro[5.5]undecan-3-yl)-N-(2-(2,6-dioxopiperidin-3-yl)-1-oxoisoindolin-4-yl)acetamid |
| 51 | N-(2-((5-Chloro-2-((4-(4-(4-(2-(1-(2-(2,6-dioxopiperidin-3-yl)-1,3-dioxoisoindolin-5-yl)piperidin-4-yl)ethyl)piperazin-1-yl)piperidin-1-yl)-2-methoxyphenyl)amino)pyrimidin-4-yl)amino)phenyl)-N-methylmethansulfonamid |
| 52 | N-(2-((5-Chloro-2-((4-(4-(4-(2-((2-(2,6-dioxopiperidin-3-yl)-1,3-dioxoisoindolin-4-yl)oxy)ethyl)piperazin-1-yl)piperidin-1-yl)-2-methoxyphenyl)amino)pyrimidin-4-yl)amino)phenyl)-N-methylmethansulfonamid |
| 53 | N-(2-((5-Chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-2-methoxyphenyl)amino)pyrimidin-4-yl)amino)phenyl)-N-methylmethansulfonamid |
| 54 | N-(2-((5-Chloro-2-((4-(4-((1-(3-(2-(2,6-dioxopiperidin-3-yl)-1,3-dioxoisoindolin-4-yl)propanoyl)piperidin-4-yl)methyl)piperazin-1-yl)-2-methoxyphenyl)amino)pyrimidin-4-yl)amino)phenyl)-N-methylmethansulfonamid |
| 56 | (2R,4R)-1-((2-(4-((4-(4-((5-Chloro-4-((2-(N-methylmethylsulfonamido)phenyl)amino)pyrimidin-2-yl)amino)-3-methoxyphenyl)piperazin-1-yl)methyl)piperidin-1-yl)acetyl)-L-valyl)-4-hydroxy-N-(4-(4-methylthiazol-5-yl)benzyl)pyrrolidin-2-carboxamid |
| 58 | N-(2-((5-Chloro-2-((4-(4-(4-(2-((2-(2,6-dioxopiperidin-3-yl)-1,3-dioxoisoindolin-5-yl)amino)ethyl)piperazin-1-yl)piperidin-1-yl)-2-methoxyphenyl)amino)pyrimidin-4-yl)amino)phenyl)-N-methylmethansulfonamid |
| 59 | N-(2-((5-Chloro-2-((4-(4-((1-((1-(2-(2,6-dioxopiperidin-3-yl)-1,3-dioxoisoindolin-5-yl)piperidin-4-yl)methyl)piperidin-4-yl)methyl)piperazin-1-yl)-2-methoxyphenyl)amino)pyrimidin-4-yl)amino)phenyl)-N-methylmethansulfonamid |
| 60 | N-(2-((5-Chloro-2-((4-(4-(4-((1-(2-(2,6-dioxopiperidin-3-yl)-1,3-dioxoisoindolin-5-yl)azetidin-3-yl)methyl)piperazin-1-yl)piperidin-1-yl)-2-methoxyphenyl)amino)pyrimidin-4-yl)amino)phenyl)-N-methylmethansulfonamid |
| 61 | N-(2-((5-Chloro-2-((4-(4-((1-((1-(2-(2,6-dioxopiperidin-3-yl)-1,3-dioxoisoindolin-5-yl)azetidin-3-yl)methyl)piperidin-4-yl)methyl)piperazin-1-yl)-2-methoxyphenyl)amino)pyrimidin-4-yl)amino)phenyl)-N-methylmethansulfonamid |
| 62 | N-(2-((5-Chloro-2-((4-(4-((4-(((2-(2,6-dioxopiperidin-3-yl)-1,3-dioxoisoindolin-4-yl)amino)methyl)piperidin-1-yl)methyl)piperidin-1-yl)-2-methoxyphenyl)amino)pyrimidin-4-yl)amino)phenyl)-N-methylmethansulfonamid |
| 63 | N-(2-((5-Chloro-2-((4-(4-((4-(((2-(2,6-dioxopiperidin-3-yl)-1,3-dioxoisoindolin-5-yl)amino)methyl)piperidin-1-yl)methyl)piperidin-1-yl)-2-methoxyphenyl)amino)pyrimidin-4-yl)amino)phenyl)-N-methylmethansulfonamid |
| 64 | N-(2-((5-Chloro-2-((4-(4-((1-(3-(2-(2,6-dioxopiperidin-3-yl)-1,3-dioxoisoindolin-5-yl)propanoyl)piperidin-4-yl)methyl)piperazin-1-yl)-2-methoxyphenyl)amino)pyrimidin-4-yl)amino)phenyl)-N-methylmethansulfonamid |
| 65 | N-(2-((5-Chloro-2-((4-(4-((1-((2-(2,6-dioxopiperidin-3-yl)-1,3-dioxoisoindolin-4-yl)glycyl)piperidin-4-yl)methyl)piperazin-1-yl)-2-methoxyphenyl)amino)pyrimidin-4-yl)amino)phenyl)-N-methylmethansulfonamid |
| 66 | N-(2-((5-Chloro-2-((4-(4-((1-((2-(2,6-dioxopiperidin-3-yl)-1,3-dioxoisoindolin-5-yl)glycyl)piperidin-4-yl)methyl)piperazin-1-yl)-2-methoxyphenyl)amino)pyrimidin-4-yl)amino)phenyl)-N-methylmethansulfonamid |
| 67 | N-(2-((5-Chloro-2-((4-(4-((4-(2-(2,6-dioxopiperidin-3-yl)-1,3-dioxoisoindolin-5-yl)piperazin-1-yl)methyl)piperidin-1-yl)-2-methoxyphenyl)amino)pyrimidin-4-yl)amino)phenyl)-N-methylmethansulfonamid |
| 68 | N-(2-((5-Chloro-2-((4-(4-((4-(2-(2,6-dioxopiperidin-3-yl)-1,3-dioxoisoindolin-5-yl)piperidin-4-yl)methyl)piperazin-1-yl)-2-methoxyphenyl)amino)pyrimidin-4-yl)amino)phenyl)-N-methylmethansulfonamid |
| 69 | N-(2-((5-Chloro-2-((4-(4-((1-((2-(2,6-dioxopiperidin-3-yl)-1,3-dioxoisoindolin-4-yl)oxy)propanoyl)piperidin-4-yl)methyl)piperazin-1-yl)-2-methoxyphenyl)amino)pyrimidin-4-yl)amino)phenyl)-N-methylmethansulfonamid |
| 70 | N-(2-((5-Chloro-2-((4-(4-((1-((2-(2,6-dioxopiperidin-3-yl)-1,3-dioxoisoindolin-4-yl)-L-alanyl)piperidin-4-yl)methyl)piperazin-1-yl)-2-methoxyphenyl)amino)pyrimidin-4-yl)amino)phenyl)-N-methylmethansulfonamid |
| 71 | N-(2-((5-Chloro-2-((4-(4-(4-((1-(2-(2,6-dioxopiperidin-3-yl)-1,3-dioxoisoindolin-5-yl)piperidin-4-yl)methyl)piperazin-1-yl)piperidin-1-yl)-2-methoxyphenyl)amino)pyrimidin-4-yl)amino)phenyl)-N-methylmethansulfonamid |
| 74 | N-(2-((5-Chloro-2-((4-(4-(4-(2-((2-(2,6-dioxopiperidin-3-yl)-1,3-dioxoisoindolin-4-yl)amino)ethyl)piperazin-1-yl)piperidin-1-yl)-2-methoxy-5-methylphenyl)amino)pyrimidin-4-yl)amino)phenyl)-N-methylmethansulfonamid |
| 75 | N-(2-((5-Chloro-2-((4-(4-((1-((2-(2,6-dioxopiperidin-3-yl)-1,3-dioxoisoindolin-4-yl)-L-alanyl)piperidin-4-yl)methyl)piperazin-1-yl)-2-methoxy-5-methylphenyl)amino)pyrimidin-4-yl)amino)phenyl)-N-methylmethansulfonamid |
| 76 | N-(2-((5-Chloro-2-((4-(4-(4-((1-(2-(2,6-dioxopiperidin-3-yl)-1,3-dioxoisoindolin-5-yl)piperidin-4-yl)methyl)piperazin-1-yl)piperidin-1-yl)-2-methoxy-5-methylphenyl)amino)pyrimidin-4-yl)amino)phenyl)-N-methylmethansulfonamid |
| 77 | N-(2-((5-Chloro-2-((4-(4-(4-(2-(1-(2-(2,6-dioxopiperidin-3-yl)-1,3-dioxoisoindolin-5-yl)piperidin-4-yl)ethyl)piperazin-1-yl)piperidin-1-yl)-2-methoxy5-methylphenyl)amino)pyrimidin-4-yl)amino)phenyl)-N-methylmethansulfonamid |
| 78 | N-(2-((5-Chloro-2-((4-(4-((4-((2-(2,6-dioxopiperidin-3-yl)-1,3-dioxoisoindolin-4-yl)-L-alanyl)piperazin-1-yl)methyl)piperidin-1-yl)-2-methoxy-5-methylphenyl)amino)pyrimidin-4-yl)amino)phenyl)-N-methylmethansulfonamid |
| 79 | N-(2-((5-Chloro-2-((4-(4-((4-(2-(2,6-dioxopiperidin-3-yl)-1,3-dioxoisoindolin-5-yl)piperazin-1-yl)methyl)piperidin-1-yl)-2-methoxy-5-methylphenyl)amino)pyrimidin-4-yl)amino)phenyl)-N-methylmethansulfonamid |
| 80 | N-(2-((5-Chloro-2-((4-(4-((1-((2-(2,6-dioxopiperidin-3-yl)-1,3-dioxoisoindolin-4-yl)glycyl)piperidin-4-yl)methyl)piperazin-1-yl)-2-methoxy-5-methylphenyl)amino)pyrimidin-4-yl)amino)phenyl)-N-methylmethansulfonamid |
| 81 | N-(2-((5-Chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-2-methoxy-5-methylphenyl)amino)pyrimidin-4-yl)amino)phenyl)-N-methylmethansulfonamid |
| 82 | N-(2-((5-Chloro-2-((4-(4-((1-((2S)-2-((2-(2,6-dioxopiperidin-3-yl)-1,3-dioxoisoindolin-4-yl)oxy)propanoyl)piperidin-4-yl)methyl)piperazin-1-yl)-2-methoxy-5-methylphenyl)amino)pyrimidin-4-yl)amino)phenyl)-N-methylmethansulfonamid |

2. Verbindung gemäß Anspruch 1 oder ein pharmazeutisch akzeptables Salz davon,
wobei es sich bei der Verbindung um eine der folgenden Verbindungen handelt:
| Verbindung Nr. | IUPAC-Benennung |
|---|---|
| 11 | (2R,4R)-1-((S)-2-(2-(4-(1-(4-((5-Chloro-4-((2-(N-methylmethylsulfonamido)phenyl)amino)pyrimidin-2-yl)amino)-3-methoxyphenyl)piperidin-4-yl)piperazin-1-yl)acetamido)-3,3-dimethylbutanoyl)-4-hydroxy-N-(4-(4-methylthiazol-5-yl)benzyl)pyrrolidin-2-carboxamid |
| 19 | (2R,4R)-1-((S)-2-(2-(4-(1-(4-((5-Chloro-4-((2-(N-methylmethylsulfonamido)phenyl)amino)pyrimidin-2-yl)amino)-2-fluorophenyl)piperidin-4-yl)piperazin-1-yl)acetamido)-3,3-dimethylbutanoyl)-4-hydroxy-N-(4-(4-methylthiazol-5-yl)benzyl)pyrrolidin-2-carboxamid |
| 20 | (2R,4R)-1-((S)-2-(3-(4-(1-(4-((5-Chloro-4-((2-(N-methylmethylsulfonamido)phenyl)amino)pyrimidin-2-yl)amino)-3-methoxyphenyl)piperidin-4-yl)piperazin-1-yl)propanamido)-3,3-dimethylbutanoyl)-4-hydroxy-N-(4-(4-methylthiazol-5-yl)benzyl)pyrrolidin-2-carboxamid |
| 22 | 2-(4-(1-(4-((5-Chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1-oxoisoindolin-4-yl)acetamid |
| 24 | 2-(4-((1-(4-((5-Chloro-4-((2-(N-methylmethylsulfonamido)phenyl)amino)pyrimidin-2-yl)amino)-3-methoxyphenyl)piperidin-4-yl)methyl)piperazin-1-yl)-N-(2-(2,6-dioxopiperidin-3-yl)-1-oxoisoindolin-4-yl)acetamid |
| 26 | 2-(4-((4-(4-((5-Chloro-4-((2-(N-methylmethylsulfonamido)phenyl)amino)pyrimidin-2-yl)amino)-3-methoxyphenyl)piperazin-1-yl)methyl)piperidin-1-yl)-N-(2-(2,6-dioxopiperidin-3-yl)-1-oxoisoindolin-4-yl)acetamid |
| 27 | (2R,4R)-1-((S)-2-(5-(4-((1-(4-((5-Chloro-4-((2-(N-methylmethylsulfonamido)phenyl)amino)pyrimidin-2-yl)amino)-3-methoxyphenyl)piperidin-4-yl)methyl)piperazin-1-yl)acetamido)-3,3-dimethylbutanoyl)-4-hydroxy-N-(4-(4-methylthiazol-5-yl)benzyl)pyrrolidin-2-carboxamid |
| 28 | (2R,4R)-1-((S)-2-(2-(4-((4-(4-((5-Chloro-4-((2-(N-methylmethylsulfonamido)phenyl)amino)pyrimidin-2-yl)amino)-3-methoxyphenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamid |
| 31 | (2R,4R)-1-((S)-2-(2-(9-(4-((5-Chloro-4-((2-(N-methylmethylsulfonamido)phenyl)amino)pyrimidin-2-yl)amino)-3-methoxyphenyl)-3,9-diazaspiro[5,5]undecan-3-yl)acetamido)-3,3-dimethylbutanoyl)-4-hydroxy-N-(4-(4-methylthiazol-5-yl)benzyl)pyrrolidin-2-carboxamid |
| 35 | (2S,4R)-1-((S)-2-(2-(4-((4-(3-Chloro-4-((5-chloro-4-((2-(N-methylmethylsulfonamido)phenyl)amino)pyrimidin-2-yl)amino)phenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamid |
| 36 | (2S,4R)-1-((S)-2-(2-(4-((4-(4-((5-Chloro-4-((2-(N-methylmethylsulfonamido)phenyl)amino)pyrimidin-2-yl)amino)2-fluorophenyl)piperazin-1-yl)methyl)piperidin-1-yl)acetamido)-3,3-dimethylbutanoyl)-4-hydroxy-N-(4-(4-methylthiazol-5-yl)benzyl)pyrrolidin-2-carboxamid |
| 39 | N-(2-((5-Chloro-2-((4-(4-(2-(3-((2-(2,6-dioxopiperidin-3-yl)-1-oxoisoindolin-4-yl)oxy)azetidin-1-yl)ethyl)piperazin-1-yl)-3-fluorophenyl)amino)pyrimidin-4-yl)amino)phenyl)-N-methylmethansulfonamid |
| 40 | N-(2-((5-Chloro-2-((4-(4-(2-((1-(2-(2,6-dioxopiperidin-3-yl)-1,3-dioxoisoindolin-5-yl)azetidin-3-yl)oxy)ethyl)piperazin-1-yl)-3-fluorophenyl)amino)pyrimidin-4-yl)amino)phenyl)-N-methylmethansulfonamid |
| 43 | N-(2-((5-Chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-3-fluorophenyl)amino)pyrimidin-4-yl)amino)phenyl)-N-methylmethansulfonamid |
| 45 | N-(2-((5-Chloro-2-((4-(4-(4-(2-((2-(2,6-dioxopiperidin-3-yl)-1,3-dioxoisoindolin-4-yl)amino)ethyl)piperazin-1-yl)piperidin-1-yl)-2-methoxyphenyl)amino)pyrimidin-4-yl)amino)phenyl)-N-methylmethansulfonamid |
| 46 | N-(2-((5-Chloro-2-((4-(4-((1-(2-((2-(2,6-dioxopiperidin-3-yl)-1,3-dioxoisoindolin-4-yl)oxy)ethyl)piperidin-4-yl)methyl)piperazin-1-yl)-3-fluorophenyl)amino)pyrimidin-4-yl)amino)phenyl)-N-methylmethansulfonamid |
| 47 | 2-(4-(1-(4-((5-Chloro-4-((2-(N-methylmethylsulfonamido)phenyl)amino)pyrimidin-2-yl)amino)-3-methoxyphenyl)piperidin-4-yl)piperazin-1-yl)-N-(2-(2,6-dioxopiperidin-3-yl)-1-oxoisoindolin-5-yl)acetamid |
| 51 | N-(2-((5-Chloro-2-((4-(4-(4-(2-(1-(2-(2,6-dioxopiperidin-3-yl)-1,3-dioxoisoindolin-5-yl)piperidin-4-yl)ethyl)piperazin-1-yl)piperidin-1-yl)-2-methoxyphenyl)amino)pyrimidin-4-yl)amino)phenyl)-N-methylmethansulfonamid |
| 53 | N-(2-((5-Chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-2-methoxyphenyl)amino)pyrimidin-4-yl)amino)phenyl)-N-methylmethansulfonamid |
| 54 | N-(2-((5-Chloro-2-((4-(4-((1-(3-(2-(2,6-dioxopiperidin-3-yl)-1,3-dioxoisoindolin-4-yl)propanoyl)piperidin-4-yl)methyl)piperazin-1-yl)-2-methoxyphenyl)amino)pyrimidin-4-yl)amino)phenyl)-N-methylmethansulfonamid |
| 59 | N-(2-((5-Chloro-2-((4-(4-((1-((1-(2-(2,6-dioxopiperidin-3-yl)-1,3-dioxoisoindolin-5-yl)piperidin-4-yl)methyl)piperidin-4-yl)methyl)piperazin-1-yl)-2-methoxyphenyl)amino)pyrimidin-4-yl)amino)phenyl)-N-methylmethansulfonamid |
| 62 | N-(2-((5-Chloro-2-((4-(4-((4-(((2-(2,6-dioxopiperidin-3-yl)-1,3-dioxoisoindolin-4-yl)amino)methyl)piperidin-1-yl)methyl)piperidin-1-yl)-2-methoxyphenyl)amino)pyrimidin-4-yl)amino)phenyl)-N-methylmethansulfonamid |
| 63 | N-(2-((5-Chloro-2-((4-(4-((4-(((2-(2,6-dioxopiperidin-3-yl)-1,3-dioxoisoindolin-5-yl)amino)methyl)piperidin-1-yl)methyl)piperidin-1-yl)-2-methoxyphenyl)amino)pyrimidin-4-yl)amino)phenyl)-N-methylmethansulfonamid |
| 65 | N-(2-((5-Chloro-2-((4-(4-((1-((2-(2,6-dioxopiperidin-3-yl)-1,3-dioxoisoindolin-4-yl)glycyl)piperidin-4-yl)methyl)piperazin-1-yl)-2-methoxyphenyl)amino)pyrimidin-4-yl)amino)phenyl)-N-methylmethansulfonamid |
| 66 | N-(2-((5-Chloro-2-((4-(4-((1-((2-(2,6-dioxopiperidin-3-yl)-1,3-dioxoisoindolin-5-yl)glycyl)piperidin-4-yl)methyl)piperazin-1-yl)-2-methoxyphenyl)amino)pyrimidin-4-yl)amino)phenyl)-N-methylmethansulfonamid |
| 68 | N-(2-((5-Chloro-2-((4-(4-((4-(2-(2,6-dioxopiperidin-3-yl)-1,3-dioxoisoindolin-5-yl)piperidin-4-yl)methyl)piperazin-1-yl)-2-methoxyphenyl)amino)pyrimidin-4-yl)amino)phenyl)-N-methylmethansulfonamid |
| 69 | N-(2-((5-Chloro-2-((4-(4-((1-((2-(2,6-dioxopiperidin-3-yl)-1,3-dioxoisoindolin-4-yl)oxy)propanoyl)piperidin-4-yl)methyl)piperazin-1-yl)-2-methoxyphenyl)amino)pyrimidin-4-yl)amino)phenyl)-N-methylmethansulfonamid |
| 70 | N-(2-((5-Chloro-2-((4-(4-((1-((2-(2,6-dioxopiperidin-3-yl)-1,3-dioxoisoindolin-4-yl)-L-alanyl)piperidin-4-yl)methyl)piperazin-1-yl)-2-methoxyphenyl)amino)pyrimidin-4-yl)amino)phenyl)-N-methylmethansulfonamid |
| 71 | N-(2-((5-Chloro-2-((4-(4-(4-((1-(2-(2,6-dioxopiperidin-3-yl)-1,3-dioxoisoindolin-5-yl)piperidin-4-yl)methyl)piperazin-1-yl)piperidin-1-yl)-2-methoxyphenyl)amino)pyrimidin-4-yl)amino)phenyl)-N-methylmethansulfonamid |
| 74 | N-(2-((5-Chloro-2-((4-(4-(4-(2-((2-(2,6-dioxopiperidin-3-yl)-1,3-dioxoisoindolin-4-yl)amino)ethyl)piperazin-1-yl)piperidin-1-yl)-2-methoxy-5-methylphenyl)amino)pyrimidin-4-yl)amino)phenyl)-N-methylmethansulfonamid |
| 75 | N-(2-((5-Chloro-2-((4-(4-((1-((2-(2,6-dioxopiperidin-3-yl)-1,3-dioxoisoindolin-4-yl)-L-alanyl)piperidin-4-yl)methyl)piperazin-1-yl)-2-methoxy-5-methylphenyl)amino)pyrimidin-4-yl)amino)phenyl)-N-methylmethansulfonamid |
| 76 | N-(2-((5-Chloro-2-((4-(4-(4-((1-(2-(2,6-dioxopiperidin-3-yl)-1,3-dioxoisoindolin-5-yl)piperidin-4-yl)methyl)piperazin-1-yl)piperidin-1-yl)-2-methoxy-5-methylphenyl)amino)pyrimidin-4-yl)amino)phenyl)-N-methylmethansulfonamid |
| 77 | N-(2-((5-Chloro-2-((4-(4-(4-(2-(1-(2-(2,6-dioxopiperidin-3-yl)-1,3-dioxoisoindolin-5-yl)piperidin-4-yl)ethyl)piperazin-1-yl)piperidin-1-yl)-2-methoxy5-methylphenyl)amino)pyrimidin-4-yl)amino)phenyl)-N-methylmethansulfonamid |
| 78 | N-(2-((5-Chloro-2-((4-(4-((4-((2-(2,6-dioxopiperidin-3-yl)-1,3-dioxoisoindolin-4-yl)-L-alanyl)piperazin-1-yl)methyl)piperidin-1-yl)-2-methoxy-5-methylphenyl)amino)pyrimidin-4-yl)amino)phenyl)-N-methylmethansulfonamid |
| 80 | N-(2-((5-Chloro-2-((4-(4-((1-((2-(2,6-dioxopiperidin-3-yl)-1,3-dioxoisoindolin-4-yl)glycyl)piperidin-4-yl)methyl)piperazin-1-yl)-2-methoxy-5-methylphenyl)amino)pyrimidin-4-yl)amino)phenyl)-N-methylmethansulfonamid |
| 81 | N-(2-((5-Chloro-2-((4-(4-((1-(2-((2-(2,6-dioxopiperidin-3-yl)-1,3-dioxoisoindolin-4-yl)oxy)acetyl)piperidin-4-yl)methyl)piperazin-1-yl)-2-methoxy-5-methylphenyl)amino)pyrimidin-4-yl)amino)phenyl)-N-methylmethansulfonamid |
| 82 | N-(2-((5-Chloro-2-((4-(4-((1-((2S)-2-((2-(2,6-dioxopiperidin-3-yl)-1,3-dioxoisoindolin-4-yl)oxy)propanoyl)piperidin-4-yl)methyl)piperazin-1-yl)-2-methoxy-5-methylphenyl)amino)pyrimidin-4-yl)amino)phenyl)-N-methylmethansulfonamid |

3. Zusammensetzung, umfassend eine Verbindung gemäß Anspruch 1 oder Anspruch 2 oder ein pharmazeutisch akzeptables Salz davon und einen pharmazeutisch akzeptablen Träger.

4. Pharmazeutische Zusammensetzung zur Verwendung bei der Behandlung oder Vorbeugung einer Krebserkrankung mit einer EGFR-Mutation, umfassend die Verbindung gemäß Anspruch 1 oder Anspruch 2 oder ein pharmazeutisch akzeptables Salz davon als Wirkstoff.

5. Pharmazeutische Zusammensetzung zur Verwendung nach Anspruch 4, wobei es sich bei der Krebserkrankung um Lungenkrebs, Leberkrebs, Speiseröhrenkrebs, Magenkrebs, Dickdarmkrebs, Dünndarmkrebs, Bauchspeicheldrüsenkrebs, Melanom, Brustkrebs, Mundhöhlenkrebs, Hirntumor, Schilddrüsenkrebs, Nebenschilddrüsenkrebs, Nierenkrebs, Gebärmutterhalskrebs, Sarkom, Prostatakrebs, Harnröhrenkrebs, Blasenkrebs, Hodenkrebs, Blutkrebs, Lymphom, Hautkrebs oder Fibroadenom handelt.

6. Pharmazeutische Zusammensetzung zur Verwendung nach Anspruch 5, wobei es sich bei der Krebserkrankung um Lungenkrebs handelt.

7. Pharmazeutische Zusammensetzung zur Verwendung nach Anspruch 6, wobei es sich bei dem Lungenkrebs um nicht-kleinzelligen Lungenkrebs handelt.

## Revendications

1. Composé ou sel pharmaceutiquement acceptable de celui-ci, lequel composé est l'un quelconque des composés suivants :
| Composé N° | Nom UICPA |
|---|---|
| | |
| 2 | 5-(4-(1-(4-((5-chloro-4-((2-(N-méthylméthylsulfonamido)phényl)amino)pyrimidin-2-yl)amino)-3-méthoxyphényl)pipéridin-4-yl)pipérazin-1-yl)-N-(2-(2,6-dioxopipéridin-3-yl)-1,3-dioxoisoindolin-4-yl)pentanamide |
| 6 | (2R,4R)-1-((S)-2-(5-(4-(4-((5-chloro-4-((2-(N-méthylméthylsulfonamido)phényl)amino)pyrimidin-2-yl)amino)-3-méthoxyphényl)pipérazin-1-yl)pentanamido)-3,3-diméthylbutanoyl)-4-hydroxy-N-(4-(4-méthylthiazol-5-yl)benzyl)pyrrolidin-2-carboxamide |
| 7 | (2R,4R)-1-((S)-2-(5-(4-(1-(4-((5-chloro-4-((2-(N-méthylméthylsulfonamido)phényl)amino)pyrimidin-2-yl)amino)-3-méthoxyphényl)pipéridin-4-yl)pipérazin-1-yl)pentanamido)-3,3-diméthylbutanoyl)-4-hydroxy-N-(4-(4-méthylthiazol-5-yl)benzyl)pyrrolidin-2-carboxamide |
| 11 | (2R,4R)-1-((S)-2-(2-(4-(1-(4-((5-chloro-4-((2-(N-méthylméthylsulfonamido)phényl)amino)pyrimidin-2-yl)amino)-3-méthoxyphényl)pipéridin-4-yl)pipérazin-1-yl)acétamido)-3,3-diméthylbutanoyl)-4-hydroxy-N-(4-(4-méthylthiazol-5-yl)benzyl)pyrrolidin-2-carboxamide |
| 13 | N-(2-((5-chloro-2-((4-(4-(4-(2-(2-((2-(2,6-dioxopipéridin-3-yl)-1,3-dioxoisoindolin-4-yl)oxy)éthoxy)éthyl)pipérazin-1-yl)pipéridin-1-yl)-2-méthoxyphényl)amino)pyrimidin-4-yl)amino)phényl)-N-méthylméthanesulfonamide |
| 14 | N-(2-((5-chloro-2-((4-(4-(4-(2-(2-((2-(2,6-dioxopiperidin-3-yl)-1,3-dioxoisoindolin-4-yl)oxy)éthoxy)éthyl)pipérazin-1-yl)pipéridin-1-yl)-2-méthoxyphényl)amino)pyrimidin-4-yl)amino)phényl)-N-méthylméthanesulfonamide |
| 15 | N-(2-((5-chloro-2-((4-(4-(4-(4-((2-(2,6-dioxopipéridin-3-yl)-1,3-dioxoisoindolin-4-yl)oxy)butyl)pipérazin-1-yl)pipéridin-1-yl)-2-méthoxyphényl)amino)pyrimidin-4-yl)amino)phényl)-N-méthylméthanesulfonamide |
| 16 | N-(2-((5-chloro-2-((4-(4-(2-(2-((2-(2,6-dioxopipéridin-3-yl)-1,3-dioxoisoindolin-4-yl)amino)éthoxy)éthyl)pipérazin-1-yl)-2-méthoxyphényl)amino)pipéridin-4-yl)amino)phényl)-N-méthylméthanesulfonamide |
| 17 | (2R,4R)-1-((S)-2-(5-(4-(4-((5-chloro-4-((2-(N-méthylméthylsulfonamido)phényl)amino)pyrimidin-2-yl)amino)-2-fluorophényl)pipérazin-1-yl)pentanamido)-3,3-diméthylbutanoyl)-4-hydroxy-N-(4-(4-méthylthiazol-5-yl)benzyl)pyrrolidin-2-carboxamide |
| 19 | (2R,4R)-1-((S)-2-(2-(4-(1-(4-((5-chloro-4-((2-(N-méthylméthylsulfonamido)phényl)amino)pyrimidin-2-yl)amino)-2-fluorophényl)pipéridin-4-yl)pipérazin-1-yl)acétamido)-3,3-diméthylbutanoyl)-4-hydroxy-N-(4-(4-méthylthiazol-5-yl)benzyl)pyrrolidin-2-carboxamide |
| 20 | (2R,4R)-1-((S)-2-(3-(4-(1-(4-((5-chloro-4-((2-(N-méthylméthylsulfonamido)phényl)amino)pyrimidin-2-yl)amino)-3-méthoxyphényl)pipéridin-4-yl)pipérazin-1-yl)propanamido)-3,3-diméthylbutanoyl)-4-hydroxy-N-(4-(4-méthylthiazol-5-yl)benzyl)pyrrolidin-2-carboxamide |
| 22 | 2-(4-(1-(4-((5-chloro-4-((2-(N-méthylméthylsulfonamido)phényl)amino)pyrimidin-2-yl)amino)-3-méthoxyphényl)pipéridin-4-yl)pipérazin-1-yl)-N-(2-(2,6-dioxopipéridin-3-yl)-1-oxoisoindolin-4-yl)acétamide |
| 24 | 2-(4-((1-(4-((5-chloro-4-((2-(N-méthylméthylsulfonamido)phényl)amino)pyrimidin-2-yl)amino)-3-méthoxyphényl)pipéridin-4-yl)méthyl)pipérazin-1-yl)-N-(2-(2,6-dioxopiperidin-3-yl)-1-oxoisoindolin-4-yl)acetamide |
| 26 | 2-(4-((4-(4-((5-chloro-4-((2-(N-méthylméthylsulfonamido)phényl)amino)pyrimidin-2-yl)amino)-3-méthoxyphényl)pipérazin-1-yl)méthyl)pipéridin-1-yl)-N-(2-(2,6-dioxopipéridin-3-yl)-1-oxoisoindolin-4-yl)acétamide |
| 27 | (2R,4R)-1-((S)-2-(5-(4-((1-(4-((5-chloro-4-((2-(N-méthylméthylsulfonamido)phényl)amino)pyrimidin-2-yl)amino)-3-méthoxyphényl)pipéridin-4-yl)méthyl)pipérazin-1-yl)acétamido)-3,3-diméthylbutanoyl)-4-hydroxy-N-(4-(4-méthylthiazol-5-yl)benzyl)pyrrolidin-2-carboxamide |
| 28 | (2R,4R)-1-((S)-2-(2-(4-((4-(4-((5-chloro-4-((2-(N-méthylméthylsulfonamido)phényl)amino)pyrimidin-2-yl)amino)-3-méthoxyphényl)pipérazin-1-yl)méthyl)pipéridin-1-yl)acétamido)-3,3-dimethylbutanoyl)-4-hydroxy-N-(4-(4-methylthiazol-5-yl)benzyl)pyrrolidin-2-carboxamide |
| 31 | (2R,4R)-1-((S)-2-(2-(9-(4-((5-chloro-4-((2-(N-méthylméthylsulfonamido)phényl)amino)pyrimidin-2-yl)amino)-3-méthoxyphényl)-3,9-diazaspiro[5,5]undécan-3-yl)acétamido)-3,3-diméthylbutanoyl)-4-hydroxy-N-(4-(4-méthylthiazol-5-yl)benzyl)pyrrolidin-2-carboxamide |
| 33 | N-(2-((5-chloro-2-((4-(4-(2-((1-(2-(2,6-dioxopipéridin-3-yl)-1,3-dioxoisoindolin-5-yl)azétidin-3-yl)oxy)éthyl)pipérazin-1-yl)-2-méthoxyphényl)amino)pyrimidin-4-yl)amino)phényl)-N-méthylméthanesulfonamide |
| 35 | (2S,4R)-1-((S)-2-(2-(4-((4-(3-chloro-4-((5-chloro-4-((2-(N-méthylméthylsulfonamido)phényl)amino)pyrimidin-2-yl)amino)phényl)pipérazin-1-yl)méthyl)pipéridin-1-yl)acétamido)-3,3-diméthylbutanoyl)-4-hydroxy-N-(4-(4-méthylthiazol-5-yl)benzyl)pyrrolidin-2-carboxamide |
| 36 | (2S,4R)-1-((S)-2-(2-(4-((4-(4-((5-chloro-4-((2-(N-méthylméthylsulfonamido)phényl)amino)pyrimidin-2-yl)amino)-2-fluorophényl)pipérazin-1-yl)méthyl)pipéridin-1-yl)acétamido)-3,3-diméthylbutanoyl)-4-hydroxy-N-(4-(4-méthylthiazol-5-yl)benzyl)pyrrolidin-2-carboxamide |
| 37 | (2S,4R)-1-((S)-2-(2-(3-((4-(4-((5-chloro-4-((2-(N-méthylméthylsulfonamido)phényl)amino)pyrimidin-2-yl)amino)-3-méthoxyphényl)pipérazin-1-yl)méthyl)azétidin-1-yl)acétamido)-3,3-diméthylbutanoyl)-4-hydroxy-N-(4-(4-méthylthiazol-5-yl)benzyl)pyrrolidin-2-carboxamide |
| 39 | N-(2-((5-chloro-2-((4-(4-(2-(3-((2-(2,6-dioxopipéridin-3-yl)-1-oxoisoindolin-4-yl)oxy)azétidin-1-yl)éthyl)pipérazin-1-yl)-3-fluorophényl)amino)pyrimidin-4-yl)amino)phényl)-N-méthylméthanesulfonamide |
| 40 | N-(2-((5-chloro-2-((4-(4-(2-((1-(2-(2,6-dioxopiperidin-3-yl)-1,3-dioxoisoindolin-5-yl)azétidin-3-yl)oxy)éthyl)pipérazin-1-yl)-3-fluorophényl)amino)pyrimidin-4-yl)amino)phényl)-N-méthylméthanesulfonamide |
| 42 | N-(2-((5-chloro-2-((4-(4-(4-((2-(2,6-dioxopipéridin-3-yl)-1,3-dioxoisoindolin-5-yl)méthyl)pipérazin-1-yl)pipéridin-1-yl)-3-fluorophényl)amino)pyrimidin-4-yl)amino)phényl)-N-méthylméthanesulfonamide |
| 43 | N-(2-((5-chloro-2-((4-(4-((1-(2-((2-(2,6-dioxopipéridin-3-yl)-1,3-dioxoisoindolin-4-yl)oxy)acétyl)pipéridin-4-yl)méthyl)pipérazin-1-yl)-3-fluorophényl)amino)pyrimidin-4-yl)amino)phényl)-N-méthylméthanesulfonamide |
| 44 | N-(2-((5-chloro-2-((4-(4-(4-(((2S)-1-(2-(2,6-dioxopiperidin-3-yl)-1,3-dioxoisoindolin-5-yl)pyrrolidin-2-yl)méthyl)pipérazin-1-yl)pipéridin-1-yl)-2-méthoxyphényl)amino)pyrimidin-4-yl)amino)phényl)-N-méthylméthanesulfonamide |
| 45 | N-(2-((5-chloro-2-((4-(4-(4-(2-((2-(2,6-dioxopipéridin-3-yl)-1,3-dioxoisoindolin-4-yl)amino)éthyl)pipérazin-1-yl)pipéridin-1-yl)-2-méthoxyphényl)amino)pyrimidin-4-yl)amino)phényl)-N-méthylméthanesulfonamide |
| 46 | N-(2-((5-chloro-2-((4-(4-((1-(2-((2-(2,6-dioxopipéridin-3-yl)-1,3-dioxoisoindolin-4-yl)oxy)ethyi)piperidin-4-yl)methyl)piperazin-1-yl)-3-fluorophényl)amino)pyrimidin-4-yl)amino)phényl)-N-méthylméthanesulfonamide |
| 47 | 2-(4-(1-(4-((5-chloro-4-((2-(N-méthylméthylsulfonamido)phényl)amino)pyrimidin-2-yl)amino)-3-méthoxyphényl)pipéridin-4-yl)pipérazin-1-yl)-N-(2-(2,6-dioxopipéridin-3-yl)-1-oxoisoindolin-5-yl)acétamide |
| 48 | 2-(4-((4-(4-((5-chloro-4-((2-(N-methylmethylsulfonamido)phenyl)amino)pyrimidin-2-yl)amino)-2-fluorophényl)pipérazin-1-yl)méthyl)pipéridin-1-yl)-N-(2-(2,6-dioxopipéridin-3-yl)-1-oxoisoindolin-4-yl)acétamide |
| 50 | 2-(9-(4-((5-chloro-4-((2-(N-méthylméthylsulfonamido)phényl)amino)pyrimidin-2-yl)amino)-3-méthoxyphényl)-3,9-diazaspiro[5.5]undécan-3-yl)-N-(2-(2,6-dioxopipéridin-3-yl)-1-oxoisoindolin-4-yl)acétamide |
| 51 | N-(2-((5-chloro-2-((4-(4-(4-(2-(1-(2-(2,6-dioxopipéridin-3-yl)-1,3-dioxoisoindolin-5-yl)pipéridin-4-yl)éthyl)pipérazin-1-yl)pipéridin-1-yl)-2-méthoxyphényl)amino)pyrimidin-4-yl)amino)phényl)-N-méthylméthanesulfonamide |
| 52 | N-(2-((5-chloro-2-((4-(4-(4-(2-((2-(2,6-dioxopipéridin-3-yl)-1,3-dioxoisoindolin-4-yl)oxy)éthyl)pipérazin-1-yl)pipéridin-1-yl)-2-méthoxyphényl)amino)pyrimidin-4-yl)amino)phényl)-N-méthylméthanesulfonamide |
| 53 | N-(2-((5-chloro-2-((4-(4-((1-(2-((2-(2,6-dioxopiperidin-3-yl)-1,3-dioxoisoindolin-4-yl)oxy)acétyl)pipéridin-4-yl)méthyl)pipérazin-1-yl)-2-méthoxyphényl)amino)pyrimidin-4-yl)amino)phényl)-N-méthylméthanesulfonamide |
| 54 | N-(2-((5-chloro-2-((4-(4-((1-(3-(2-(2,6-dioxopipéridin-3-yl)-1,3-dioxoisoindolin-4-yl)propanoyl)pipéridin-4-yl)méthyl)pipérazin-1-yl)-2-méthoxyphényl)amino)pyrimidin-4-yl)amino)phényl)-N-méthylméthanesulfonamide |
| 56 | (2R,4R)-1-((2-(4-((4-(4-((5-chloro-4-((2-(N-méthylméthylsulfonamido)phényl)amino)pyrimidin-2-yl)amino)-3-méthoxyphényl)pipérazin-1-yl)méthyl)pipéridin-1-yl)acétyl)-L-valyl)-4-hydroxy-N-(4-(4-méthylthiazol-5-yl)benzyl)pyrrolidin-2-carboxamide |
| 58 | N-(2-((5-chloro-2-((4-(4-(4-(2-((2-(2,6-dioxopipéridin-3-yl)-1,3-dioxoisoindolin-5-yl)amino)éthyl)pipérazin-1-yl)pipéridin-1-yl)-2-méthoxyphényl)amino)pyrimidin-4-yl)amino)phényl)-N-méthylméthanesulfonamide |
| 59 | N-(2-((5-chloro-2-((4-(4-((1-((1-(2-(2,6-dioxopipéridin-3-yl)-1,3-dioxoisoindolin-5-yl)pipéridin-4-yl)méthyl)pipéridin-4-yl)méthyl)pipérazin-1-yl)-2-méthoxyphényl)amino)pyrimidin-4-yl)amino)phényl)-N-méthylméthanesulfonamide |
| 60 | N-(2-((5-chloro-2-((4-(4-(4-((1-(2-(2,6-dioxopipéridin-3-yl)-1,3-dioxoisoindolin-5-yl)azétidin-3-yl)méthyl)pipérazin-1-yl)pipéridin-1-yl)-2-méthoxyphényl)amino)pyrimidin-4-yl)amino)phényl)-N-méthylméthanesulfonamide |
| 61 | N-(2-((5-chloro-2-((4-(4-((1-((1-(2-(2,6-dioxopipéridin-3-yl)-1,3-dioxoisoindolin-5-yl)azétidin-3-yl)méthyl)pipéridin-4-yl)méthyl)pipérazin-1-yl)-2-méthoxyphényl)amino)pyrimidin-4-yl)amino)phényl)-N-méthylméthanesulfonamide |
| 62 | N-(2-((5-chloro-2-((4-(4-((4-(((2-(2,6-dioxopipéridin-3-yl)-1,3-dioxoisoindolin-4-yl)amino)méthyl)pipéridin-1-yl)méthyl)pipéridin-1-yl)-2-méthoxyphényl)amino)pyrimidin-4-yl)amino)phényl)-N-méthylméthanesulfonamide |
| 63 | N-(2-((5-chloro-2-((4-(4-((4-(((2-(2,6-dioxopipéridin-3-yl)-1,3-dioxoisoindolin-5-yl)amino)methyl)piperidin-1-yl)methyl)piperidin-1-yl)-2-méthoxyphényl)amino)pyrimidin-4-yl)amino)phényl)-N-méthylméthanesulfonamide |
| 64 | N-(2-((5-chloro-2-((4-(4-((1-(3-(2-(2,6-dioxopipéridin-3-yl)-1,3-dioxoisoindolin-5-yl)propanoyl)pipéridin-4-yl)méthyl)pipérazin-1-yl)-2-méthoxyphényl)amino)pyrimidin-4-yl)amino)phényl)-N-méthylméthanesulfonamide |
| 65 | N-(2-((5-chloro-2-((4-(4-((1-((2-(2,6-dioxopiperidin-3-yl)-1,3-dioxoisoindolin-4-yl)glycyl)pipéridin-4-yl)méthyl)pipérazin-1-yl)-2-méthoxyphényl)amino)pyrimidin-4-yl)amino)phényl)-N-méthylméthanesulfonamide |
| 66 | N-(2-((5-chloro-2-((4-(4-((1-((2-(2,6-dioxopipéridin-3-yl)-1,3-dioxoisoindolin-5-yl)glycyl)pipéridin-4-yl)méthyl)pipérazin-1-yl)-2-méthoxyphényl)amino)pyrimidin-4-yl)amino)phényl)-N-méthylméthanesulfonamide |
| 67 | N-(2-((5-chloro-2-((4-(4-((4-(2-(2,6-dioxopipéridin-3-yl)-1,3-dioxoisoindolin-5-yl)pipérazin-1-yl)méthyl)pipéridin-1-yl)-2-méthoxyphényl)amino)pyrimidin-4-yl)amino)phényl)-N-méthylméthanesulfonamide |
| 68 | N-(2-((5-chloro-2-((4-(4-((4-(2-(2,6-dioxopipéridin-3-yl)-1,3-dioxoisoindolin-5-yl)pipéridin-4-yl)méthyl)pipérazin-1-yl)-2-méthoxyphényl)amino)pyrimidin-4-yl)amino)phényl)-N-méthylméthanesulfonamide |
| 69 | N-(2-((5-chloro-2-((4-(4-((1-((2-(2,6-dioxopipéridin-3-yl)-1,3-dioxoisoindolin-4-yl)oxy)propanoyl)pipéridin-4-ylyméthyl)pipérazin-1-yl)-2-méthoxyphényl)amino)pyrimidin-4-yl)amino)phényl)-N-méthylméthanesulfonamide |
| 70 | N-(2-((5-chloro-2-((4-(4-((1-((2-(2,6-dioxopipéridin-3-yl)-1,3-dioxoisoindolin-4-yl)-L-alanyl)pipéridin-4-yl)méthyl)pipérazin-1-yl)-2-méthoxyphényl)amino)pyrimidin-4-yl)amino)phényl)-N-méthylméthanesulfonamide |
| 71 | N-(2-((5-chloro-2-((4-(4-(4-((1-(2-(2,6-dioxopipéridin-3-yl)-1,3-dioxoisoindolin-5-yl)pipéridin-4-yl)méthyl)pipérazin-1-yl)pipéridin-1-yl)-2-méthoxyphényl)amino)pyrimidin-4-yl)amino)phényl)-N-méthylméthanesulfonamide |
| 74 | N-(2-((5-chloro-2-((4-(4-(4-(2-((2-(2,6-dioxopipéridin-3-yl)-1,3-dioxoisoindolin-4-yl)amino)éthyl)pipérazin-1-yl)pipéridin-1-yl)-2-méthoxy-5-méthylphényl)amino)pyrimidin-4-yl)amino)phényl)-N-méthylméthanesulfonamide |
| 75 | N-(2-((5-chloro-2-((4-(4-((1-((2-(2,6-dioxopipéridin-3-yl)-1,3-dioxoisoindolin-4-yl)-L-alanyl)pipéridin-4-yl)méthyl)pipérazin-1-yl)-2-méthoxy-5-méthylphényl)amino)pyrimidin-4-yl)amino)phényl)-N-méthylméthanesulfonamide |
| 76 | N-(2-((5-chloro-2-((4-(4-(4-((1-(2-(2,6-dioxopipéridin-3-yl)-1,3-dioxoisoindolin-5-yl)pipéridin-4-yl)méthyl)pipérazin-1-yl)pipéridin-1-yl)-2-méthoxy-5-méthylphényl)amino)pyrimidin-4-yl)amino)phényl)-N-méthylméthanesulfonamide |
| 77 | N-(2-((5-chloro-2-((4-(4-(4-(2-(1-(2-(2,6-dioxopipéridin-3-yl)-1,3-dioxoisoindolin-5-yl)pipéridin-4-yl)éthyl)pipérazin-1-yl)pipéridin-1-yl)-2-méthoxy-5-méthylphényl)amino)pyrimidin-4-yl)amino)phényl)-N-méthylméthanesulfonamide |
| 78 | N-(2-((5-chloro-2-((4-(4-((4-((2-(2,6-dioxopipéridin-3-yl)-1,3-dioxoisoindolin-4-yl)-L-alanyl)pipérazin-1-yl)méthyl)pipéridin-1-yl)-2-méthoxy-5-méthylphényl)amino)pyrimidin-4-yl)amino)phényl)-N-méthylméthanesulfonamide |
| 79 | N-(2-((5-chloro-2-((4-(4-((4-(2-(2,6-dioxopipéridin-3-yl)-1,3-dioxoisoindolin-5-yl)pipérazin-1-yl)méthyl)pipéridin-1-yl)-2-méthoxy-5-méthylphényl)amino)pyrimidin-4-yl)amino)phényl)-N-méthylméthanesulfonamide |
| 80 | N-(2-((5-chloro-2-((4-(4-((1-((2-(2,6-dioxopipéridin-3-yl)-1,3-dioxoisoindolin-4-yl)glycyl)pipéridin-4-yl)méthyl)pipérazin-1-yl)-2-méthoxy-5-méthylphényl)amino)pyrimidin-4-yl)amino)phényl)-N-méthylméthanesulfonamide |
| 81 | N-(2-((5-chloro-2-((4-(4-((1-(2-((2-(2,6-dioxopipéridin-3-yl)-1,3-dioxoisoindolin-4-yl)oxy)acétyl)pipéridin-4-yl)méthyl)pipérazin-1-yl)-2-méthoxy-5-méthylphényl)amino)pyrimidin-4-yl)amino)phényl)-N-méthylméthanesulfonamide |
| 82 | N-(2-((5-chloro-2-((4-(4-((1-((2S)-2-((2-(2,6-dioxopipéridin-3-yl)-1,3-dioxoisoindolin-4-yl)oxy)propanoyl)pipéridin-4-yl)méthyl)pipérazin-1-yl)-2-méthoxy-5-méthylphényl)amino)pyrimidin-4-yl)amino)phényl)-N-méthylméthanesulfonamide |

2. Composé selon la revendication 1 ou sel pharmaceutiquement acceptable de celui-ci, lequel composé est l'un quelconque des composés suivants :
| Composé N° | Nom UICPA |
|---|---|
| 11 | (2R,4R)-1-((S)-2-(2-(4-(1-(4-((5-chloro-4-((2-(N-méthylméthylsulfonamido)phényl)amino)pyrimidin-2-yl)amino)-3-méthoxyphényl)pipéridin-4-yl)pipérazin-1-yl)acétamido)-3,3-diméthylbutanoyl)-4-hydroxy-N-(4-(4-méthylthiazol-5-yl)benzyl)pyrrolidin-2-carboxamide |
| 19 | (2R,4R)-1-((S)-2-(2-(4-(1-(4-((5-chloro-4-((2-(N-méthylméthylsulfonamido)phényl)amino)pyrimidin-2-yl)amino)-2-fluorophényl)pipéridin-4-yl)pipérazin-1-yl)acétamido)-3,3-diméthylbutanoyl)-4-hydroxy-N-(4-(4-méthylthiazol-5-yl)benzyl)pyrrolidin-2-carboxamide |
| 20 | (2R,4R)-1-((S)-2-(3-(4-(1-(4-((5-chloro-4-((2-(N-méthylméthylsulfonamido)phényl)amino)pyrimidin-2-yl)amino)-3-méthoxyphényl)pipéridin-4-yl)pipérazin-1-yl)propanamido)-3,3-diméthylbutanoyl)-4-hydroxy-N-(4-(4-méthylthiazol-5-yl)benzyl)pyrrolidin-2-carboxamide |
| 22 | 2-(4-(1-(4-((5-chloro-4-((2-(N-méthylméthylsulfonamido)phényl)amino)pyrimidin-2-yl)amino)-3-méthoxyphényl)pipéridin-4-yl)pipérazin-1-yl)-N-(2-(2,6-dioxopipéridin-3-yl)-1-oxoisoindolin-4-yl)acétamide |
| 24 | 2-(4-((1-(4-((5-chloro-4-((2-(N-méthylméthylsulfonamido)phényl)amino)pyrimidin-2-yl)amino)-3-méthoxyphényl)pipéridin-4-yl)méthyl)pipérazin-1-yl)-N-(2-(2,6-dioxopipéridin-3-yl)-1-oxoisoindolin-4-yl)acétamide |
| 26 | 2-(4-((4-(4-((5-chloro-4-((2-(N-méthylméthylsulfonamido)phényl)amino)pyrimidin-2-yl)amino)-3-méthoxyphényl)pipérazin-1-yl)méthyl)pipéridin-1-yl)-N-(2-(2,6-dioxopipéridin-3-yl)-1-oxoisoindolin-4-yl)acétamide |
| 27 | (2R,4R)-1-((S)-2-(5-(4-((1-(4-((5-chloro-4-((2-(N-méthylméthylsulfonamido)phényl)amino)pyrimidin-2-yl)amino)-3-méthoxyphényl)pipéridin-4-yl)méthyl)pipérazin-1-yl)acétamido)-3,3-diméthylbutanoyl)-4-hydroxy-N-(4-(4-méthylthiazol-5-yl)benzyl)pyrrolidin-2-carboxamide |
| 28 | (2R,4R)-1-((S)-2-(2-(4-((4-(4-((5-chloro-4-((2-(N-méthylméthylsulfonamido)phényl)amino)pyrimidin-2-yl)amino)-3-méthoxyphényl)pipérazin-1-yl)méthyl)pipéridin-1-yl)acétamido)-3,3-diméthylbutanoyl)-4-hydroxy-N-(4-(4-méthylthiazol-5-yl)benzyl)pyrrolidin-2-carboxamide |
| 31 | (2R,4R)-1-((S)-2-(2-(9-(4-((5-chloro-4-((2-(N-méthylméthylsulfonamido)phényl)amino)pyrimidin-2-yl)amino)-3-méthoxyphényl)-3,9-diazaspiro[5,5]undécan-3-yl)acétamido)-3,3-dimethylbutanoyl)-4-hydroxy-N-(4-(4-methylthiazol-5-yl)benzyl)pyrrolidin-2-carboxamide |
| 35 | (2S,4R)-1-((S)-2-(2-(4-((4-(3-chloro-4-((5-chloro-4-((2-(N-méthylméthylsulfonamido)phényl)amino)pyrimidin-2-yl)amino)phényl)pipérazin-1-yl)méthyl)pipéridin-1-yl)acétamido)-3,3-diméthylbutanoyl)-4-hydroxy-N-(4-(4-méthylthiazol-5-yl)benzyl)pyrrolidin-2-carboxamide |
| 36 | (2S,4R)-1-((S)-2-(2-(4-((4-(4-((5-chloro-4-((2-(N-méthylméthylsulfonamido)phényl)amino)pyrimidin-2-yl)amino)-2-fluorophényl)pipérazin-1-yl)méthyl)pipéridin-1-yl)acétamido)-3,3-diméthylbutanoyl)-4-hydroxy-N-(4-(4-méthylthiazol-5-yl)benzyl)pyrrolidin-2-carboxamide |
| 39 | N-(2-((5-chloro-2-((4-(4-(2-(3-((2-(2,6-dioxopipéridin-3-yl)-1-oxoisoindolin-4-yl)oxy)azétidin-1-yl)éthyl)pipérazin-1-yl)-3-fluorophényl)amino)pyrimidin-4-yl)amino)phényl)-N-méthylméthanesulfonamide |
| 40 | N-(2-((5-chloro-2-((4-(4-(2-((1-(2-(2,6-dioxopipéridin-3-yl)-1,3-dioxoisoindolin-5-yl)azétidin-3-yl)oxy)éthyl)pipérazin-1-yl)-3-fluorophényl)amino)pyrimidin-4-yl)amino)phényl)-N-méthylméthanesulfonamide |
| 43 | N-(2-((5-chloro-2-((4-(4-((1-(2-((2-(2,6-dioxopipéridin-3-yl)-1,3-dioxoisoindolin-4-yl)oxy)acétyl)pipéridin-4-yl)méthyl)pipérazin-1-yl)-3-fluorophényl)amino)pyrimidin-4-yl)amino)phényl)-N-méthylméthanesulfonamide |
| 45 | N-(2-((5-chloro-2-((4-(4-(4-(2-((2-(2,6-dioxopipéridin-3-yl)-1,3-dioxoisoindolin-4-yl)amino)éthyl)pipérazin-1-yl)pipéridin-1-yl)-2-méthoxyphényl)amino)pyrimidin-4-yl)amino)phényl)-N-méthylméthanesulfonamide |
| 46 | N-(2-((5-chloro-2-((4-(4-((1-(2-((2-(2,6-dioxopipéridin-3-yl)-1,3-dioxoisoindolin-4-yl)oxy)éthyl)pipéridin-4-yl)méthyl)pipérazin-1-yl)-3-fluorophényl)amino)pyrimidin-4-yl)amino)phényl)-N-méthylméthanesulfonamide |
| 47 | 2-(4-(1-(4-((5-chloro-4-((2-(N-méthylméthylsulfonamido)phényl)amino)pyrimidin-2-yl)amino)-3-méthoxyphényl)pipéridin-4-yl)pipérazin-1-yl)-N-(2-(2,6-dioxopipéridin-3-yl)-1-oxoisoindolin-5-yl)acétamide |
| 51 | N-(2-((5-chloro-2-((4-(4-(4-(2-(1-(2-(2,6-dioxopipéridin-3-yl)-1,3-dioxoisoindolin-5-yl)pipéridin-4-yl)éthyl)pipérazin-1-yl)pipéridin-1-yl)-2-méthoxyphényl)amino)pyrimidin-4-yl)amino)phényl)-N-méthylméthanesulfonamide |
| 53 | N-(2-((5-chloro-2-((4-(4-((1-(2-((2-(2,6-dioxopipéridin-3-yl)-1,3-dioxoisoindolin-4-yl)oxy)acétyl)pipéridin-4-yl)méthyl)pipérazin-1-yl)-2-méthoxyphényl)amino)pyrimidin-4-yl)amino)phényl)-N-méthylméthanesulfonamide |
| 54 | N-(2-((5-chloro-2-((4-(4-((1-(3-(2-(2,6-dioxopipéridin-3-yl)-1,3-dioxoisoindolin-4-yl)propanoyl)pipéridin-4-yl)méthyl)pipérazin-1-yl)-2-méthoxyphényl)amino)pyrimidin-4-yl)amino)phényl)-N-méthylméthanesulfonamide |
| 59 | N-(2-((5-chloro-2-((4-(4-((1-((1-(2-(2,6-dioxopipéridin-3-yl)-1,3-dioxoisoindolin-5-yl)pipéridin-4-yl)méthyl)pipéridin-4-yl)méthyl)pipérazin-1-yl)-2-méthoxyphényl)amino)pyrimidin-4-yl)amino)phényl)-N-méthylméthanesulfonamide |
| 62 | N-(2-((5-chloro-2-((4-(4-((4-(((2-(2,6-dioxopipéridin-3-yl)-1,3-dioxoisoindolin-4-yl)amino)méthyl)pipéridin-1-yl)méthyl)pipérid in-1-yl)-2-méthoxyphényl)amino)pyrimidin-4-yl)amino)phényl)-N-méthylméthanesulfonamide |
| 63 | N-(2-((5-chloro-2-((4-(4-((4-(((2-(2,6-dioxopipéridin-3-yl)-1,3-dioxoisoindolin-5-yl)amino)méthyl)pipéridin-1-yl)méthyl)pipéridin-1-yl)-2-méthoxyphényl)amino)pyrimidin-4-yl)amino)phényl)-N-méthylméthanesulfonamide |
| 65 | N-(2-((5-chloro-2-((4-(4-((1-((2-(2,6-dioxopipéridin-3-yl)-1,3-dioxoisoindolin-4-yl)glycyl)pipéridin-4-yl)méthyl)pipérazin-1-yl)-2-méthoxyphényl)amino)pyrimidin-4-yl)amino)phényl)-N-méthylméthanesulfonamide |
| 66 | N-(2-((5-chloro-2-((4-(4-((1-((2-(2,6-dioxopipéridin-3-yl)-1,3-dioxoisoindolin-5-yl)glycyl)pipéridin-4-yl)méthyl)pipérazin-1-yl)-2-méthoxyphényl)amino)pyrimidin-4-yl)amino)phényl)-N-méthylméthanesulfonamide |
| 68 | N-(2-((5-chloro-2-((4-(4-((4-(2-(2,6-dioxopipéridin-3-yl)-1,3-dioxoisoindolin-5-yl)pipéridin-4-yl)méthyl)pipérazin-1-yl)-2-méthoxyphényl)amino)pyrimidin-4-yl)amino)phényl)-N-méthylméthanesulfonamide |
| 69 | N-(2-((5-chloro-2-((4-(4-((1-((2-(2,6-dioxopipéridin-3-yl)-1,3-dioxoisoindolin-4-yl)oxy)propanoyl)pipéridin-4-yl)méthyl)pipérazin-1-yl)-2-méthoxyphényl)amino)pyrimidin-4-yl)amino)phényl)-N-méthylméthanesulfonamide |
| 70 | N-(2-((5-chloro-2-((4-(4-((1-((2-(2,6-dioxopipéridin-3-yl)-1,3-dioxoisoindolin-4-yl)-L-alanyl)pipéridin-4-yl)méthyl)pipérazin-1-yl)-2-méthoxyphényl)amino)pyrimidin-4-yl)amino)phényl)-N-méthylméthanesulfonamide |
| 71 | N-(2-((5-chloro-2-((4-(4-(4-((1-(2-(2,6-dioxopipéridin-3-yl)-1,3-dioxoisoindolin-5-yl)pipéridin-4-yl)méthyl)pipérazin-1-yl)pipéridin-1-yl)-2-méthoxyphényl)amino)pyrimidin-4-yl)amino)phényl)-N-méthylméthanesulfonamide |
| 74 | N-(2-((5-chloro-2-((4-(4-(4-(2-((2-(2,6-dioxopipéridin-3-yl)-1,3-dioxoisoindolin-4-yl)amino)éthyl)pipérazin-1-yl)pipéridin-1-yl)-2-méthoxy-5-méthylphényl)amino)pyrimidin-4-yl)amino)phényl)-N-méthylméthanesulfonamide |
| 75 | N-(2-((5-chloro-2-((4-(4-((1-((2-(2,6-dioxopipéridin-3-yl)-1,3-dioxoisoindolin-4-yl)-L-alanyl)pipéridin-4-yl)méthyl)pipérazin-1-yl)-2-méthoxy-5-méthylphényl)amino)pyrimidin-4-yl)amino)phényl)-N-méthylméthanesulfonamide |
| 76 | N-(2-((5-chloro-2-((4-(4-(4-((1-(2-(2,6-dioxopipéridin-3-yl)-1,3-dioxoisoindolin-5-yl)pipéridin-4-yl)méthyl)pipérazin-1-yl)pipéridin-1-yl)-2-méthoxy-5-méthylphényl)amino)pyrimidin-4-yl)amino)phényl)-N-méthylméthanesulfonamide |
| 77 | N-(2-((5-chloro-2-((4-(4-(4-(2-(1-(2-(2,6-dioxopipéridin-3-yl)-1,3-dioxoisoindolin-5-yl)pipéridin-4-yl)éthyl)pipérazin-1-yl)pipéridin-1-yl)-2-méthoxy-5-méthylphényl)amino)pyrimidin-4-yl)amino)phényl)-N-méthylméthanesulfonamide |
| 78 | N-(2-((5-chloro-2-((4-(4-((4-((2-(2,6-dioxopipéridin-3-yl)-1,3-dioxoisoindolin-4-yl)-L-alanyl)pipérazin-1-yl)méthyl)pipéridin-1-yl)-2-méthoxy-5-méthylphényl)amino)pyrimidin-4-yl)amino)phényl)-N-méthylméthanesulfonamide |
| 80 | N-(2-((5-chloro-2-((4-(4-((1-((2-(2,6-dioxopipéridin-3-yl)-1,3-dioxoisoindolin-4-yl)glycyl)pipéridin-4-yl)méthyl)pipérazin-1-yl)-2-méthoxy-5-méthylphényl)amino)pyrimidin-4-yl)amino)phényl)-N-méthylméthanesulfonamide |
| 81 | N-(2-((5-chloro-2-((4-(4-((1-(2-((2-(2,6-dioxopipéridin-3-yl)-1,3-dioxoisoindolin-4-yl)oxy)acétyl)pipéridin-4-yl)méthyl)pipérazin-1-yl)-2-méthoxy-5-méthylphényl)amino)pyrimidin-4-yl)amino)phényl)-N-méthylméthanesulfonamide |
| 82 | N-(2-((5-chloro-2-((4-(4-((1-((2S)-2-((2-(2,6-dioxopipéridin-3-yl)-1,3-dioxoisoindolin-4-yl)oxy)propanoyl)pipéridin-4-yl)méthyl)pipérazin-1-yl)-2-méthoxy-5-méthylphényl)amino)pyrimidin-4-yl)amino)phényl)-N-méthylméthanesulfonamide |

3. Composition comprenant un composé selon la revendication 1 ou 2 ou un sel pharmaceutiquement acceptable de celui-ci, et un excipient pharmaceutiquement acceptable.

4. Composition pharmaceutique pour utilisation dans le traitement ou la prévention d'un cancer présentant une mutation de l'EGFR, comprenant le composé selon la revendication 1 ou 2 ou un sel pharmaceutiquement acceptable de celui-ci en tant qu'ingrédient actif.

5. Composition pharmaceutique pour utilisation selon la revendication 4, dans laquelle le cancer est un cancer du poumon, cancer du foie, cancer de l'œsophage, cancer de l'estomac, cancer du côlon, cancer de l'intestin grêle, cancer du pancréas, mélanome, cancer du sein, cancer de la cavité buccale, tumeur cérébrale, cancer de la thyroïde, cancer de la parathyroïde, cancer du rein, cancer du col de l'utérus, sarcome, cancer de la prostate, cancer de l'urètre, cancer de la vessie, cancer du testicule, cancer du sang, lymphome, cancer de la peau ou fibroadénome.

6. Composition pharmaceutique pour utilisation selon la revendication 5, dans laquelle le cancer est un cancer du poumon.

7. Composition pharmaceutique pour utilisation selon la revendication 6, dans laquelle le cancer du poumon est un cancer du poumon non à petites cellules.
